# EUROPEAN PATENT APPLICATION

(11) **EP 1 443 446 A1**
(43) Date of publication of application: **04.08.2004**
(21) Application number: 02801997.4
(22) Date of filing: 04.10.2002
(51) Int. Cl.: G06F 19/00, G06F 17/30, G06N 3/00, C07K 14/00

(54) **METHOD OF PRESUMING DOMAIN LINKER REGION OF PROTEIN**

(30) Priority: 05.10.2001 JP 2001309434; 12.06.2002 JP 2002172101; 12.06.2002 JP 2002172136
(71) Applicant: Riken, Wako-shi, Saitama 351-0198 (JP)
(72) Inventor: KURODA, Yutaka; c/o RIKEN Yokohama Institute, Yokohama-shi, Kanagawa 230-0045 (JP); MIYAZAKI, Satoshi; c/o RIKEN Yokohama Institute, Yokohama-shi, Kanagawa 230-0045 (JP); TANAKA, Yoshinori; c/o RIKEN Yokohama Institute, Yokohama-shi, Kanagawa 230-0045 (JP); YOKOYAMA, Shigeyuki; c/o RIKEN Yokohama Institute, Yokohama-shi, Kanagawa 230-0045 (JP)
(74) Representative: Woods, Geoffrey Corlett
(86) International application number: PCT/JP2002/010351
(87) International publication number: WO 2003/036546

(57) **Abstract**

A domain linker region is predicted by inputting an amino-acid sequence of a protein whose structure is unknown in a hierarchical neural network having identified and learned the domain linker region.

Also, the sequence characteristics of the linker domain is identified by a statistical method, and by combining the result with the secondary structure predicting method, a domain linker predicting method for an amino-acid sequence whose structure is unknown was constructed.

## Description

A method of training a neural network to identify a linker sequence of a protein, a method of predicting / detecting a linker sequence of a protein by a neural network, a system for the prediction / detection, a program and a recording media, a method of producing / analyzing a protein fragment, a method of constructing a linker sequence database, a method of constructing a structural domain database, and a method of dividing a domain linker peptide and a protein into a structural domain.

### Field of the Invention

The present invention relates to a method of learning / predicting / detecting a protein linker sequence by a neural network and more particularly to a method of having the neural network learn a linker sequence in a multi-domain protein, a method of predicting / detecting a linker sequence from amino acid sequence information of the protein, a system for the prediction / detection, a program and a recording media, a method of manufacturing / analyzing a structural domain of a protein, a method of constructing a linker sequence database, a method of constructing a structural domain database, and a peptide having a characteristic sequence pattern in a linker sequence.

### Background art

Various individual genomes have been decoded recently, and "structural genome science" has attracted attention as an important study for analysis of systematic structure of a protein using such a large amount of genome sequence information and establishment of correlation between structural functions based on the structure.

In this structural genome study, efficient narrowing of sequences to be analyzed is required by selecting a target which is a typical protein to be coded in a genome and suitable for structural analysis. Suitability for structural determination of a protein largely depends on its molecular weight, and if the current structural determination technology, particularly NMR is used, those for which structural determination can be automated are limited to small proteins with the molecular weight of 20 to 25 thousand. Also, even if there is no technical limitation on NMR or X-ray crystal structure analysis, expression / refinement of a large protein is considerably difficult, especially when unwinding is needed. Thus, when handling a large protein, it is desired that the protein is divided into fragments by domain and each domain is analyzed.

That is, many of proteins with large molecular weights are constituted by combination of a plurality of domains like a module, and it is considered that a variety of functions is realized by the combination. Therefore, in a protein made of such a plurality of domains, quick structural analysis would be possible by dividing it into domains which are its constitutional units and by determining the structure of these domains separately. Also, accurate determination of domain boundaries is important for structural analysis with high resolution or three-dimensional structural modeling, for example.

On the contrary, when determining domain regions, their structural information is unknown in general, and actually, it is extremely difficult to divide a protein into domains correctly under such circumstances.

As a conventional method of dividing a protein into fragments, a protein limited decomposition method by protease, for example, is used experimentally. However, this method requires a great amount of time and labor and can not be effective for systematic, extensive and high-throughput structural analysis.

Thus, how a domain region in a protein can be predicted accurately becomes an important problem in the above-mentioned structural analysis.

In the meantime, there have been many trials to derive information on structure from amino-acid sequences of a protein, and protein structure predicting methods have been developed corresponding to the obtained structural information. The secondary structure of a protein has been most extensively studied structural properties, and methods of predicting the secondary structure have been proposed. These methods are based on physiochemical properties (Lim, 1974; Ptitsyn & Finkelstein, 1983), statistical analysis (Chou & Fasman, 1974; Garnier et al., 1978), pattern matching (Cohen et al., 1983; King & Sternberg, 1990, 1996), neural network (Qian & Sejnowski, 1998; Rost & Sander, 1993), and evolutionarily conserved structure (Zvelebil et al., 1987). In some cases, accuracy of the secondary structural prediction exceeds 70% (Sternberg et al., 1999). The other structural properties such as β structure (Wilmot & Thornton, 1988 ; Shepherd et al., 1999), amino acid on the protein surface (Holbook et al., 1990), center of stabilization (Dosztanyi et al., 1997), and types of structures (Chandonia & Karpus, 1995 ; Chou et al., 1998) have been studied, and their prediction have been examined.

On the contrary, a method of predicting a domain region from an amino-acid sequence has been rarely studied (Busetta & Barrans, 1984 ; Kikuchi et al., 1988). Except recent several reports (Wheelan et al., 2000 ; Romero et al., 2001), similarity of sequences have been a main method of assuming the location of a domain (Sonnhammer & Kahn, 1994 ; Heinkoff et al., 1997 ; Corpet et al., 1998 ; Kuroda et al., 2001). The methods based on similarity of sequences typically assume that the sequences conserved in various proteins (existing in common) correspond to functional or structural independent bodies and they form a domain.

These methods give useful information on virtual domain in a protein having similar sequences, but they do not intend to detect a property of the sequence to be the characteristics of a structural domain or its boundary.

However, in detecting a property of a sequence of a structural domain, the domain itself is a relatively large structural unit, and extraction of its property becomes complicated, and difficulty in handling has been pointed out.

As a method to solve such a problem, a predicting method is proposed by inventors of the present invention using a neural network focusing attention not to a domain but to a domain linker connecting two domains as structural information (see, for example, S67-1 I 1115, collection of preliminary manuscripts for the 38^{th} annual meeting of the Biophysical Society). According to this method, since a linker sequence is far shorter than a domain sequence, its sequence pattern can be recognized easily.

Also, a method of predicting a domain boundary by a simple statistical method using occurrence frequency of an amino acid in a short range is reported.

However, any of the conventional art remains at a stage for seeking a new method, paying attention to the domain linker, and characteristics of the linker sequence have not been fully extracted. As a result, prediction efficiency is not so high, and it is necessary to characterize a larger segment around the domain boundary in more detail to improve accuracy of the prediction.

Then, according to the present invention, instead of paying attention to the structural domain as structural information, a focus is placed on a domain linker connecting two structural domains, and in fixing a linker sequence, data set for extracting characteristics of sequence pattern of the domain linker is sufficiently examined, accurate information is prepared on the linker sequence, and parameters for prediction are optimized so as to provide a method, a system and a program for predicting and / or detecting. a domain linker with more reliability.

### Description of the Invention

The inventors of the present invention employed, in order to identify a sequence connecting two protein domains (linker sequence), a method of having a sequence pattern learned using a neural network and a method of representing an occurrence frequency of an amino-acid residue in a linker domain by score through statistical processing and predicting a linker sequence on a protein whose structure is unknown by combining the both methods in a mutually complementary manner so as to improve prediction efficiency. That is, in the first method, when a domain library defined by SCOP is used to divide into a linker sequence and a non-linker sequence and their respective sequence information is made to be learned separately by the neural network, it was found that there is a great difference in characteristics in amino-acid sequence between the linker and the non-linker domain including an in-domain loop. Also, it was indicated that the linker sequence has a position-dependent preference for an amino acid (Occurrence frequency of a specific amino-acid residue is high at a certain position. The specific amino acid is arranged at the position in preference.) and it was made clear that the fact is not at random. When a domain linker was actually predicted based on such knowledge, a result of a Jackknife test indicated that 58% of a predicted domain matches an actual linker domain (specificity), and 36% of a domain linker derived from SCOP was predicted (sensitivity). This prediction efficiency is more excellent than a simple method derived from a secondary structure prediction, that is, a method which assumes a long loop domain as a virtual domain linker. As a general rule, these results show that a domain linker has a local characteristic different from a loop domain.

Also, in the second method, a domain linker predicting method for an amino-acid sequence whose structure is unknown was constructed by identifying a sequence characteristic of a linker domain in a statistical method and by combining the result with a secondary structure predicting method. That is, a non-redundant sequence set was prepared for a multi-domain protein whose structure is known, a partial sequence having a loop structure was extracted from it and classified into a linker sequence and a non-linker sequence. When the occurrence frequency of each amino-acid residue was examined in each of the sequence sets, it was found out that the occurrence frequency is apparently different between the both in some types of residues. Moreover, in a sequence pattern made of 2 residues, such an example was found that the occurrence frequency was different. The characteristics obtained from these analyses were formulated and a discrimination function was gained that indicates "how much it is like linker" as a score when an arbitrary amino-acid sequence is inputted in the formula. By carrying out secondary structure prediction to a protein whose structure is unknown and by applying this discrimination function to the obtained loop candidates, a position of a domain linker could be predicted at an experimentally effective level. The present invention has been completed based on such knowledge.

The gist of the present invention is as follows.
(1) A method of training a neural network to identify a linker sequence of a protein consisting of 2 or more structural domains comprising:
   a dividing step for dividing an amino-acid sequence of a protein consisting of 2 or more structural domains of a data set into a linker sequence and a non-linker sequence;
   a window setting step for taking a window of a range of 5 to 35 residues within the amino-acid sequence of the protein consisting of two or more structural domains of the data set;
   a sequence classifying step in which, if an amino-acid residue located at the center of the window constitutes a part of the linker sequence, a numeral value is granted to classify the amino-acid sequence in the winder as a positive sequence and if the amino-acid residue located at the center of the window constitutes a part of the non-linker sequence, a numeral value is granted to classify the amino-acid sequence in the window as a negative sequence; and
   a learning step for repeatedly learning to optimize a weight parameter of a hierarchical neural network by a back-propagation method,
   in which a value representing an amino-acid sequence in the window in numerals is input to the hierarchical neural network to acquire an output value, the error between the output value and the numeral value which classifies the amino-acid sequence in the window either as a positive sequence or as a negative sequence is calculated, and the weight parameter of the hierarchical neural network is so detemined that the error becomes minimal.
(2) A method of predicting a linker sequence of a protein whose structure is unknown comprising:
   a window setting step for taking a window of a range of 5 to 35 residues within an amino-acid sequence of a protein whose structure is unknown;
   an input / output step for obtaining an output value by inputting a value of the amino-acid sequence in the window represented in numerals into a hierarchical neutral network having trained by the method of (1);
   a predicted value granting step for granting the output value to an amino-acid residue located at the center of the window as a predicted value;
   a step of repeating the input / output step and the predicted value granting step, with the position of the window being moved within a desired range of the amino-acid sequence of the protein whose structure is unknown; and
   a linker sequence predicting step for predicting as a linker sequence a region cosisting of amino-acid residues with the predicted values larger than a preset threshold value.
(3) A method as set forth in (2) comprising, following the step of repeating the input / output step and the predicted value granting step:
   an average value calculating step for obtaining an average value by taking a new window of a range more than the predetermined number of residues within the amino-acid sequence of the protein whose structure is unknown and smoothing the predicted values over the amino-acid residues within this window; and
   a step for repeating the average value calculating step, with the position of the new window being moved within a desired range of the amino-acid sequence of the protein whose structure is unknown, and in the linker sequence predicting step, a linker sequence is predicted by the threshold with respect to the average value of the predicted values.
(4) A method as set forth in (3), wherein in the linker sequence predicting step, if the largest of the predicted values for the amino-acid residues in a region consisting of amino-acid residues whose average value of the predicted values, is larger than a preset threshold value is larger than a preset cut-off value, that region is predicted as a linker sequence.
(5) A system for predicting a linker sequence of a protein whose structure is unknown comprising an amino-acid sequence input means for inputting numerals that represent the amino-acid sequence of the protein whose structure is unknown, a window setting means for taking a window in the amino-acid sequence of the protein whose structure is unknown, an in-window amino-acid sequence input means by which numerals that represent the amino-acid sequence in the window, are input into a hierarchical neural network trained to identify the linker sequence of a protein consisting of 2 or more structural domains, an output value calculating means for having the hierarchical neural network calculate an output value, a predicted value granting means for granting the output value to the amino-acid residue located at the center of the window as a predicted value, a window-position moving means for moving the position of the window within a desired range of the amino-acid sequence of the protein whose structure is unknown, a smoothing window setting means for taking a new window of a range more than the predetermined number of residues in the amino-acid sequence of the protein whose structure is unknown, an average value calculating means for obtaining an average value by smoothing predicted values over the amino-acid residues in the new window, a smoothing window moving means for moving the position of the new window within a desired range of the amino-acid sequence of the protein whose structure is unknown, and a linker sequence predicting means for predicting as a linker sequence a region consisting of the amino-acid residues whose average value of the predicted values is larger than a preset threshold value.
(6) A program for having a computer function as a system for predicting a linker sequence of a protein whose structure is unknown characterized in that the system comprises an amino-acid sequence input means for inputting numerals that represent the amino-acid sequence of the protein whose structure is unknown, a window setting means for taking a window in the amino-acid sequence of the protein whose structure is unknown, an in-window amino-acid sequence input means by which numerals that represent the amino-acid sequence in the window are input into a hierarchical neural network trained to identify the linker sequence of a protein consisting of 2 or more structural domains, an output value calculating means for having the hierarchical neural network calculate an output value, a predicted value granting means for granting the output value to the amino-acid residue located at the center of the window as a predicted value, a window-position moving means for moving the position of the window within a desired range of the amino-acid sequence of the protein whose structure is unknown, a smoothing window setting-means for taking a new window of a range more than the predetermined number of residues in the amino-acid sequence of the protein whose structure is unknown, an average value calculating means for obtaining an average value by smoothing predicted values over the amino-acid residues in the new window, a smoothing window moving means for moving the position of the new window within a desired range of the amino-acid sequence of the protein whose structure is unknown, and a linker sequence predicting means for predicting as a linker sequence a region consisting of the amino-acid residues whose average value of the predicted values is larger than a preset threshold value.
(7) A computer readable recording medium having recorded thereon a program for having a computer function as a system for predicting a linker sequence of a protein whose structure is unknown characterized in that the system comprises an amino-acid sequence input means for inputting numerals that represent the amino-acid sequence of the protein whose structure is unknown, a window setting means for taking a window in the amino-acid sequence of the protein whose structure is unknown, an in-window amino-acid sequence input means by which numerals that represent the amino-acid sequence in the window are input into a hierarchical neural network trained to identify the linker sequence of a protein consisting of 2 or more structural domains, an output value calculating means for having the hierarchical neural network calculate an output value, a predicted value granting means for granting the output value to the amino-acid residue located at the center of the window as a predicted value, a window-position moving means for moving the position of the window within a desired range of the amino-acid sequence of the protein whose structure is unknown, a smoothing window setting means for taking a new window of a range more than the predetermined number of residues in the amino-acid sequence of the protein whose structure is unknown, an average value calculating means for obtaining an average value by smoothing predicted values over the amino-acid residues in the new window, a smoothing window moving means for moving the position of the new window within a desired range of the amino-acid sequence of the protein whose structure is unknown, and a linker sequence predicting means for predicting as a linker sequence a region consisting of the amino-acid residues whose average value of the predicted values is larger than a preset threshold value.
(8) A method of producing a protein fragment corresponding to one or more structural domains located closer to the N-terminal side than a predicted linker sequence comprising a step for producing at least one of the protein fragments obtained by cutting off a protein at any of the following portions (i), (ii) or (iii):
   (i) an arbitrary portion of at least one linker sequence predicted by the method as set forth in any of (2) through (4);
   (ii) any of portions located between the C-terminal of at least one linker sequence predicted by the method as set forth in any of (2) through (4) and the 50^{th} amino-acid residue as counted therefrom to the C-terminal side of the protein; or
   (iii) any of portions located between the N-terminal of at least one linker sequence predicted by the method as set forth in any of (2) through (4) and the 15^{th} amino-acid residue as counted therefrom to the N-terminal side of the protein.
(9) A method of producing a protein fragment corresponding to one or more structural domains located closer to the C-terminal side than a predicted linker sequence comprising a step for producing at least one of the protein fragments obtained by cutting off a protein at any of the following portions (i), (iv) or (v):
   (i) an arbitrary portion of at least one linker sequence predicted by the method as set forth in any of (2) through (4);
   (iv) any of portions located between the N-terminal of at least one linker sequence predicted by the method as set forth in any of (2) through (4) and the 50^{th} amino-acid residue as counted therefrom to the N-terminal side of the protein; or
   (v) any of portions located between the C-terminal of at least one linker sequence predicted by the method as set forth in any of (2) through (4) and the 15^{th} amino-acid residue as counted therefrom to the C-terminal side of the protein.
(10) A method of analyzing a protein fragment corresponding to one or more structural domains located closer to the N-terminal side than a predicted linker sequence comprising a step for analyzing at least one of the protein fragments obtained by cutting off a protein at any of the following portions (i), (ii) or (iii):
   (i) an arbitrary portion of at least one linker sequence predicted by the method as set forth in any of (2) through (4);
   (ii) any of portions located between the C-terminal of at least one linker sequence predicted by the method as set forth in any of (2) through (4) and the 50^{th} amino-acid residue as counted therefrom to the C-terminal side of the protein; or
   (iii) any of portions located between the N-terminal of at least one linker sequence predicted by the method as set forth in any of (2) through (4) and the 15^{th} amino-acid residue as counted therefrom to the N-terminal side of the protein.
(11) A method of analyzing a protein fragment corresponding to one or more structural domains located closer to the C-terminal side than a predicted linker sequence comprising a step for analyzing at least one of the protein fragments obtained by cutting off a protein at any of the following portions (i), (iv) or (v):
   (i) an arbitrary portion of at least one linker sequence predicted by the method as set forth in any of (2) through (4);
   (iv) any of portions located between the N-terminal of at least one linker sequence predicted by the method as set forth in any of (2) through (4) and the 50^{th} amino-acid residue counted therefrom to the N-terminal side of the protein; or
   (v) any of portions located between the C-terminal of at least one linker sequence predicted by the method as set forth in any of (2) through (4) and the 15^{th} amino-acid residue as counted therefrom to the C-terminal side of the protein.
(12) A method of constructing a linker sequence database comprising a step for recording in a recording medium the amino-acid sequence data for the linker sequence predicted by the method as set forth in any of (2) through (4).
(13) A method of constructing a structural domain database comprising a step for recording in a recording medium the amino-acid sequence data for the structural domain obtained by cutting off a protein at an arbitrary portion of at least one linker sequence predicted by the method as set forth in any of the (2) through (4).
(14) A peptide which has a sequence pattern satisfying the conditions of (i) and (ii) below and can function as a domain linker of a multi-domain protein:
   (i) when a sequence fragment consisting of 19 residues in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈{0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
      the value of the following g(x) should be in a range of 0.5 to 1.0:$\text{g} {\text{(x) = τ(ν}}_{\text{0}} {\text{+ν}}_{\text{1}} {\text{f}}_{\text{1}} {\text{(x)+ν}}_{\text{2}} {\text{f}}_{\text{2}} \text{(x))}$ 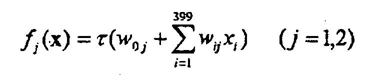 $\text{τ} \text{(} \text{u} \text{) = 1/(1 +} {\text{e}}^{\text{-u}} \text{)}$ (where a combination of wᵢⱼ(i=0, ·····, 399; j=1,2) and vⱼ(j=0, 1, 2) is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
   (ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.
(15) A method of predicting a region having a sequence pattern satisfying the conditions of (i) and (ii) below as a linker sequence of protein:
   (i) when a sequence fragment consisting of 19 residues in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈{0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
      the value of the following g(x) should be in a range of 0.5 to 1.0:$\text{g} {\text{(x) = τ(ν}}_{\text{0}} {\text{+ ν}}_{\text{1}} {\text{f}}_{\text{1}} {\text{(x) + ν}}_{\text{2}} {\text{f}}_{\text{2}} \text{(x))}$ 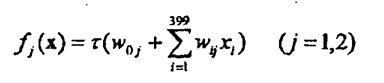 $\text{τ(} \text{u} \text{) = 1/(1+} {\text{e}}^{\text{-}} {\text{}}^{\text{u}} \text{)}$ (where a combination of wᵢⱼ(i=0, ·····, 399; j=1,2) and vⱼ(j=0, 1, 2) is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
   (ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.
(16) A method of dividing a protein into structural domains characterized in that the protein is cut off at an arbitrary portion of a region having a sequence pattern satisfying the conditions of (i) and (ii) below:
   (i) when a sequence fragment consisting of 19 residues in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈{0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
      the value of the following g(x) sould be in a range of 0.5 to 1.0:$\text{g} {\text{(x) = τ(ν}}_{\text{0}} {\text{+ν}}_{\text{1}} {\text{f}}_{\text{1}} {\text{(x)+ν}}_{\text{2}} {\text{f}}_{\text{2}} \text{(x))}$ 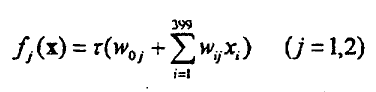 $\text{τ(} \text{u} \text{)=1/(1+} {\text{e}}^{\text{-}} {\text{}}^{\text{u}} \text{)}$ (where a combination of wᵢⱼ(i=0,·····, 399; j=1,2) and vⱼ(j=0, 1, 2)is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
   (ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.
(17) A method of producing a protein fragment comprising a step for producing at least one of the protein fragments obtained by cutting off a protein at an arbitrary portion of a region having a sequence pattern satisfying the conditions of (i) and (ii) below:
   (i) when a sequence fragment consisting of 19 residues in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈{0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
      the value of the following g(x) should be in a range of 0.5 to 1.0:$\text{g} {\text{(x) = τ(ν}}_{\text{0}} {\text{+ ν}}_{\text{1}} {\text{f}}_{\text{1}} \text{(x) +} {\text{ν}}_{\text{2}} {\text{f}}_{\text{2}} \text{(x))}$ 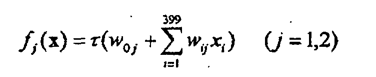 $\text{τ(} \text{u} \text{) = 1/(1} {\text{+e}}^{\text{-u}} \text{)}$ (where a combination of wᵢⱼ(i=0,·····, 399; j=1,2) and vⱼ(j=0, 1, 2)is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
   (ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.
(18) A method of analyzing a protein fragment comprising a step for analyzing at least one of the protein fragments obtained by cutting off protein at an arbitrary portion of a region having a sequence pattern satisfying the conditions of (i) and (ii) below:
   (i) when a sequence fragment consisting of 19 residues in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈{0,1} (} \text{i} \text{=1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
      the value of the following g(x) should be in a range of 0.5 to 1.0:$\text{g} {\text{(x) = τ(ν}}_{\text{0}} {\text{+ ν}}_{\text{t}} {\text{f}}_{\text{1}} {\text{(x) + ν}}_{\text{2}} {\text{f}}_{\text{2}} \text{(x))}$ 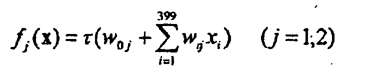 $\text{τ(} \text{u} \text{) = 1/(1+} {\text{e}}^{\text{-u}} \text{)}$ (where a combination of wᵢⱼ(i=0,·····, 399; j=1,2) and vⱼ(j=0, 1, 2)is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
   (ii) a central residue of the sequence fragment x=(x₁, x₂, ·····, x₃₉₉) with the value of g(x) in the range of 0.5 to 1.0 should be included, with an amino acid within 9 residues before and after the central residue being optionally further included.
(19) A method of producing a new multi-domain protein by designing a new linker sequence with a peptide having a sequence pattern satisfying the conditions of (i) and (ii) below and by connecting at least two protein fragments:
   (i) when a sequence fragment consisting of 19 in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈ {0,1} (} \text{i} \text{=1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
      the value of the following g(x) shoud be in a range of 0.5 to 1.0:$\text{g} {\text{(x) = τ(ν}}_{\text{0}} {\text{+ ν}}_{\text{1}} {\text{f}}_{\text{1}} \text{(x) +} {\text{ν}}_{\text{2}} {\text{f}}_{\text{2}} \text{(x))}$ 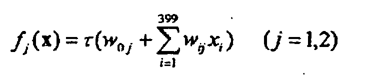 $\text{τ(} \text{u} \text{) = 1/(1 +} {\text{e}}^{\text{-}} {\text{}}^{\text{u}} \text{)}$ (where a combination of wᵢⱼ(i=0,·····, 399; j=1,2) and vⱼ(j=0, 1, 2)is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
   (ii) a central residue of the sequence fragment x=(x₁, x₂, ·····, x₃₉₉) with the value of g(x) in the range of 0.5 to 1.0 should be included, with an amino acid within 9 residues before and after the central residue being optionally further included.
(20) A method comprising:
   i) a step for extracting a linker sequence and a non-linker loop sequence from a database of multi-domain proteins of known structures; and
   ii) a step for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino-acid residue Xₐₐ occurring in a linker sequence and a non-linker loop sequence, respectively) and the probabilities P_{XaaYaa(m)}^{L} and P_{XaaYaa(m)}^{N} of occurrence of the amino-acid residues Xₐₐ and Yₐₐ as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring in the linker sequence and the non-linker loop sequence, respectively, as interrupted by m amino acid residues (the order of Xₐₐ and Yₐₐ does not matter)), said method predicting and/or detecting a linker sequence in a multi-domain protein of unknown structure from the characteristics in terms of the amino-acid sequence of the linker sequence extracted in step i).
(21) A system comprising:
   i) a means for extracting a linker sequence and a non-linker loop sequence from a database of multi-domain proteins of known structures i; and
   ii) a means for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino-acid residue Xₐₐ occurring in a linker sequence and a non-linker loop sequence, respectively) and the probabilities P_{XaaYaa(m)}^{L} and P_{XaaYaa(m)}^{N} of occurrence of the amino-acid residues Xₐₐ and Yₐₐ as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring in the linker sequence and then-linker loop sequence, respectively, as interrupted by m amino acid residues (the order of Xₐₐ and Yₐₐ does not matter)), said system predicting and/or detecting. a linker sequence in a multi-domain protein of unknown structure from the characteristics in terms of the amino-acid sequence of the linker sequence extracted by the means of i).
(22) A program for having a computer function as a system for predicting and / or detecting a linker sequence in a multi-domain protein of unknown structure from the characateristics in terms of its amino acid sequence, the system comprising:
   i) a means for extracting a linker sequence and a non-linker loop sequence from a database of multi-domain proteins of known structures; and
   ii) a means for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino-acid residue Xₐₐ occurring in a linker sequence and a non-linker loop sequence, respectively) and the probabilities P_{XaaYaa(m)}^{L} and P_{XaaYaa(m)}^{N} of occurrence of the amino-acid residues Xₐₐ and Yₐₐ as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring in the linker sequence and the non-linker loop sequence, respectively, as interrupted by m amino acid residues (the order of Xₐₐ and Yₐₐ does not matter)).
(23) A structural domain predicting method comprising a step in which a protein fragment generated by cutting off a multi-domain protein of unknown structure at any of the portions of a linker sequence in the multi-domain protein after it was predicted by the method as set forth in (20) is predicted as a structural domain.
(24) A protein producing method comprising a step for producing a protein having the same amino-acid sequence as the structural domain predicted by the method as set forth in (23).
(25) A protein analyzinig method comprising a step for analyzing a protein having the same amino-acid sequence as the structural domain predicted by the method as set forth in (23).
(26) A system for calculating a parameter of an occurrence trend of an amino-acid residue comprising:
   i) a means for extracting a linker sequence and a non-linker loop sequence from a database of multi-domain proteins of known structures;
   ii) a means for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino acid residue Xₐₐ occurring in a linker sequence and a non-linker loop sequence, respectively)
   iii) a means for obtaining an occurrence trend parameter S_{Xaa} of the amino-acid residue Xₐₐ by the following equation :${\text{S}}_{\text{Xaa}} {\text{=log (P}}_{\text{Xaa}} {\text{}}^{\text{L}} {\text{/P}}_{\text{Xaa}} {\text{}}^{\text{N}} \text{)}$ (where S_{Xaa}=0 if there is no statistically significant difference between P_{Xaa}^{L} and P_{Xaa}^{N}).
(27) A program for having a computer function as a system for calculating a parameter representing an occurrence trend of an arbitrary amino-acid residue, the system comprising:
   i) a means for extracting a linker sequence and a non-linker loop sequence from a database of multi-domain proteins of known structures;
   ii) a means for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino acid residue Xₐₐ occurring in a linker sequence and a non-linker loop sequence, respectively); and
   iii) a means for obtaining an occurrence trend parameter S_{Xaa} of the amino acid residue Xₐₐ by the following equation:${\text{S}}_{\text{Xaa}} {\text{=log (P}}_{\text{Xaa}} {\text{}}^{\text{L}} {\text{/P}}_{\text{Xaa}} {\text{}}^{\text{N}} \text{)}$ (where S_{Xaa}=0 if there is no statistically significant difference between P_{Xaa}^{L} and P_{Xaa}^{N}).
(28) A system for calculating a parameter of an appearance trend of an amino-acid residue pair comprising:
   i) a means for extracting a linker sequence and a non-linker loop sequence from a database of multi-domain proteins of known structures;
   ii) a means for obtaining, based on statistical processing of amino acid sequence of each domain, the probabilities P_{XaaYaa(m)}^{L} and P_{XaaYaa(m)}^{N} of occurrence of amino-acid residues Xₐₐ and Yₐₐ (the order of Xₐₐ and Yₐₐ does not matter) as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring (the order of Xₐₐ and Yₐₐ does not matter) in a linker sequence and a non-linker loop sequence, respectively, as interrupted by m amino-acid residues (m is an integer, m = 0, 1, 2)) for the cases where m is 0, 1 and 2, respectively; and
   iii) a means for obtaining an occurrence trend parameter S_{XaaYaa(m)} of the pair of amino acid residues Xₐₐ and Yₐₐ by the following equation:${\text{S}}_{\text{XaaYaa(m)}} {\text{=log (P}}_{\text{XaaYaa(m)}} {\text{}}^{\text{L}} {\text{/P}}_{\text{XaaYaa(m)}} {\text{}}^{\text{N}} \text{)}$ (where S_{Xaa}=0 if there is no statistically significant difference between P_{XaaYaa(m)}^{L} and P_{XaaYaa(m)}^{N}).
(29) A program for having a computer function as a system for calculating a parameter representing an occurrence trend of an arbitrary amino-acid residue pair, the system comprising:
   i) a means for extracting a linker sequence and a non-linker loop sequence from a database of multi-domain proteins of known structures;
   ii) a means for obtaining, based on statistical processing of amino acid sequence of each domain, the probabilities P_{XaaYaa(m)}^{L} and P_{XaaYaa(m)}^{N} of occurrence of amino-acid residues Xₐₐ and Yₐₐ (the order of Xₐₐ and Yₐₐ does not matter) as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring (the order of Xₐₐ and Yₐₐ does not matter) in a linker sequence and a non-linker loop sequence, respectively, as interrupted by m amino-acid residues (m is an integer, m = 0, 1, 2)) for the cases where m is 0, 1 and 2, respectively; and
   iii) a means for obtaining an occurrence trend parameter S_{XaaYaa(m)} of the pair of amino-acid residues Xₐₐ and Yₐₐ by the following equation:${\text{S}}_{\text{XaaYaa(m)}} {\text{=log (P}}_{\text{XaaYaa(m)}} {\text{}}^{\text{L}} {\text{/P}}_{\text{XaaYaa(m)}} {\text{}}^{\text{N}} \text{)}$ (where S_{Xaa}=0 if there is no statistically significant difference between P_{XaaYaa(m)}^{L} and P_{XaaYaa(m)}^{N}).
(30) A system for obtaining a linker degree determination score F₁ for an amino-acid sequence with L₁ amino-acid residues (L₁ is an integer of 1 or more but not more than 21), the system comprising:
   i) a means for obtaining a linker trend score F₁s of an amino-acid residue Aₖ by the following equation: 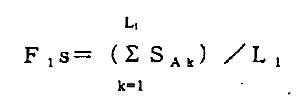 (where S_{Ak}=log(P_{Ak}^{L}/P_{Ak}^{N})
      where S_{Ak}=0 if there is no statistically significant difference between P_{Ak}^{L} and P_{Ak}^{N};
      P_{Ak}^{L} and P_{Ak}^{N} are the probabilities of the amino-acid residue Aₖ occurring in a linker sequence and a non-linker loop sequence, respectively);
   ii) a means for obtaining a linker trend score F₁p of the pair of amino-acid residues Aₖ and Aₖ₊₍ₘ₊₁₎, as interrupted by m arbitrary amino-acid residues (m is an integer, m = 0, 1, 2), by the following equation: 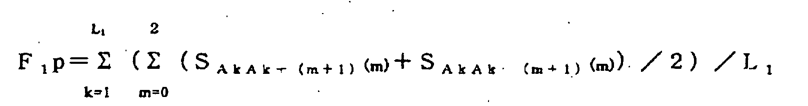 (where S_{AkAk+(m+1)(m)}=log(P_{AkAk+(m+1)(m)}^{L}/P_{AkAk+(m+1)(m)}^{N}) and S_{AkAk-(m+1)(m)}=log(P_{AkAk-(m+1)(m)}^{L}/P_{AkAk-(m+1)(m)}^{N})
      where S_{AkAk+(m+1)(m)}=0 or S_{AkAk-(m+1)(m)}=0 if there is no statistically significant difference between P_{AkAk+(m+1)(m)}^{L} and P_{AkAk+(m+1)(m)}^{N} or between P_{AkAk-(m+1)(m)}^{L} and P_{AkAk-(m+1)(m)}^{N};
      P_{AkAk+(m+1)(m)}^{L} and P_{AkAk+(m+1)(m)}^{N} are the probabilities of the arbitrary amino-acid residues Aₖ and Aₖ₊₍ₘ₊₁₎ occurring in a linker sequence and a non-linker loop sequence, respectively (the order of Aₖ and Aₖ₊₍ₘ₊₁₎ does not matter), and P_{AkAk-(m+1)(m)}^{L} and P_{AkAk-(m+1)(m)}^{N} are the probabilities of the arbitrary amino-acid residues Aₖ and Aₖ₋₍ₘ₊₁₎ occurring in the linker sequence and the non-linker loop sequence, respectively (the order of Aₖ and Aₖ₋₍ₘ₊₁₎ occurring does not matter)); and
   iii) a means for obtaining a linker degree determination score F₁ by the following equation below:${\text{F}}_{\text{1}} {\text{=F}}_{\text{1}} {\text{s+ α}}_{\text{1}} {\text{F}}_{\text{1}} \text{p}$ (where 0≦α₁≦1).
(31) A program for having a computer function as a system for obtaining a linker degree determination score F₁ for an amino-acid sequence with L₁ amino-acid residues (L₁ is an integer of 1 or more but not more than 21), the system comprising:
   i) a means for obtaining a linker trend score F₁s of an amino-acid residue Aₖ by the following equation: 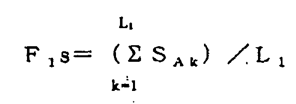 (where S_{Ak}=log(P_{Ak}^{L}/P_{Ak}^{N})
      where S_{Ak}=0 if there is no statistically significant difference between P_{Ak}^{L} and P_{Ak}^{N};
      P_{Ak}^{L} and P_{Ak}^{N} are the probabilities of the amino-acid residue Aₖ occurring in a linker sequence and a non-linker loop sequence, respetively);
   ii) a means for obtaining a linker trend score F₁ₚ of the pair of amino-acid residues Aₖ and Aₖ₊₍ₘ₊₁₎, as interrupted by m arbitrary amino-acid residues (m is an integer, m = 0, 1, 2), by the following equation: 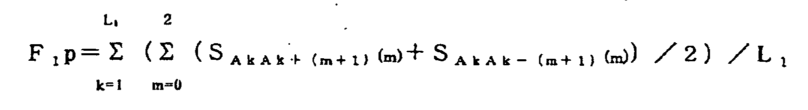 (where S_{AkAk+(m+1)(m)}=log(P_{AkAk+(m+1)(m)}^{L}/P_{AkAk+(m+1)(m)}^{N}) and S_{AkAk-(m+1)(m)}=log(P_{AkAk-(m+1)(m)}^{L}/P_{AkAk-(m+1)(m)}^{N})
      where S_{AkAk+(m+1)(m)}=0 or S_{AkAk-(m+1)(m)}=0 if, there is no statistically significant difference between P_{AkAk+(m+1)(m)}^{L} and P_{AkAk+(m+1)(m)}^{N} or between P_{AkAk-(m+1)(m)}^{L} and P_{AkAk-(m+1)(m)}^{N};
      P_{AkAk+(m+1)(m)}^{L} and P_{AkAk+(m+1)(m)}^{N} are the probabilities of the arbitrary amino-acid residues Aₖ and Aₖ₊₍ₘ₊₁₎ occurring in a linker sequence and a non-linker loop sequence, respectively (the order of Aₖ and Aₖ₊₍ₘ₊₁₎ does not matter), and P_{AkAk-(m+1)(m)}^{L} and P_{AkAk-(m+1)(m)}^{N} are the probabilities of the arbitrary amino-acid residues Aₖ and Aₖ₋₍ₘ₊₁₎ occurring in the linker sequence and the non-linker loop sequence, respectively (the order of Aₖ and Aₖ₋₍ₘ₊₁₎ does not matter)); and
   iii) a means for obtaining a linker degree determination score F₁ by the following equation:${\text{F}}_{\text{1}} {\text{=F}}_{\text{1}} {\text{s+ α}}_{\text{1}} {\text{F}}_{\text{1}} \text{p}$ (where 0≦ α₁ ≦ 1).
(32) A method of obtaining a linker degree determination score F₁₁(i) for an amino-acid residue Ai at a position i in an amino-acid sequence with L₂ amino-acid residues (L₂ is an integer of 22 or more) by taking a window of w amino-acid residues before and after the amino-acid residue at the position i (i is an integer of 1 or more but not more than L₂) comprising:
   i) a step for obtaining a linker trend determination score F₁₁s(i) of an amino-acid residue Aₖ by the folloing equation: 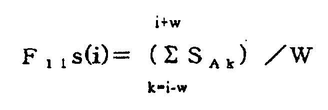 (where W is the window width, and W=2w+1,${\text{S}}_{\text{Ak}} {\text{=log(P}}_{\text{Ak}} {\text{}}^{\text{L}} {\text{/P}}_{\text{Ak}} {\text{}}^{\text{N}} \text{)}$ where S_{Ak}=0 if there is no statistically significant difference between P_{Ak}^{L} and P_{Ak}^{N};
      P_{Ak}^{L} and P_{Ak}^{N} are the probabilities of the amino-acid residue Aₖ occurring in a linker sequence and a non-linker loop sequence, respectively);
   ii) a step for obtaining the linker trend score F₁₁p(i) of the pair of amino-acid residues Aᵢ and Aᵢ₊₍ₘ₊₁₎, as interrupted by m arbitrary amino-acid residues (m is an integer, m = 0, 1, 2), by the following equation: 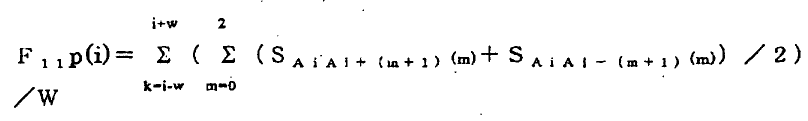 (where S_{AiAi+(m+1)(m)}=log(P_{AiAi+(m+1)(m)}^{L}/P_{AiAi+(m+1)(m)}^{N}) and
      S_{AiAi-(m+1)(m)}=log(P_{AiAi-(m+1)(m)}^{L}/P_{AiAi(m+1)(m)}^{N})
      where S_{AiAi+(m+1)(m)}=0 or S_{AiAi-(m+1)(m)}=0 if there is no statistically significant diffrerence between P_{AiAi+(m+1)(m)}^{L} and P_{AiAi+(m+1)(m)}^{N} or between P_{AiAi-(m+1)(m)}^{L} and P_{AiAi-(m+1)(m)}^{N};
      P_{AiAi+(m+1)(m)}^{L} and P_{AiAi+(m+1)(m)}^{N} are the probabilities of the pair of the arbitrary amino-acid residues Aᵢ and Aᵢ₊₍ₘ₊₁₎ occurring in a linker sequence and a non-linker loop sequence, respectively (the order of A, and Aᵢ₊₍ₘ₊₁₎ does not matter), and P_{AiAi-(m+1)(m)}^{L} and P_{AiAi-(m+1)(m)}^{N} are the probabilities of the pair of the arbitrary amino-acid residues Aᵢ and Aᵢ₋₍ₘ₊₁₎ occurring in the linker sequence and the non-linker loop sequence, respectively (the order of Aᵢ and Aᵢ₋₍ₘ₊₁₎ does not matter)); and
   iii) a step for obtaining the linker degree determination score F₁₁(i) of the amino-acid residue Ai at the position i by the following equation:${\text{F}}_{\text{11}} {\text{(i)=F}}_{\text{11}} {\text{s(i)+ α}}_{\text{11}} {\text{F}}_{\text{11}} \text{p(i)}$ (where 0≦ α₁₁ ≦ 1).
(33) A system for obtaining a linker degree determination score F₁₁(i) for an amino-acid residue Ai at a position i in an amino-acid sequence with L₂ amino-acid residues (L₂ is an integer of 22 or more) by taking a window of w amino-acid residues before and after the amino-acid residue at the position i (i is an integer of 1 or more but not more than L₂) comprising:
   i) a step for obtaining a linker trend determination score F₁₁s(i) of an amino-acid residue Aₖ by following equation: 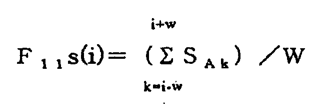 (where W is the window width, and W=2w+1,${\text{S}}_{\text{Ak}} {\text{=log(P}}_{\text{Ak}} {\text{}}^{\text{L}} {\text{/P}}_{\text{Ak}} {\text{}}^{\text{N}} \text{)}$ where S_{Ak}=0 if there is no statistically significant difference between P_{Ak}^{L} and P_{Ak}^{N};
      P_{Ak}^{L} and P_{Ak}^{N} are the probabilities of the amino-acid residue Aₖ occurring in a linker sequence and a non-linker loop sequence, respectively);
   ii) a step for obtaining the linker trend score F₁₁p(i) of the pair of amino-acid residues A, and Aᵢ₊₍ₘ₊₁₎, as interrupted by m arbitrary amino-acid residues (m is an integer, m = 0, 1, 2), by the following equation: 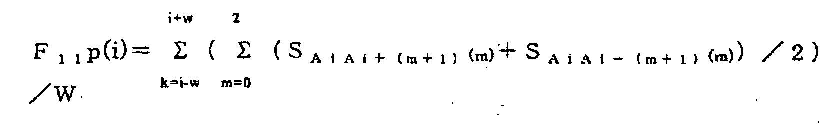 (where S_{AiAi+(m+1)(m)}=log(P_{AiAi+(m+1)(m)}^{L}/P_{AiAi+(m+1)(m)}^{N}) and
      S_{AiAi-(m+1)(m)}=log(P_{AiAi-(m+1)(m)}^{L}/P_{AiAi-(m+1)(m)}^{N})
      where S_{AiAi+(m+1)(m)}=0 or S_{AiAi-(m+1)(m)}=0 if there is no statistically significant diffrerence between P_{AiAi+(m+1)(m)}^{L} and P_{AiAi+(m+1)(m)}^{N} or between P_{AiAi-(m+1)(m)}^{L} and P_{AiAi-(m+1)(m)}^{N};
      P_{AiAi+(m+1)(m)}^{L} and P_{AiAi+(m+1)(m)}^{N} are the probabilities of the pair of the arbitrary amino-acid residues Aᵢ and Aᵢ₊₍ₘ₊₁₎ occurring in a linker sequence and a non-linker loop sequence, respectively (the order of Aᵢ and Aᵢ₊₍ₘ₊₁₎ does not matter), and P_{AiAi-(m+1)(m)}^{L} and P_{AiAi-(m+1)(m)}^{N} are the probabilities of the pair of the arbitrary amino-acid residues Aᵢ and Aᵢ₋₍ₘ₊₁₎ occurring in the linker sequence and the non-linker loop sequence, respectively (the order of Aᵢ and Aᵢ₋₍ₘ₊₁₎ does not matter)); and
   iii) a step for obtaining the linker degree determination score F₁₁(i) of the amino-acid residue Ai at the position i by the following equation:${\text{F}}_{\text{11}} {\text{(i)=F}}_{\text{11}} {\text{s(i)+ α}}_{\text{11}} {\text{F}}_{\text{11}} \text{p(i)}$ (where 0≦ α₁₁≦ 1).
(34) A program for having a computer function as a system for obtaining a linker degree determination score F₁₁(i) for an amino-acid residue Ai at a position i in an amino-acid sequence with L₂ amino-acid residues (L₂ is an integer of 22 or more) by taking a window of w amino-acid residues before and after the amino-acid residue at the position i (i is an integer of 1 or more but not more than L₂), the system comprising:
   i) a step for obtaining a linker trend score F₁₁s(i) of an amino-acid residue Aₖ by the following equation: 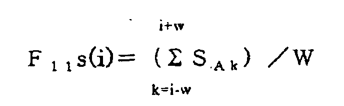 (where W is the window width, and W=2w+1,${\text{S}}_{\text{Ak}} {\text{=log(P}}_{\text{Ak}} {\text{}}^{\text{L}} {\text{/P}}_{\text{Ak}} {\text{}}^{\text{N}} \text{)}$ where S_{Ak}=0 if there is no statistically significant diffrerence between P_{Ak}^{L} and P_{Ak}^{N};
      P_{Ak}^{L} and P_{Ak}^{N} are the probabilities of the amino-acid residue Aₖ occurring in a linker sequence and a non-linker loop sequence, respectively);
   ii) a step for obtaining the linker trend score F₁₁p(i) of the pair of amino-acid residues Aᵢ and Aᵢ₊₍ₘ₊₁₎, as interrupted by m arbitrary amino-acid residues (m is an integer, m = 0, 1, 2), by the following equation: 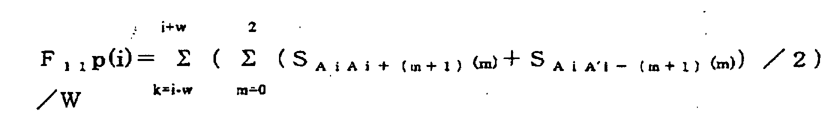 (where S_{AiAi+(m+1)(m)}=log(P_{AiAi+(m+1)(m)}^{L}/P_{AiAi+(m+1)(m)}^{N}) and
      S_{AiAi-(m+1)(m)}=log(P_{AiAi-(m+1)(m)}^{L}/P_{AiAi-(m+1)(m)}^{N})
      where S_{AiAi+(m+1)(m)}=0 or S_{AiAi-(m+1)(m)}=0 if there is no statistically significant diffrerence between P_{AiAi+(m+1)(m)}^{L} and P_{AiAi+(m+1)(m)}^{N} or between P_{AiAi-(m+1)(m)}^{L} and P_{AiAi-(m+1)(m)}^{N};
      P_{AiAi+(m+1)(m)}^{L} and P_{AiAi+(m+1)(m)}^{N} are the probabilities of the pair of the arbitrary amino-acid residues Aᵢ and Aᵢ₊₍ₘ₊₁₎ occurring in a linker sequence and a non-linker loop sequence, respectively (the order of Aᵢ and Aᵢ₊₍ₘ₊₁₎ does not matter), and P_{AiAi-(m+1)(m)}^{L} and P_{AiAi-(m+1)(m)}^{N} are the probabilities of the pair of the arbitrary amino-acid residues Aᵢ and Aᵢ₋₍ₘ₊₁₎ occurring in the linker sequence and the non-linker loop sequence, respectively (the order of Aᵢ and Aᵢ₋₍ₘ₊₁₎ does not matter)); and
   iii) a step for obtaining the linker degree determination score F₁₁(i) of the amino acid residue Ai at the position i by the following equation:${\text{F}}_{\text{11}} {\text{(i)=F}}_{\text{11}} {\text{s(i)+α}}_{\text{11}} {\text{F}}_{\text{11}} \text{p(i)}$ (where 0≦α₁₁≦1).
(35) A method by which a linker degree determination score F₁₂(i) of an amino-acid residue Ai at a position i in an amino-acid sequence seq.0 with L₂ amino-acid residues (L₂ is an interger of 22 or more) for which the existence of n homologous sequences seq.1∼seq.n (n is an integer of 1 or more) is known is obtained by taking a window with w amino-acid residues before and after the amino-acid residue at the position i (i is an integer of 1 or more but not more than 22), the method comprising:
   i) a step for identifying an amino-acid residue Aᵢ^{k} in a seq.k (k is an integer of 1 or more but not more than n) corresponding to an amino-acid residue Ai⁰ at a position i in the seq.0 by aligning seq.0 and seq.1∼seq.n;
   ii) a step for obtaining parameters S'_{Ai}, S'_{AiAi+(m+1)}(m) and S'_{AiAi-(m+1)}(m) for the amino-acid residue Ai at the position i by the following equation: 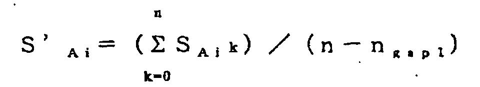 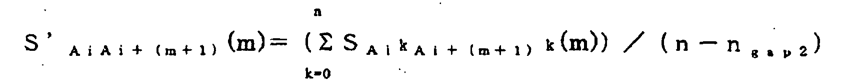 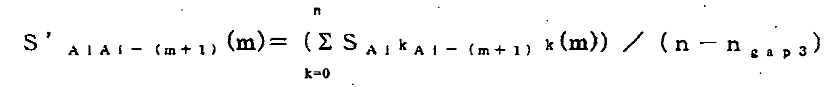 (where n_{gap1} is the number of gaps occurring in Aᵢ^{k},${\text{S}}_{\text{Ai}} {\text{k=log(P}}_{\text{Ai}} {\text{k}}^{\text{L}} {\text{/P}}_{\text{Ai}} {\text{k}}^{\text{N}} \text{)}$ where S_{Ai}k=0 if there is no statistically significant difference between P_{Ai}k^{L} and P_{Ai}k^{N};
      P_{Ai}k^{L} and P_{Ai}k^{N} are the probabilities of the amino-acid residue Aᵢ^{k} occurring in a linker sequence and a non-linker loop sequence, respectively;
      wherein n_{gap2} is the number of gaps occurring in Aᵢ^{k} or Aᵢ₊₍ₘ₊₁₎^{k}, S_{Ai}k_{Ai+(m+1)}k(m)= log(P_{Ai}k_{Ai+(m+1)}k₍ₘ₎^{L}/P_{Ai}k_{Ai+(m+1)}k₍ₘ₎^{N})
      where S_{Ai}k_{Ai+(m+1)}k₍ₘ₎=0 if there is no statistically significant difference between P_{Ai}k_{Ai+(m+1)}k₍ₘ₎^{L} and P_{Ai}k_{Ai+(m+1)}k₍ₘ₎^{N};
      P_{Ai}k_{Ai+(m+1)}k₍ₘ₎^{L} and P_{Ai}k_{Ai+(m+1)}k₍ₘ₎^{N} are the probabilities of the amino-acid residues Aᵢ^{k} and Aᵢ₊₍ₘ₊₁₎^{k} occurring in a linker sequence and a non-linker loop sequence, respectively (the order of Aᵢ^{k} and Aᵢ₊₍ₘ₊₁₎^{k} does not matter) as interrupted by m arbitrary amino-acid residues (m is an integer, m = 0, 1, 2);
      and wherein n_{gap3} is the number of gaps occurring in Aᵢ^{k} or Aᵢ₋₍ₘ₊₁₎^{k}, S_{Ai}k_{Ai-(m+1)}k(m)= log(P_{Ai}k_{Ai-(m+1)}k₍ₘ₎^{L}/P_{Ai}k_{Ai-(m+1)}k₍ₘ₎^{N})
      where S_{Ai}k_{Ai-(m+1)}k₍ₘ₎=0 if there is no statistically significant difference between P_{Ai}k_{Ai-(m+1)}k₍ₘ₎^{L} and P_{Ai}k_{Ai-(m+1)}k₍ₘ₎^{N};
      P_{Ai}k_{Ai-(m+1)}k₍ₘ₎^{L} and P_{Ai}k_{Ai-(m+1)}k₍ₘ₎^{N} are the probabilities of the amino-acid residues Aᵢ^{k} and Aᵢ₋₍ₘ₊₁₎^{k} occurring in a linker sequence and a non-linker loop sequence, respectively (the order of Aᵢ^{k} and Aᵢ₋₍ₘ₊₁₎^{k} does not matter) as interrupted by m arbitrary amino-acid residues (m is an integer, m = 0, 1, 2));
   iii) a step for obtaining a linker trend score F₁₂s(i) of an amino-acid residue by the following equation: 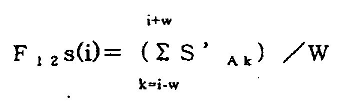
   iv) a step for obtaining a linker trend score F₁₂p(i) of an arbitrary amino-acid residue pair by the following equation: 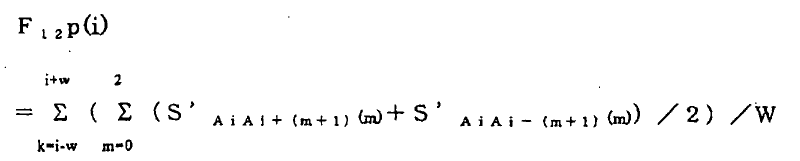 and
   v) a step for obtaining the linker degree determination score F₁₂(i) for the amino-acid residue Ai at the position i by the following equation:${\text{F}}_{\text{12}} {\text{(i)=F}}_{\text{12}} {\text{s(i)+ α}}_{\text{12}} {\text{F}}_{\text{12}} \text{p(i)}$ (where 0≦ α₁₂≦ 1).
(36) A system by which a linker degree determination score F₁₂(i) of an amino-acid residue Ai at a position i in an amino-acid sequence seq.0 with L₂ amino-acid residues (L₂ is an interger of 22 or more) for which the existence of n homologous sequences seq.1∼seq.n (n is an integer of 1 or more) is known is obtained by taking a window with w amino-acid residues before and after the amino-acid residue at the position i (i is an integer of 1 or more but not more than 22), the system comprising:
   i) a means for identifying an amino-acid residue Aᵢ^{k} in a seq.k (k is an integer of 1 or more but not more than n) corresponding to an amino-acid residue Ai⁰ at the position i in the seq.0 by aligning seq.0 and seq.1∼seq.n;
   ii) a means for obtaining parameters for the amino-acid residue Ai at the position i, S'_{Ai}, S'_{AiAi+(m+1)}(m) and S'_{AiAi-(m+1)}(m), by the following equation: 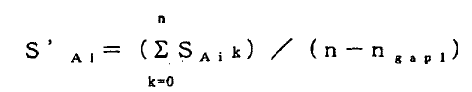 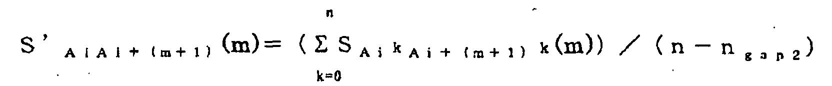 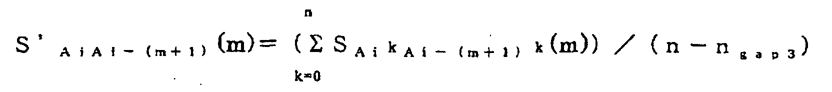 (where n_{gap1} is the number of gaps occurring in Aᵢ^{k},${\text{S}}_{\text{Ai}} {\text{k=log(P}}_{\text{Ai}} {\text{k}}^{\text{L}} {\text{/P}}_{\text{Ai}} {\text{k}}^{\text{N}} \text{)}$ where S_{Ai}^{k}=0 if there is no statistically significant difference between P_{Ai}k^{L} and P_{Ai}k^{N};
      P_{Ai}k^{L} and P_{Ai}k^{N} are the probabilities of the amino-acid residue Aᵢ^{k} occurring in a linker sequence and a non-linker loop sequence, respectively;
      wherein n_{gap2} is the number of gaps occurring in Aᵢ^{k} or Aᵢ₊₍ₘ₊₁₎^{k}, S_{Ai}k_{Ai+(m+1)}k(m)= log(P_{Ai}k_{Ai+(m+1)}k₍ₘ₎^{L}/P_{Ai}k_{Ai+(m+1)}k₍ₘ₎^{N})
      where S_{Ai}k_{Ai+(m+1)}k₍ₘ₎=0 if there is no statistically significant difference between P_{Ai}k_{Ai+(m+1)}k₍ₘ₎^{L} and P_{Ai}k_{Ai+(m+1)}k₍ₘ₎^{N};
      P_{Ai}k_{Ai+(m+1)}k₍ₘ₎^{L} and P_{Ai}k_{Ai+(m+1)}k₍ₘ₎^{N} are the probabilities of the amino-acid residues Aᵢ^{k} and Aᵢ₊₍ₘ₊₁₎^{k} occurring in the linker sequence and the non-linker loop sequence, respectively (the order of Aᵢ^{k} and Aᵢ₊₍ₘ₊₁₎^{k} does not matter) as interrupted by m arbitrary amino-acid residues (m is an integer, m = 0, 1, 2);
      and wherein n_{gap3} is the number of gaps occurring in Aᵢ^{k} or Aᵢ₋₍ₘ₊₁₎^{k}, S_{Ai}k_{Ai-(m+1)}k₍ₘ₎= log(P_{Ai}k_{Ai-(m+1)}k₍ₘ₎^{L}/P_{Ai}k_{Ai-(m+1)}k₍ₘ₎^{N})
      where S_{Ai}k_{Ai-(m+1)}k₍ₘ₎=0 if there is no statistically significant difference between P_{Ai}k_{Ai-(m+1)}k₍ₘ₎^{L} and P_{Ai}k_{Ai-(m+1)}k₍ₘ₎^{N};
      P_{Ai}k_{Ai-(m+1)}k₍ₘ₎^{L} and P_{Ai}k_{Ai-(m+1)}k₍ₘ₎^{N} are the probabilities of the amino-acid residues Aᵢ^{k} and Aᵢ₋₍ₘ₊₁₎^{k} occurring in the linker sequence and the non-linker loop sequence, respectively (the order of Aᵢ^{k} and Aᵢ₋₍ₘ₊₁₎^{k} does not matter) as interrupted by m arbitrary amino acid residues (m is an integer, m = 0, 1, 2));
   iii) a means for obtaining a linker trend score F₁₂s(i) of an amino-acid residue by the following equation; 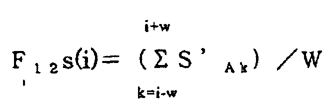
   iv) a means for obtaining a linker trend score F₁₂p(i) of an arbitrary amino-acid residue pair by the following equation; 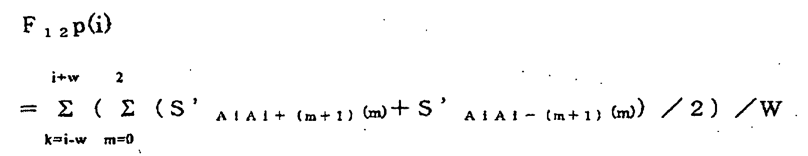 and
   v) a means for obtaining the linker degree determination score F₁₂(i) for the amino-acid residue Ai at the position i by the following equation:${\text{F}}_{\text{12}} {\text{(i)=F}}_{\text{12}} {\text{s(i)+ α}}_{\text{12}} {\text{F}}_{\text{12}} \text{p(i)}$ (where 0≦α₁₂≦ 1).
(37) A program for having a computer function as a system by which a linker degree determination score F₁₂(i) of an amino-acid residue Ai at a position i in an amino-acid sequence seq.0 with L₂ amino-acid residues (L₂ is an interger of 22 or more) for which the existence of n homologous sequences seq.1∼seq.n (n is an integer of 1 or more) is known is obtained by taking a window with w amino-acid residues before and after the amino-acid residue at the position i (i is an integer of 1 or more but not more than 22), the system comprising:
   i) a means for identifying an amino acid residue Aᵢ^{k} in a seq.k (k is an integer of 1 or more but not more than n) corresponding to an amino-acid residue Ai⁰ at the position i in the seq.0 by aligning seq.0 and seq.1∼seq.n;
   ii) a means for obtaining parameters for the amino-acid residue Ai at the position i, S'_{Ai}, S'_{AiAi+(m+1)}(m) and S'_{AiAi-(m+1)}(m), by the following equation: 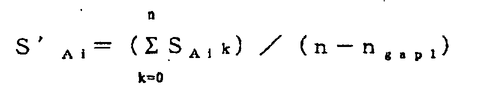 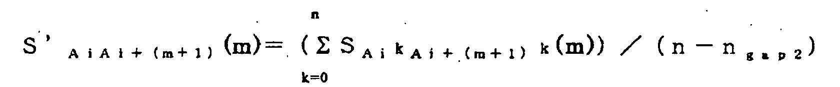 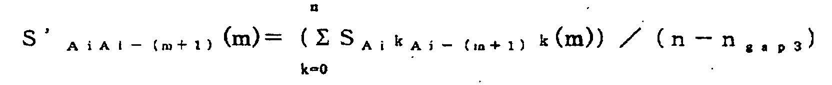 (where n_{gap1} is the number of gaps occurring in Aᵢ^{k},${\text{S}}_{\text{Ai}} {\text{k=log(P}}_{\text{Ai}} {\text{k}}^{\text{L}} {\text{/P}}_{\text{Ai}} {\text{k}}^{\text{N}} \text{)}$ where S_{Ai}k=0 if there is no statistically significant difference between P_{Ai}k^{L} and P_{Ai}k^{N};
      P_{Ai}k^{L} and P_{Ai}k^{N} are the probabilities of the amino-acid residue Aᵢ^{k} occurring in a linker sequence and a non-linker loop sequence, respectively;
      wherein n_{gap2} is the number of gaps occurring in Aᵢ^{k} or Aᵢ₊₍ₘ₊₁₎^{k}, S_{Ai}k_{Ai+(m+1)}k₍ₘ₎= log(P_{Ai}k_{Ai+(m+1)}k₍ₘ₎^{L}/P_{Ai}k_{Ai+(m+1)}k₍ₘ₎^{N})
      where S_{Ai}k_{Ai+(m+1)}k₍ₘ₎=0 if there is no statistically significant difference between P_{Ai}k_{Ai+(m+1)}k₍ₘ₎^{L} and P_{Ai}k_{Ai+(m+1)}k₍ₘ₎^{N};
      P_{Ai}k_{Ai+(m+1)}k₍ₘ₎^{L} and P_{Ai}k_{Ai+(m+1)}k₍ₘ₎^{N} are the probabilities of the amino-acid residues Aᵢ^{k} and Aᵢ₊₍ₘ₊₁₎^{k} occurring in the linker sequence and the non-linker loop sequence, respectively (the order of Aᵢ^{k} and Aᵢ₊₍ₘ₊₁₎^{k} does not matter) as interrupted by m arbitrary amino-acid residues (m is an integer, m = 0, 1, 2);
      and wherein n_{gap3} is the number of gaps occurring in Aᵢ^{k} or Aᵢ₋₍ₘ₊₁₎^{k},${\text{S}}_{\text{Ai}} {\text{k}}_{\text{Ai-(m+1)}} {\text{k(m)=log(P}}_{\text{Ai}} {\text{k}}_{\text{Ai-(m+1)}} {\text{k}}_{\text{(m)}} {\text{}}^{\text{L}} {\text{/P}}_{\text{Ai}} {\text{k}}_{\text{Ai-(m+1)}} {\text{k}}_{\text{(m)}} {\text{}}^{\text{N}} \text{)}$ where S_{Ai}k_{Ai-(m+1)}k₍ₘ₎=0 if there is no statistically significant difference between P_{Ai}k_{Ai-(m+1)}k₍ₘ₎^{L} and P_{Ai}k_{Ai-(m+1)}k₍ₘ₎^{N};
      P_{Ai}k_{Ai-(m+1)}k₍ₘ₎^{L} and P_{Ai}k_{Ai-(m+1)}k₍ₘ₎^{N} are the probabilities of the amino-acid residues Aᵢ^{k} and Aᵢ₋₍ₘ₊₁₎^{k} occurring in the linker sequence and the non-linker loop sequence, respectively (the order of Aᵢ^{k} and Aᵢ₋₍ₘ₊₁₎^{k} does not matter) as interrupted by m arbitrary amino-acid residues (m is an integer, m = 0, 1, 2);
   iii) a means for obtaining a linker trend score F₁₂s(i) of an amino-acid residue by the following equation; 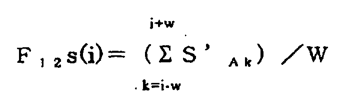
   iv) a means for obtaining a linker trend score F₁₂p(i) of an arbitrary amino-acid residue pair by the following equation; 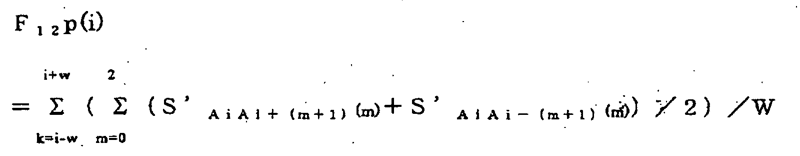 and
   v) a means for obtaining the linker degree determination score F₁₂(i) for the amino-acid residue Ai at the position i by the following equation:${\text{F}}_{\text{12}} {\text{(i)=F}}_{\text{12}} {\text{s(i)+α}}_{\text{12}} {\text{F}}_{\text{12}} \text{p(i)}$ (where 0≦ α₁₂≦ 1).
(38) A method of predicting a domain linker portion comprising:
   i) a step for obtaining a linker degree determination score of an amino-acid residue Ai at a position i in an amino-acid sequence with L₂ amino-acid residues (L₂ is an interger of 22 or more) according to the method as set forth in (32) or (35) (however, a linker degree determination score need not be obtained for 0 to 50 residues at the N and C terminals of the amino-acid sequence);
   ii) a step for executing secondary-structure prediction on the amino acid sequence and predicting which regions will take a loop structure;
   iii) a step for obtaining regions which are found likely to take a loop structre in the secondary-structure prediction and whose linker degree determination score is greater than 0; and
   iv) a step for predicting for each of the regions obtained in iii) that the position at which the linker degree determination score takes a maximum value is the position at which the domain linker exists.
(39) A system for predicting a domain linker portion comprising:
   i) a means for obtaining a linker degree determination score of an amino acid residue Ai at a position i in an amino-acid sequence with L₂ amino-acid residues (L₂ is an interger of 22 or more) according to the method as set forth in (32) or (35) (however, a linker degree determination score need not be obtained for 0 to 50 residues at the N and C terminals of the amino-acid sequence);
   ii) a means for executing secondary-structure prediction on the amino-acid sequence and predicting which regions will take a loop structure;
   iii) a means for obtaining regions which are found likely to take a loop structure in the secondary-structure prediction and whose linker degree determination score is greater than 0; and
   iv) a means for predicting for each of the regions obtained in iii) that the position at which the linker degree determination score takes a maximum value is the position at which the domain linker exists.
(40) A program for having a computer function as a system for predicting a domain linker portion, the system comprising:
   i) a means for obtaining a linker degree determination score of an amino-acid residue Ai at a position i in an amino-acid sequence with L₂ amino-acid residues (L₂ is an interger of 22 or more) according to the method as set forth in (32) or (35) (however, a linker degree determination score need not be obtained for 0 to 50 residues at the N and C terminals of the amino-acid sequence);
   ii) a means for executing secondary-structure prediction on the amino-acid sequence and predicting which regions will take a loop structure;
   iii) a means for obtaining regions which are found likely to take a loop structure in the secondary-structure prediction and whose linker degree determination score is greater than 0; and
   iv) a means for predicting for each of the regions obtained in iii) that the position at which the linker degree determination score takes a maximum value is the position at which the domain linker exists.
(41) A method of constructing an amino-acid sequence database comprising:
   i) a step for obtaining a linker degree determination score of an amino-acid residue Ai at a position i in an amino-acid sequence with L₂ amino-acid residues (L₂ is an interger of 22 or more) according to the method as set forth in (32) or (35) (however, a linker degree determination score need not be obtained for 0 to 50 residues at the N and C terminals of the amino-acid sequence);
   ii) a step for executing secondary-structure prediction on the amino-acid sequence and predicting which regions will take a loop structure;
   iii) a step for obtaining regions which are found likely to take a loop structure in the secondary-structure prediction and whose linker degree determination score is greater than 0;
   iv) a step for selecting from the regions obtained in iii) the one whose maximum value of the linker degree determination score is greater than a lower limit value; and
   v) a step for recording in a recording medium the amino-acid sequence of the region selected in iv).
(42) A domain linker peptide made of the same amino-acid sequence as the amino-acid sequence of a region whose maximum value of a linker degree determination score is greater than a lower limit value, and which was obtained by a method comprising:
   i) a step for obtaining a linker degree determination score of an amino-acid residue Ai at a position i in an amino-acid sequence with L₂ amino acid residues (L₂ is an interger of 22 or more) according to a method as set forth in (32) or (35) (however, a linker degree determination score need not be obtained for 0 to 50 residues at the N and C terminals of the amino acid sequence);
   ii) a step for executing secondary-structure prediction on the amino-acid sequence and predicting which regions will take a loop structure;
   iii) a step for obtaining regions which are found likely to take a loop structure in the secondary-structure prediction and whose linker trend determination score is greater than 0; and
   iv) a step for selecting from the regions obtained in iii) the one whose maximum value of the linker degree determination score is greater than the lower limit value.
(43) A method of predicting a structural domain comprising a step for predicting about an amino-acid sequence with L₂ amino-acid residues (L₂ is an interger of 22 or more) that a sequence fragment generated by cutting off the amino-acid sequence at any portion of a region including the domain linker portion predicted by the method as set forth in (38) or the position at which a domain linker exists is a structural domain.
(44) A method as set forth in (43), wherein if n domain linker portions are predicted, t of them (t is an integer of 1 or more but not more than n) is selected, all the patterns for cutting an amino acid sequence at that position are considererd, and all the sequence fragments obtained are predicted as structural domains.
(45) A system for predicting a structural domain comprising a means for predicting about an amino-acid sequence with L₂ amino-acid residues (L₂ is an interger of 22 or more) that a sequence fragment generated by cutting off the amino-acid sequence at any portion of a region including the domain linker portion predicted by the method as set forth in (38) or the position at which a domain linker exists is a structural domain.
(46) A program for having a computer function as a system for predicting a structural domain, the system comprising a means for predicting about an amino-acid sequence with L₂ amino-acid residues (L₂ is an interger of 22 or more) that a sequence fragment generated by cutting off the amino-acid sequence at any portion of a region including the domain linker portion predicted by the method as set forth in (38) or the position at which a domain linker exists is a structural domain.
(47) A method of constructing an amino-acid sequence database comprising a step in which concerning an amino-acid sequence with L₂ amino-acid residues (L₂ is an interger of 22 or more), the amino-acid sequence of a sequence fragment generated by cutting off the first-mentioned amino-acid sequence at any portion of a region including the domain linker portion predicted by the method as set forth in (38) or the portion at which a domain linker exists is recorded in a recording medium.
(48) A method of producing a protein comprising a step for producing a protein having the same amino-acid sequence as the structural domain predicted by the method as set forth in (43).
(49) A method of analyzing a protein comprising a step for analyzing a protein having the same amino-acid sequence as the structural domain predicted by the method as set forth in (43).
(50) A method of producing a protein comprising designing a new multi-domain protein generated by connecting at least 2 protein fragments with a domain linker peptide as set forth in (42) and producing this multi-domain protein.

In this description, a "structural domain region" refers to a local region in an amino-acid sequence of a protein, in which a polypeptide chain is folded to form a compact and stable structure. It is needless to say that this polypeptide folding structure is formed in an intact protein, but the structure can also be formed solely or by association with low molecules (ligand, heavy atom, peptide, nucleic acid, etc.) when a structural domain is cut off from a protein.

The "structural domain" means a protein fragment in which a polypeptide chain in a structural domain is folded to form a structure. Since the structural domain can form a structure independently of other portions of a protein, it is also a functionally independent unit in many cases.

A "multi-domain protein" is a protein comprised of two or more structural domains.

A "domain linker" is a sequence taking a loop structure connecting adjacent two structural domains among structures of multi-domain proteins. Usually, the domain linker is a peptide chain shorter than the structural domain.

A "non-linker loop" is a sequence taking a loop structure in a structural domain.

In the fields of structural biology and molecular biology, terms such as "functional domain region" and "functional domain" may be used. The "functional domain region" is a local region in an amino-acid sequence in a protein and a sequence in which a polypeptide chain is folded so as to exert a specific function. It is needless to say that this polypeptide folding structure is formed in an intact protein, but the structure can also be formed solely or by association with low molecules (ligand, heavy atom, peptide, nucleic acid, etc.) when a structural domain is cut off from a protein. The "functional domain" is a protein fragment in which a polypeptide chain of the functional domain region is folded so as to exert a specific function.

The structural domain may solely constitute a functional domain, but a plurality of structural domains may constitute a functional domain. Conversely, it can be said that the functional domain consists of one or more structural domains. Therefore, since the structural domain is a basic structural unit in a structure of a protein, it is also an indispensable unit in analysis of a molecular function of a protein. In the present invention, a relation between an amino-acid sequence not with the functional domain but with the structural domain will be examined.

A "window" is an amino-acid sequence of a certain length (10 residues, for example) in an amino-acid sequence of an intact protein. The window is effective in obtaining characteristics of the residues at the center of the window based on the characteristics of the residues in the region. In a preferred embodiment of the present invention, the window was used for calculating an output value of a neural network and for averaging the output values. Also, in another preferred embodiment of the present invention, the window was used for locally smoothing a numeral value which can be obtained continuously over the full length of a protein.

In this description, "-" indicates a range including numeral values set forth before and after the symbol as a minimum value and a maximum value, respectively.

This description includes specifications and / or drawings in the Japanese Patent Application Nos. 2001-309434 and 2002-172101, underlying the right of priority of the present application.

### Brief Description of the Drawings

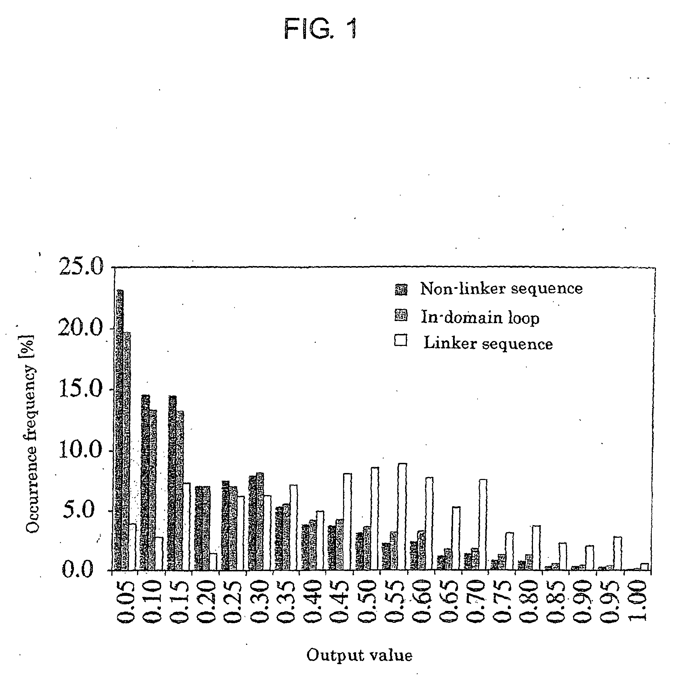

Fig. 1 shows distribution of average values of neural network output values for a linker sequence and a non-linker sequence. Black and white bar graphs represent distribution of sequence segments corresponding to the linker sequence and the non-linker sequence, respectively. Gray bar graphs represent distribution of in-domain loop sequence. The output values were calculated using a three-layer neural network after learning with the window size of 19 and the number of hidden units of 2 and averaged using a smoothing window of 19 residues (See the section on the smoothing window of Materials & Methods). Averaging of the output values (for positions of the residues in its smoothing window) decreases occurrence of the linker sequence of the average output value at 1.0. For evaluation, a 10-fold Jackknife test was used.

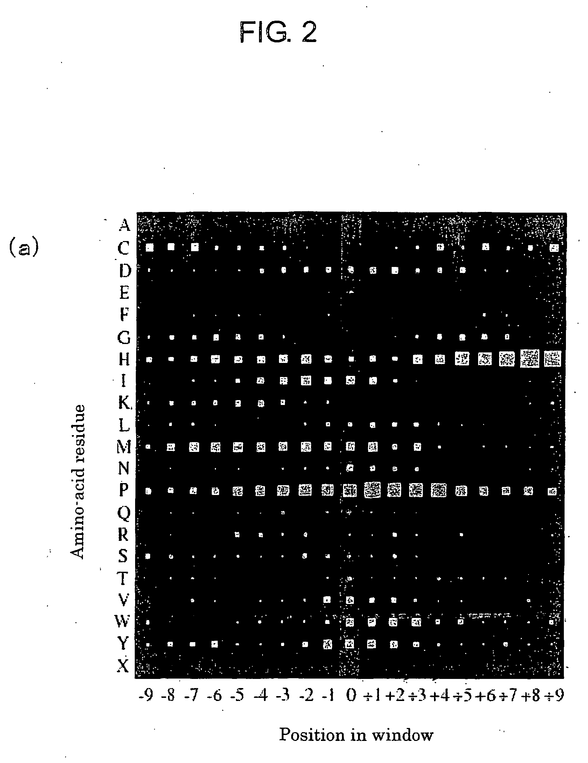

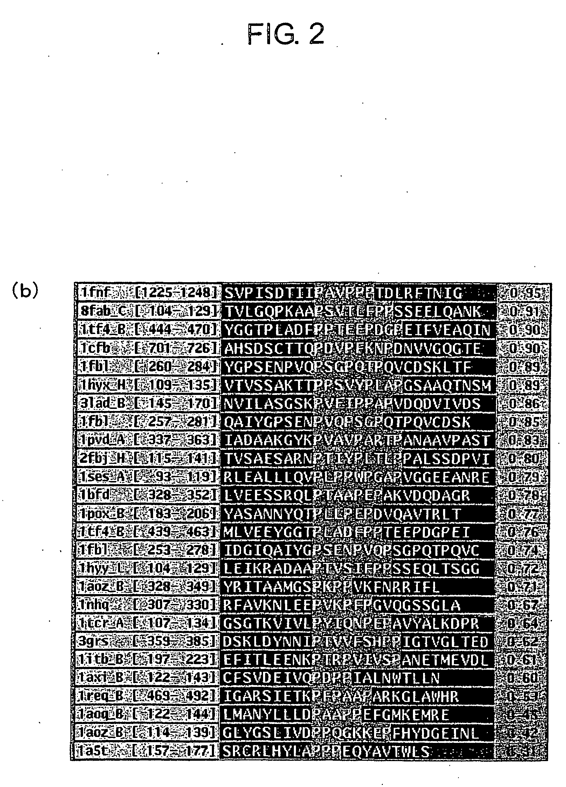

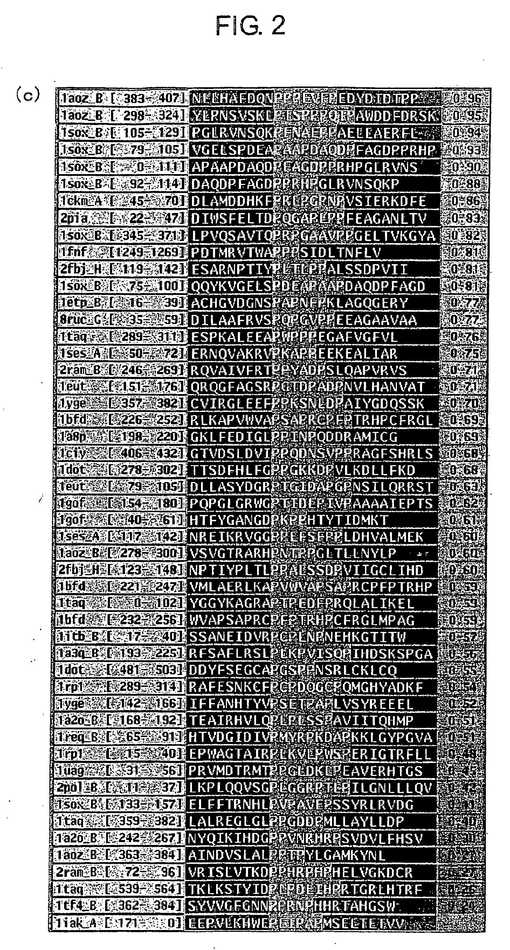

Fig. 2(a) shows a Hinton diagram of optimized weight parameters. The parameter values were shown by positive and negative in red and blue squares, respectively. The parameters were calculated using a neural network without hidden units and explained as contribution of residues for discriminating the domain linker and the non-linker. 10 sets of the independent optimized parameters obtained by the 10-fold Jackknife test were standardized and averaged. We used the window size of 19 residues. (b, c) Proline-rich segments in a domain linker (b) and proline-rich segments inn other regions (c). A sequence of all the segments including at least 3 residues of proline in 9 residues existing in 74 multi-domain proteins (Table 1) (proline-rich segment) is shown. The length of the proline-rich segment is varied from 3 to 9 residues. The praline-rich segment is highlighted, and adjacent 9 residues on both sides are listed in Table. The residues are colored according to contribution in the Hinton diagram (Fig. 2a). That is, proline is in red, histidine is in blue, and the other amino acids are in white. Identifiers of protein chains are shown on the left with their starting and ending amino-acid residues. The neural network output values smoothed for the proline-rich segment are averaged for the range of the segment and shown on the right. The green hue is in proportion to the output value of the neural network from 0.0 (black) to 1.0 (light green). This value is not shown for the lower row in Fig. 2c. That is because the proline-rich segment is close to the C terminal of a protein sequence and its smoothed output value could not be obtained. The output value was calculated by the neural network after learning with the window size of 19 and the number of hidden units of 2 and smoothed using the smoothing window of 19 residues.

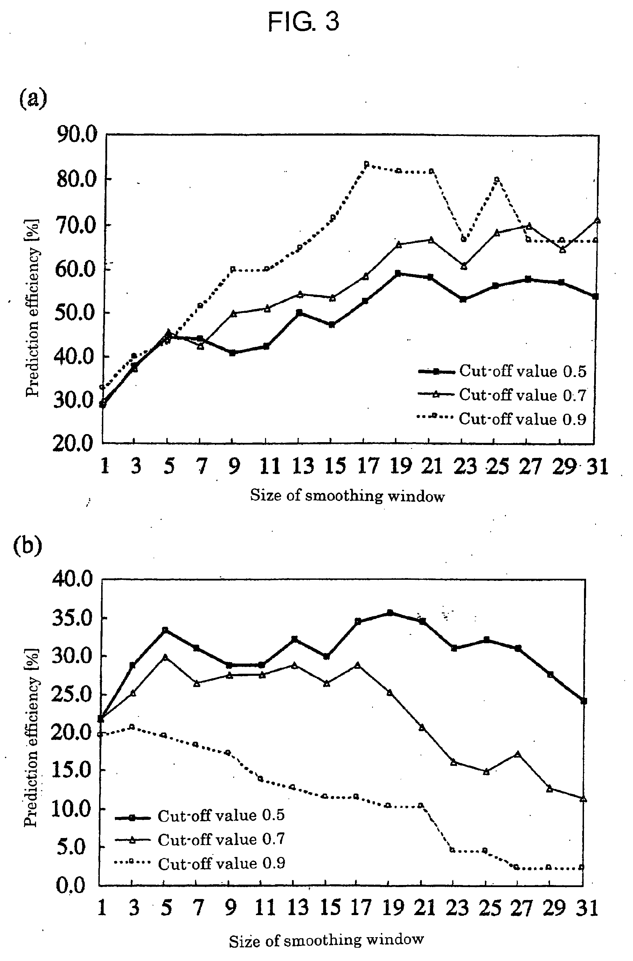

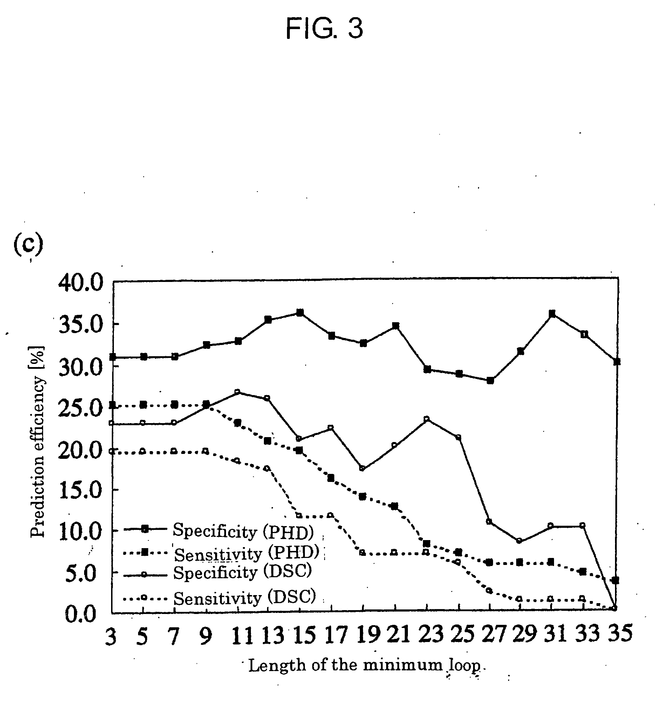

Fig. 3 (a, b) shows efficiency of domain linker prediction by the neural network. The domain linker in a protein sequence was predicted with a threshold value of 0.5. Also, the efficiency predicting the predicted region in the first rank was evaluated using the 10-fold Jackknife test: (a) Cases where the domain linker corresponding to SCOP derived domain linker (specificity) is predicted. (b) How much share of all the SCOP derived domain linker sequences is held by the SCOP derived domain linker sequences correctly predicted by the neural network (sensitivity). The horizontal axis indicates the size of the smoothing window. The prediction efficiency was obtained using a cut-off value of 0.5 (black circle and |bold solid line), 0.7 (white triangle and thin solid line) and 0.9 (while circle and dotted line). (c) Prediction efficiency of domain linker by DSC, PHD. The domain linker was predicted as follows using a secondary structure predicting program. Assume that the loop region predicted by DSC, PHD is ranked based on its length and that a longer loop region has a tendency to become a domain linker, the longest loop region was predicted as a domain linker. As in Fig. 3a, by changing the length of the loop domain used for prediction, two values (specificity, solid line; sensitivity, broken line) were calculated (horizontal axis). The 10-fold Jackknife test result of production by DSC, PHD is shown with white circles and black squares.
Fig. 4 shows ranking of the predicted domain linkers. The prediction was carried out with the 19-residue smoothing window, threshold value and cut-off value of 0.5 and evaluated using the 10-fold Jackknife test. Occurrence frequency of the linker in the predicted region is shown (black, correct prediction; white wrong prediction). The total of predicted regions was 139, in which 47 corresponded to correct prediction, while 92 were wrong.

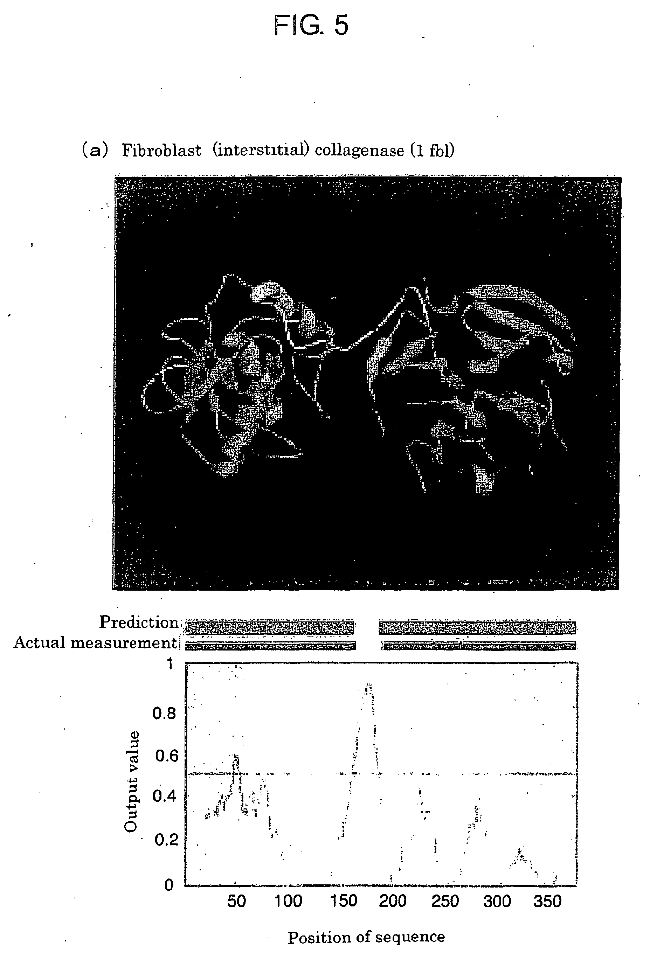

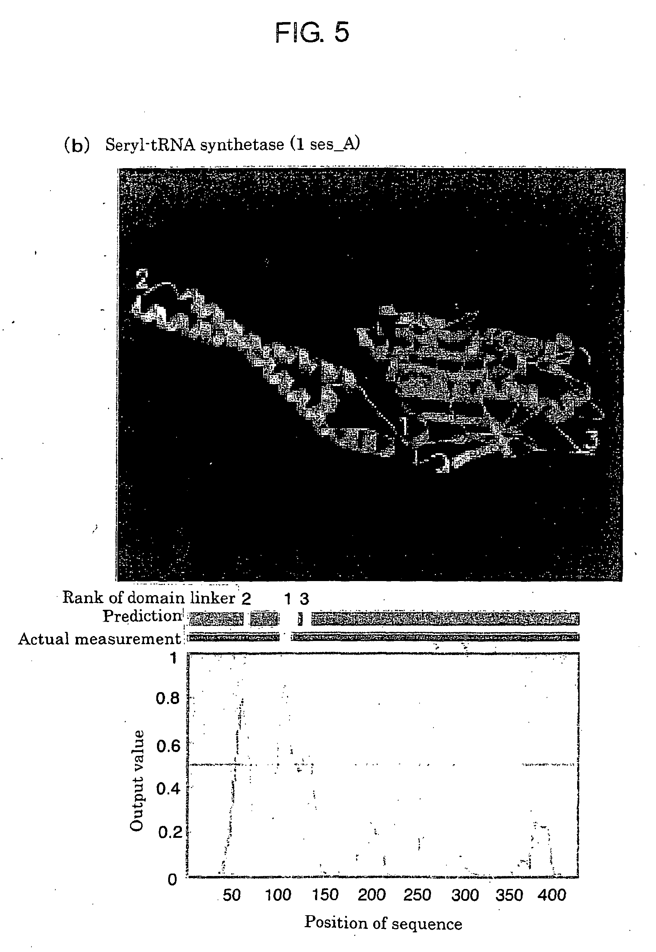

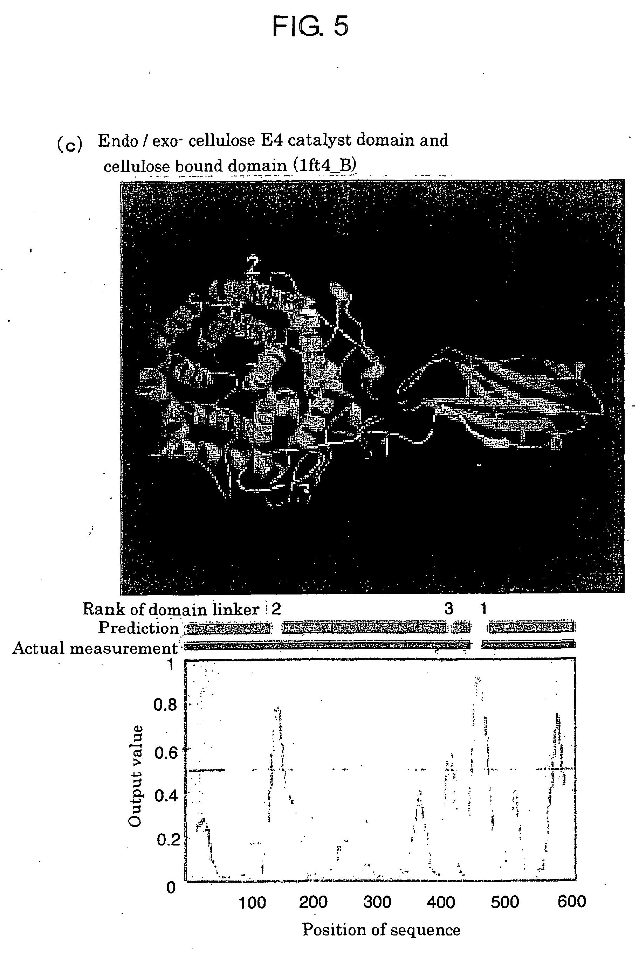

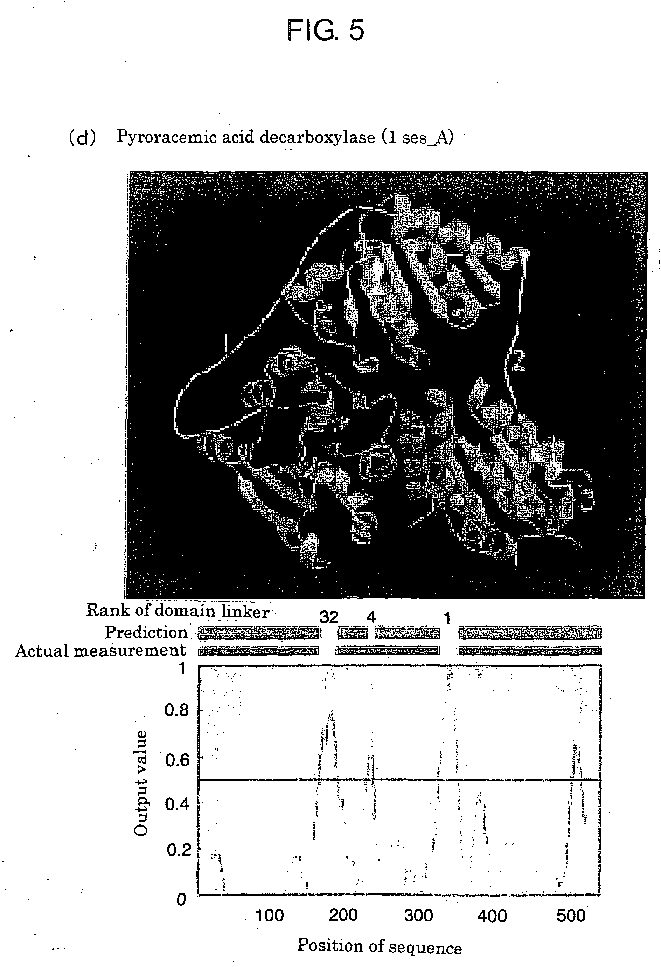

Fig. 5 shows a success example of the domain linker prediction. The prediction was carried out with the 19-residue smoothing window, the threshold value and the cut-off value of 0.5. In each example, the lower plot indicates an output value of the neural network (smoothed output value, blue; raw data, light red) against the number of residues. The above diagram shows a ribbon representation (prepared using Molscript and Raster 3D). Here, the predicted domain linker is labeled according to its rank (when two or more regions are predicted), and the regions with boundaries determined by the predicted domain linker were colored to indicate the difference.
Fig. 6 is a failure example of domain linker prediction. The prediction was carried out as in Fig. 5.
Fig. 7 shows a neural network used for sequence classification.
Fig. 8 shows the sequence classification. When a residue at the center of the window is a domain linker, it shall be 0, and when it is not, it shall be 0.
Fig. 9 shows sequence encoding. Each amino-acid residue is represented by a 21-bit binary number. Only the bit at the corresponding residue position is 1, while the others are 0. The 21^{st} bit corresponds to a non-standard amino acid.
Fig. 10 shows a neuron model.
Fig. 11 shows a three-layer neural network.
Fig. 12 is a flow chart for explaining 1 preferred embodiment of how to learn a neural network according to the present invention.
Fig. 13 is a flowchart for explaining 1 preferred embodiment of a method of predicting a linker sequence of a protein according to the present invention.
Fig. 14 is a block diagram showing constitution of a linker sequence predicting system according to the present invention.
Fig. 15 is a block diagram showing functions of a linker sequence predicting system according to the present invention.
Fig. 16 shows distribution of output values of a neural network for residues in and outside a domain linker.
Fig. 17 is a table prepared by extracting a linker sequence portion from a multi-domain protein database with known structure.
Fig. 18 is a table prepared by extracting a linker sequence portion from a multi-domain protein database with known structure.
Fig. 19 a table prepared by extracting a linker sequence portion from a multi-domain protein database with known structure.
Fig. 20 is a flowchart explaining an operation of a linker sequence predicting / detecting system according to a preferred embodiment of the 18^{th} invention of the present application or a preferred embodiment of the 19^{th} invention of the present application.
Fig. 21 is a block diagram showing constitution of a linker sequence predicting / detecting system according to a preferred embodiment of the present invention.
Fig. 22 is a block diagram showing functions of a linker sequence predicting / detecting system according to a preferred embodiment of the 19^{th} invention of the present application.
Fig. 23 is a flowchart of a method of predicting a structural domain according to a preferred embodiment of the 21^{st} invention of the present application.
Fig. 24 is a flowchart explaining an operation of a trend parameter calculating system for a single amino-acid residue according to a preferred embodiment of the 24^{th} invention of the present application.
Fig. 25 is a block diagram explaining functions of a trend parameter calculating system for a single amino-acid residue according to a preferred embodiment of the 24^{th} invention of the present application.
Fig. 26 is a flowchart explaining an operation of a trend parameter calculating system for an amino-acid residue pair according to a preferred embodiment of the 26^{th} invention of the present application.
Fig. 27 is a block diagram explaining functions of a trend parameter calculating system for an amino-acid residue pair according to a preferred embodiment of the 26^{th} invention of the present application.
Fig. 28 is a flowchart explaining an operation of a trend parameter calculating system for an amino-acid residue pair according to a preferred embodiment of the 28^{th} invention of the present application.
Fig. 29 is a block diagram explaining functions of a system for obtaining a linker degree discrimination score F₁s according to a preferred embodiment of the 28^{th} invention of the present application.
Fig. 30 is a flowchart explaining an operation of a system for obtaining a linker degree discrimination score F₂(i) according to a preferred embodiment of the 30^{th} invention of the present application.
Fig. 31 is a block diagram explaining functions of a system for obtaining a linker degree discrimination score F₂(i) according to a preferred embodiment of the 30^{th} invention of the present application.
Fig. 32 is a flowchart explaining an operation of a method of obtaining a linker degree discrimination score F₁₂(i) according to a preferred embodiment of the 33^{rd} invention of the present application or a system for obtaining a linker degree discrimination score F₁₂(i) of the 34^{th} invention of the present application.
Fig. 33 is a block diagram explaining functions of a system for obtaining a linker degree discrimination score F₁₂(i) according to a preferred embodiment of the 34^{th} invention of the present application.
Fig. 34 is a flowchart explaining an operation of a method of predicting a domain linker portion according to a preferred embodiment of the 36^{th} invention of the present application or a predicting system for a domain linker portion according to a preferred embodiment of the 37^{th} invention of the present application.
Fig. 35 is a block diagram explaining functions of a predicting system for a domain linker portion according to a preferred embodiment of the 37^{th} invention of the present application.
Fig. 36 is a flowchart explaining an operation of a method of predicting a domain linker portion according to a preferred embodiment of the 36^{th} invention of the present application or a predicting system for a domain linker portion according to another preferred embodiment of the 37^{th} invention of the present application.
Fig. 37 is a block diagram explaining functions of a predicting system for a domain linker portion according to another preferred embodiment of the 37^{th} invention of the present application.
Fig. 38 is a flowchart explaining an operation of a system for predicting a structural domain according to a preferred embodiment of the 42^{nd} invention of the present application.
Fig. 39 is a block diagram explaining functions of a system for predicting a structural domain according to a preferred embodiment of the 42^{nd} invention of the present application.
Fig. 40 is a flowchart explaining an operation of a system for predicting a structural domain according to another preferred embodiment of the 42^{nd} invention of the present application.
Fig. 41 is a block diagram explaining functions of a system for predicting a structural domain according to another preferred embodiment of the 42^{nd} invention of the present application.
Fig. 42 shows distribution of sequence length.
Fig. 43 shows the length of a sequence (number of amino-acid residues) for each of a linker sequence and a non-linker loop sequence.
Fig. 44 shows a probability of occurrence of an amino-acid residue for each of a linker sequence and a non-linker loop sequence.
Fig. 45 shows how to obtain a single amino-acid residue trend parameter.
Fig. 46 shows grouping and alignment of a linker sequence.
Fig. 47 shows a probability of occurrence of an amino-acid residue pair with 0 piece of an arbitrary amino-acid residue between them for each of a linker sequence and a non-linker loop sequence.
Fig. 48 shows a probability of occurrence of an amino-acid residue pair with 1 piece of an arbitrary amino-acid residue between them for each of a linker sequence and a non-linker loop sequence.
Fig. 49 shows a probability of occurrence of an amino-acid residue pair with 2 pieces of an arbitrary amino-acid residue between them for each of a linker sequence and a non-linker loop sequence.
Fig. 50 shows how to obtain an amino-acid residue pair trend parameter.
Fig. 51 is a distribution map showing distribution state of scores of each sequence by executing a calculation for a linker degree discrimination score according to a preferred embodiment of the 28^{th} invention of the present application for prepared 242 pieces of a linker sequence and 3381 pieces of non-linker sequence with F₁s for the horizontal axis and F₁p for the vertical axis.
Fig. 52 shows a result of domain linker prediction.
Fig. 53 shows how to take a window.
Fig. 54 shows aligned sequences of seq.0 and seq. 1 through seq. n and how to take a window.

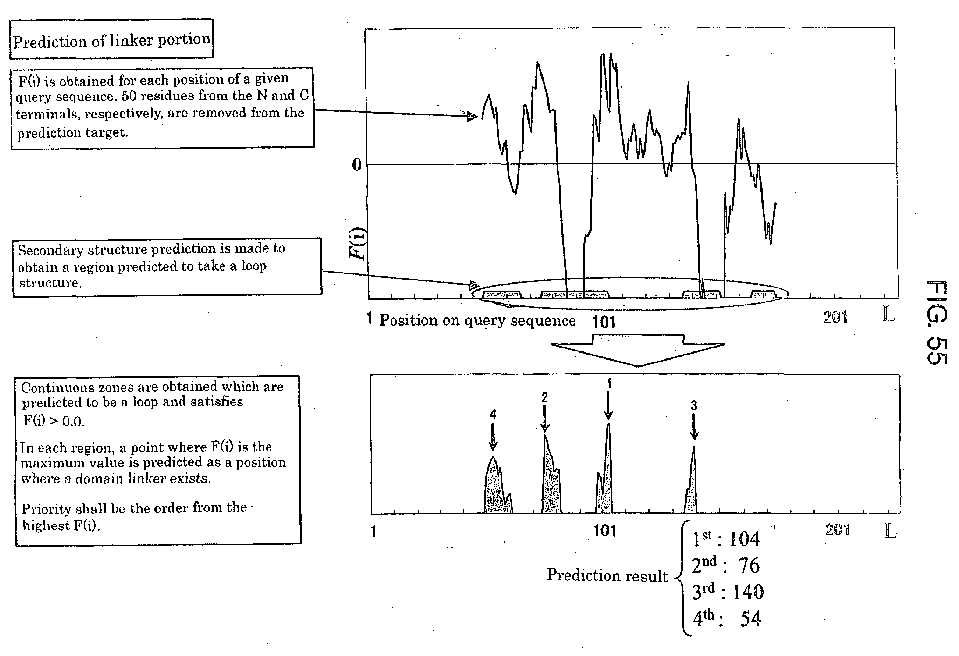

Fig. 55 shows an outline of a predicting method of a domain linker portion.

### Brief Description of Numerals

1: Computer
2. CPU
3: ROM
4: RAM
5: Input part
6: Sending / receiving part
7: Display part
8: Hard disk drive
9: CD-ROM drive
10: CD-ROM
11: Amino-acid sequence input part
12: Window setting part
13: In-window amino-acid sequence input part
14: Output value calculation part
15: Predicted value granting part
16: Window position moving part
17: Smoothing window setting part
18: Average value calculation part
19: Smoothing window moving part
20: Linker sequence prediction part
101: Computer
102. CPU
103: ROM
104: RAM
105: Input part
106: Sending / receiving part
107: Display part
108: Hard disk drive
109: CD-ROM drive
110: CD-ROM
1021: Linker sequence extraction part
1022: Non-linker loop sequence extraction part
1023: P_{Xaa}^{L} calculation part
1024: P_{XaaYaa(m)}^{L} calculation part
1031: Linker sequence extraction part
1032: Non-linker loop sequence extraction part
1033: P_{Xaa}^{L} calculation part
1034: P_{XaaYaa (m)}^{L} calculation part
1035: S_{Xaa} calculation part
1041: Linker sequence extraction part
1042: Non-linker loop sequence extraction part
1043: P_{Xaa}^{L} calculation part
1044: P_{XaaYaa (m)}^{L} calculation part
1045: S_{XaaYaa (m)} calculation part
1051:F₁s calculation part
1052:F₁p calculation part
1053:F₁ calculation part
1071:F₁₁s (i) calculation part
1072:F₁₁p (i) calculation part
1073:F₁₁ (i) calculation part
1081: Aᵢ^{k} identification part
1082:S'_{Ai}, S' _{AiAi+(m+1)}(m) and S' _{AiAi-(m+1)}(m) calculation part
1083:F₁₂s (i) calculation part
1084:F₁₂p (i) calculation part
1085:F₁₂ (i) calculation part
1091:F₁₁s (i) calculation part
1092:F₁₁p (i) calculation part
1093:F₁₁ (i) calculation part
1094: Secondary structure prediction part
1095: Region search part
1096: Domain linker existing position prediction part
1101: Aᵢ^{k} identification part
1102:S'_{Ai}, S'_{AiAi+(m+1)}(m) and S'_{AiAi-(m+1)}(m) calculation part
1103:F₁₂s (i) calculation part
1104:F₁₂p (i) calculation part
1105:F₁₂ (i) calculation part
1106: Secondary structure prediction part
1107: Region search part
1108: Domain linker existing position prediction part
1201:F₁₁s (i) calculation part
1202:F₁₁p (i) calculation part
1203:F₁₁ (i) calculation part
1204: Secondary structure prediction part
1205: Region search part
1206: Domain linker existing position prediction part
1207: Structural domain prediction part
1301: Aᵢ^{k} identification part
1302:S'_{Ai}, S' _{AiAi+(m+1)}(m) and S' _{AiAi-(m+1)}(m) calculation part
1303:F₁₂s (i) calculation part
1304:F₁₂p (i) calculation part
1305:F₁₂ (i) calculation part
1306: Secondary structure prediction part
1307: Region search part
1308: Domain linker existing position prediction part
1309: Structural domain prediction part

### Best Mode for Carrying-out of the Invention

A suitable mode for carrying out the present invention will be described below referring to the attached drawings. In Figs, 12, 13, 20, 23, 24, 26, 28, 30, 32, 34, 36, 38 and 40, S indicates each step.

The first invention of the present application is a method of having a neural network identify and learn a linker sequence of a protein consisting of 2 or more structural domains comprising:
a dividing step for dividing an amino-acid sequence of a protein consisting of 2 or more structural domains of a data set into a linker sequence and a non-linker sequence;
a window setting step for taking a window of a range of 5 to 35 residues within the amino-acid sequence of the protein consisting of two or more structural domains of the data set;
a sequence classifying step in which, if an amino-acid residue located at the center of the window constitutes a part of the linker sequence, a numeral value is granted to classify the amino-acid sequence in the window positive sequence and if the amino-acid residue located at the center of the window constitutes a part of the non-linker sequence, a numeral value is granted to classify the amino-acid sequence in the window as a negative sequence; and
a learning step for repeatedly learning to optimize a weight parameter of a hierarchical neural network in a back-propagation method,
and the back-propagation method is a method to determine the weight parameter of the hierarchical neural network by inputting a value which represents an amino-acid sequence in the window in a numeral value so as to acquire an output value and by calculating an error between the output value and the numeral value which classifies the amino-acid sequence in the window as a positive sequence or a negative sequence so that the error becomes the minimum.

In the above method, it is advantageous that, before the dividing step for dividing an amino-acid sequence of a protein of a data set into a linker sequence and a non-linker sequence, a data set of an amino-acid sequence of a protein consisting of 2 or more structural domains whose structure is known is created.

In the above method, as a value representing an amino-acid sequence in a numeral value, a numeral value which converted the amino-acid sequence into a binary code can be exemplified. Also, the amino-acid sequence can be represented by a numeral value of 1 when it is classified as a positive sequence, while by a numeral value of 0 when classified as a negative sequence, or these numeral values can be switched (reversed).

The number of hidden units of a neural network may be 0 through 2. In general, the larger this number is, the input / output relations at a higher level can be learned, but when the number of data in a data set is small, the restriction prevents full learning of the high-level correspondence between the amino-acid sequence and structural information, and the effect of setting the number of hidden units to a large number can not be gained. Therefore, in the present invention, for the purpose of decreasing useless variables as much as possible, it is desirable that the range is 0 through 2, but it might become desirable to have a range of 2 or more due to future expansion of the database.

The window size is 5 to 35 amino-acid residues, but more preferably 10 to 35 residues, and furthermore preferably 19 residues. If the window size is less than 5 residues, characteristics of a sequence pattern can not be fully extracted, and full learning effect can not be expected. On the contrary, if it is larger than 35 residues, the number of variables to be determined by learning increases and if the number of learning data is smaller than the number of variables to be determined, "memorization" (phenomenon that even fine characteristics of learning data is extracted) is apt to occur, and learning efficiency tends to degrade.

It is advantageous that the above sequence classifying process and the learning process are repeated by moving the position of the window in a desired range of the amino-acid sequence of a protein of a data set (for example, a range excluding up to 60 residues respectively from the N terminal and the C terminal).

Also, it is advantageous that the above dividing process, window setting process, sequence classifying process and the learning process are executed for the amino-acid sequence of all the proteins in the created data set.

The amino-acid residue located at the center of the window can be an amino-acid residue located in, the neighborhood of the center of the window. For example, if the total of the amino-acid residues in a window is 2n+1 pieces, the (n+1)th amino-acid from the 1^{st} amino acid in the window can be cited as an amino-acid residue located at the center of the window, and if the total of the amino-acid residues in a window is 2n pieces, the nth or the (n+1)th amino-acid from the 1^{st} amino acid in the window can be cited as an amino-acid residue located at the center of the window.

The back-propagation method is described in detail in Rumelhalt, 1986.

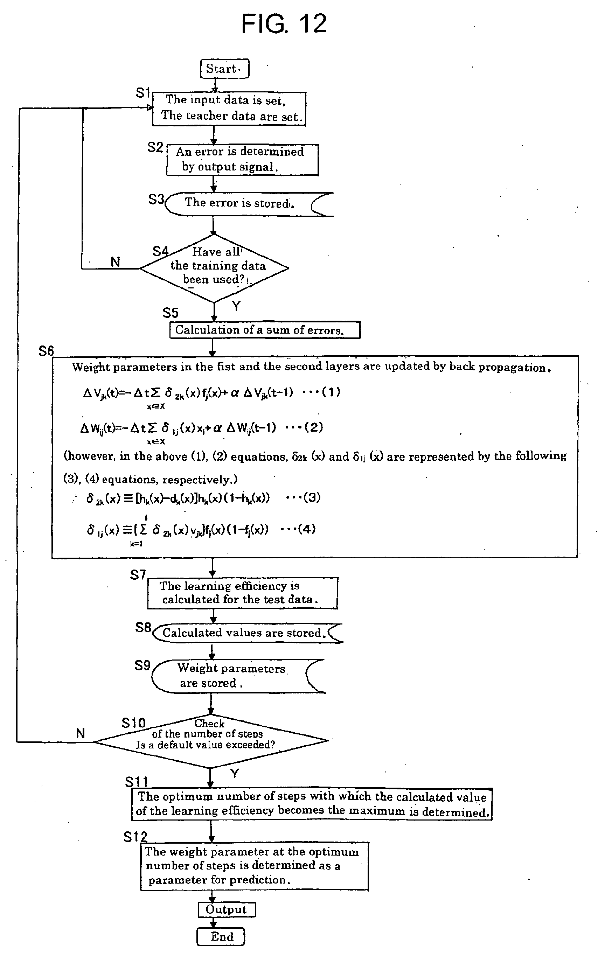

Fig. 12 is a flow chart for explaining 1 preferred embodiment of how to learn a neural network according to the present invention. Here, a three-layer feed-forward type neural network is used.

First, a data set of amino-acid sequences of proteins whose structure is known and which consists of 2 or more structural domains is prepared. In creating a data set, appropriate protein structures registered in PDB, for example, may be selected.

Each protein in the data set is divided into a linker sequence and a non-linker sequence.

Then, for the protein in the data set, a window is taken in the amino-acid sequence, and if a residue at the center of the window constitutes a part of the linker sequence, the amino-acid sequence in the window is classified as a positive sequence, while a residue at the center of the window constitutes a part of the non-linker sequence, the amino-acid sequence in the window is classified as a negative sequence. This classification process is to be learned by a neural network thereafter, but before that, it is advantageous that input data and teacher data are converted into a binary code. For learning, it is advantageous to use the back-propagation method.

In order to evaluate learning efficiency, the data set is equally divided into the one for training and the other for test. The proportion of the data set for training to the data set for test may be 9:1. In the predicting method by a neural network, the Jackknife method (Chou et al., 1998) can be used as a method for evaluating its prediction efficiency. In this Jackknife method, the data set is divided into 10 groups, in which learning is executed for 9 groups of them, and after tests are made for the rest, this is repeated for all the combinations. By using this method, all the data can be statistically processed as a test data, and even if the number of data sets is small, restriction by the data set number can be overcome. If the number of data sets is sufficient, this method is not necessarily required, and the proportion of training data to test data in evaluating the prediction efficiency can be selected as appropriate. The training data and the test data can be used as fixed or by various combinations. For example, in examining learning conditions, it is advantageous to use the training data and the test data as fixed. Also, once the learning conditions are determined, it is advantageous to make prediction after executing learning with various combinations of training data and test data.

The input data and the teacher data are set (S1). The input data corresponds to an amino-acid sequence in a window taken in the amino-acid sequence of a protein in the data set. The teacher data is correct output to the input data (that is, whether the central residue of the inputted amino-acid sequence constitutes a part of a domain linker or not).

An output signal is obtained from the neural network to which the input data is inputted so as to determine an error from the teacher data (S2).

The error determined in S2 is stored (S3).

It is judged whether the steps of S1 through S3 are carried out for all the training data or not (S4), and if the judgment result is No, the steps of S1 through S3 are carried out for unprocessed training data.

For all the training data, a sum of errors between the output signal and the teacher data is calculated (S5).

By the back-propagation method, a 1-layer and a 2-layer weight parameters (Vⱼₖ, Wᵢⱼ) are updated (S6). 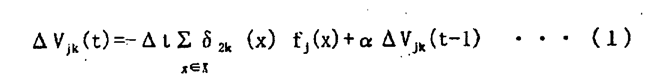 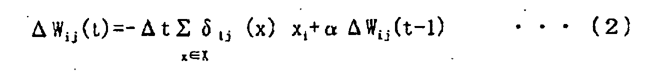 (however, in the above (1), (2) equations, δ₂ₖ (x) and δ₁ⱼ (x) are represented by the following (3), (4) equations, respectively.)${\text{δ}}_{\text{2k}} {\text{(x) ≡ [h}}_{\text{k}} {\text{(x)-d}}_{\text{k}} {\text{(x)]h}}_{\text{k}} {\text{(x) (1-h}}_{\text{k}} \text{(x))}$

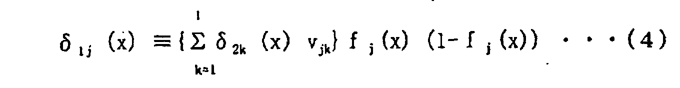

Then, the learning efficiency is calculated for the test data (S7). For the calculation of the learning efficiency, the test data was inputted in the neural network to obtain an output value, and if the output value (predicted value) of the neural network is not less than 0.5, it was classified as a linker sequence, while if it is 0.5 or less, it was considered to be classified as a non-linker sequence, and its rate of correct answers was calculated.

The calculated value of learning efficiency calculated in S7 is stored (S8).

The weight parameter updated in S6 is stored (S9).

It is judged whether the number of learning steps exceeds a default value or not (S10), and if not, the steps of S1 through S9 are carried out. If the number of learning steps exceeds the default value, the program goes on to S11.

The optimum number of steps with which the calculated value of the learning efficiency becomes the maximum is determined (S11).

The weight parameter at the optimum number of steps is determined as a parameter for prediction (S12). When the training data and the test data are used in various combinations, the optimum number of steps is determined per combination, and parameters for prediction are obtained for the number of combinations. In predicting a linker sequence of a protein, it is advantageous that a series of processing for prediction is executed for each parameter and the obtained prediction results are averaged at the end (Since the prediction results of the neural network is put out in numeral values, these values are averaged.)

It is advantageous that an output device puts out parameters for prediction.

The 2^{nd} invention of the present application provides a method of predicting a linker sequence of a protein whose structure is unknown comprising:
a window setting step for taking a window of a range of 5 to 35 residues within an amino-acid sequence of a protein whose structure is unknown;
an input / output step for obtaining an output value by inputting a value of the amino-acid sequence in the window represented in a numeral value in a hierarchical neutral network having learned in the above method;
a predicted value granting step for granting the output value to an amino-acid residue located at the center of the window as a predicted value;
a step in which the input /output step and the predicted value granting step are repeated by moving the position of the window in a desired range of the amino-acid sequence of the protein whose structure is unknown; and
a linker sequence predicting step for predicting a region made of an amino-acid residue with the predicted value larger than a preset threshold value as a linker sequence.

It is advantageous that, following the step in which the input /output step and the predicted value granting step are repeated, an average value calculating step for obtaining an average value by taking a new window of a range more than a predetermined number of residues within the amino-acid sequence of the protein whose structure is unknown and by smoothing the predicted values among the amino-acid residues within this window; and
a step for repeating the average value calculating step by moving the position of the new window within a desired range of the amino-acid sequence of the protein whose structure is unknown may be included. In this case, in the linker sequence predicting step, it is advantageous that a linker sequence is predicted by the threshold to the average value of the predicted value.

In the above predicting method, a protein whose structure is unknown may be an intact protein or a protein fragment. An amino-acid sequence of a protein is the type and arrangement order of an amino acid constituting the protein (amino-acid sequence).

As an amino-acid sequence of a protein whose structure is unknown, there can be amino-acid sequences of proteins registered in various databases (for example, GeneBank, Protein Data Bank (PDB), SWISSPROT, etc.), amino-acid sequences of newly analyzed proteins, etc.

The "protein whose structure is unknown" shall include those proteins whose structure of the entire range is unknown and those proteins whose part of the structure is known but the rest is unknown.

As a desired range of an amino-acid sequence of a protein whose structure is unknown to move the position of a window, the range excluding up to 60 residues respectively from the N terminal and the C terminal of the protein can be cited, but not limited to that range.

The window size is 5 to 35 amino-acid residues, but more preferably 10 to 35 residues and furthermore preferably 19 residues.

In the above linker sequence predicting method, before the window setting process, a value representing an amino-acid sequence of a protein whose structure is unknown in a numeral value may be inputted.

In the above method, a region made of an amino-acid residue whose average value of predicted values is larger than a threshold value set in advance may be predicted as a linker sequence, and if the largest of the predicted values of the amino-acid residue in a region made of an amino-acid residue whose average value of predicted values is larger than a preset threshold value is larger than a preset cut-off value, the region may be predicted as a linker sequence.

The threshold value is to determine how much allowance is given to the size of a region predicted as a domain linker. If the threshold value is set lower, the size of a predicted region gets larger. If the size of the predicted region gets larger, prediction becomes rough, but the correct answer rate of the prediction is improved.

The cut-off value adjusts specificity (proportion of correct answers in domain linkers predicted by the neural network) and sensitivity (proportion of those which can be predicted by the neural network among actual domain linkers). If the cut-off value is set large, the sensitivity is lowered (that is, domain linkers which can be predicted are limited), but on the contrary, the specificity gets higher (the possibility of correct answer gets high for the predicted regions).

In the predicting method of the present invention, a window is taken in an amino-acid sequence of a given protein, an output value of the neural network for the amino-acid sequence in the window is calculated and the obtained output value (real value in a range of 0.0 to 1.0) is granted as a predicted value of a domain linker trend of the residue at the center of the above window.

Here, since the above output value is relatively easily fluctuated, in order to obtain a prediction result with higher reliability, it is desirable to average the obtained output values. That is, a window for averaging (referred to as a smoothing window) is taken in an amino-acid sequence in the above protein, predicted values granted to each of the amino-acid residues are averaged among the amino-acid residues in this smoothing window, and the obtained average value is made as a predicted value of the domain linker trend of the residue at the center of the above smoothing window.

The size of this smoothing window may only be larger than a predetermined number of residues, for example, not less than 10 amino-acid residues or more preferably, 19 residues. In the range smaller than 10 residues, prediction efficiency is lowered, and linker prediction with high reliability becomes difficult.

In the present invention, based on the averaged predicted value so obtained, in identifying whether the sequence including the amino-acid residue to which this predicted value is given is a domain linker or not, a threshold value and a cut-off value for the predicted value are set and the range larger than set values of the threshold value and the cut-off value is defined as a domain linker. It is preferable that the threshold value and the cut-off value are 0.5 through 1.0. In the range lower than 0.5, the sensitivity for detecting a portion to be a linker sequence can be sufficiently secured but the accuracy (specificity) to be the linker sequence gets lower.

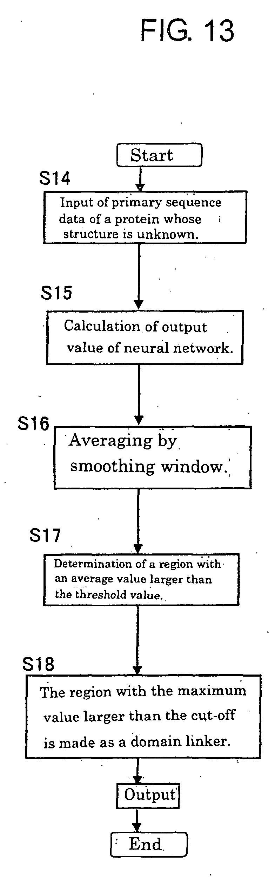

Fig. 13 is a flow chart for explaining 1 preferred embodiment of a method of predicting a linker sequence of a protein according to the present invention.

First, data of an amino-acid sequence of a protein (amino-acid sequence) whose structure is unknown is inputted (S14). The data to be inputted may be, for example, an amino-acid sequence of a protein whose structure is unknown represented in a numeral value.

An output value of a neural network is calculated (S15). When the step of S 15 is explained in more detail, a process in which a window is set in an amino-acid sequence of a protein whose structure is unknown, the amino-acid sequence data in the window is inputted in the above hierarchical neural network having learned and an output value is calculated is carried out for all the window positions. The output value of the neural network is granted to its central residue as a predicted value indicating whether the residue at the center of the amino-acid sequence in the window constitutes a part of a linker sequence or not.

Then, the predicted value is averaged among amino-acid residues in the smoothing window (averaging window) (S16). The smoothing window is a new window set in the amino-acid sequence of the protein whose structure is unknown for averaging the predicted value. The position of this smoothing window is moved within a desired range in the amino-acid sequence of the protein whose structure is unknown so as to average the predicted value.

A region made of an amino-acid residue whose average value is larger than the threshold value is determined (S17).

A region where the largest average value of the predicted values of the amino-acid residues in the region determined in S17 is larger than a cut-off value is made as a linker sequence (S18). Or the region determined in S17 may be the linker sequence.

It is advantageous that the linker sequence is outputted to an output device.

The 3^{rd} invention of the present application is a system for predicting a linker sequence of a protein whose structure is unknown (hereinafter referred to as "linker sequence predicting system") comprising an amino-acid sequence input means for inputting a value of the amino-acid sequence of the protein whose structure is unknown represented in a numeral value, a window setting means for taking a window in the amino-acid sequence of the protein whose structure is unknown, an in-window amino-acid sequence input means for inputting the value of the amino-acid sequence in the window represented in a numeral value into a hierarchical neural network having identified and learned the linker sequence of the protein consisting of 2 or more structural domains, an output value calculating means for having the hierarchical neural network calculate an output value, a predicted value granting means for granting the output value to the amino-acid residue located at the center of the window as a predicted value, a window-position moving means for moving the position of the window in a desired range of the amino-acid sequence of the protein whose structure is unknown, a smoothing window setting means for taking a new window of a range more than the predetermined number of residues in the amino-acid sequence of the protein whose structure is unknown, an average value calculating means for obtaining an average value by smoothing predicted values among the amino-acid residues in the new window, a smoothing window moving means for moving the position of the new window within a desired range of the amino-acid sequence of the protein whose structure is unknown, and a linker sequence predicting means for predicting a region consisting of the amino-acid residues with the average value of the predicted value larger than a preset threshold value as a linker sequence.

The window size is 5 to 35 amino-acid residues, but more preferably 10 to 35 residues, and furthermore preferably 19 residues.

The size of the new window may be not less than the predetermined number of residues, for example, not less than 10 amino-acid residues and more preferably 19 residues.

As a hierarchical neural network having identified and learned a linker sequence of a protein consisting of 2 or more structural domains, a neural network having learned by the method of the first invention of the present application is preferable.

As a desired range of an amino-acid sequence of a protein whose structure is unknown in which the position of the window and the smoothing window are to be moved, the range excluding up to 60 residues from the N terminal and the C terminal respectively of the protein can be cited, but not limited to that range.

The 4^{th} invention of the present application provides a program for having a computer function as a system for predicting a linker sequence of a protein whose structure is unknown characterized in that the system comprises an amino-acid sequence input means for inputting a value of the amino-acid sequence of the protein whose structure is unknown represented in a numeral value, a window setting means for taking a window in the amino-acid sequence of the protein whose structure is unknown, an in-window amino-acid sequence input means for inputting the value of the amino-acid sequence in the window represented in a numeral value into a hierarchical neural network having identified learned the linker sequence of the protein consisting of 2 or more structural domains, an output value calculating means for having the hierarchical neural network calculate an output value, a predicted value granting means for granting the output value to the amino-acid residue located at the center of the window as a predicted value, a window-position moving means for moving the position of the window in a desired range of the amino-acid sequence of the protein whose structure is unknown, a smoothing window setting means for taking a new window of a range more than the predetermined number of residues in the amino-acid sequence of the protein whose structure is unknown, an average value calculating means for obtaining an average value by smoothing predicted values among the amino-acid residues in the new window, a smoothing window moving means for moving the position of the new window within a desired range of the amino-acid sequence of the protein whose structure is unknown, and a linker sequence predicting means for predicting a region consisting of the amino-acid residues with the average value of the predicted value larger than a preset threshold value as a linker sequence.

The 5^{th} invention of the present application provides a computer readable recording medium which recorded a program for having a computer function as a system for predicting a linker sequence of a protein whose structure is unknown characterized in that the system comprises an amino-acid sequence input means for inputting a value of the amino-acid sequence of the protein whose structure is unknown represented in a numeral value, a window setting means for taking a window in the amino-acid sequence of the protein whose structure is unknown, an in-window amino-acid sequence input means for inputting the value of the amino-acid sequence in the window represented in a numeral value into a hierarchical neural network having identified and learned the linker sequence of the protein consisting of 2 or more structural domains, an output value calculating means for having the hierarchical neural network calculate an output value, a predicted value granting means for granting the output value to the amino-acid residue located at the center of the window as a predicted value, a window-position moving means for moving the position of the window in a desired range of the amino-acid sequence of the protein whose structure is unknown, a smoothing window setting means for taking a new window of a range more than the predetermined number of residues in the amino-acid sequence of the protein whose structure is unknown, an average value calculating means for obtaining an average value by smoothing predicted values among the amino-acid residues in the new window, a smoothing window moving means for moving the position of the new window within a desired range of the amino-acid sequence of the protein whose structure is unknown, and a linker sequence predicting means for predicting a region consisting of the amino-acid residues with the average value of the predicted value larger than a preset threshold value as a linker sequence.

This recording medium which recorded the program may be ROM itself of the linker sequence predicting system or CD-ROM or the like which can be read when the recording medium is inserted into a program reading device such as a CD-ROM drive provided as an external memory unit. Or the above recording medium may be a magnetic tape, cassette tape, flexible disk, hard disk, MO / MD / DVD, etc. or semiconductor memory.

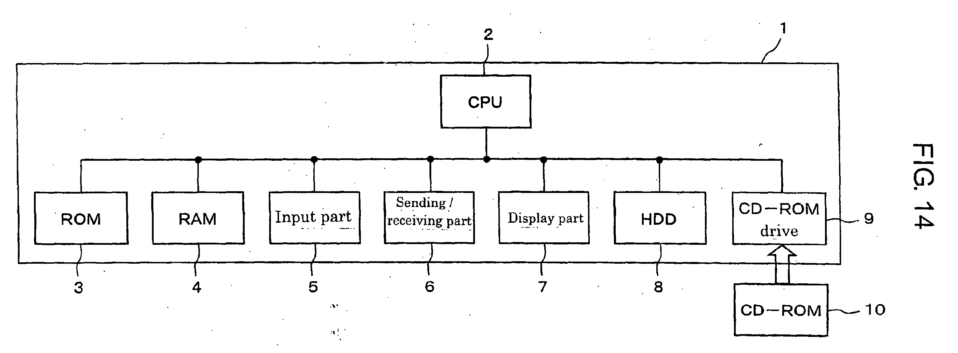

Fig. 14 is a block diagram showing constitution of a linker sequence predicting system according to the present invention. This system comprises a computer 1 provided with a CPU 2, a ROM 3, a RAM 4, an input part 5, a sending / receiving part 6, a display part 7, a hard disk drive 8 and a CD-ROM drive 9. Instead of a CD-ROM 10, a rewritable CD-R or CD-RW can be used as a recording medium. In that case, instead of the CD-ROM drive 9, a drive for CD-R or for CD-RW is provided. Instead of the CD-ROM 10, DVD, ZiP, MO, PD and their media can be used as a medium for maintaining information and a drive corresponding to it can be provided.

The CPU 2 controls the entire linker sequence predicting system according to the program stored in the ROM 3, the RAM 4 or the hard disk drive (HDD) 8 and executes the linker sequence predicting processing which will be described later. The ROM 3 stores programs and so on for commanding processing required for operation of the linker sequence predicting system. The RAM 4 temporarily stores data required for execution of the linker sequence predicting processing. The input part 5 includes a keyboard, mouse, etc. manipulated when inputting conditions necessary for execution of the linker sequence predicting system. The sending / receiving part 6 executes sending / receiving processing of data through a communication line based on the command of the CPU 2. The display part 7 executes processing for displaying input information, output information, etc. based on the command from the CPU 2. The hard disk drive (HDD) 8 stores the linker sequence predicting program, data sets, etc., reads out the stored program, data sets, etc. based on the command of the CPU 2 and stores them in the RAM 43, for example, The CD-ROM drive 9 reads out a program, data or the like from the stored program, data sets, etc. stored in the CD-ROM 10 based on the command of the CPU 2 and stores them in the hard disk drive (HDD) 8, for example,

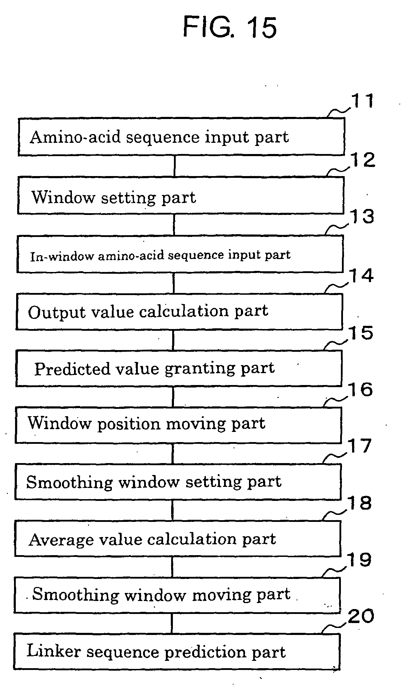

Fig. 15 is a block diagram explaining functions of the linker sequence predicting system according to the present invention. To an amino-acid sequence input part 11, a value representing an amino-acid sequence of a protein whose structure is unknown in a numeral value is inputted. In a window setting part 12, a window is set in an amino-acid sequence of a protein whose structure is unknown. In an in-window amino-acid sequence input part 13, a value representing an amino-acid sequence in the window in a numeral value is inputted into a hierarchical neural network having identified and learned a linker sequence of a protein consisting of 2 or more structural domains. In an output value calculation part 14, an output value is calculated by the hierarchical neural network. At a predicted value granting part 15, the output value is granted as a predicted value to an amino-acid residue located at the center of the window. In a window position moving part 16, the position of a window is moved in a desired range of the amino-acid sequence of the protein whose structure is unknown. In a smoothing window setting part 17, a new window in a range larger than the predetermined number of residues is set in the amino-acid sequence of the protein whose structure is unknown. In-an average value calculation part 18, a predicted value is averaged among the amino-acid residues in the new window so as to obtain an average value. In a smoothing window moving part 19, the position of the new window is moved in a desired range of the amino-acid sequence of the protein whose structure is unknown. In a linker sequence prediction part 20, a region consisting of an amino-acid residue whose average value of the predicted value is larger than a preset threshold value is predicted as a linker sequence.

The 6^{th} invention of the present application provides a method of producing a protein fragment corresponding to one or more structural domains located on the side of an N-terminal from a predicted linker sequence comprising a step for producing at least one of the protein fragments obtained by cutting off a protein at any of the following portions (i), (ii) or (iii):
(i) an arbitrary portion of at least one linker sequence predicted by the above method;
(ii) any of portions located between a C-terminal of at least one linker sequence predicted by the above method and the 50^{th} amino-acid residue counted therefrom to the C-terminal side of the protein; or
(iii) any of portions located between the N-terminal of at least one linker sequence predicted by the above method and the 15^{th} amino-acid residue counted therefrom to the N-terminal side of the protein.

By this method, a protein can be cut off without breaking the structure of a structural domain existing on the side of the N terminal of the predicted linker sequence so as to obtain a protein fragment.

The above (ii) portion exists between the C terminal of at least one linker sequence predicted by the above method and the 50^{th} amino-acid residue counted therefrom to the C-terminal side of the protein, but preferably existing between the C terminal of the linker sequence and the 30^{th} amino-acid residue counted therefrom to the C-terminal side of the protein.

Also, the above (iii) portion exists between the N terminal of at least one linker sequence predicted by the above method and the 15^{th} amino-acid residue counted therefrom to the N-terminal side of the protein, but preferably existing between the N terminal of the linker sequence and the 10^{th} amino-acid residue counted therefrom to the N-terminal side of the protein.

The 7^{th} invention of the present application provides a method of producing a protein fragment corresponding to one or more structural domains located on the side of a C-terminal from a predicted linker sequence comprising a step for producing at least one of the protein fragments obtained by cutting off a protein at any of the following portions (i), (iv) or (v):
(i) an arbitrary portion of at least one linker sequence predicted by the above method;
(iv) any of portions located between an N-terminal of at least one linker sequence predicted by the above method and the 50^{th} amino-acid residue counted therefrom to the N-terminal side of the protein; or
(v) any of portions located between the C-terminal of at least one linker sequence predicted by the above method and the 15^{th} amino-acid residue counted therefrom to the C-terminal side of the protein.

By this method, a protein can be cut off without breaking the structure of a structural domain existing on the side of the C terminal of the predicted linker sequence so as to obtain a protein fragment.

The above (iv) portion exists between the N terminal of at least one linker sequence predicted by the above method and the 50^{th} amino-acid residue counted therefrom to the N-terminal side of the protein, but preferably existing between the N terminal of the linker sequence and the 30^{th} amino-acid residue counted therefrom to the N-terminal side of the protein.

Also, the above (v) portion exists between the C terminal of at least one linker sequence predicted by the above method and the 15^{th} amino-acid residue counted therefrom to the C-terminal side of the protein, but preferably existing between the C terminal of the linker sequence and the 10^{th} amino-acid residue counted therefrom to the C-terminal side of the protein.

For manufacture of a protein fragment, any publicly known method, that is, a chemical synthesizing method, genetic engineering method, etc. may be used.

The 8^{th} invention of the present application provides a method of analyzing a protein fragment corresponding to one or more structural domains located on the side of an N-terminal from a predicted linker sequence comprising a step for analyzing at least one of the protein fragments obtained by cutting off a protein at any of the following portions (i), (ii) or (iii):
(i) an arbitrary portion of at least one linker sequence predicted by the above method;
(ii) any of portions located between a C-terminal of at least one linker sequence predicted by the above method and the 50^{th} amino-acid residue counted therefrom to the C-terminal side of the protein; or
(iii) any of portions located between the N-terminal of at least one linker sequence predicted by the above method and the 15^{th} amino-acid residue counted therefrom to the N-terminal side of protein.

By this method, a protein can be cut off without breaking the structure of a structural domain existing on the side of the N terminal of the predicted linker sequence so as to analyze the structure of a protein fragment.

The above (ii) portion exists between the C terminal of at least one linker sequence predicted by the above method and the 50^{th} amino-acid residue counted therefrom to the C-terminal side of the protein, but preferably existing between the C terminal of the linker sequence and the 30^{th} amino-acid residue counted therefrom to the C-terminal side of the protein.

Also, the above (iii) portion exists between the N terminal of at least one linker sequence predicted by the above method and the 15^{th} amino-acid residue counted therefrom to the N-terminal side of the protein, but preferably existing between the N terminal of the linker sequence and the 10^{th} amino-acid residue counted therefrom to the N-terminal side of the protein.

The 9^{th} invention of the present application provides a method of analyzing a protein fragment corresponding to one or more structural domains located on the side of a C-terminal from a predicted linker sequence comprising a step for analyzing at least one of the protein fragments obtained by cutting off a protein at any of the following portions (i), (iv) or (v):
(i) an arbitrary portion of at least one linker sequence predicted by the above method;
(iv) any of portions located between an N-terminal of at least one linker sequence predicted by the above method and the 50^{th} amino-acid residue counted therefrom to the N-terminal side of the protein; or
(v) any of portions located between the C-terminal of at least one linker sequence predicted by the above method and the 15^{th} amino-acid residue counted therefrom to the C-terminal side of the protein.

By this method, a protein can be cut off without breaking the structure of a structural domain existing on the side of the C terminal of the predicted linker sequence so as to analyze the structure of a protein fragment.

The above (iv) portion exists between the N terminal of at least one linker sequence predicted by the above method and the 50^{th} amino-acid residue counted therefrom to the N-terminal side of the protein, but preferably existing between the N terminal of the linker sequence and the 30^{th} amino-acid residue counted therefrom to the N-terminal side of the protein.

Also, the above (v) portion exists between the C terminal of at least one linker sequence predicted by the above method and the 15^{th} amino-acid residue counted therefrom to the N-terminal side of the protein, but preferably existing between the C terminal of the linker sequence and the 10^{th} amino-acid residue counted therefrom to the C-terminal side of the protein.

As analysis of a protein fragment, in addition to the X-ray crystal structure analysis, protein structure analysis by NMR, etc., measurement of various bioactivities can be cited.

In the above manufacture / analyzing methods of a protein fragment, the protein fragment is a concept including a structural domain.

In order to cut off a protein, any publicly known method, that is, an enzymic method using protease, chemical decomposition method to cut off a peptide chain using chemicals, etc. may be used.

The 10^{th} invention of the present application provides a method of constructing a linker sequence database comprising a step for recording amino-acid sequence data of the linker sequence predicted by the above method in a recording medium.

The 11^{th} invention of the present application provides a method of constructing a structural domain database comprising a step for recording amino-acid sequence data of the structural domain obtained by cutting off a protein at an arbitrary portion of at least one linker sequence predicted by the above method in a recording medium.

As a recording medium, a magnetic tape, cassette-tape, flexible disk, hard disk, MO / MD / DVD, etc. or semiconductor memory can be cited.

The 12^{th} invention of the present application provides a peptide which has a sequence pattern satisfying the conditions of (i) and (ii) below and can function as a domain linker of a multi-domain protein:
(i) when a sequence fragment consisting of continuous 19 residues is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈ {0,1} (} \text{i} \text{=1,···,399))}$ (where, x = (x₁, x₂,·····, x₃₉₉) is a 399-bit (= 19 x 21) binary sequence obtained as a result of arrangement in a series of 21-bit binary sequences corresponding to the type of an amino acid according to the sequence of the 19 residues of the sequence fragment, and the bit sequence corresponds to, in order, "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" and for the 21-bit binary sequence, only those matching the type of the amino acid of the represented residues are 1, while the others are 0.)
   the value of the following g(x) is in a range of 0.5 to 1.0.$\text{g} {\text{(x)=τ(ν}}_{\text{0}} \text{+} {\text{ν}}_{\text{1}} {\text{f}}_{\text{1}} \text{(x)+} {\text{ν}}_{\text{2}} {\text{f}}_{\text{2}} \text{(x))}$ 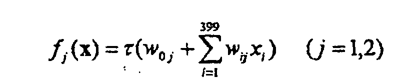 $\text{r} \text{(} \text{u} \text{) = 1/(1 +} {\text{e}}^{\text{-}} {\text{}}^{\text{u}} \text{)}$ (where a combination of wᵢⱼ(i=0,·····, 399; j=1,2) and vⱼ(j=0, 1, 2) is selected from a group consisting of a combination of Group 1 in Table A, a combination of Group 2 in Table B, a combination of Group 3 in Table C, a combination of Group 4 in Table D, a combination of Group 5 in Table E, a combination of Group 6 in Table F, a combination of Group 7 in Table G, a combination of Group 8 in Table H, a combination of group 9 in Table I, and a combination of Group 10 in Table J.)
(ii) a central residue of the sequence fragment x=(x₁, x₂,·····, x₃₉₉) with the value of g(x) in the range of 0.5 to 1.0 may be included, and an amino acid within 9 residues before and after the central residue may further be included.

The above peptide may consist only of the sequence pattern satisfying the conditions in the above (i) and (ii) or may include other amino-acid sequences as long as it can function as a domain linker of a multi-domain protein.

The range of the numeral values of g(x) is preferably 0.5 - 1.0. If the value is lower than 0.5, prediction accuracy is lowered and it causes a problem in reliability.

The 13^{th} invention of the present application provides a method of predicting a region having a sequence pattern satisfying the conditions of the above (i) and (ii) as a linker sequence of protein. For example, by detecting a sequence pattern satisfying the conditions of the above (i) and (ii) from amino-acid sequences of proteins registered in various databases (for example, GeneBank, PDB, SWISSPROT, etc.), amino-acid sequences of newly analyzed proteins, etc., a region having the sequence pattern can be predicted as a linker sequence.

The 14^{th} invention of the present application provides a method of dividing a protein into structural domains characterized in that the protein is cut off at an arbitrary portion of a region having a sequence pattern satisfying the conditions of the above (i) and (ii).

In order to cut off a protein, any publicly known method, that is, an enzymic method using protease, chemical decomposition method to cut off a peptide chain using chemicals, etc. may be used.

The 15^{th} invention of the present application provides a method of producing a protein fragment comprising a step for producing at least one of the protein fragments obtained by cutting off a protein at an arbitrary portion of a region having a sequence pattern satisfying the conditions of the above (i) and (ii).

For manufacture of a protein fragment, any publicly known method, that is, a chemical synthesizing method, genetic engineering method, etc. may be used.

The 16^{th} invention of the present application provides a method of analyzing a protein fragment comprising a step for analyzing at least one of the protein fragments obtained by cutting off protein at an arbitrary portion of a region having a sequence pattern satisfying the conditions of the above (i) and (ii)

As analysis of a protein fragment, in addition to the X-ray crystal structure analysis, protein structure analysis by NMR, etc., measurement of various bioactivities can be cited.

In the above manufacture / analyzing methods of a protein fragment, the protein fragment is a concept including a structural domain.

In order to cut off a protein, any publicly known method, that is, an enzymic method using protease, chemical decomposition method to cut off a peptide chain using chemicals, etc. may be used.

The 17^{th} invention of the present application provides a method of producing a new multi-domain protein by designing a new domain linker using a peptide having a sequence pattern satisfying the conditions of the above (i) and (ii) and by connecting at least two protein fragments.

For manufacture of a protein fragment, any publicly known method, that is, a chemical synthesizing method, genetic engineering method, etc. may be used.

The 18^{th} invention of the present application provides a method of predicting and / or detecting a linker sequence in a multi-domain protein sequence whose structure is unknown from characteristics of the above linker sequence on an amino-acid sequence comprising:
i) a step for extracting a linker sequence and a non-linker loop sequence from a database of multi-domain protein whose structure is known; and
ii) a step for obtaining, based on statistical processing of amino-acid sequence of each domain, probabilities P_{Xaa}^{L} and P_{Xaa}^{N} of occurrence of an amino-acid residue Xₐₐ (where P_{Xaa}^{L} and P_{Xaa}^{N} are probabilities of occurrence of the amino-acid residue Xₐₐ in a linker sequence and a non-linker loop sequence, respectively) and probabilities P_{XaaYaa(m)}^{L} and P_{XaaYaa(m)}^{N} of occurrence of the amino-acid residues Xₐₐ and Yₐₐ with m pieces (m is an integer, m = 0, 1, 2) of arbitrary amino-acid residues between them (where P_{XaaYaa(m)}^{L} and P_{XaaYaa(m)}^{N} are probabilities of occurrence of the amino-acid residues Xₐₐ and Yₐₐ in the linker sequence and the non-linker loop sequence, respectively, with m pieces of amino acid residues between them (the order of Xₐₐ and Yₐₐ does not matter)).

In the 18^{th} invention of the present application, the above multi-domain protein database whose structure is known provides both amino-acid sequences and structural coordinates of a protein. They are created by, for example, open databases such as SCOP, nr-PDB, etc. Also, as an example of a selecting method, DSSP, Visual inspection can be cited, but not limited to them.

In the 18^{th} invention of the present application, a linker sequence and a non-linker loop sequence are extracted from the above multi-domain protein database whose structure is known, and an amino-acid sequence corresponding to each region is used as a data set.

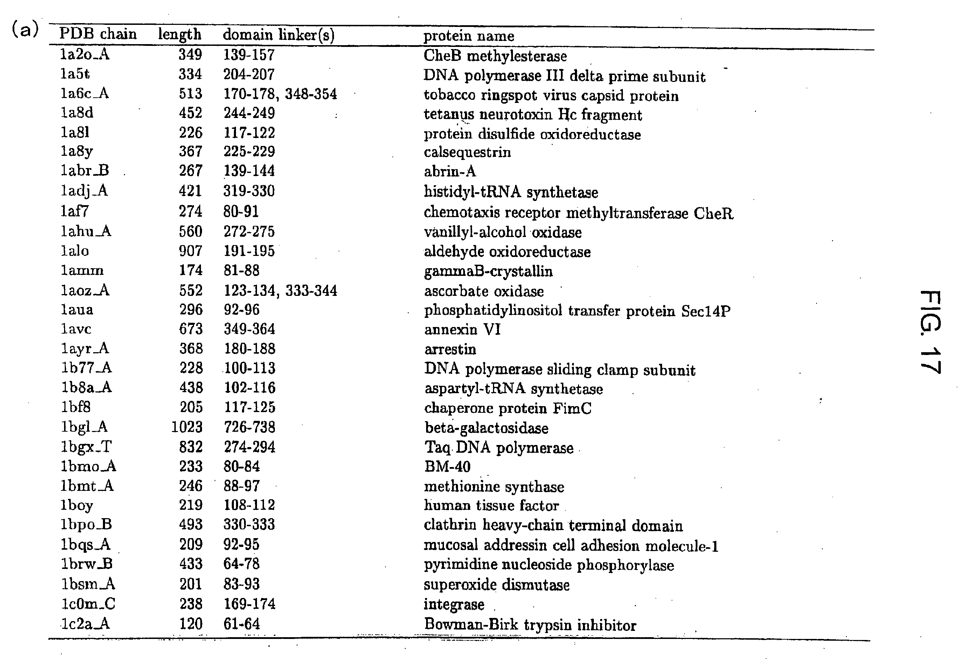

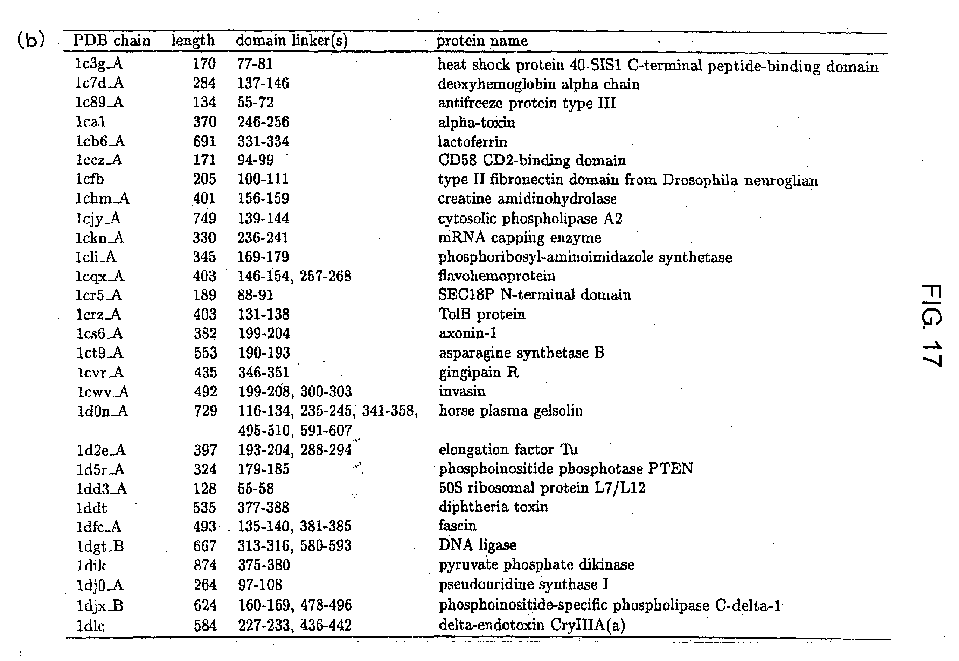

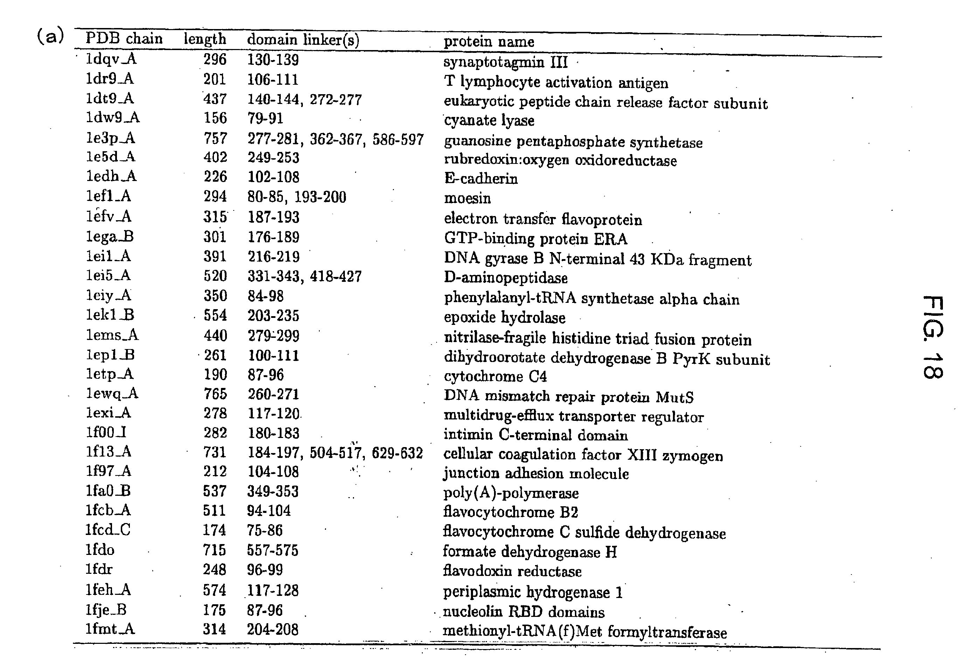

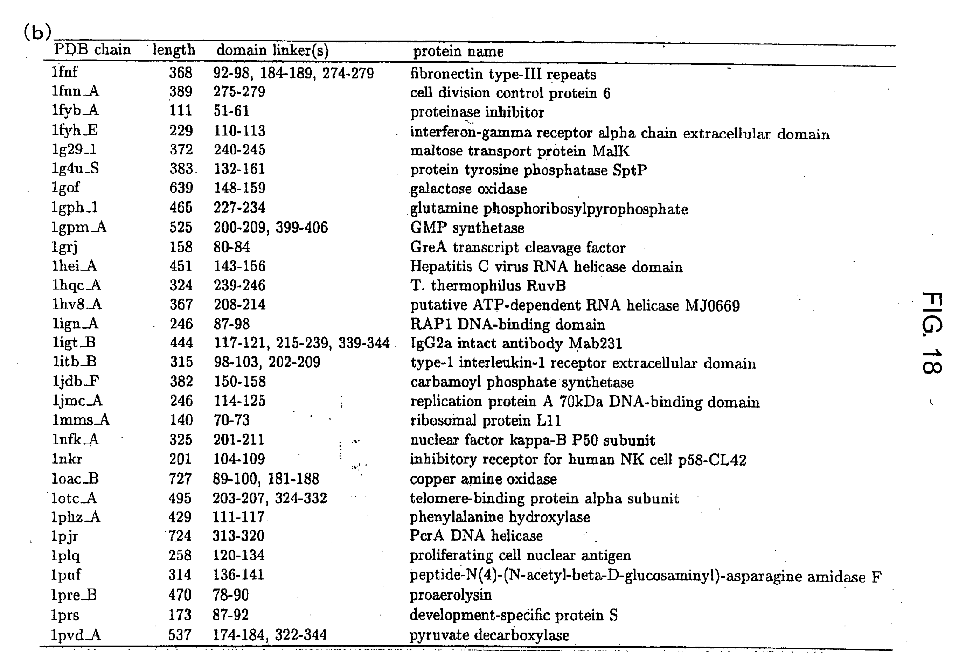

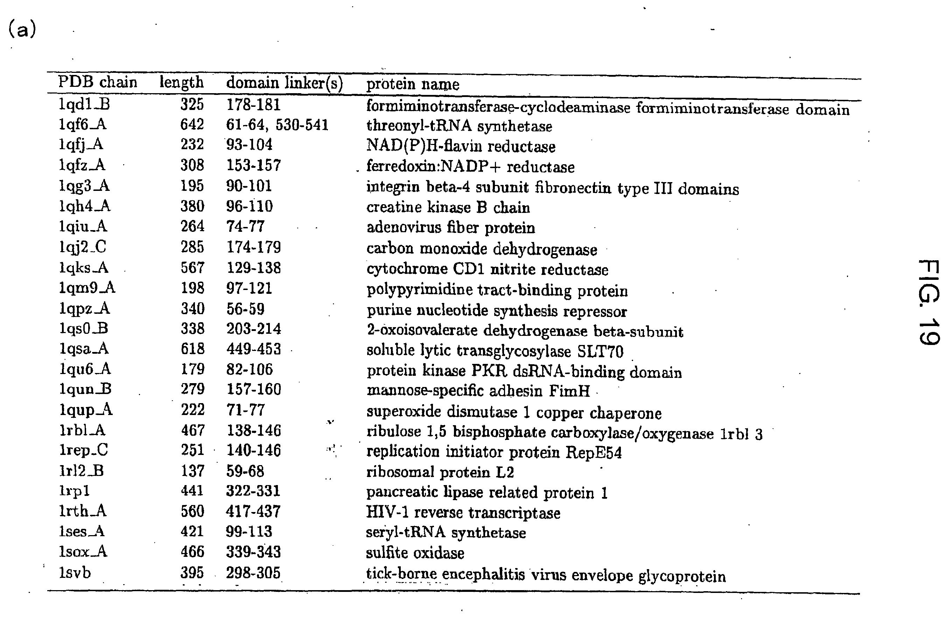

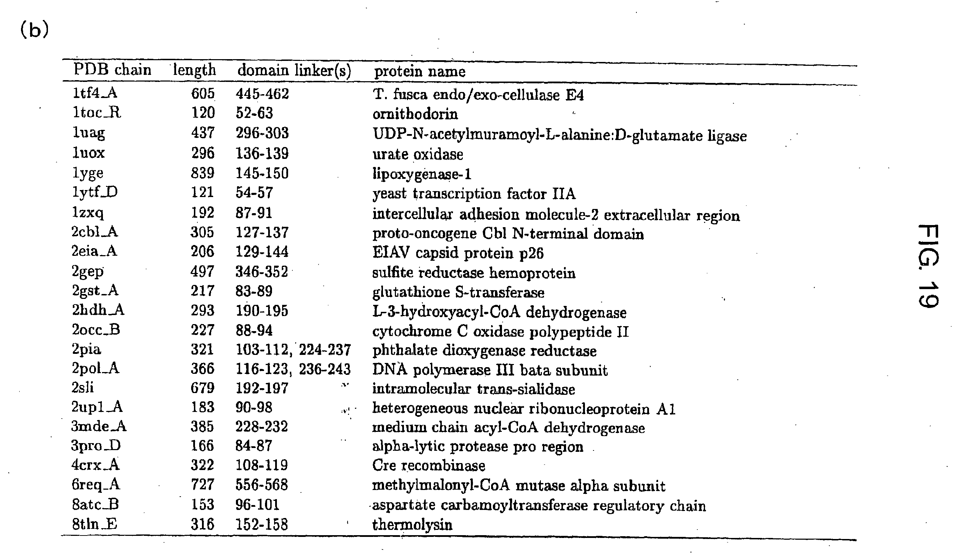

Figs. 17 through 19 show an example of so extracted linker sequences. As shown in Table of Fig. 17, it is advantageous to prepare PDB chain, length, position of the linker sequence, name of the protein, etc. as a data set.

On the other hand, the above non-linker loop sequence is a loop sequence in the above multi-domain protein database whose structure is known from which the above linker sequence and regions located at both N/C terminals are removed.

When extracting these linker sequences and non-linker loop sequences, the following standard can be used.

First, a loop sequence with the length indicated by DSSP or the like of 4 residues or more is extracted. Those including a domain boundary defined by the open database such as SCOP in this loop region or at the terminal of the loop sequence are classified as a linker sequence, while those other than the linker sequence and not located at either of the N/C terminals are classified as a non-linker loop sequence.

Also, based on statistical processing of amino-acid sequence of the above linker sequence and the above non-linker loop sequence, probabilities P_{Xaa}^{L} and P_{Xaa}^{N} of occurrence of an amino-acid residue Xₐₐ and probabilities P_{XaaYaa(m)}^{L} and P_{XaaYaa(m)}^{N} of occurrence of the amino-acid residues Xₐₐ and Yₐₐ with m pieces (m is an integer, m = 0, 1, 2) of arbitrary amino-acid residues between them can be obtained as follows.

First, when the total number of amino-acid residues included in an amino-acid sequence of a target linker sequence (or a non-linker loop sequence) is Nₜₒₜₐₗ and an occurrence-frequency of an amino-acid residue Xₐₐ in the amino-acid sequence is N_{Xaa},${\text{P}}_{\text{Xaa}} {\text{}}^{\text{L}} {\text{=N}}_{\text{Xaa}} {\text{/N}}_{\text{total}} {\text{(P}}_{\text{Xaa}} {\text{}}^{\text{N}} {\text{=N}}_{\text{Xaa}} {\text{/N}}_{\text{total}} \text{)}$

Also, when all the partial sequence patterns of the length m + 2 (m is an integer, m = 0, 1, 2) included in the amino-acid sequence of the target linker sequence (or the non-linker loop sequence) is Nₜₒₜₐₗ₍ₘ₎ and the occurrence frequency of the amino-acid residues Xₐₐ and Yₐₐ in the amino-acid sequence with m pieces of arbitrary amino-acid residues between them (the order of Xₐₐ and Yₐₐ does not matter) is N_{XaaYaa(m)},${\text{P}}_{\text{XaaYaa(m)}} {\text{}}^{\text{L}} {\text{=N}}_{\text{XaaYaa(m)}} {\text{/N}}_{\text{total(m)}}$${\text{(P}}_{\text{XaaYaa(m)}} {\text{}}^{\text{N}} {\text{=N}}_{\text{XaaYaa(m)}} {\text{/N}}_{\text{total(m)}} \text{)}$

These P_{Xaa}^{L} and P_{XaaYaa(m)}^{L} (or P_{Xaa}^{N} and P_{XaaYaa(m)}^{N}) can be used for predicting / detecting a linker sequence in the multi-domain protein whose structure is unknown.

Also, in the 18^{th} invention of the present application, it is preferable that, when extracting a linker sequence and a non-linker loop sequence, they are divided into longer ones and shorter ones according to the length of the amino-acid sequence in each extracted region, occurrence probabilities of amino acids are obtained separately for the longer case and the shorter case, and characteristics of the sequence in each case is formulated so that the linker sequence is predicted applying a discrimination function in each case. In this way, by reflecting the trend of "how much it is like linker" in the domain linker prediction, prediction accuracy can be improved. In this case, it is preferable that the number L_{L} of amino-acid residues of longer amino-acid sequences is in a range of 8 to 50 residues both inclusive, or more preferably in a range of 10 to 50 residues both inclusive. It is preferable that the number Ls of amino-acid residues of longer amino-acid sequences is in a range of 4 to 12 residues both inclusive, or more preferably in a range of 4 to 9 residues both inclusive. By dividing the length of the amino-acid sequence in the loop region according to the above range and by extracting characteristics from each of them, more accurate discrimination functions can be obtained, and prediction with high accuracy is enabled.

When domain linker prediction was actually carried out with 10 ≦ L_{L} ≦ 50, 4 ≦ L_{S} ≦ 9, 52% of the predicted domain matched an actual linker sequence (specificity), and 45% of the domain linker derived from SCOP was predicted (sensitivity).

The 19^{th} invention of the present application provides a system of predicting and / or detecting a linker sequence in a multi-domain protein whose structure is unknown from characteristics of the above linker sequence on an amino-acid sequence (hereinafter referred to as "linker sequence predicting / detecting system") comprising:
i) a means for extracting a linker sequence and a non-linker loop sequence from a database of multi-domain protein whose structure is known; and
ii) a step for obtaining, based on statistical processing of amino-acid sequence of each domain, probabilities P_{Xaa}^{L} and P_{Xaa}^{N} of occurrence of an amino-acid residue Xₐₐ (where P_{Xaa}^{L} and P_{Xaa}^{N} are probabilities of occurrence of the amino-acid residue Xₐₐ in a linker sequence and a non-linker loop sequence, respectively) and probabilities P_{XaaYaa(m)}^{L} and P_{XaaYaa(m)}^{N} of occurrence of the amino-acid residues Xₐₐ and Yₐₐ with m pieces (m is an integer, m = 0, 1, 2) of arbitrary amino-acid residues between them (where P_{XaaYaa(m)}^{L} and P_{XaaYaa(m)}^{N} are probabilities of occurrence of the amino-acid residues Xₐₐ and Yₐₐ in the linker sequence and the non-linker loop sequence, respectively, with m pieces of amino acid residues between them (the order of Xₐₐ and Yₐₐ does not matter)).

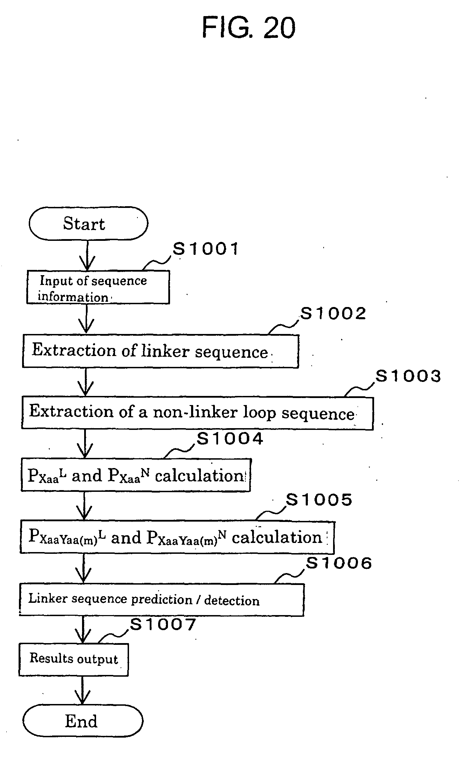

Fig. 20 is a flowchart explaining an operation of the linker sequence predicting / detecting system according to a preferred embodiment of the 18^{th} invention of the present application or a preferred embodiment of the 19^{th} invention of the present application.

At Step S1001, sequence information is inputted from the multi-domain protein database whose structure is known. At Step S1002, a linker sequence is extracted. At Step S1003, a non-linker loop sequence is also extracted. And at Step S1004, based on statistical processing of the amino-acid sequence of each sequence, probabilities P_{Xaa}^{L} and P_{Xaa}^{N} of occurrence of an amino-acid residue Xₐₐ is obtained. Then, at Step S1005, based on statistical processing of the amino-acid sequence of each sequence, probabilities P_{XaaYaa(m)}^{L} and P_{XaaYaa(m)}^{N} of occurrence of the amino-acid residues Xₐₐ and Yₐₐ with m pieces (m is an integer, m = 0, 1, 2) of arbitrary amino-acid residues between them (the order of Xₐₐ and Yₐₐ does not matter) is obtained. At Step S1006, using P_{Xaa}^{L} and P_{XaaYaa(m)}^{L} (P_{Xaa}^{N} and P_{XaaYaa(m)}^{N}), a linker sequence in the multi-domain protein whose structure is unknown is predicted and / or detected. At Step S1007, the result is outputted. The result output indicates, for example, predicted amino-acid sequences, position, length, priority, etc.. of the predicted linker sequence.

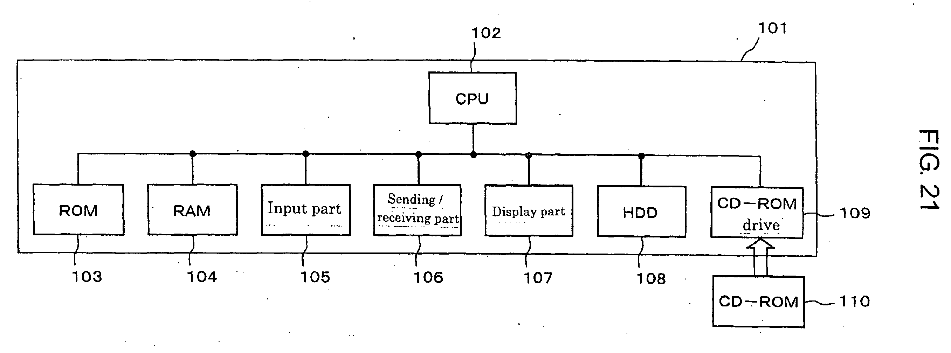

Fig. 21 is a block diagram showing constitution of a linker sequence predicting / detecting system according to a preferred embodiment of the present invention. This system comprises a computer 101 provided with a CPU 102, a ROM 103, a RAM 104, an input part 105, a sending / receiving part 106, a display part 107, a hard disk drive 108 and a CD-ROM drive 109. Instead of a CD-ROM 110, a rewritable CD-R or CD-RW can be used as a recording medium. In that case, instead of the CD-ROM drive 109, a drive for CD-R or for CD-RW is provided. Instead of the CD-ROM 110, DVD, ZiP, MO, PD and their media can be used as a medium for holding information and a drive corresponding to it can be provided.

The CPU 102 controls the entire linker sequence predicting system according to the program stored in the ROM 103, the RAM 104 or the hard disk drive (HDD) 108 and executes the linker sequence predicting processing which will be described later. The ROM 103 stores programs and so on for commanding processing required for operation of the linker sequence predicting system. The RAM 104 temporarily stores data required for execution of the linker sequence predicting processing. The input part 105 includes a keyboard, mouse, etc. manipulated when inputting conditions necessary for execution of the linker sequence predicting system. The sending / receiving part 106 executes sending / receiving processing of data through a communication line based on the command of the CPU 102. The display part 107 executes processing for displaying input information, output information, etc. based on the command from the CPU 102. The hard disk drive (HDD) 108 stores the linker sequence predicting program, data sets, etc. (See Figs. 17 through 19), reads out the stored program, data sets, etc. based on the command of the CPU 102 and stores them in the RAM 104, for example, The CD-ROM drive 109 reads out a program, data or the like from the stored program, data sets, etc. stored in the CD-ROM 110 based on the command of the CPU 102 and stores them in the hard disk drive (HDD) 108, for example,

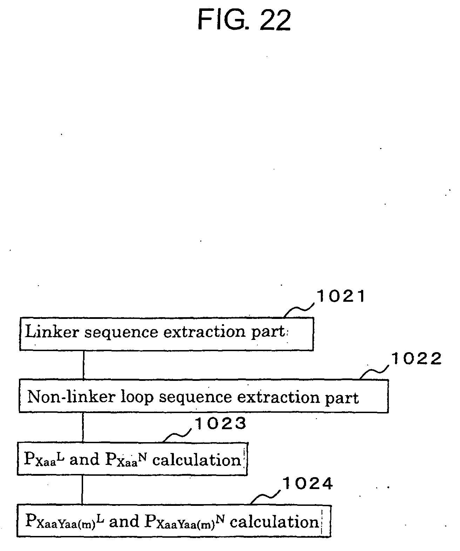

Fig. 22 is a block diagram showing functions of a linker sequence predicting / detecting system according to a preferred embodiment of the 19^{th} invention of the present application. In a linker sequence extraction part 1021, a linker sequence portion is extracted from a multi-domain protein database whose structure is known. In a non-linker loop sequence extraction part 1022, a non-linker sequence portion is extracted from the multi-domain protein database whose structure is known. In a P_{Xaa}^{L} (as well as P_{Xaa}^{N}) calculation part 1023, based on statistical processing of the amino-acid sequences of the linker sequence portion and the non-linker loop sequence portion, probabilities P_{Xaa}^{L} (P_{Xaa}^{N}) of occurrence of an amino-acid residue Xₐₐ is obtained. In a P_{XaaYaa(m)}^{L} (as well as P_{XaaYaa(m)}^{N}) calculation part 1024, based on statistical processing of the amino-acid sequences of the linker sequence portion and the non-linker loop sequence portion, probabilities P_{XaaYaa(m)}^{L} (as well as P_{XaaYaa(m)}^{N}) of occurrence of the amino-acid residues Xₐₐ and Yₐₐ with m pieces (m is an integer, m = 0, 1, 2) of arbitrary amino-acid residues between them (the order of Xₐₐ and Yₐₐ does not matter) is obtained.

The 20^{th} invention of the present application provides a program for having a computer function as the system of the 19^{th} invention of the present application.

The 21^{st} invention of the present application provides a structural domain predicting method comprising a step for predicting as a structural domain a protein fragment generated by cutting off, at any of portions of a linker sequence in a multi-domain protein whose structure is unknown predicted by the method of the 18^{th} invention of the present application, the multi-domain protein.

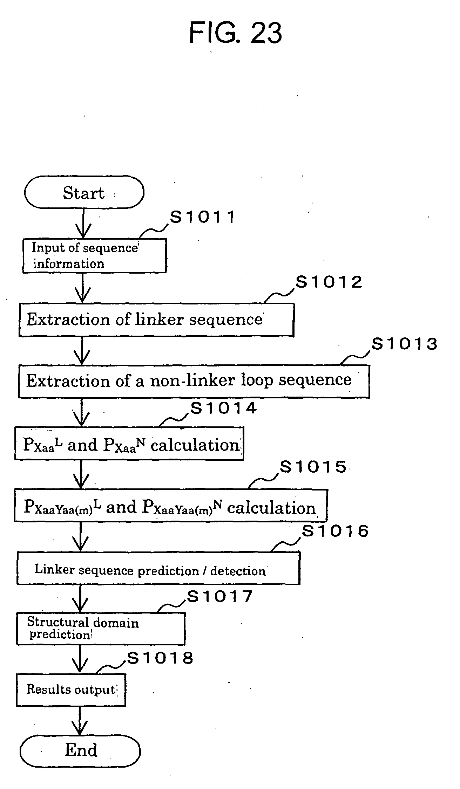

Fig. 23 is a flowchart of a method of predicting a structural domain according to a preferred embodiment of the 21^{st} invention of the present application. Steps S1011 through S1016 are the same as Steps S1001 through 1006 in Fig. 2. At step S1017, a protein fragment generated by cutting off the multi-domain protein at any of portions of a linker sequence predicted at S1016 is predicted as a structural domain. At Step S1018, the result is outputted. The result output indicates, for example, predicted amino-acid sequences, position, size, etc. of the predicted structural domain.

The 22^{nd} invention of the present application is a protein producing method comprising a step for producing a protein having the same amino-acid sequence as the structural domain predicted by the method of the 21^{st} invention of the present application. For manufacture of a protein fragment, any publicly known method, that is, a chemical synthesizing method, genetic engineering method, etc. may be used.

The 23^{rd} invention of the present application is a protein analyzing method comprising a step for analyzing a protein having the same amino-acid sequence as the structural domain predicted by the method of the 21^{st} invention of the present application. As analysis of a protein fragment, in addition to the X-ray crystal structure analysis, protein structure analysis by NMR, etc., measurement of various bioactivities can be cited.

The 24^{th} invention of the present application provides a system for calculating an occurrence trend parameter of an amino-acid residue comprising:
i) a means for extracting a linker sequence and a non-linker loop sequence from a database of multi-domain protein whose structure is known;
ii) a means for obtaining, based on statistical processing of amino-acid sequence of each domain, probabilities P_{Xaa}^{L} and P_{Xaa}^{N} of occurrence of an amino-acid residue Xₐₐ (where P_{Xaa}^{L} and P_{Xaa}^{N} are probabilities of occurrence of the amino acid residue Xₐₐ in a linker sequence and a non-linker loop sequence, respectively); and
iii) a means for obtaining an occurrence trend parameter S_{Xaa} of the amino-acid residue Xₐₐ by a following equation:${\text{S}}_{\text{Xaa}} {\text{=log (P}}_{\text{Xaa}} {\text{}}^{\text{L}} {\text{/P}}_{\text{Xaa}} {\text{}}^{\text{N}} \text{)}$ (where, if there is no statistically significant difference between P_{Xaa}^{L} and P_{Xaa}^{N}, it shall be S_{Xaa}=0.).

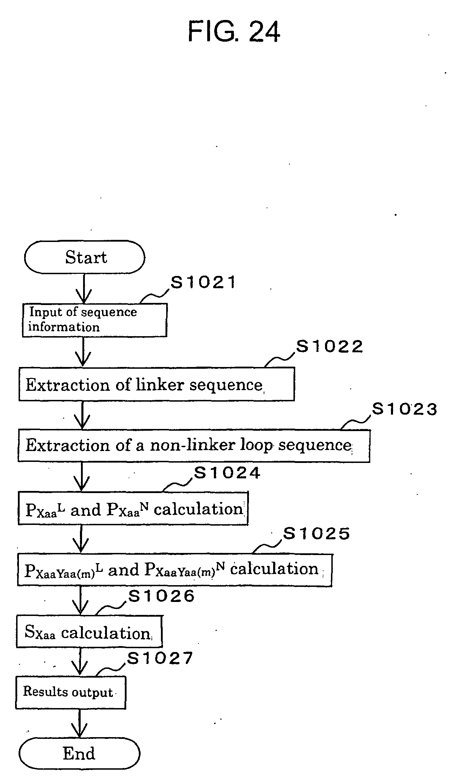

Fig. 24 is a flowchart explaining an operation of a system for calculating an occurrence trend parameter for a single amino-acid residue according to a preferred embodiment of the 24th invention of the present application. Steps S1021 through S1025 are the same as Steps S1001 through 1005 in Fig. 20. At Step S 1026, an occurrence trend parameter S_{Xaa} of the amino-acid residue Xₐₐ is obtained by an equation of S_{Xaa}=log(P_{Xaa}^{L}/P_{Xaa}^{N})(however, if there is no statistically significant difference between P_{Xaa}^{L} and P_{Xaa}^{N}, it shall be S_{Xaa}=0). At Step S1027, a calculated value of the occurrence trend parameter S_{Xaa} of the amino-acid residue Xₐₐ obtained at Step S1026 is outputted. The result output indicates, for example, a value of S_{Xaa} for each amino-acid residue. Step S1027 may be omitted. If the result is to be used for the next processing (calculation processing of discrimination scores, for example), Step S1027 is omitted.

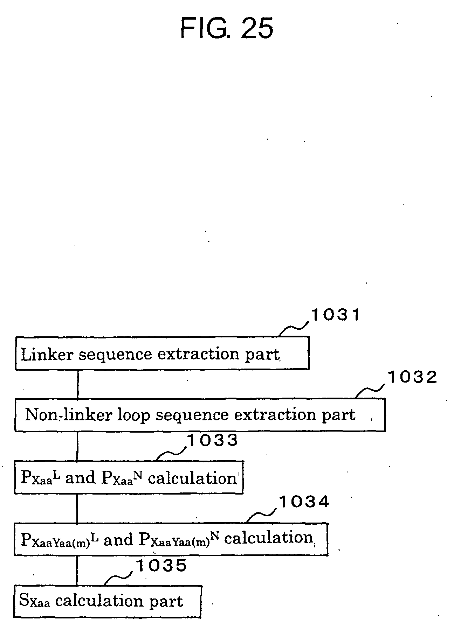

The occurrence trend parameter calculating system for an arbitrary amino-acid residue according to the 24^{th} invention of the present application is realized by a computer similar to that shown in Fig. 21, which is provided with, for example, a linker sequence extraction part 1031, a non-linker sequence extraction part 1032, a P_{Xaa}^{L} (P_{Xaa}^{N}) calculation part 1033, a P_{XaaYaa(m)}^{L} (P_{XaaYaa(m)}^{N}) calculation part 1034 and a S_{Xaa} calculation part 1035 shown in Fig. 25. The linker sequence extraction part 1031, the non-linker sequence extraction part 1032, the P_{Xaa}^{L} (P_{Xaa}^{N}) calculation part 1033 and the P_{XaaYaa(m)}^{L} (P_{XaaYaa(m)}^{N}) calculation part 1034 are the same as the linker sequence extraction part 1021, the non-linker sequence extraction part 1022, the P_{Xaa}^{L} (P_{Xaa}^{N}) calculation part 1023, and the P_{XaaYaa(m)}^{L} (P_{XaaYaa(m)}^{N}) calculation part 1024 in Fig. 22, respectively. In the S_{Xaa} calculation part 1035, the occurrence trend parameter S_{Xaa} of the amino-acid residue Xₐₐ is obtained by the equation of S_{Xaa}=log(P_{Xaa}^{L}/P_{Xaa}^{N})(however, if there is no statistically significant difference between P_{Xaa}^{L} and P_{Xaa}^{N}, it shall be S_{Xaa}=0).

The 25^{th} invention of the present application provides a program for having a computer function as a system of the 24^{th} invention of the present application.

The 26^{th} invention of the present application provides a system for calculating an occurrence trend parameter of an amino-acid residue pair comprising
i) a means for extracting a linker sequence and a non-linker loop sequence from a database of multi-domain protein whose structure is known;
ii) a means for obtaining, based on statistical processing of amino acid sequence of each domain, probabilities P_{XaaYaa(m)}^{L} and P_{XaaYaa(m)}^{N} of occurrence of amino-acid residues Xₐₐ and Yₐₐ (the order of Xₐₐ and Yₐₐ does not matter) with m pieces (m is an integer, m = 0, 1, 2) of arbitrary amino-acid residues between them (where P_{XaaYaa(m)}^{L} and P_{XaaYaa(m)}^{N} are probabilities of occurrence of the amino-acid residues Xₐₐ and Yₐₐ (the order of Xₐₐ and Yₐₐ does not matter) in a linker sequence and a non-linker loop sequence, respectively, with m pieces of amino-acid residues between them) for the cases where m is 0, 1 and 2, respectively; and
iii) a means for obtaining an occurrence trend parameter S_{XaaYaa(m)} of the amino acid residue pair Xₐₐ and Yₐₐ by a following equation:${\text{S}}_{\text{XaaYaa(m)}} {\text{=log (P}}_{\text{XaaYaa(m)}} {\text{}}^{\text{L}} {\text{/P}}_{\text{XaaYaa(m)}} {\text{}}^{\text{N}} \text{)}$ (where, if there is no statistically significant difference between P_{XaaYaa(m)}^{L} and P_{XaaYaa(m)}^{N}, it shall be S_{Xaa}=0.).

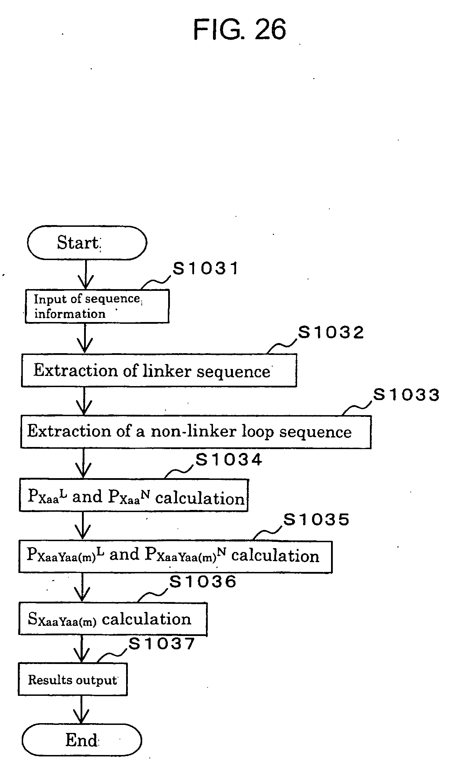

Fig. 26 is a flowchart explaining an operation of an occurrence trend parameter calculating system for an amino-acid residue pair according to a preferred embodiment of the 26^{th} invention of the present application. Steps S1031 through S1035 are the same as Steps S1001 through 1005 in Fig. 20. At Step S1036, an occurrence trend parameter S_{XaaYaa(m)} of the amino-acid residue pair Xₐₐ and Yₐₐ is obtained by an equation of S_{XaaYaa(m)} =log (P_{XaaYaa(m)}^{L}/P_{XaaYaa(m)}^{N}) (however, if there is no statistically significant difference between P_{XaaYaa(m)}^{L} and P_{XaaYaa(m)}^{N}, it shall be S_{Xaa}=0). At Step S1037, a calculated value of the occurrence trend parameter S_{XaaYaa(m)} of the amino-acid residue pair Xₐₐ and Yₐₐ obtained at Step S1036 is outputted. The result output indicates, for example, a value of S_{XaaYaa(m)} for each amino-acid residue pair. Step S1037 may be omitted. If the result is to be used for the next processing (calculation processing of discrimination scores, for example), Step S1037 is omitted.

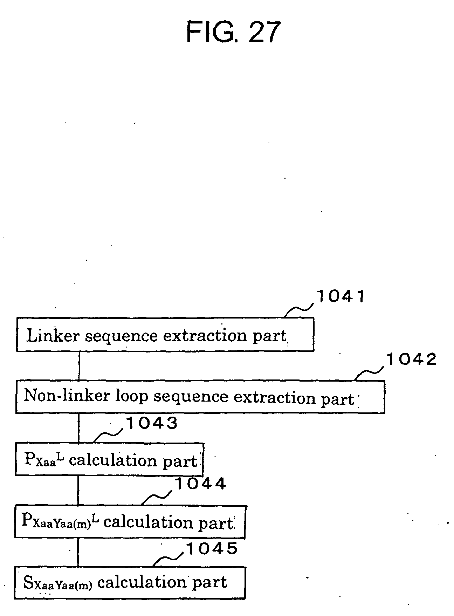

The occurrence trend parameter calculating system for an arbitrary amino-acid residue pair according to the 26^{th} invention of the present application is realized by a computer similar to that shown in Fig. 21, which is provided with, for example, a linker sequence extraction part 1041, a non-linker sequence extraction part 1042, a P_{Xaa}^{L} (P_{Xaa}^{N}) calculation part 1043, a P_{XaaYaa}(m)^{L} (P_{XaaYaa(m)}^{N}) calculation part 1044 and a S_{XaaYaa(m)} calculation part 1045 shown in Fig. 27. The linker sequence extraction part 1041, the non-linker sequence extraction part 1042, the P_{Xaa}^{L} (P_{Xaa}^{N}) calculation part 1043 and the P_{XaaYaa(m)}^{L} (P_{XaaYaa(m)}^{N}) calculation part 1044 are the same as the linker sequence extraction part 1021, the non-linker sequence extraction part 1022, the P_{Xaa}^{L} (P_{Xaa}^{N}) calculation part 1023, and the P_{XaaYaa(m)}^{L} (P_{XaaYaa(m)}^{N}) calculation part 1024 in Fig. 22, respectively. In the S_{XaaYaa(m)} calculation part 1045, the occurrence trend parameter S_{XaaYaa(m)} of the amino-acid residue pair Xₐₐ and Yₐₐ is obtained by the equation of S_{XaaYaa(m)} =log (P_{XaaYaa(m)}^{L}/P_{XaaYaa(m)}^{N}) (however, if there is no statistically significant difference between P_{XaaYaa(m)}^{L} and P_{XaaYaa(m)}^{N}, it shall be S_{Xaa}=0).

The 27^{th} invention of the present application provides a program for having a computer function as a system of the 26^{th} invention of the present application.

The 28^{th} invention of the present application provides a system for obtaining a linker degree discrimination score F₁ for an amino-acid sequence with L₁ pieces (L₁ is an integer from 1 or more to 21 or less) of amino-acid residues, the system comprising:
i) a means for obtaining a linker trend score F₁s of an amino-acid residue Aₖ by an equation below: 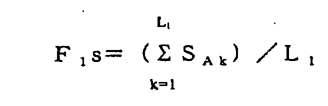 (in the equation, S_{Ak}=log(P_{Ak}^{L}/P_{Ak}^{N})
   where, if there is no statistically significant difference between P_{Ak}^{L} and P_{Ak}^{N}, it shall be S_{Ak}=0.
   Here, P_{Ak}^{L} and P_{Ak}^{N} are probabilities of occurrence of the amino-acid residue Aₖ in a linker sequence and a non-linker loop sequence, respectively.);
ii) a means for obtaining a linker trend score F₁p of an amino-acid residue pair Aₖ and Aₖ₊₍ₘ₊₁₎ with m pieces (m is an integer, m = 0, 1, 2) of arbitrary amino-acid residues between them by an equation below: 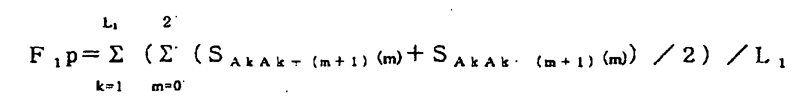 (in the equation, S_{AkAk+(m+1)(m)}=log(P_{AkAk+(m+1)(m)}^{L}/P_{AkAk+(m+1)(m)}^{N}) and S_{AkAk-(m+1)(m)}=log(P_{AkAk-(m+1)(m)}^{L}/P_{AkAk-(m+1)(m)}^{N})
   where, if there is no statistically significant difference between P_{AkAk+(m+1)(m)}^{L} and P_{AkAk+(m+1)(m)}^{N}, or P_{AkAk-(m+1)(m)}^{L} and P_{AkAk-(m+1)(m)}^{N}, it shall be S_{AkAk+(m+1)(m)}=0, or S_{AkAk-(m+1)(m)}=0.
   Here, P_{AkAk+(m+1)(m)}^{L} and P_{AkAk+(m+1)(m)}^{N} are probabilities of occurrence of the arbitrary amino-acid residues Aₖ and Aₖ₊₍ₘ₊₁₎ in a linker sequence and a non-linker loop sequence, respectively (the order of Aₖ and Aₖ₊₍ₘ₊₁₎ does not matter), and P_{AkAk-(m+1)(m)}^{L} and P_{AkAk-(m+1)(m)}^{N} are probabilities of occurrence of the arbitrary amino-acid residues Aₖ and Aₖ₋₍ₘ₊₁₎ in the linker sequence and the non-linker loop sequence, respectively (the order of Aₖ and Aₖ₋₍ₘ₊₁₎ does not matter)); and
iii) a means for obtaining a linker degree discrimination score Fi by an equation below:${\text{F}}_{\text{1}} {\text{=F}}_{\text{1}} {\text{s+α}}_{\text{1}} {\text{F}}_{\text{1}} \text{p}$ (in the equation, 0≦ α₁ ≦ 1)

A linker sequence set is a set of amino-acid sequences including at least one linker sequence, and those obtained by extracting a linker sequence portion from a multi-domain protein database whose structure is known can be cited, for example.

A non-linker loop sequence set is a set of amino-acid sequences including at least one non-linker loop sequence, and those obtained by extracting a non-linker sequence portion from a multi-domain protein database whose structure is known can be cited, for example.

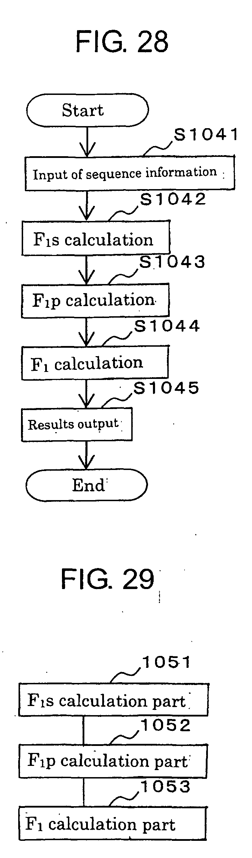

Fig. 28 is a flowchart explaining an operation of a trend score calculating system for an amino-acid residue pair according to a preferred embodiment of the 28^{th} invention of the present application. At Step S1041, sequence information is inputted. The sequence information to be inputted may be any sequence information such as, for example, amino-acid sequence information from the multi-domain protein database whose structure is known, amino-acid sequence information from the multi-domain protein database whose structure is unknown, sequence information not registered in the database but newly found, etc. At Step S1042, an occurrence trend score F₁s of an arbitrary amino-acid residue is obtained by the following equation: 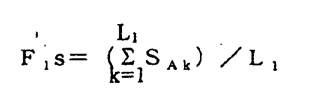 (in the equation, S_{Ak}=log(P_{Ak}^{L}/P_{Ak}^{N})
(where, P_{Ak}^{L} is an occurrence probability of an amino-acid residue Aₖ in a linker sequence set, while P_{Ak}^{N} is an occurrence probability of an amino-acid residue Aₖ in a non-linker sequence set, but if there is no statistically significant difference between P_{Ak}^{L} and P_{Ak}^{N}, it shall be S_{Ak}=0.)

At step S1043, an occurrence trend score F₁p of an amino-acid residue pair is obtained by the following equation: 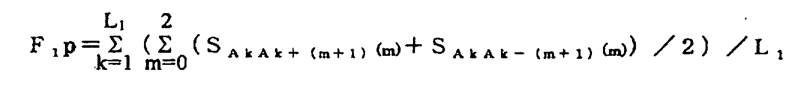 (in the equation, S_{AkAk+(m+1)(m)}=log(P_{AkAk+(m+1)(m)}^{L}/P_{AkAk+(m+1)(m)}^{N})
(where, P_{AkAk+(m+1)(m)}^{L} is an occurrence probability of the arbitrary amino-acid residues Aₖ and Aₖ₊₍ₘ₊₁₎ in a linker sequence set with m pieces (m is an integer, m = 0, 1, 2) of arbitrary amino-acid residues between them (the order of Aₖ and Aₖ₊₍ₘ₊₁₎ does not matter), while P_{AkAk+(m+1)(m)}^{N} is an occurrence probability of the arbitrary amino-acid residues Aₖ and Aₖ₊₍ₘ₊₁₎ in a non-linker sequence set with m pieces (m is an integer, m = 0, 1, 2) of arbitrary amino-acid residues between them (the order of Aₖ and Aₖ₊₍ₘ₊₁₎ does not matter), but if there is no statistically significant difference between P_{AkAk+(m+1)(m)}^{L} and P_{AkAk+(m+1)(m)}^{N}, it shall be S_{AkAk+(m+1)(m)}=0).
(in the equation, S_{AkAk-(m+1)(m)}=log(P_{AkAk-(m+1)(m)}^{L}/P_{AkAk-(m+1)(m)}^{N})
(where, P_{AkAk-(m+1)(m)}^{L} is an occurrence probability of the arbitrary amino-acid residues Aₖ and Aₖ₋₍ₘ₊₁₎ in a linker sequence set with m pieces (m is an integer, m = 0, 1, 2) of arbitrary amino-acid residues between them (the order of Aₖ and Aₖ₋₍ₘ₊₁₎ does not matter), while P_{AkAk-(m+1)(m)}^{N} is an occurrence probability of the arbitrary amino-acid residues Aₖ and Aₖ₋₍ₘ₊₁₎ in a non-linker sequence set with m pieces (m is an integer, m = 0, 1, 2) of arbitrary amino-acid residues between them (the order of Aₖ and Aₖ₋₍ₘ₊₁₎ does not matter), but if there is no statistically significant difference between P_{AkAk-(m+1)(m)}^{L} and P_{AkAk-(m+1)(m)}^{N}, it shall be S_{AkAk-(m+1)(m)}=0).

At Step S1044, the linker degree discrimination score Fi is obtained by an equation below:${\text{F}}_{\text{1}} {\text{=F}}_{\text{1}} {\text{s+α}}_{\text{1}} {\text{F}}_{\text{1}} \text{p}$ (in the equation, 0≦ α₁≦1)

At Step S1045, the linker degree discrimination score F₁ obtained at Step S1044 is outputted. The result output indicates, for example, an amino-acid residue, a value of F₁ of each amino-acid sequence, etc. Step S1045 may be omitted. If the result is to be used for the next processing (construction processing of domain linker database, for example), Step S1045 is omitted.

The system for obtaining the linker degree discrimination score F₁s of the 28^{th} invention of the present invention is realized by a computer similar to that shown in Fig. 21, which is provided with, for example, an Fis calculation part 1051, an F₁p calculation part 1052, and an F₁ calculation part 1053. In the F₁s calculation part 1051, the occurrence trend score F₁s of an amino-acid residue is obtained by the above equation. In the F₁p calculation part 1052, the occurrence trend score F₁p of an amino-acid residue pair is obtained by the above equation. In the F₁ calculation part 1053, the linker degree discrimination score F₁ is obtained by the above equation

The 29^{th} invention of the present application provides a program for having a computer function as a system of the 28^{th} invention of the present application.

The 30^{th} invention of the present application provides a method of obtaining a linker degree discrimination score F₁₁(i) for an amino-acid residue Ai at a position i in an amino-acid sequence with L₂ pieces (L₂ is an integer of 22 or more) of amino-acid residues by taking a window of w pieces of amino-acid residues before and after the amino-acid residue at the position i (i is an integer from 1 or more to L₂ or less) comprising:
i) a step for obtaining a linker trend score F₁₁s(i) of an amino-acid residue Aₖ by an equation below: 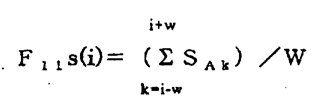 (in the equation, W is a window width, and W=2w+1,${\text{S}}_{\text{Ak}} {\text{=log(P}}_{\text{Ak}} {\text{}}^{\text{L}} {\text{/P}}_{\text{Ak}} {\text{}}^{\text{N}} \text{)}$ where, if there is no statistically significant difference between P_{Ak}^{L} and P_{Ak}^{N}, it shall be S_{Ak}=0.
   Here, P_{Ak}^{L} and P_{Ak}^{N} are probabilities of occurrence of the amino-acid residue Aₖ in a linker sequence and a non-linker loop sequence, respectively.);
ii) a step for obtaining the linker trend score F₁₁p(i) of an amino-acid residue pair Aᵢ and Aᵢ₊₍ₘ₊₁₎ with m pieces (m is an integer, m = 0, 1, 2) of arbitrary amino-acid residues between them by an equation below: 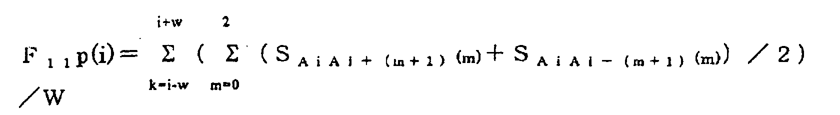 (in the equation, S_{AiAi+(m+1)(m)}=log(P_{AiAi+(m+1)(m)}^{L}/P_{AiAi+(m+1)(m)}^{N}), and S_{AiAi-(m+1)(m)}=log(P_{AiAi-(m+1)(m)}^{L}/P_{AiAi-(m+1)(m)}^{N})
   where, if there is no statistically significant difference between P_{AiAi+(m+1)(m)}^{L} and P_{AiAi+(m+1)(m)}^{N}, or P_{AiAi-(m+1)(m)}^{L} and P_{AiAi-(m+1)(m)}^{N}, it shall be S_{AiAi+(m+1)(m)}=0, or S_{AiAi-(m+1)(m)}=0.
   Here, P_{AiAi+(m+1)(m)}^{L} and P_{AiAi+(m+1)(m)}^{N} are probabilities of occurrence of the arbitrary amino-acid residue pair Aᵢ and Aᵢ₊₍ₘ₊₁₎ in a linker sequence and a non-linker loop sequence, respectively (the order of Aᵢ and Aᵢ₊₍ₘ₊₁₎ does not matter), and P_{AiAi-(m+1)(m)}^{L} and P_{AiAi-(m+1)(m)}^{N} are probabilities of occurrence of the arbitrary amino-acid residues Aᵢ and Aᵢ₋₍ₘ₊₁₎ in the linker sequence and the non-linker loop sequence, respectively (the order of Aᵢ and Aᵢ₋₍ₘ₊₁₎ does not matter)); and
iii) a step for obtaining the linker degree discrimination score F₁₁(i) of the amino-acid residue Ai at the position i by an equation below:${\text{F}}_{\text{11}} {\text{(i)=F}}_{\text{11}} {\text{s(i)+α}}_{\text{11}} {\text{F}}_{\text{11}} \text{p(i)}$
(in the equation, 0≦ α₁₁≦1)

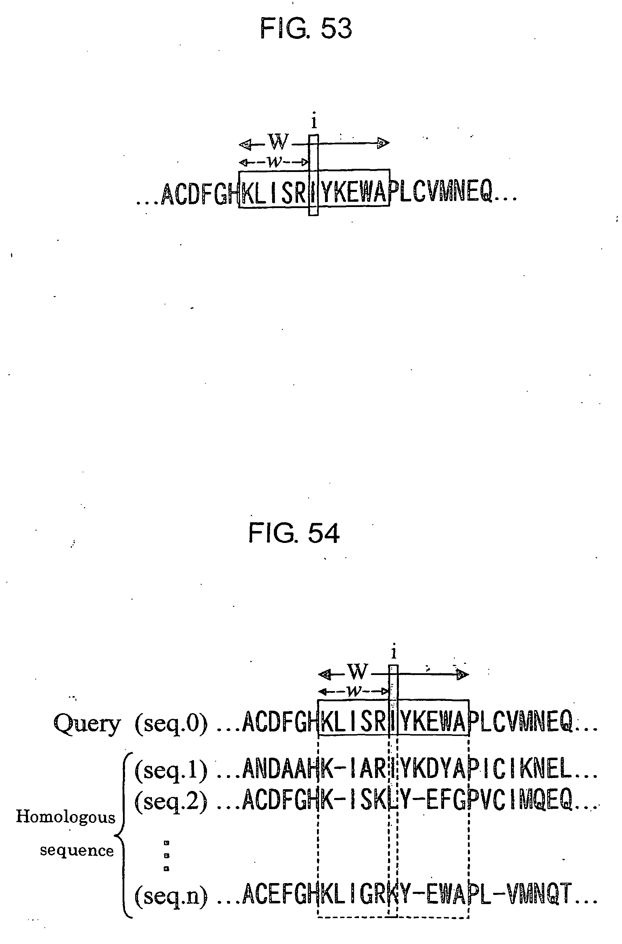

In Fig. 53, how to take a window is shown.

The window width W is preferably 5 through 21, more preferably 9 through 13.

The 31^{st} invention of the present invention provides a system for obtaining a linker degree discrimination score F₁₁(i) for an amino-acid residue Ai at a position i in an amino-acid sequence with L₂ pieces (L₂ is an integer of 22 or more) of amino-acid residues by taking a window of w pieces of amino-acid residues before and after the amino-acid residue at the position i (i is an integer from 1 or more to L₂ or less) comprising:
i) a means for obtaining a linker trend score F₁₁s(i) of an amino-acid residue Aₖ by an equation below: 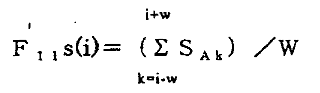 (in the equation, W is a window width, and W=2w+1,${\text{S}}_{\text{Ak}} {\text{=log(P}}_{\text{Ak}} {\text{}}^{\text{L}} {\text{/P}}_{\text{Ak}} {\text{}}^{\text{N}} \text{)}$ where, if there is no statistically significant difference between P_{Ak}^{L} and P_{Ak}^{N}, it shall be S_{Ak}=0.
   Here, P_{Ak}^{L} and P_{Ak}^{N} are probabilities of occurrence of the amino-acid residue Aₖ in a linker sequence and a non-linker loop sequence, respectively.);
ii) a means for obtaining the linker trend score F₁₁p(i) of an amino-acid residue pair Aᵢ and Aᵢ₊₍ₘ₊₁₎ with m pieces (m is an integer, m = 0, 1, 2) of arbitrary amino-acid residues between them by an equation below: 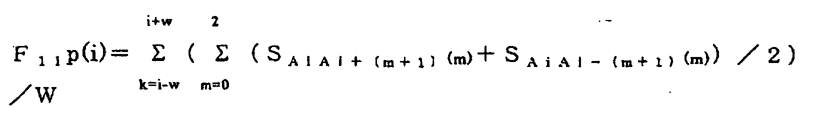 (in the equation, S_{AiAi+(m+1)(m)}=log(P_{AiAi+(m+1)(m)}^{L}/P_{AiAi+(m+1)(m)}^{N}), and S_{AiAi-(m+1)(m)}=log(P_{AiAi-(m+1)(m)}^{L}/P_{AiAi-(m+1)(m)}^{N})
   where, if there is no statistically significant difference between P_{AiAi+(m+1)(m)}^{L} and P_{AiAi+(m+1)(m)}^{N}, or P_{AiAi-(m+1)(m)}^{L} and P_{AiAi-(m+1)(m)}^{N}, it shall be S_{AiAi+(m+1)(m)}=0, or S_{AiAi-(m+1)(m)}=0.
   Here, P_{AiAi+(m+1)(m)}^{L} and P_{AiAi+(m+1)(m)}^{N} are probabilities of occurrence of the arbitrary amino-acid residue pair Aᵢ and Aᵢ₊₍ₘ₊₁₎ in a linker sequence and a non-linker loop sequence, respectively (the order of Aᵢ and Aᵢ₊₍ₘ₊₁₎ does not matter), and P_{AiAi-(m+1)(m)}^{L} and P_{AiAi-(m+1)(m)}^{N} are probabilities of occurrence of the arbitrary amino-acid residue pair Aᵢ and Aᵢ₋₍ₘ₊₁₎ in the linker sequence and the non-linker loop sequence, respectively (the order of Aᵢ and Aᵢ₋₍ₘ₊₁₎ does not matter)); and
iii) a means for obtaining the linker degree discrimination score F₁₁(i) of the amino-acid residue Ai at the position i by an equation below:${\text{F}}_{\text{11}} {\text{(i)=F}}_{\text{11}} {\text{s(i)+α}}_{\text{11}} {\text{F}}_{\text{11}} \text{p(i)}$
(in the equation, 0≦ α₁₁≦1)

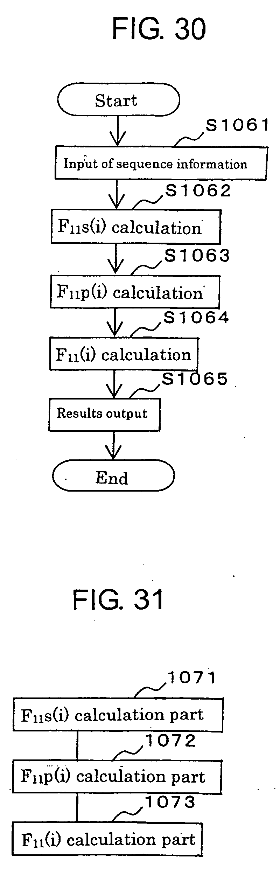

Fig. 30 is a flowchart explaining an operation of a system for obtaining a linker degree discrimination score F₁₁(i) according to a preferred embodiment of the 30^{th} invention of the present application or a system for obtaining a linker degree discrimination score F₁₁(i) according to a preferred embodiment of the 31^{st} invention of the present application.

At Step S1061, sequence information is inputted. The sequence information to be inputted may be any sequence information such as, for example, sequence information from the multi-domain protein database whose structure is known, sequence information from the multi-domain protein database whose structure is unknown, sequence information not registered in the database but newly found, etc.

At Step S1062, an occurrence trend score F₁₁s(i) of an arbitrary amino-acid residue is obtained by the following equation: 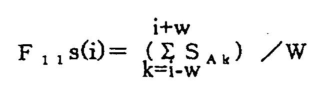 (in the equation, W is a window width, and W=2w+1,${\text{S}}_{\text{Ak}} {\text{=log(P}}_{\text{Ak}} {\text{}}^{\text{L}} {\text{/P}}_{\text{Ak}} {\text{}}^{\text{N}} \text{)}$ (where, P_{Ak}^{L} is an occurrence probability of an amino-acid residue Aₖ in a linker sequence set, while P_{AK}^{N} is an occurrence probability of an amino-acid residue Aₖ in a non-linker sequence set, but if there is no statistically significant difference between P_{Ak}^{L} and P_{AK}^{N}, it shall be S_{Ak}=0.)

At step S1063, an occurrence trend score F₁₁p(i) of an amino-acid residue pair is obtained by the following equation: 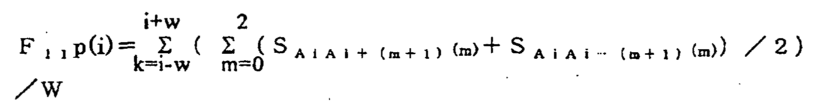 (in the equation, S_{AiAi+(m+1)(m)}=log(P_{AiAi+(m+1)(m)}^{L}/P_{AiAi+(m+1)(m)}^{N}))
(where, P_{AiAi+(m+1)(m)}^{L} is an occurrence probability of the arbitrary amino-acid residues Aᵢ and Aᵢ₊₍ₘ₊₁₎ in a linker sequence set with m pieces (m is an integer, m = 0, 1, 2) of arbitrary amino-acid residues between them (the order of Aᵢ and Aᵢ₊₍ₘ₊₁₎ does not matter), while P_{AiAi+(m+1)(m)}^{N} is an occurrence probability of the arbitrary amino-acid residues Aᵢ and Aᵢ₊₍ₘ₊₁₎ in a non-linker sequence set with m pieces (m is an integer, m = 0, 1, 2) of arbitrary amino-acid residues between them (the order of Aᵢ and Aᵢ₊₍ₘ₊₁₎ does not matter), but if there is no statistically significant difference between P_{AiAi+(m+1)(m)}^{L} and P_{AiAi+(m+1)(m)}^{N}, it shall be S_{AiAi+(m+1)(m)}=0).${\text{S}}_{\text{AiAi-(m+1)(m)}} {\text{=log(P}}_{\text{AiAi-(m+1)(m)}} {\text{}}^{\text{L}} {\text{/P}}_{\text{AiAi-(m+1)(m)}} {\text{}}^{\text{N}} \text{))}$ (where, P_{AiAi-(m+1)(m)}^{L} is an occurrence probability of the arbitrary amino-acid residues Aᵢ and Aᵢ₋₍ₘ₊₁₎ in a linker sequence set with m pieces (m is an integer, m = 0, 1, 2) of arbitrary amino-acid residues between them (the order of Aᵢ and Aᵢ₋₍ₘ₊₁₎ does not matter), while P_{AiAi+(m+1)(m)}^{N} is an occurrence probability of the arbitrary amino-acid residues Aᵢ and Aᵢ₋₍ₘ₊₁₎ in a non-linker sequence set with m pieces (m is an integer, m = 0, 1, 2) of arbitrary amino-acid residues between them (the order of Aᵢ and Aᵢ₋₍ₘ₊₁₎ does not matter), but if there is no statistically significant difference between P_{AiAi-(m+1)(m)}^{L} and P_{AiAi-(m+1)(m)}^{N}, it shall be S_{AiAi-(m+1)(m)}=0).

At Step S1064, the linker degree discrimination score F₁₁(i) is obtained by an equation below:${\text{F}}_{\text{11}} {\text{(i)=F}}_{\text{11}} {\text{s(i)+α}}_{\text{11}} {\text{F}}_{\text{11}} \text{p(i)}$ (in the equation, 0≦ α₁₁ ≦ 1)

Steps S1062 to S1064 are executed for all the amino-acid residues Ai at the position i existing in the range of 1 or more to L₂ or less.

At Step S1065, the linker degree discrimination score F₁₁(i) obtained at Step S1064 is outputted. The result output indicates, for example, an amino-acid sequence, the position i and a value of corresponding F₁₁(i), etc. Step S1065 may be omitted. If the result is to be used for the next processing (prediction processing of domain linker, for example), Step S1065 is omitted.

The system for obtaining the linker degree discrimination score F₁₁(i) of the 31^{st} invention of the present invention is realized by a computer similar to that shown in Fig. 21, which is provided with, for example, an F₁₁s(i) calculation part 1071, an F₁₁p(i) calculation part 1072, and an F₁₁(i) calculation part 1073. In the F₁₁s(i) calculation part 1071, the F₁₁p(i)calculation part 1072, and the F₁₁(i) calculation part 1073, F₁₁s(i), F₁₁p(i) and the linker degree discrimination score F₁₁(i) is obtained by the above equations, respectively.

The 32^{nd} invention of the present application provides a program for having a computer function as a system of the 31^{st} invention of the present application.

The 33^{rd} invention of the present application provides a method of obtaining a linker degree discrimination score F₁₂(i) of an amino-acid residue Ai at a position i in an amino-acid sequence seq.0 with L₂ pieces (L₂ is an integer of 22 or more) of amino-acid residues for which existence of n pieces (n is an integer of 1 or more) of homologous sequences seq.1∼seq.n is known by taking a window with w pieces of the amino-acid residues before and after the amino-acid residue at the position i (i is an integer from 1 or more to 22 or less) comprising:
i) a step for identifying an amino-acid residue Aᵢ^{k} in a seq.k (k is an integer from 1 or more and n or less) corresponding to an amino-acid residue Ai⁰ at a position i in the seq.0 by aligning seq.0 and seq.1∼seq.n;
ii) a step for obtaining parameters S'_{Ai}, S'_{AiAi+(m+1)}(m) and S'_{AiAi-(m+1)}(m) of the amino-acid residue Ai at the position i by an equation below: 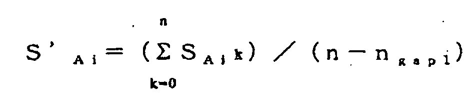 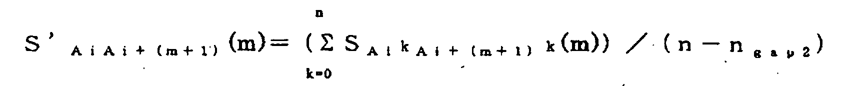 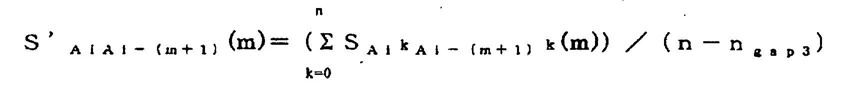 (in the equation, n_{gap1} is the number of gaps occurring in Aᵢ^{k},${\text{S}}_{\text{Ai}} {\text{k=log(P}}_{\text{Ai}} {\text{k}}^{\text{L}} {\text{/P}}_{\text{Ai}} {\text{k}}^{\text{N}} \text{)}$ where, if there is no statistically significant difference between P_{Ai}k^{L} and P_{Ai}k^{N}, it shall be S_{Ai}k=0.
   Here, P_{Ai}k^{L} and P_{Ai}k^{N} are probabilities of occurrence of the amino-acid residue Aᵢ^{k} in a linker sequence and a non-linker loop sequence, respectively.
   Also, in the equation, n_{gap2} is the number of gaps occurring in Aᵢ^{k} or Aᵢ₊₍ₘ₊₁₎ ^{k},${\text{S}}_{\text{Ai}} {\text{k}}_{\text{Ai+(m+1)}} {\text{k(m)= log(P}}_{\text{Ai}} {\text{k}}_{\text{Ai+(m+1)}} {\text{k}}_{\text{(m)}} {\text{}}^{\text{L}} {\text{/P}}_{\text{Ai}} {\text{k}}_{\text{Ai+(m+1)}} {\text{k}}_{\text{(m)}} {\text{}}^{\text{N}} \text{)}$ where, if there is no statistically significant difference between P_{Ai}k_{Ai+(m+1)}k₍ₘ₎^{L} and P_{Ai}k_{Ai+(m+1)}k ₍ₘ₎^{N}, it shall be S_{Ai}k_{Ai+(m+1)}k ₍ₘ₎=0.
   Here, P_{Ai}k_{Ai+(m+1)}k₍ₘ₎^{L} and P_{Ai}k_{Ai+(m+1)}k₍ₘ₎^{N} are probabilities of occurrence of the arbitrary amino-acid residues Aᵢ^{k} and Aᵢ₊₍ₘ₊₁₎^{k} in a linker sequence and a non-linker loop sequence, respectively (the order of Aᵢ^{k} and Aᵢ₊₍ₘ₊₁₎^{k} does not matter) with m pieces (m is an integer, m = 0, 1, 2) of arbitrary amino-acid residues between them.
   Moreover, in the equation, n_{gap3} is the number of gaps occurring in Aᵢ^{k} or Aᵢ₋₍ₘ₊₁₎ ^{k},${\text{S}}_{\text{Ai}} {\text{k}}_{\text{Ai-(m+1)}} {\text{k(m)= log(P}}_{\text{Ai}} {\text{k}}_{\text{Ai-(m+1)}} {\text{k}}_{\text{(m)}} {\text{}}^{\text{L}} {\text{/P}}_{\text{Ai}} {\text{k}}_{\text{Ai-(m+1)}} {\text{k}}_{\text{(m)}} {\text{}}^{\text{N}} \text{)}$ where, if there is no statistically significant difference between P_{Ai}k_{Ai-(m+1)}k₍ₘ₎^{L} and P_{Ai}k_{Ai-(m+1)}k₍ₘ₎^{N}, it shall be S_{Ai}k_{Ai-(m+1)}k₍ₘ₎=0.
   Here, P_{Ai}k_{Ai-(m+1)}k₍ₘ₎^{L} and P_{Ai}k_{Ai-(m+1)}k₍ₘ₎^{N} are probabilities of occurrence of the amino-acid residues Aᵢ^{k} and Aᵢ₋₍ₘ₊₁₎^{k} in a linker sequence and a non-linker loop sequence, respectively (the order of Aᵢ^{k} and Aᵢ₋₍ₘ₊₁₎^{k} does not matter) with m pieces (m is an integer, m = 0, 1, 2) of arbitrary amino-acid residues between them.);
iii) a step for obtaining a linker trend score F₁₂s(i) of an amino-acid residue by an equation below: 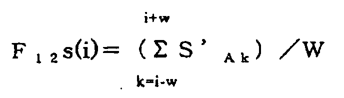
iv) a step for obtaining a linker trend score F₁₂p(i) of an arbitrary amino-acid residue pair by an equation below: and 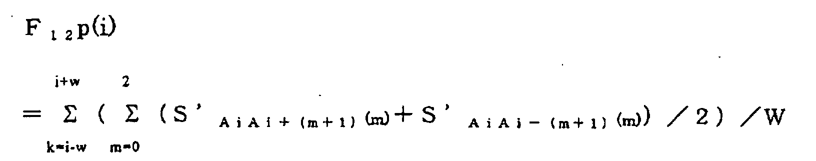
v) a step for obtaining the linker degree discrimination score F₁₂(i) of the amino-acid residue Ai at the position i by an equation below:${\text{F}}_{\text{12}} {\text{(i)=F}}_{\text{12}} {\text{s(i)+α}}_{\text{12}} {\text{F}}_{\text{12}} \text{p(i)}$
(in the equation, 0≦ α₁₂ ≦ 1)

In Fig. 54, sequences of aligned seq.0 and seq.1 through seq.n and how to take a window are shown.

The 34^{th} invention of the present application is a system for obtaining a linker degree discrimination score F₁₂(i) of an amino-acid residue Ai at a position i in an amino-acid sequence seq.0 with L₂ pieces (L₂ is an integer of 22 or more) of amino-acid residues for which existence of n pieces (n is an integer of 1 or more) of homologous sequences seq.1∼seq.n is known, by taking a window with w pieces of amino-acid residues before and after the amino-acid residue at the position i (i is an integer from 1 or more to 22 or less) comprising:
i) a means for identifying an amino-acid residue Aᵢ^{k} in a seq.k (k is an integer from 1 or more and n or less) corresponding to an amino-acid residue Ai⁰ at the position i in the seq.0 by aligning seq.0 and seq.1∼seq.n;
ii) a means for obtaining parameters of the amino-acid residue Ai at the position i, S'_{Ai}, S'_{AiAi+(m+1)}(m) and S'_{AiAi-(m+1)}(m) by an equation below: 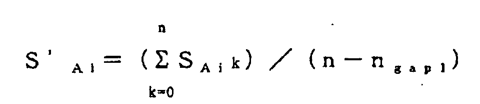 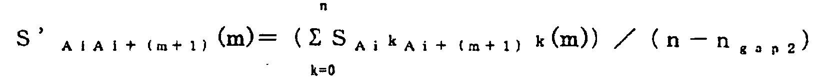 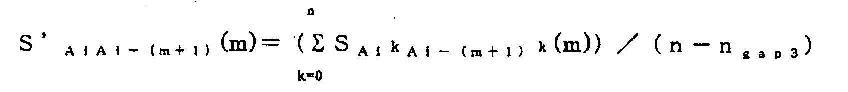 (in the equation, n_{gap1} is the number of gaps occurring in Aᵢ^{k} ,${\text{S}}_{\text{Ai}} {\text{k=log(P}}_{\text{Ai}} {\text{k}}^{\text{L}} {\text{/P}}_{\text{Ai}} {\text{k}}^{\text{N}} \text{)}$ where, if there is no statistically significant difference between P_{Ai}k^{L} and P_{Ai}k^{N}, it shall be S_{Ai}^{k}=0.
   Here, P_{Ai}k^{L} and P_{Ai}k^{N} are probabilities of occurrence of the amino-acid residue Aᵢ^{k} in a linker sequence and a non-linker loop sequence, respectively.
   Also, in the equation, n_{gap2} is the number of gaps occurring in Aᵢ^{k} or Aᵢ₊₍ₘ₊₁₎^{k},${\text{S}}_{\text{Ai}} {\text{k}}_{\text{Ai+(m+1)}} {\text{k(m)= log(P}}_{\text{Ai}} {\text{k}}_{\text{Ai+(m+1)}} {\text{k}}_{\text{(m)}} {\text{}}^{\text{L}} {\text{/P}}_{\text{Ai}} {\text{k}}_{\text{Ai+(m+1)}} {\text{k}}_{\text{(m)}} {\text{}}^{\text{N}} \text{)}$ where, if there is no statistically significant difference between P_{Ai}k_{Ai+(m+1)}k₍ₘ₎^{L}and P_{Ai}k_{Ai+(m+1)}k₍ₘ₎^{N}, it shall be S_{Ai}k_{Ai+(m+1)}k₍ₘ₎=0.
   Here, P_{Ai}k_{Ai+(m+1)}k₍ₘ₎^{L} and P_{Ai}k_{Ai+(m+1)}k₍ₘ₎^{N} are probabilities of occurrence of the amino-acid residues Aᵢ^{k} and Aᵢ₊₍ₘ₊₁₎^{k} in the linker sequence and the non-linker loop sequence, respectively (the order of Aᵢ^{k} and Aᵢ₊₍ₘ₊₁₎^{k} does not matter) with m pieces (m is an integer, m = 0, 1, 2) of arbitrary amino-acid residues between them.
   Moreover, in the equation, n_{gap3} is the number of gaps occurring in Aᵢ^{k} or Aᵢ₋₍ₘ₊₁₎^{k},${\text{S}}_{\text{Ai}} {\text{k}}_{\text{Ai-(m+1)}} {\text{k(m)= log(P}}_{\text{Ai}} {\text{k}}_{\text{Ai-(m+1)}} {\text{k}}_{\text{(m)}} {\text{}}^{\text{L}} {\text{/P}}_{\text{Ai}} {\text{k}}_{\text{Ai-(m+1)}} {\text{k}}_{\text{(m)}} {\text{}}^{\text{N}} \text{)}$ where, if there is no statistically significant difference between P_{Ai}k_{Ai-(m+1)}k₍ₘ₎^{L} and P_{Ai}k_{Ai-(m+1)}k₍ₘ₎^{N}, it shall be S_{Ai}k_{Ai-(m+1)}k₍ₘ₎=0.
   Here, P_{Ai}k_{Ai-(m+1)}k₍ₘ₎^{L} and P_{Ai}k_{Ai-(m+1)}k₍ₘ₎^{N} are probabilities of occurrence of the amino-acid residues Aᵢ^{k} and Aᵢ₋₍ₘ₊₁₎^{k} in the linker sequence and the non-linker loop sequence, respectively (the order of Aᵢ^{k} and Aᵢ₋₍ₘ₊₁₎^{k} does not matter) with m pieces (m is an integer, m = 0, 1, 2) of arbitrary amino acid residues between them.);
iii) a means for obtaining a linker trend score F₁₂s(i) of an amino-acid residue by an equation below; 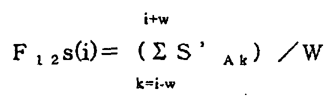
iv) a means for obtaining a linker trend score F₁₂p(i) of an arbitrary amino-acid residue pair by an equation below; and 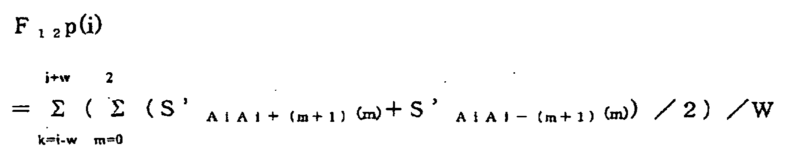
v) a means for obtaining the linker degree discrimination score F₁₂(i) of the amino-acid residue Ai at the position i by an equation below.${\text{F}}_{\text{12}} {\text{(i)=F}}_{\text{12}} {\text{s(i)+α}}_{\text{12}} {\text{F}}_{\text{12}} \text{p(i)}$
(in the equation, 0≦ α₁₂≦1)

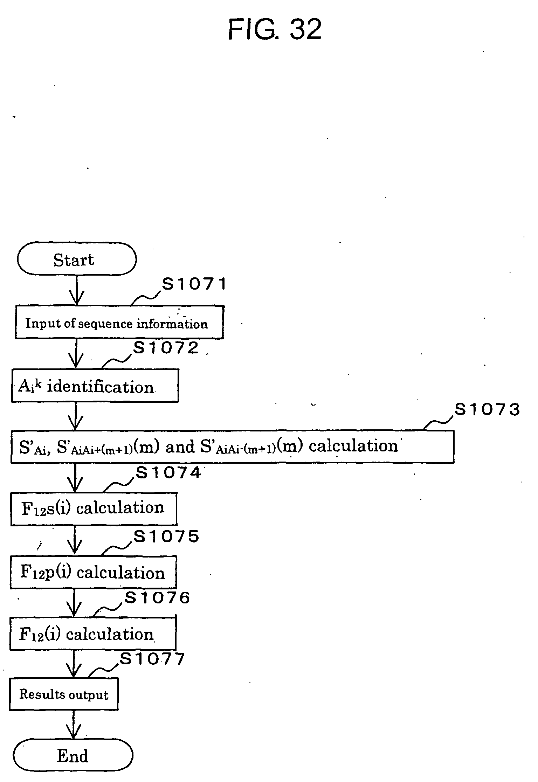

Fig. 32 is a flowchart explaining an operation of a method of obtaining a linker degree discrimination score F₁₂(i) according to a preferred embodiment of the 33^{rd} invention of the present application or a system for obtaining a linker degree discrimination score F₁₂(i) of the 34^{th} invention of the present application.

At Step S1071, sequence information is inputted. The sequence information to be inputted may be any sequence information such as, for example, sequence information from the multi-domain protein database whose structure is known, sequence information from the multi-domain protein database whose structure is unknown, sequence information not registered in the database but newly found, etc.

At Step S1072, the amino-acid residue Aᵢ^{k} in the seq.k (k is an integer from 1 or more and n or less) corresponding to the amino-acid residue Ai⁰ at the position i in the seq.0 is identified by aligning seq.0 and seq.1∼seq.n;
k is an integer

At Step S1073, the parameters S'_{Ai}, S'_{AiAi+(m+1)}(m) and S'_{AiAi-(m+1)}(m) of the amino-acid residue Ai at the position i are obtained by an equation below: 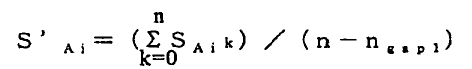 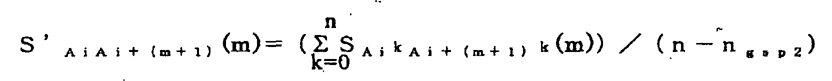 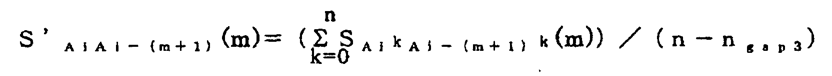 (in the equation, n_{gap1} is the number of gaps occurring in Aᵢ^{k} ,${\text{S}}_{\text{Ai}} {\text{k=log(P}}_{\text{Ai}} {\text{k}}^{\text{L}} {\text{/P}}_{\text{Ai}} {\text{k}}^{\text{N}} \text{)}$ (where, P_{Ai}k^{L} is an occurrence probability of the amino-acid residue Aᵢ^{k} in a linker sequence and P_{Ai}k^{N} is an occurrence probability of the amino-acid residue Aᵢ^{k} in a non-linker loop sequence, but if there is no statistically significant difference between P_{Ai}k^{L} and P_{Ai}k^{N}, it shall be S_{Ai}^{k}=0.)
(in the equation, n_{gap2} is the number of gaps occurring in Aᵢ^{k} or Aᵢ₊₍ₘ₊₁₎^{k},${\text{S}}_{\text{Ai}} {\text{k}}_{\text{Ai+(m+1)}} {\text{k(m)= log(P}}_{\text{Ai}} {\text{k}}_{\text{Ai+(m+1)}} {\text{k}}_{\text{(m)}} {\text{}}^{\text{L}} {\text{/P}}_{\text{Ai}} {\text{k}}_{\text{Ai+(m+1)}} {\text{k}}_{\text{(m)}} {\text{}}^{\text{N}} \text{)}$ (in the equation, P_{Ai}k_{Ai+(m+1)}k₍ₘ₎^{L} is an occurrence probability of the amino-acid residues Aᵢ^{k} and Aᵢ₊₍ₘ₊₁₎^{k} in the linker sequence set (the order of Aᵢ^{k} and Aᵢ₊₍ₘ₊₁₎^{k} does not matter) with m pieces (m is an integer, m = 0, 1, 2) of arbitrary amino-acid residues between them, and P_{Ai}k_{Ai+(m+1)}k₍ₘ₎^{N} is an occurrence probability of the amino-acid residues Aᵢ^{k} and Aᵢ₊₍ₘ₊₁₎^{k} in the non-linker sequence set (the order of Aᵢ^{k} and Aᵢ₊₍ₘ₊₁₎^{k} does not matter) with m pieces (m is an integer, m = 0, 1, 2) of arbitrary amino-acid residues between them, but if there is no statistically significant difference between P_{Ai}k_{Ai+(m+1)}k₍ₘ₎^{L}and P_{Ai}k_{Ai+(m+1)}k ₍ₘ₎^{N}, it shall be S_{Ai}k_{Ai+(m+1)}k₍ₘ₎=0.
(in the equation, n_{gap3} is the number of gaps occurring in Aᵢ^{k} or Aᵢ₋₍ₘ₊₁₎^{k},${\text{S}}_{\text{Ai}} {\text{k}}_{\text{Ai-(m+1)}} {\text{k(m)= log(P}}_{\text{Ai}} {\text{k}}_{\text{Ai-(m+1)}} {\text{k}}_{\text{(m)}} {\text{}}^{\text{L}} {\text{/P}}_{\text{Ai}} {\text{k}}_{\text{Ai-(m+1)}} {\text{k}}_{\text{(m)}} {\text{}}^{\text{N}} \text{)}$ (in the equation, P_{Ai}k_{Ai-(m+1)}k₍ₘ₎^{L} is an occurrence probability of the amino-acid residues Aᵢ^{k} and Aᵢ₋₍ₘ₊₁₎^{k} in the linker sequence set (the order of Aᵢ^{k} and Aᵢ₋₍ₘ₊₁₎^{k} does not matter) with m pieces (m is an integer, m = 0, 1, 2) of arbitrary amino acid residues between them, and P_{Ai}k_{Ai-(m+1)}k₍ₘ₎^{N} is an occurrence probability of the amino-acid residues Aᵢ^{k} and Aᵢ₋₍ₘ₊₁₎^{k} in the non-linker loop sequence set (the order of Aᵢ^{k} and Aᵢ₋₍ₘ₊₁₎^{k} does not matter) with m pieces (m is an integer, m = 0, 1, 2) of arbitrary amino acid residues between them, but if there is no statistically significant difference between P_{Ai}k_{Ai-(m+1)}k₍ₘ₎^{L} and P_{Ai}k_{Ai-(m+1)}k₍ₘ₎^{N}, it shall be S_{Ai}k_{Ai-(m+1)}k₍ₘ₎=0..);

At Step S1074, the single amino-acid residue trend score F₁₂s(i) is obtained by an equation below;

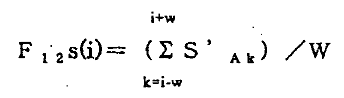

At Step S1075, the occurrence trend score F₁₂p(i) of an arbitrary amino-acid residue pair by an equation below:

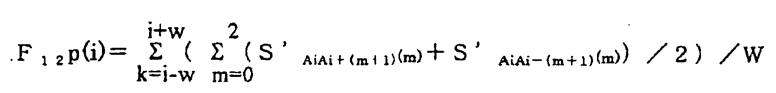

At Step S1076, the linker degree discrimination score F₁₂(i) of the amino-acid residue Ai at the position i by an equation below.${\text{F}}_{\text{12}} {\text{(i)=F}}_{\text{12}} {\text{s(i)+α}}_{\text{12}} {\text{F}}_{\text{12}} \text{p(i)}$ (in the equation, 0 ≦ α₁₂ ≦ 1)

Steps S1072 to S1076 are executed for all the amino-acid residues Ai at the position i existing in the range of 1 or more to L₂ or less.

At Step S1077, the linker degree discrimination score F₁₂(i) obtained at Step S1076 is outputted. The result output indicates, for example, an amino-acid sequence, the position i and a value of corresponding F₁₂(i), etc. Step S1077 may be omitted. If the result is to be used for the next processing (prediction processing of domain linker, for example), Step S1077 is omitted.

The system for obtaining the linker degree discrimination score F₁₂(i) of the 34^{th} invention of the present invention is realized by a computer similar to that shown in Fig. 21, which is provided with, for example, an Aᵢ^{k} identification part 1081, an S'_{Ai}, S'_{AiAi+(m+1)}(m) and S'_{AiAi-(m+1)}(m) calculation part 1082, an F₁₂s(i) calculation part 1083, and an F₁₂ₚ(i) calculation part 1084, and an F₁₂(i) calculation part 1085. In the Aᵢ^{k} identification part 1081, the amino-acid residue Aᵢ^{k} in the seq.k (k is an integer from 1 or more and n or less) corresponding to the amino-acid residue Ai⁰ at the position i in the seq.0 is identified by aligning seq.0 and seq.1∼seq.n. In the S'_{Ai}, S'_{AiAi+(m+1)}(m) and S'_{AiAi-(m+1)}(m) calculation part 1082, the parameters S'_{Ai}, S'_{AiAi+(m+1)}(m) and S'_{AiAi-(m+1)}(m) of the amino-acid residue Ai at the position i are obtained by an above equation. In the F₁₂s(i) calculation part 1083, the F₁₂p(i) calculation part 1084, and the F₁₂(i) calculation part 1085, respectively, F₁₂s(i), F₁₂p(i) and F₁₂(i) are obtained by the above equations, respectively.

The 35^{th} invention of the present application provides a program having a computer function as a system of the 34^{th} invention of the present application.

The 36^{th} invention of the present application provides a method of predicting a domain linker portion comprising:
i) a step for obtaining a linker degree discrimination score of an amino-acid residue Ai at a position i in an amino-acid. sequence with L₂ pieces (L₂ is an integer of 22 or more) of amino-acid residues according to the method of the 30^{th} or the 33^{rd} invention of the present application (however, a linker degree discrimination score does not have to be obtained for 0 to 50 residues at the N and C terminals of the amino-acid sequence);
ii) a step for obtaining a region predicted to take a loop structure for the amino-acid sequence by executing secondary-structure prediction;
iii) a step for obtaining a region which is predicted to take the loop structure in the secondary-structure prediction and whose linker degree discrimination score is larger than 0; and
iv) a step for predicting for each region in iii) a position
where the linker degree discrimination score becomes the maximum value as a position where the domain linker exists.

Fig. 54 shows an outline of the method of predicting a domain linker portion. In Fig., a query sequence is an amino-acid sequence of seq.0, and F(i) is a linker degree discrimination score (the above F₁, F₂(i), F₁₁(i) and F₁₂(i), for example).

The secondary structure prediction can be executed using a program such as DSC (by R. D. King, M. J. E. Sternberg (1996)) or the like.

The 37^{th} invention of the present application provides a system for predicting a domain linker portion comprising:
i) a means for obtaining a linker degree discrimination score of an amino acid residue Ai at a position i in an amino-acid sequence with L₂ pieces (L₂ is an integer of 22 or more) of amino-acid residues according to the method of the 30^{th} or the 33^{rd} invention of the present application (however, a linker degree discrimination score does not have to be obtained for 0 to 50 residues at the N and C terminals of the amino-acid sequence);
ii) a means for obtaining a region predicted to take a loop structure for the amino-acid sequence by executing secondary-structure prediction;
iii) a means for obtaining a region which is predicted to take the loop structure in the secondary-structure prediction and whose linker degree discrimination score is larger than 0; and
iv) a means for predicting for each region in iii) a position where the linker degree discrimination score becomes the maximum value as a position where the domain linker exists.

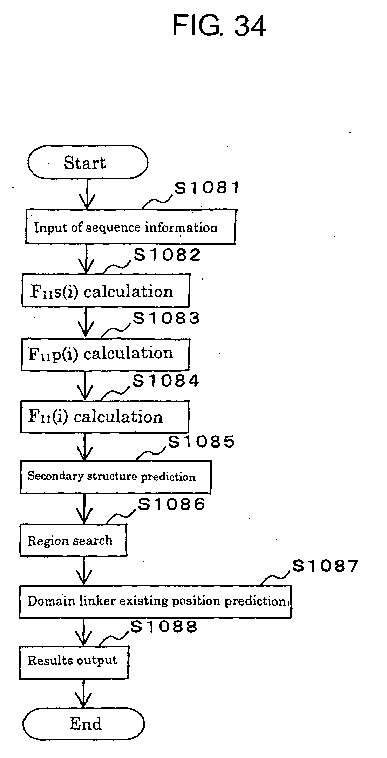

Fig. 34 is a flowchart explaining an operation of a method of predicting a domain linker portion according to a preferred embodiment of the 36^{th} invention of the present application or a predicting system for a domain linker portion according to a preferred embodiment of the 37^{th} invention of the present application.

Steps S1081 through S1084 are the same as Steps S1061 through S1064 in Fig. 30. At Step S1085, a region predicted to take a loop structure is obtained for the amino-acid sequence with L₂ pieces (L₂ is an integer of 22 or more) of amino-acid residues by executing secondary-structure prediction. At Step S1086, a region which is predicted to take the loop structure in the secondary-structure prediction and whose linker degree discrimination score is larger than 0 is obtained. At Step S1087, a position where the linker degree discrimination score becomes the maximum value is predicted as a position where the domain linker exists for each region obtained at Step S1086. At Step S1077, the result is outputted. The result output indicates, for example, the predicted sequences, the position, length, priority, e.tc. of the predicted linker sequence.

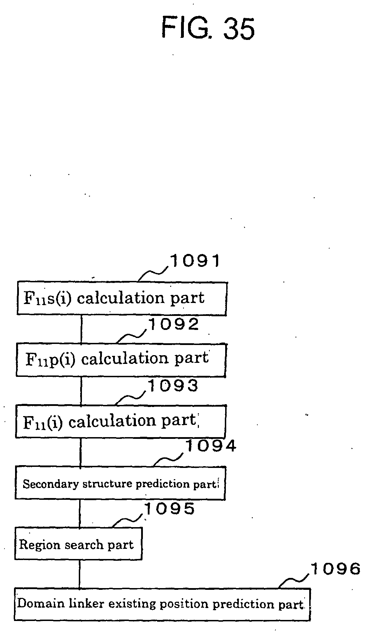

A preferred embodiment of the predicting system of a domain linker portion of the 37^{th} invention of the present application shown in Fig. 34 is realized by a computer similar to that shown in Fig. 21, which is provided with, for example, an F₁₁s(i) calculation part 1091, an F₁₁p(i) calculation part 1092, and an F₁₁(i) calculation part 1093, a secondary structure prediction part 1094, a region search part 1095 and a domain linker existing position prediction part 1096 shown in Fig. 35. The F₁₁s(i) calculation part 1091, the F₁₁p(i) calculation part 1092, and the F₁₁(i) calculation part 1093 are the same as an F₁₁s(i) calculation part 1071, an F₁₁p(i) calculation part 1072, and an F₁₁(i) calculation part 1073 in Fig. 31, respectively. In the secondary structure prediction part 1094, secondary structure prediction is executed for the amino-acid sequence with L₂ pieces (L₂ is an integer of 22 or more) of amino-acid residues, and a region predicted to take a loop structure is obtained. In the region search part 1095, a region which is predicted to take the loop structure in the secondary-structure prediction and whose linker degree discrimination score is larger than 0 is obtained. In the domain linker existing position prediction part 1096, a position where the linker degree discrimination score becomes the maximum value is predicted as a position where the domain linker exists for each region obtained in the region search part 1095.

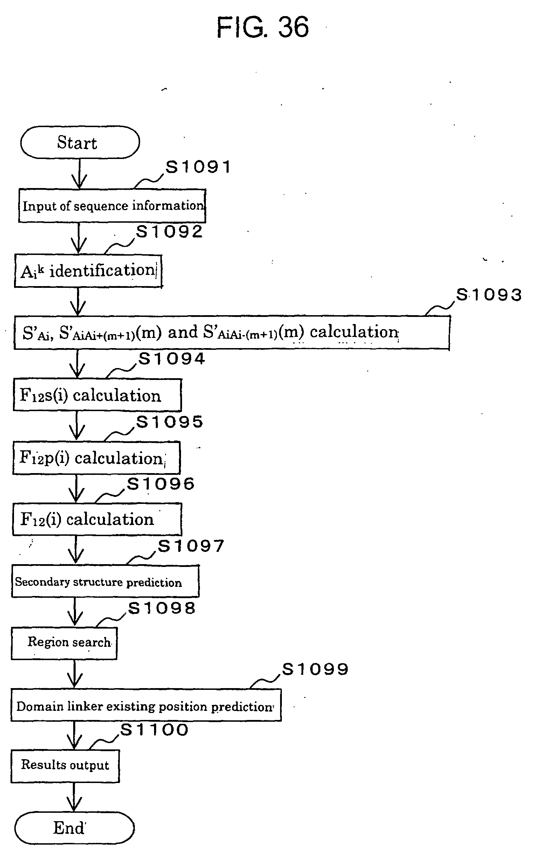

Fig. 36 is a flowchart explaining an operation of a method of predicting a domain linker portion according to a preferred embodiment of the 36^{th} invention of the present application or a predicting system for a domain linker portion according to a preferred embodiment of the 37^{th} invention of the present application.

Steps S1091 through S1096 are the same as Steps S1071 through S1076 in Fig. 32. Steps S1097 through S1100 are the same as Steps S1085 through S1088 in Fig. 34.

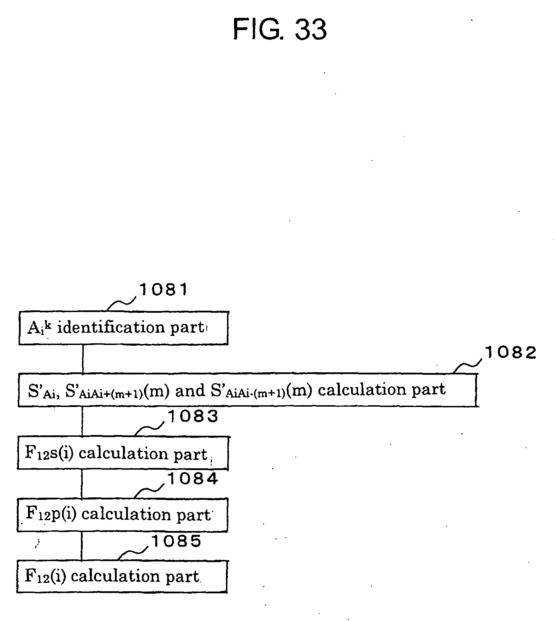

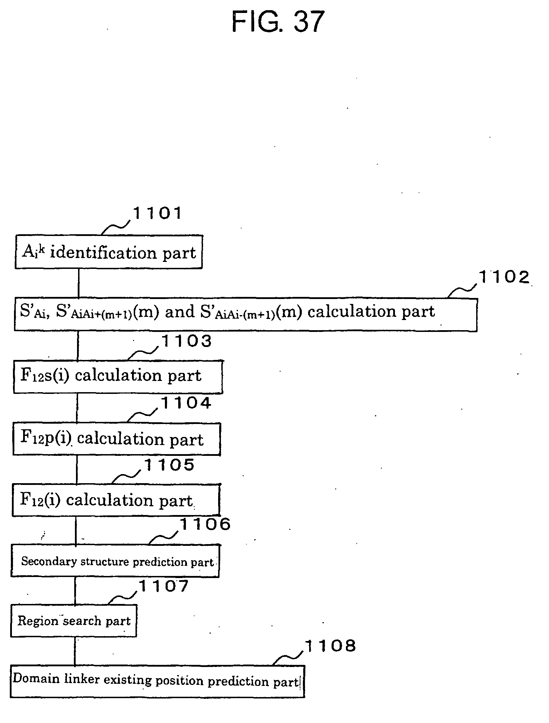

Another preferred embodiment of the predicting system of a domain linker portion of the 37^{th} invention of the present application shown in Fig. 36 is realized by a computer similar to that shown in Fig. 21, which is provided with, for example, an Aᵢ^{k} identification part 1101, an S'_{Ai}, S'_{AiAi+(m+1)}(m) and S'_{AiAi-(m+1)}(m) calculation part 1102, an F₁₂s(i) calculation part 1103, and an F₁₂p(i) calculation part 1104, an F₁₂(i) calculation part 1105, a secondary structure prediction part 1106, a region search part 1107, and a domain linker existing position prediction part 1108 shown in Fig. 37. The Aᵢ^{k} identification part 1101, the S'_{Ai}, S'_{AiAi+(m+1)}(m) and S'_{AiAi-(m+1)}(m) calculation part 1102, the F₁₂s(i) calculation part 1103, and the F₁₂p(i) calculation part 1104, the F₁₂(i) calculation part 1105 are the same as the Aᵢ^{k} identification part 1081, the S'_{Ai}, S'_{AiAi+(m+1)}(m) and S'_{AiAi-(m+1)}(m) calculation part 1082, the F₁₂s(i) calculation part 1083, and the F₁₂p(i) calculation part 1084, the F₁₂(i) calculation part 1085 in Fig. 33, respectively. The secondary structure prediction part 1106, the region search part 1107, and the domain linker existing position prediction part 1108 are the same as the secondary structure prediction part 1094, the region search part 1095, and the domain linker existing position prediction part 1096 in Fig. 35, respectively.

The 38^{th} invention of the present application provides a program for having a computer function as a system of the 37^{th} invention of the present application.

The 39^{th} invention of the present application provides a method of constructing an amino-acid sequence database comprising:
i) a step for obtaining a linker degree discrimination score of an amino-acid residue Ai at a position i in an amino-acid sequence with L₂ pieces (L₂ is an integer of 22 or more) of amino-acid residues according to the method of the 30^{th} or the 33^{rd} invention of the present application (however, a linker degree discrimination score does not have to be obtained for 0 to 50 residues at the N and C terminals of the amino-acid sequence);
ii) a step for obtaining a region predicted to take a loop structure for the amino-acid sequence by executing secondary-structure prediction;
iii) a step for obtaining a region which is predicted to take the loop structure in the secondary-structure prediction and whose linker degree discrimination score is larger than 0;
iv) a step for selecting a region from those obtained in iii) whose maximum value of the linker degree discrimination score is larger than a lower limit value; and
v) a step for recording an amino-acid sequence of a region selected in iv) in a recording medium.

The lower limit value in the step iv) is preferably any value not less than 0, and preferably any value from 0.0 to 1.0.

In the step v), as a recording medium for recording the amino-acid sequence of a region selected in iv) may be a magnetic tape, cassette tape, flexible disk, hard disk, CD-ROM, MO / MD / DVD, etc. or semiconductor memory.

The 40^{th} invention of the present application provides a domain linker peptide made of an amino-acid sequence which is the same as the amino-acid sequence in a region whose maximum value of a linker degree discrimination score is larger than a lower limit value, obtained from a method comprising:
i) a step for obtaining a linker degree discrimination score of an amino-acid residue Ai at a position i in an amino-acid sequence with L₂ pieces (L₂ is an integer of 22 or more) of amino acid residues according to a method of the 30^{th} or the 33^{rd} invention of the present application (however, a linker degree discrimination score does not have to be obtained for 0 to 50 residues at the N and C terminals of the amino acid sequence);
ii) a step for obtaining a region predicted to take a loop structure for the amino-acid sequence by executing secondary-structure prediction;
iii) a step for obtaining a region which is predicted to take the loop structure in the secondary-structure prediction and whose linker trend discrimination score is larger than 0; and
iv) a step for selecting a region from those obtained in iii) whose maximum value of the linker degree discrimination score is larger than the lower limit value.

The 41^{st} invention of the present application provides a method of predicting a structural domain comprising a step for predicting, concerning an amino-acid sequence with L₂ pieces (L₂ is an integer of 22 or more) of amino-acid residues, a sequence fragment generated by cutting off the amino-acid sequence at any portion of a region including a domain linker portion or a domain-linker existing position predicted by the method of the 36^{th} invention of the present application as a structural domain. In this 41^{st} invention of the present application, if n pieces of domain linker portions are predicted, t piece(s) (t is an integer from 1 or more to n or less) among them is (are) selected, all the patterns for cutting an amino acid sequence at that position are considered, and all the obtained sequence fragments may be predicted as structural domains.

The 42^{nd} invention of the present application provides a system for predicting a structural domain (hereinafter referred to as "structural domain predicting system") comprising a means for predicting, concerning an amino-acid sequence with L₂ pieces (L₂ is an integer of 22 or more) of amino-acid residues, a sequence fragment generated by cutting off the amino-acid sequence at any portion of a region including a domain linker portion or a domain-linker existing position predicted by the method of the 36^{th} invention of the present application as a structural domain.

The structural domain may be those existing in a multi-domain protein.

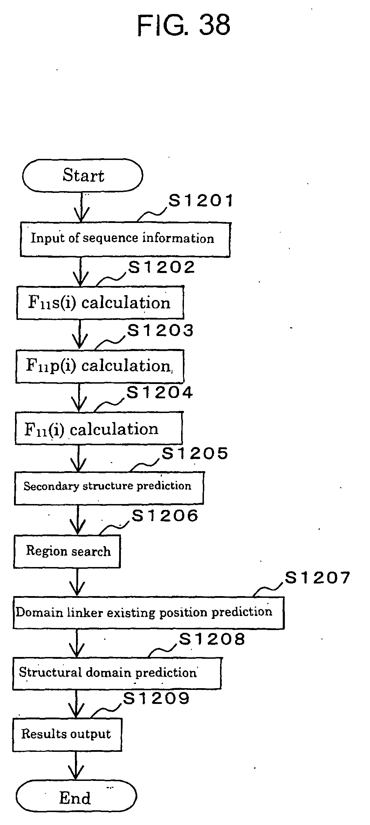

Fig. 38 is a flowchart explaining an operation of a structural domain predicting system according to a preferred embodiment of the 42^{nd} invention of the present application.

Steps S1201 through S1207 are the same as Steps S1081 through S1087 in Fig. 34, respectively. At Step S1208, a sequence fragment generated by cutting off the amino-acid sequence with L₂ pieces (L₂ is an integer of 22 or more) of amino-acid residues at any portion of a region including a domain linker portion or a domain-linker existing position predicted at Step S1207 is predicted as a structural domain. At Step S1209, the result is outputted. The result output indicates, for example, predicted amino-acid sequences, position and size of the predicted linker sequence, etc.

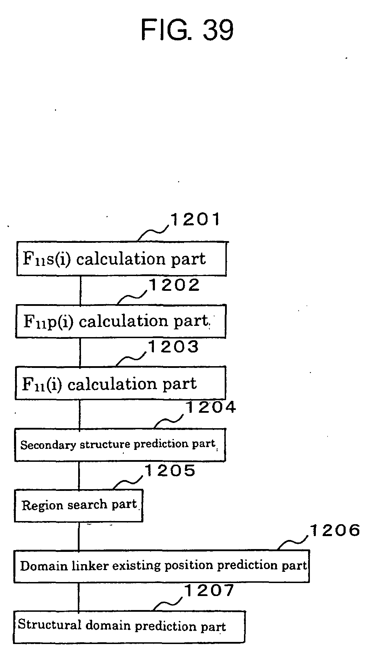

A preferred embodiment of the structural domain predicting system of the 42^{nd} invention of the present application shown in Fig. 38 is realized by a computer similar to that shown in Fig. 21, which is provided with, for example, an F₁₁s(i) calculation pant 1201, an F₁₁p(i) calculation part 1202, and an F₁₁(i) calculation part 1203, a secondary structure prediction part 1204, a region search part 1205, a domain linker existing position prediction part 1206 and a structural domain prediction part 1207 shown in Fig. 39. The F₁₁s(i) calculation part 1201, the F₁₁p(i) calculation part 1202, and the F₁₁(i) calculation part 1203, the secondary structure prediction part 1204, the region search part 1205, and the domain linker existing position prediction part 1206 are the same as the F₁₁s(i) calculation part 1091, the F₁₁p(i) calculation part 1092, and the F₁₁(i) calculation part 1093, the secondary structure prediction part 1094 and the region search part 1095 in Fig. 35, respectively. In the structural domain prediction part 1207, a sequence fragment generated by cutting off the amino-acid sequence with L₂ pieces (L₂ is an integer of 22 or more) of amino-acid residues at any portion of a region including a domain linker portion or a domain-linker existing position predicted in the domain linker existing position prediction part 1206 is predicted as a structural domain.

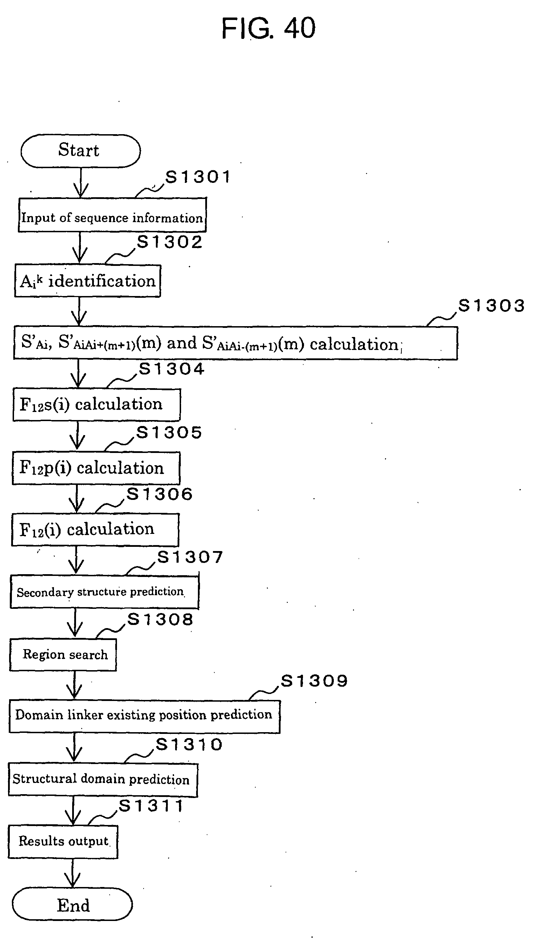

Fig. 40 is a flowchart explaining an operation of a system for predicting a structural domain according to another preferred embodiment of the 42^{nd} invention of the present application.

Steps S1301 through S1309 are the same as Steps S1091 through S1099 in Fig. 36, respectively. Steps S1310 through S1311 are the same as Steps S1208 through S1209 in Fig. 38, respectively.

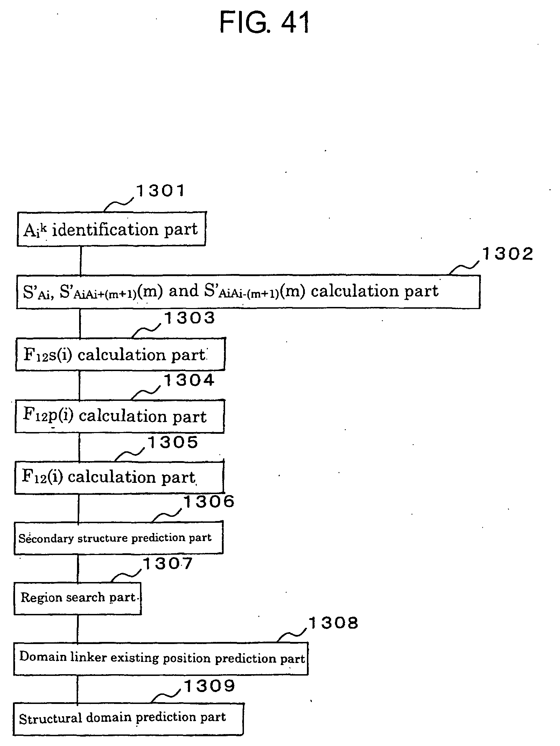

Another preferred embodiment of the structural domain predicting system of the 42^{nd} invention of the present application shown in Fig. 40 is realized by a computer similar to that shown in Fig. 21, which is provided with, for example, an Aᵢ^{k} identification part 1301, an S'_{Ai}, S'_{AiAi+(m+1)}(m) S'_{AiAi-(m+1)}(m) calculation part 1302, an F₁₂s(i) calculation part 1303, and an F₁₂p(i) calculation part 1304, an F₁₂(i) calculation part 1305, a secondary structure prediction part 1306, a region search part 1307, and a domain linker existing position prediction part 1308 and a structural domain prediction part 1309 shown in Fig. 41. The Aᵢ^{k} identification part 1301, the S'_{Ai}, S'_{AiAi+(m+1)}(m) and S'_{AiAi-(m+1)}(m) calculation part 1302, the F₁₂s(i) calculation part 1303, and the F₁₂p(i) calculation part 1304, the F₁₂(i) calculation part 1305, the secondary structure prediction part 1306, the region search part 1307 and the domain linker existing position prediction part 1308 are the same as the Aᵢ^{k} identification part 1101, the S'_{Ai}, S'_{AiAi+(m+1)}(m) and S'_{AiAi-(m+1)}(m) calculation part 1102, the F₁₂s(i) calculation part 1103, and the F₁₂p(i) calculation part 1104, the F₁₂(i) calculation part 1105, the secondary structure prediction part 1106, the region search part 1107, and the domain linker existing position prediction part 1108 shown in Fig. 37. The structural domain prediction part 1309 is the same as the structural prediction part 1207 in Fig. 39.

The 43^{rd} invention of the present application provides a program for having a computer function as a system of the 42^{nd} invention of the present application.

The 44^{th} invention of the present application provides a method of constructing an amino-acid sequence database comprising a step for recording in a recording medium, concerning an amino-acid sequence with L₂ pieces (L₂ is an integer of 22 or more) of amino-acid residues, the amino-acid sequence of a sequence fragment generated by cutting off the amino-acid sequence at any portion of a region including a domain linker portion or a domain-linker existing position predicted by the method of the 36^{th} invention of the present application.

The 45^{th} invention of the present application provides a method of manufacturing a protein comprising a step for manufacturing a protein having the same amino-acid sequence as the structural domain predicted by the method of the 41^{st} invention of the present application.

The 46^{th} invention of the present application provides a method of analyzing a protein comprising a step for analyzing a protein having the same amino-acid sequence as the structural domain predicted by the method of the 41^{st} invention of the present application.

The 47^{th} invention of the present application provides a method of manufacturing a protein comprising designing a new multi-domain protein which is a domain linker peptide of the 40^{th} invention of the present application and is generated by connecting at least 2 protein fragments and manufacturing this multi-domain protein.

As above, the present invention is constituted by a first method using a neural network as in the 1^{st} to the 17^{th} inventions and a second method using statistical processing of occurrence frequency of an amino acid as in the 18^{th} to the 47^{th} inventions, and it is preferable that those methods are used in the complementary manner in identification of a linker. That is, even if a correct prediction result can not be obtained with the first method for a region to be predicted, there is a case that a correct answer can be derived if the second method is used, and vice versa. Also, by checking the results of the both, more reliable linker identification can be achieved. In any case, by combining these methods for various prediction candidates, a domain linker region in a protein can be correctly identified at the probability of about 65%.

The present invention will be explained in detail according to the embodiments. These embodiments are only for illustration of the present invention and do not limit the scope of the present invention.

### [Embodiment 1] Characterization and prediction of a linker sequence by neural network

### Result

### (a) Domain sequence analysis

First, it was examined if local sequence characteristics exist in a domain linker and if they can be extracted by a neural network. Segments derived from a multi-domain protein are classified into "linker sequence" and "non-linker sequence" depending on whether the amino-acid residue at its center is included in the domain linker or not (See the section on materials and methods). These classified sequences were used for learning of the neural network.

### Optimization of learning conditions

Here, the conditions by which the neural network is efficiently trained were examined, and the size of the window (Table 2a) and the number of hidden units (Table 2b) were optimized so as to achieve the maximum learning effect.

The effect of the window size was evaluated by the proportion of the number of times of correct classification of linkers and non-linkers against the number of times of wrong classification. The result in Table 2a shows that the correct answer rate is slightly lowered with increase of the window size, while the correct answer rate of the linker sequence rises up to the window size 19 and then, gradually drops. This fact indicates that most of the characteristics of the sequences required for identification of the domain linker is included in 19 amino-acid residues. In the meantime, the drop in the correct answer rate of the linker sequence was found in the window size not less than 19 as with the drop in the correct answer rate of the non-linker sequence. This drop does not relate to the total of the characteristics of the sequences. That is because the once the window reaches a size enough to include all the characteristics of the sequence, the correct answer rate becomes constant but does not drop. We assumed that this drop was caused by the increase of the number of parameters brought into a larger window size, and the data set of the limited size would prevent the neural network from operating in the optimum state with the larger window size. Here, as the optimum condition, the window size of the 19 amino-acid residues was adopted.

We further examined the effect of the number of hidden units (Table 2b). In theory, the neural network in the case where there are not any hidden units can detect only independent contribution of each amino acid to the domain linker (first order features). When the hidden units are brought into, the ability of neural network to extract higher-level characteristics such as a relation between an amino-acid pair and the domain linker, for example, is improved (Qian & Sejnowski, 1988). However, in our research, increase of the number of hidden units did not remarkably improve the learning effect (Table 2b). The reason why the learning efficiency was not improved can be briefly explained by non-existence of higher-level characteristics in the linker sequence. However, as with the observation of the window size, the learning effect might be affected by reduction of the data size and too many parameters. Considering the calculation time or the fact that there is no effect even after introduction of many parameters, we decided to use the neural network with the number of hidden units set to 0 or 2 (zero means a two-layer network).

### Effect of the size of data set in learning

In order to evaluate how the size of the data set affects the learning effect, we examined if the correct answer rate depends on the size of the training data set or not. The correct answer rate of linker sequence classification did not become flat even after the current data set got large (Table 2c), it is expected that the learning efficiency will be improved if more data is available. In other words, the data set used here is not sufficient to fully extract the characteristics of the domain linker. However, despite these limitations, the characteristics of the detectable linker sequences could be extracted using the neural network, which will be described below.

### Identification of linker sequence and non-linker sequence

The ability of the neural network to identify the linker and the non linker can be examined by distribution of output values of these neural networks (Fig. 1). We calculated output values of the linker sequences and the non-linker sequences and averaged these values over the smoothing window of 19 residues. The distribution of output values of the linker sequences were obviously different from the distribution of the output values of the non-linker sequences even though there are some overlaps (white and black bar graphs respectively in Fig. 1). The output values of the linker sequences tend to be higher (those with the output values distributing above 0.4 amount to 60.3% of the entire linker sequences), while the non-linker sequences and the in-domain loops indicate lower values (those with the output values of 0.2 or less are 59.1% and 53.3%, respectively).

### Characterization of the linker sequence

The characteristics on the sequence extracted from the two:layer neural network can be visualized using the Hinton diagram (Rumelhart et al.,1986) (Fig. 2). In the case of the two-layer network, the respective weight parameter values are explained as contribution of a corresponding amino-acid residue to the difference between the linker sequence and the non-linker sequence (type of the amino acid and the position in the window). We observed that there is a high correlation between these weight parameters and the occurrence frequency of an amino acid at the respective position (no data shown). The Hinton diagram obviously indicates that proline is a strong determinant amino-acid residue. This fact matches the result of the amino-acid composition analysis (occurrence frequency of proline is 13.9% in the domain linker and 5.3% in the whole data). However, the characteristics depending on the position are also observed for the other residues whose content in the domain linker is almost equal to the content in the whole data set. For example, a histidine residue indicates obviously negative distribution at the C terminal, but this position corresponds to the C terminal of the domain linker, that is, the N terminal of the subsequent domain. Methionine, isoleucine, tyrosine and tryptophan also show negative distribution. In general, hydrophobic amino acids tend to show negative distribution, while hydrophilic amino acids contributes on the positive side. These results highlight the ability to efficiently extract characteristics of the sequence not known from the averaged amino-acid composition value with a neural network.

### Proline-rich segment

As observed both in the amino-acid composition and the Hinton diagram, the domain linker has a characteristic of highly frequent occurrence of proline (the average number of proline residues in a domain linker is 1.65). However, some in-domain sequences also have portions with locally high proline content. Then, we assumed that the difference between the linker sequence and the non-linker sequence is the contents of other amino acids. We examined the characteristics of a short segment including at least 3 prolines in 9 residues (proline-rich segment). Most of the proline-rich segments belong to the in-domain region (50 in in-domain region against 26 in the domain linker), and most of them overlap the in-domain loop region. Figs. 2b and 2c show all the proline-rich segments corresponding to the domain linker and the in-domain region, respectively, with the sequence of the 9 residues adjoining to the both ends. Interestingly, the domain linkers in the proline-rich segment and its adjoining sequences rarely include histidine (Fig. 2b). On the other hand, in the sequence located in the domain, histidine occurs relatively frequently (Fig. 2c). For example, though there are only 5 residues of histidine in the former sequence, while 38 residues are observed in the latter. Moreover, there are many histidine located at the C terminal of the sequence belonging to the in-domain region (against 13 of them on the half of the N terminal side, there are 25 on the half of the C terminal side). These evidences verify the characteristics found in the Hinton diagram and shows that histidine is an important clue in identification of the domain linker and the in-domain loop regions.

### (b) Prediction of domain linker in sequence of protein

In this section, the ability of a neural network to predict a domain linker in an amino-acid sequence of a protein will be examined. First, a neural network having learned with the window size of 19 and the number of hidden units of 2 was used, and an output value of a protein to be examined was calculated. In order to convert the output of the neural network to prediction, the following three parameters were introduced: (1) Size of a smoothing window: The size of a window is determined, and output values exceeding this size are excluded (smooth). (2) Cut-off value: A peak is selected from the smoothed output values. (3) Threshold: A start position and an end position of a linker around the peak are determined.

### Efficiency of prediction

The efficiency of prediction was evaluated by measuring two values. One of them is a percentage indicating a proportion of a predicted region correctly assigned to a SCOP derived domain linker in all the predicted regions (specificity). (How many of predicted regions match those originally determined by SCOP as a domain linker). The other is a proportion of SCOP derived domain correctly predicted by the neural network in all the SCOP derived domain linkers (sensitivity). We examined the specificity and the sensitivity by changing two prediction parameters: size of the smoothing window and the cut-off value. The best prediction was achieved when the size of the smoothing window was fixed to 19 and the cut-off value to 0.5. Under these conditions, the specificity of the prediction was 58.8%, and the sensitivity of the prediction was 35.6% (Figs. 3a, b).

Next, we examined how the parameters of the cut-off value and the threshold value affect the prediction efficiency (Table 3). With increase of the cut-off value, the specificity rose, while the sensitivity dropped (Figs. 3a, b). In this way, the cut-off value parameter controls trade-off between the specificity and the sensitivity of prediction. On the other hand, when the threshold value is decreased, both the specificity and the sensitivity increase. This can be explained by allowance in assignment of candidate regions. This is controlled by the threshold value parameter; If the threshold value is low, the length of a predicted linker would be longer than the case where the threshold value is high. These results show that the cut-off value and the threshold value should be selected so that the balance between the specificity and the sensitivity should be desirable and that allowance in assignment of candidate regions should be desirable. In the following prediction, the value of 0.5 was used both for the cut-off value and the threshold value.

### Linker ranking

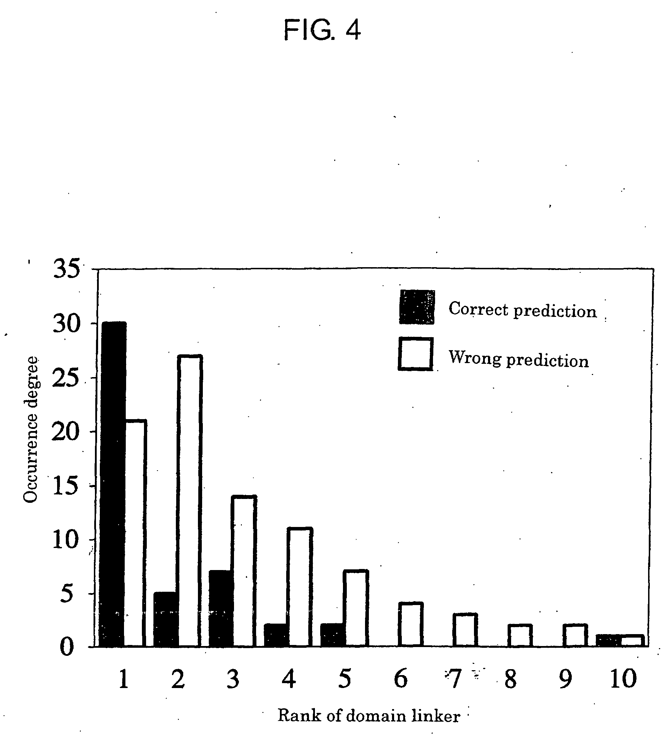

As mentioned in the section on materials and methods, we ranked the predicted candidate linkers according to their maximum smoothed output values. The correctly predicted candidate linkers were ranked at the first with preference (63.8% of all the correctly predicted candidate linkers ranked at the first), and there were few cases ranked lower (black bar graph in Fig. 4). Moreover, the candidate regions in the lower rank had wrong prediction in many cases (white bar graph in Fig. 4). These results support interrelation between our ranking and actual domain linker entity and show that selection of a sequence in the first rank can raise the specificity of prediction.

### Comparison with other methods

In order to evaluate the ability of a neural network to predict a domain linker, comparison was made with other prediction methods. A standard domain linker prediction method has not been established yet, and a simple method using secondary structural prediction was compared with our method. Here, our method is based on an intuitive assumption that a domain linker is a long loop region, and the nature of those domain linkers were ranked according to the predicted length. Also, both the specificity and the sensitivity of prediction derived from DSC or PHD were lower than the respective values obtained by the neural network by at least 10%. Moreover, the length of the predicted loop has little relation with the nature of the domain linker (Fig. 3c). These results with data shown in Fig. 2 indicate that the domain linker has a nature different from the in-domain loop region and that the nature can be distinguished by the neural network.

### Example of domain linker prediction

In Figs 5a, b, an example of correct prediction by a neural network is shown. The neural network predicted one linker in collagenase (1fb1). This was correctly assigned to a SCOP derived domain linker. For serine tRNA synthetase (1 sesA), endo / exo-cellulose E4 catalyst domain and cellulose bound domain (1ft4B), in addition to a true positive linker, a false positive linker was predicted, but when only linkers in the first rank were selected, the false positive were eliminated (Figs. 5b, c). Pyroracemic acid decarboxylase (1pvdA) has three domains, and a linker dividing these domains was predicted from the first and the second rank linkers. Actually, the region extending from the amino-acid residue positions 183 to 193 (specified in PDB) (corresponding to 174-202 in Fig. 5) was not a domain linker originally, because the domain boundary defined in SCOP is located at the center of a 3-10 helix region. Despite this fact, the neural network identified this segment as a linker.

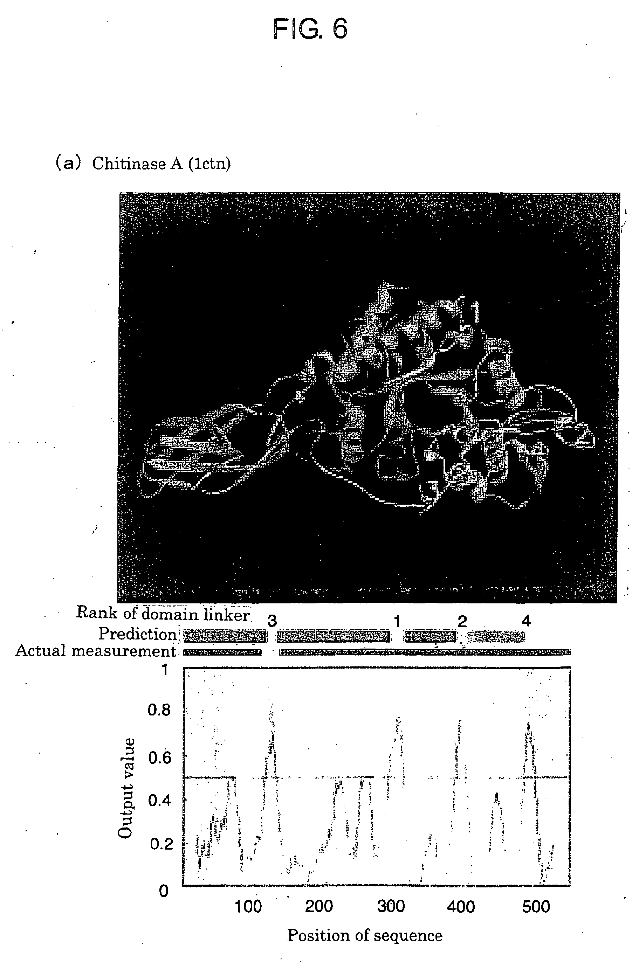

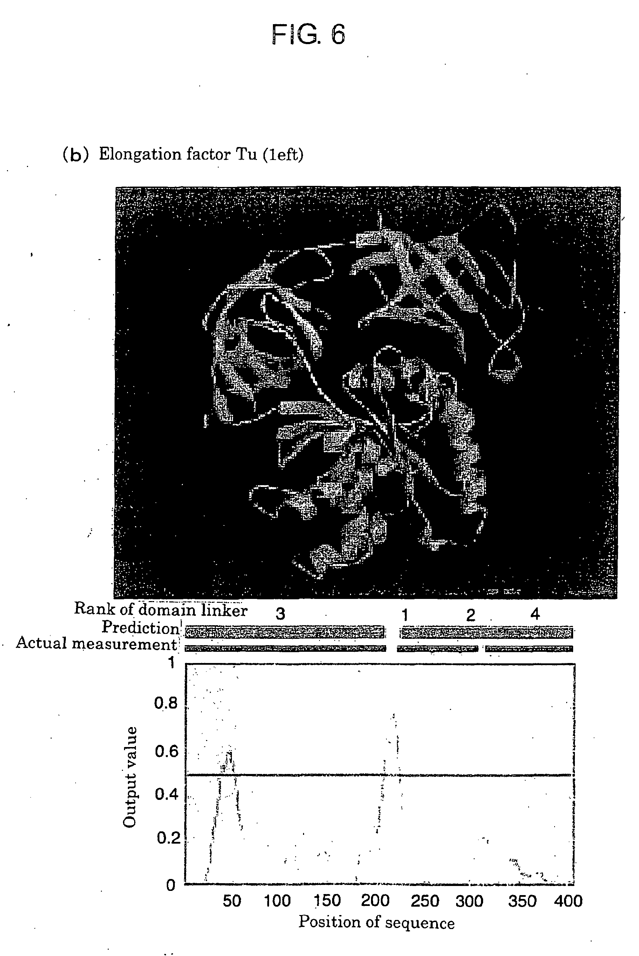

As shown in Fig. 3b, some of the observed domain linkers were not correctly predicted by the neural network. Chitinase A (1ctm) is an example that prediction was not successful. In this case, a false signal was prevailing over a true signal corresponding to a SCOP derived domain linker (Fig. 6). For some short domain linkers, output of the neural network is a weak signal or it does not put out any signal.

### Consideration

In an actual protein, since the size and structure of a domain linker are varied, definition for the domain linker is not always only one. For example, in addition to our definition, there can be definitions based on visual figures and movement of the domain. Therefore, classification of domain linkers into various types will be useful in comprehensive characterization of linker sequences. However, in our study, since the size of the data set was small, types of linkers were not analyzed in detail. Instead, a limited definition of domain linker (loop region adjacent to a domain which is structurally independent and is considered to be automatically folded) was employed. This narrow definition of domain linker seems to be suitable for recognition of characteristics of linkers by neural networks since it limits sequence patterns in the data set. However, as expected from Table 2c, if more structural data on multi-domain proteins are available in the future, the size of the data set will be larger and more detailed analysis will be enabled on more types of linker sequences.

Sequence patterns in a domain linker are suggested in the Hinton diagram (Fig. 2a). In the learning process of the neural network, the characteristics of sequences are averaged for all the linker sequences used for learning. As a result, sequences specific to individual domain linkers become inevitably vague and will not appear on the Hinton diagram. Despite that, we found characteristic occurrence patterns for some amino acids including proline and histidine. This means that the linker sequences have common local characteristics. Considering that the amino-acid composition limits characteristics to distinguish a domain linker from other regions, this result should be surprising. Actually, the local characteristics of the sequence detected by our neural network had high interrelation with occurrence frequency at each amino-acid residue position in the window. As a whole, this discovery strongly suggests that the linker sequence is characterized not only by the contents of the amino acid but its occurrence pattern in the sequence.

The Hinton diagram shows that a histidine residue is mandatory as a proline residue in discriminating a domain linker from other regions (Fig. 2a). Sequence analysis of a proline-rich segment explains a difference in occurrence frequency of histidine between the domain linker and other regions, especially with in-domain loop (Figs. 2b, c). Our prediction succeeded probably and partially because of recognition of the histidine residue by the neural network. In Figs. 2b, 2c, since the proline-rich segment has high proline content, an output value of the neural network is higher than general. However, the proline-rich segment including histidine tends to show a lower output value, and there is a strong correlation between the histidine content and the neural network output value (2b, 2c). Referring to other examples, the sequence of ifbl is (164-198, position of residue in PDB / 65-99 for the position used in Fig. 5a) including two proline-rich segments and (253-284, 154-185). The former sequence is characterized by high histidine content, while the latter does not include histidine. The neural network gives a smoothed output value lower than 0.46 to the former and a value higher than 0.62 to the latter. In this way, the position of a domain linker is correctly determined.

Assumption of a structural information amount accumulated in a local sequence is derived from prediction efficiency. In the case of blind prediction, that is, prediction without any information is roughly estimated as follows. Assume the case where a protein of amino-acid residue 300 made of two domains and the average domain size is 150. In our data set, the average domain linker size is 12.2 residues. Also, the minimum domain size is 60 residues, and when assuming that 60 residues on both ends of the protein sequence are not included in our calculation, the blind prediction gives a correct answer rate of 7% (12.2 / 300 - 60x2). On the other hand, in our study, the prediction efficiency of the neural network was 35.6% for the sensitivity and 58.8% for the specificity (Figs. 3a, 3b). In any case, improvement in efficiency from the blind prediction to the prediction by neural network (about 30 to 50%) is attributable to the structural information accumulated in the local sequence. In this way, this assumption indicates that the local sequence information can be a useful clue in detecting a domain linker. However, it also indicates that a major portion of the domain linker information is not local at the same time, and to further improve prediction, information which is not local should be taken in. Despite that, our neural network is one of rare means which can be used for detecting a virtual domain linker in sequences of a protein and has a possibility to contribute to structural and functional analysis of a large protein.

### Materials and methods

### Preparation of data

Multi-domain proteins whose structure was analyzed with resolution of 2.5 A or more and classified in SCOP database were selected from PDB (Protein Data Base). Duplication of sequences were eliminated according to the BLAST standard with the value of e of 10 • -70 (The most homologous sequences were 49% (1hyxH and 2fbjH).).

The domain linker was defined as follows. First, as determined by DSSP, a domain linker is considered to be a loop region made of at least 4 residues and include domain boundary defined by SCOP. Most of actual domain linkers corresponded to a single loop region, but in a few exceptions, it had plural loop regions in which short secondary structural elements are scattered. In these cases, not all the loop regions corresponding to them were considered as domain linkers but the only loop region was first made as a domain linker. Therefore, at the next stage of visual inspection, in order to encompass all the domain linkers, we expanded the determined region manually. Then, all the structures of the domains whose range was determined by the above defined domain linker were visually inspected. Since the SCOP definition of domain is based on the evolutionarily stored structural units, it does not match our necessary condition on the domain structure. Actually, in some multi-domain proteins, it was obviously observed that domains closely adhere to each other (e.g.: D amino-acid oxidase). Also, it seems that these SCOP defined domains can not be folded to their original structure when isolated. Moreover, we found that this ambiguity in the domain definition or domain linker definition accompanying it prevents progress of learning by a neural network. Thus, we visually examined the structure of each protein and selected only domain linkers adjoining the domain considered to take its original structure by individually and autonomously being folded. As a result, we obtained 99 domain linkers (SCOP derived) existing in 74 types of multi-domain protein. Neural network

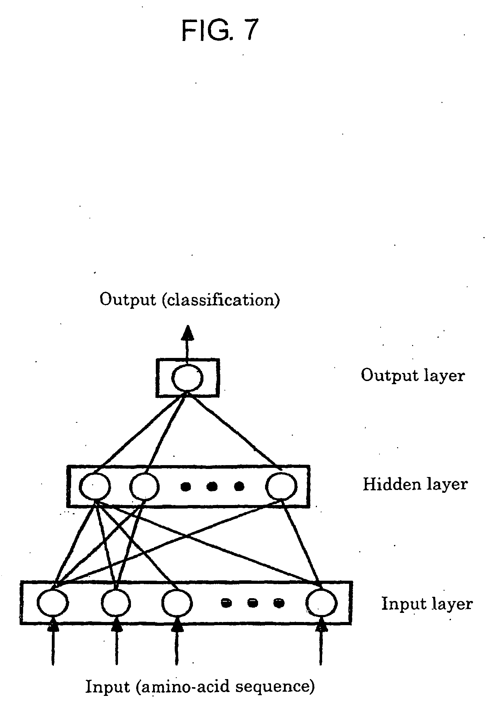

The neural network is a method for pattern recognition, and layered feed forward networks relate to input and output. The network is optimized using the back propagation algorithm so as to obtain desired input / output relations. This process is called as learning or training (for detailed explanation, see documents by Rumelhalt). In our study, in order to classify sequence segments, a neural network having a single hidden layer (Fig. 7) and a neural network having no hidden layer were used. In the learning process of the neural network, a sequence segment coded by binary system was given as an input pattern, classification of these sequence segments into the linker sequence or the non-linker sequence was made as output of 1 or 0, respectively. In this learning process, we used momentum term set to 0.9 (for predicate, Rost & Saunder was followed), and parameters of bias and weight were set in a range at random [-0.3, 0.3]. Magnitude of learning (that is, a step width of gradient drop) was made as 0.001 for the first 100 learning stages and 0.005 for the next stage. In all the stages, a correct answer rate of sequence classification was checked, and when the correct answer rate reached a peak value, the learning was stopped. In checking the correct answer rate of classification, it was considered that the case where the output value (predicted value) of neural network is not less than 0.5, it was classified to the linker sequence, while the value not more than that was classified to the non-linker sequence, and the correct answer rate was examined.

The back propagation algorithm was written in the C language, and Fujitsu's VPP700E super computer at Wako Campus, Riken was used.

### Training

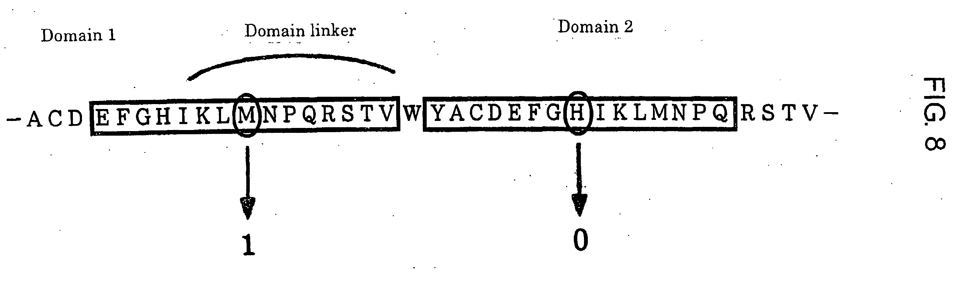

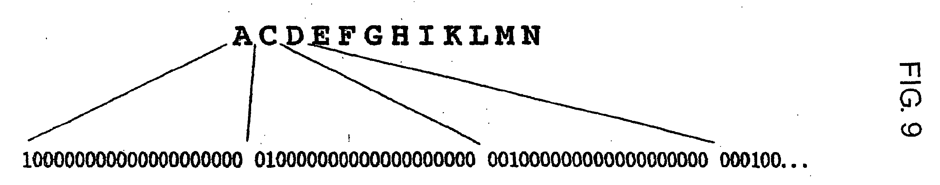

In order to extract domain linker information, we trained the neural network so that it discriminates domain linkers from non-linker sequence segments. Sequence segments of the length equal to a given window size were moved from the N terminal to the C terminal of a protein sequence and collected. Each of the sequence segments was classified to the linker sequence or the non-linker sequence according to whether the residue at its center is a part of the domain linker or not (Fig. 8). We proceeded with training using the linker sequence and the non-linker sequence at the proportion of 1:3. With this proportion, the linker and the non-linker can be discriminated most efficiently. The sequences were clearly coded. That is, each amino acid in the sequence segment was converted to 21-bit binary numbers (Fig. 9). Each bit corresponds to 20 standard amino-acid residues with the remaining corresponding to the one that can not specify an amino acid or that is not a standard amino acid. For example, the code of alanine is 100000000000000000000. In the classification of sequence, the linker was coded as 1, while the non-linker as 0.

### Test

For evaluation of learning efficiency of neural network, two methods were used. One is a single testing method, and data sets are merely divided into 2 groups, one of which is used for training and the other for testing. The proportion of data set for training to that for testing was set at 4:1. The second method is a 10-fold Jackknife test. In this method, the data set was divided into 10, in which data from 9 groups was used for learning of neural network, while the other was used to examine learning efficiency of data. This process was repeated 10 times till all the groups were used for the test. Prediction of domain linker by neural network

The first stage of linker prediction is to calculate an output value of neural network for sequence of the examined protein. Using the optimized 19-residue window, we calculated the output value of each residue in the protein sequence, and the value was made as a characteristic of the amino acid at the center of the window. Since this raw output value is extremely varied along the sequence of a protein, reliable prediction of the domain linker region was prevented. Thus, an averaged output value of the 19 residues (averaging over the 9 residues before and after) was used for the domain linker (For optimization of smoothing of this window, see the section on results).

We made the following three-stage prediction. (1) First, we assume the minimum size of a domain and ignored 60 residues at both ends of the protein. (2) We selected all the peaks from smoothed output values larger than a cut-off value. Then, a region close to the peak value having a smoothed output value larger than a threshold value was defined as a virtual domain linker (note that the cut-off value is larger or equal to the threshold value). (3) Lastly, the predicted domain linkers were ranked according to the peak value of smoothed output value (Figs 5, 6, for example). In order to evaluate prediction using this method, the Jackknife test was carried out for the data set of multi-domain proteins. Since various sequence patterns were required for training of neural network, we used the data set selected by the e value of 10⁻⁷⁰ for training. However, this data set includes sequences similar to each other, and it might affect evaluation of prediction. Then, we eliminated the sequences having the identity of full length smaller than the e value of 10⁻²⁰ (this corresponds to the fact that more than 25% of the sequences are identical) (Shown in Table 1). In the end, prediction efficiency was calculated for the set of 66 multi-domain proteins including 87 domain linkers.

### [Embodiment 2] Setting of threshold value of output value (g(X)) of neural network

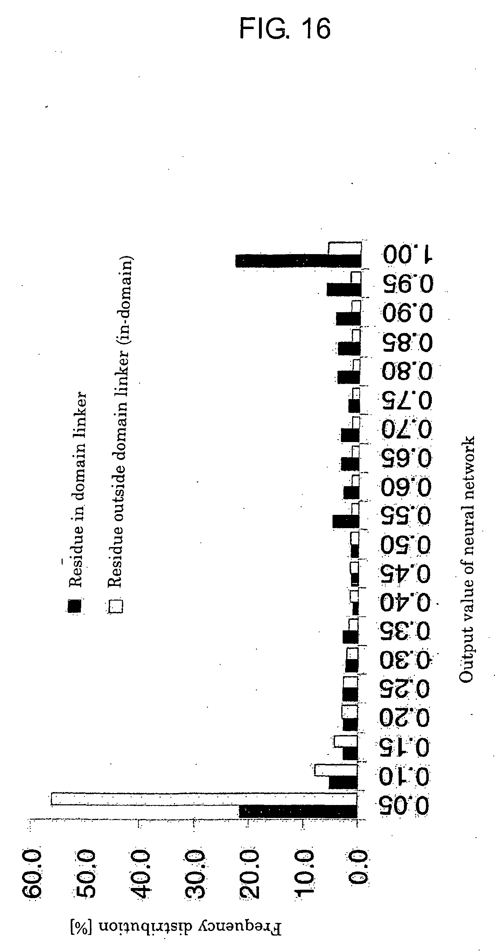

For the protein sequence of the test data used in Embodiment 1, a window of 19 residues was taken and the sequence fragment of the length of 19 residues was given to the neural network to calculate an output value (a value of 0.0 - 1.0 was obtained, and this becomes the output value for the residue at the center of the window.). The window was sequentially displaced from the N terminal to the C terminal of the protein, and output was calculated at each position. In preparing distribution, cases are classified depending on whether the residue at the center of the window is a domain linker or not, and the respective distributions were obtained. The neural network used here has three layers, and the number of the hidden units was 2. Also, distribution was obtained by the jackknife test. The results is shown in Fig. 16.

### [Embodiment 3] Preparation of domain linker database

For 86593 amino-acid sequences registered in SWISSPROT whose structure is totally unknown, prediction was made according to the method in Embodiment 1. The used neural network has three layers, and the number of hidden units was 2.

Also, prediction was (independently) made with (10 in total) neural networks optimized using 10 pieces of learning data (prepared for the Jackknife test), and the obtained 10 smoothing output values were averaged. In this averaging, the length of the smoothing window (smoothing window length) was set at 19 residues. For this average value (of 10 neural networks), an assumed linker domain was determined under the condition of the cut-off value = 0.95, threshold value = 0.5. The terminal regions (60 residues) of the protein were all included in the prediction. The linker domains were not ranked here (all the prediction domains were taken).

The amino-acid sequences predicted as linker sequences were stored in the hard disk.

### Appendix

Discussion on theoretical / methodological backgrounds has an essential meaning in setting appropriate problems (and problem solution), which can not be avoided. However, it can be an independent subject of discussion and it will be discussed separately in an appendix. Here, theoretical framework for the neural network and concrete designing of methodology based on it will be described.

### A. Neural network

### A.1. Theoretical framework of neural network

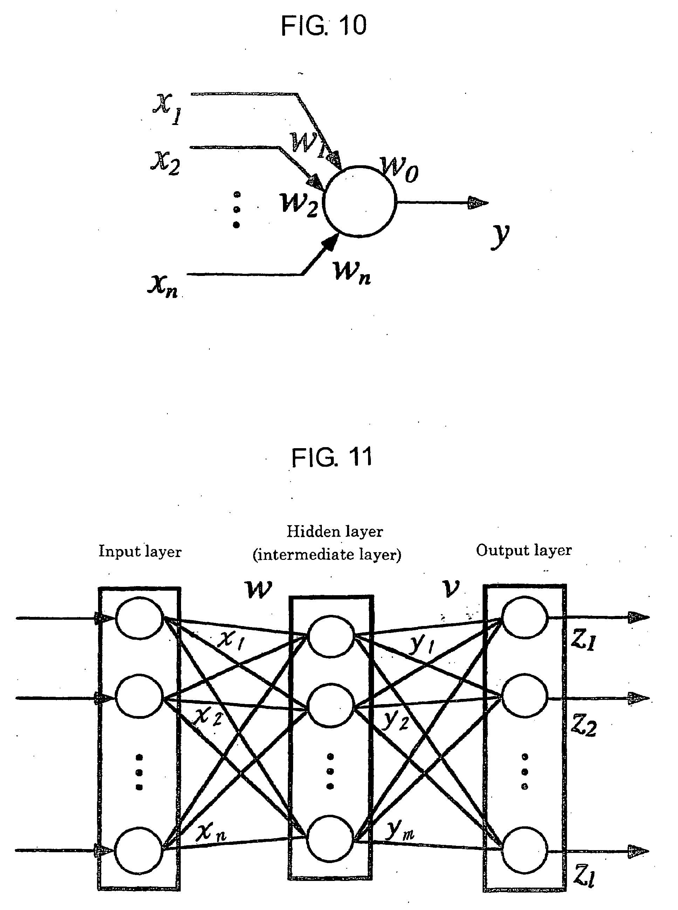

The neural network shall have the following neural model as its basic component (Fig. 10). 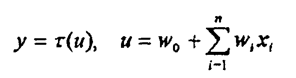 where, τ is a sigmoid function represented as follows:$\text{τ(} \text{u} \text{) =} \frac{\text{1}}{\text{1+} {\text{e}}^{\text{-α}}}$ and it takes a value of [0, 1]. In this neuron model, xᵢ is the i-th input signal coming from an axon of another neuron, wᵢ(i=1,···,n) is a degree that the input signal is strengthened by the synapse, w₀ is a threshold value, y represents an output of the neuron. That is, the input signal is weighted according to the connection strength, and whether the total u (corresponding to the internal potential of a neuron) is larger or smaller than the threshold value determines active state of the neuron (if y is 1, it is in the activated state, while if it is 9, it corresponds to the inactivated state). The connection strength can have an arbitrary real number value, and a positive value corresponds to an excitatory synapse and a negative value for an inhibitory synapse. Also, in the case of 0, it can be interpreted that there is no synapse connection.

In the neural network, neuron models are connected to each other to form a network. Here, a hierarchical feed-forward network is used. That is, neurons are arranged in the layered state so as to construct a network in which signals are transmitted from the previous layer to the next layer only in one direction. With this type of network, a neuron output in an output layer (output signal) is determined uniquely for a signal (input signal) given to a neuron in an input layer. In this sense, it can be considered as a kind of signal converter. When the connection strength / threshold value is changed, a function represented by the network is also changed, but it was proved that selection of an appropriate value can realize a non-linear continuous function ([Funahashi, 1989]). In learning, a connection strength / threshold value which can realize correct input / output relations are sought, but they can be automatically determined if the error back-propagation learning method [Rumelhart, 1986] is followed.

Referring to the three-layer neural network to be actually used in this study (Fig. 11), the error back-propagation learning method will be explained. For the input layer / hidden layer / output layer, n pieces / m pieces / 1 piece of neurons are prepared, respectively. Assuming J ≡ [0, 1], the input x and the output z of the network and the output y of the hidden layer are defined as follows:$\text{x} \text{≡ {} \text{x} \text{|} \text{x} \text{= (} {\text{x}}_{\text{1}} \text{,···,} {\text{x}}_{\text{n}} \text{),} {\text{x}}_{\text{i}} \text{∈} \text{J} \text{}}$$\text{y} \text{≡ {} \text{y} \text{|} \text{y} \text{= (} {\text{y}}_{\text{1}} \text{,···,} {\text{y}}_{\text{m}} \text{),} {\text{y}}_{\text{i}} \text{∈} \text{J} \text{}}$$\text{z} \text{≡ {} \text{z} \text{|} \text{z} \text{= (} {\text{z}}_{\text{1}} \text{,···,} {\text{z}}_{\text{l}} \text{),} {\text{z}}_{\text{i}} \text{∈} \text{J} \text{}}$ At this time, the input / output relations of the network can be understood as a function from Jⁿ to J^{l}:$\text{h} \text{=} \text{g ο f}$ Here, f is a function from Jⁿ to J^{m} realized by the hidden layer.$\text{f} \text{(} \text{x} \text{) = (} {\text{f}}_{\text{1}} \text{(} \text{x} \text{),···,} {\text{f}}_{\text{m}} \text{(} \text{x} \text{))}$ 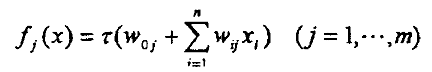 Also, g is a function from J^{m} to J^{l} realized by the output layer.$\text{g} \text{(} \text{x} \text{) = (} {\text{g}}_{\text{1}} \text{(} \text{x} \text{),···,} {\text{g}}_{\text{l}} \text{(} \text{x} \text{))}$

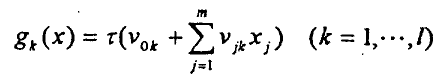

In leaning, in the error back-propagation method, an index called as an error is used as follows: 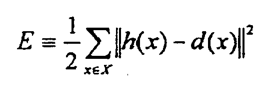 Here, d(x)=(d₁(x), ···, d₁(x)) is a correct output for the input x. X is a set of inputs x. This error E represents how far the neural network output is separated from an ideal output, and the smaller value means that it is the closer to desirable pattern identification. In learning, a dynamical system is set so as to decrease this value. 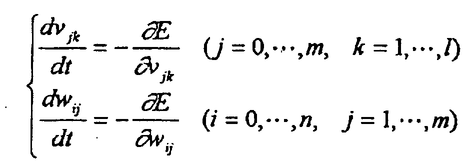 In this dynamical system, since it can be confirmed that an error E does not increase against time, if started with an appropriate weight as an initial value, the track of the dynamical system is retained at a minimum point of the error E in the end, and a desired weight can be gained. Here, the right side of the equation of the dynamical system can be concretely obtained from the definition equation of the error E as follows: 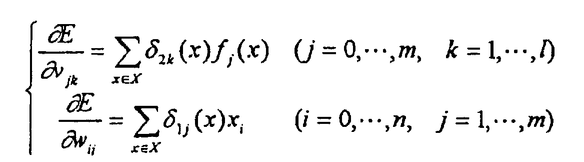 where, 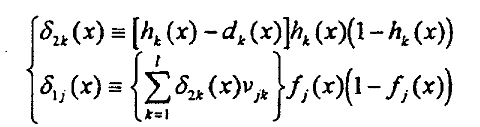 From this, the dynamical system equation can be described in more concrete form as follows: 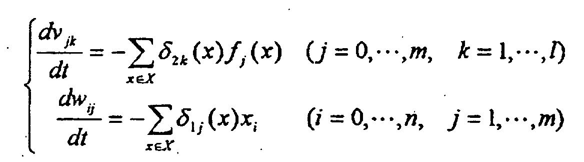 Moreover, when the left side is substituted by a difference, the following recurrence formula is derived: 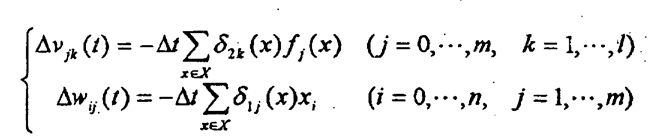 When the weights wᵢⱼ, vⱼₖ are made to evolve with time according to this recurrence formula, it can finally reach the minimum value of the error E. The above has been the principle of operation of the error back-propagation learning method.

### A.2. Improvement of learning algorithm achieved in this study

According to the above recurrence formula, all the weights wᵢⱼ, vⱼₖ in the network can optimized in principle. However, some problems occur if this learning is to be executed actually. First, it is essential to take a time width Δt small in a sense to improve the accuracy of convergence solution, but as a result, a change amount per time gets small and the number of learning times becomes enormous. Therefore, the value of Δt should be large to some extent in practice, which means the convergence gets worse. Also, once the error E reaches a minimum value which is not the smallest (local minimum), it can never get out of the current algorithm. Such a big problem still remains.

In order to solve these problems, in this study, an inertial term is added to the above recurrence formula. That is, the weight is represented by w and the following recurrence formula is set:$\text{Δ} \text{w} \text{(} \text{t} \text{) = -Δ} \text{t} \frac{\text{∂} \text{E}}{\text{∂} \text{w}} \text{+ αΔ} \text{w} \text{(} \text{t} \text{- 1)}$ Here, 0<α<1, and the closer to 1 is α, the larger is the effect of the inertial term. In the normal method, if a large value is taken for Δt, w fluctuates around the minimum value of E, and learning would not converge. On the other hand, since the new recurrence formula is changed in the direction to suppress fluctuation by the action of the inertial term, convergence of learning can be maintained even for a large Δt. Also, by decreasing fluctuation, converging speed can be considerably improved. The effect of the inertial term is also demonstrated when overcoming fine irregularity on the E curved face (when seen as a function of the weight w). Therefore, by adjusting the combination of Δt and α, the problems of increase in the number of learning times and trap by the local minimum can be avoided to some extent. As a result, after trial and error of conditions, this study was fixed to α=0.9, and Δt was set according to the given network.

### A.3. Computer environment

In carrying out the error back-propagation learning method, the algorithm was described in the program language C, and calculation was executed using the super computer VPP700E at RIKEN.

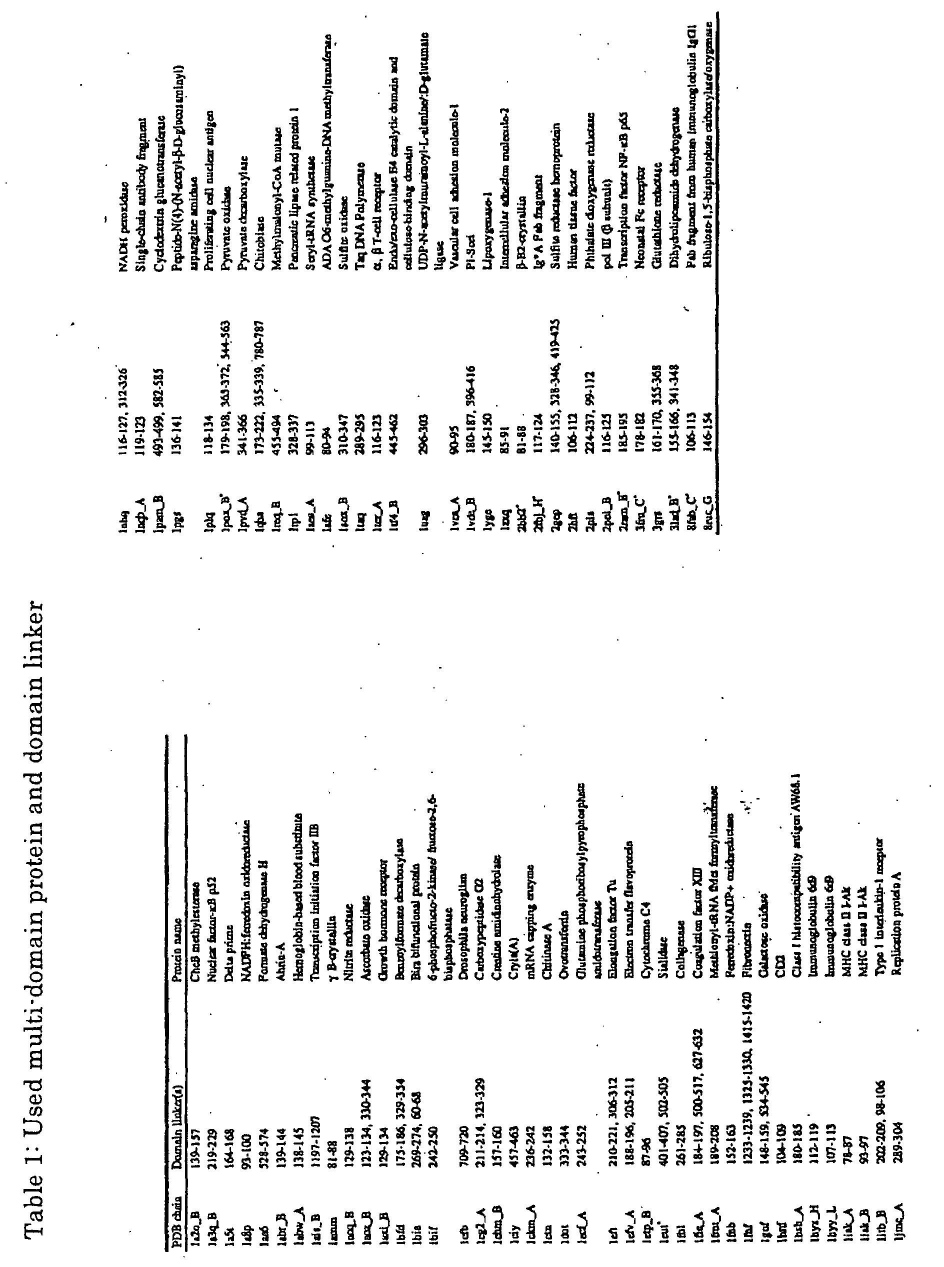

A protein chain whose structure (crystal structure with resolution of 2.5 Angstrom or more) is known and sequence is non-redundant (BLAST e value is at the level of 10⁻⁷⁰) is shown. Asterisks (*) indicate protein chains having a sequence similar to the other protein chains included in this data set (because the BLAST e value is less than 10⁻²⁰). These sequences were used for learning but they were not used for evaluation of domain linker prediction. Identification of 4-letter PDB codes and chains are on the left column. The first and the last residues of the SCOP derived domain linkers are on the center column. The names of the protein chains are on the right column.

**Table 2:**

| Conditions and learning efficiency | | |
|---|---|---|
| (a) Window size.^{a} | | |
| Window size | Linker [%] | Non-linker [%] |
| 3 | 27.8(1.2) | 91.8(0.9) |
| 5 | 34.1(2.2) | 88.3(2.0) |
| 7 | 43.9(3.5) | 84.4(2.0) |
| 9 | 46.3(2.6) | 85.4(1.7) |
| 11 | 51.1(2.8) | 84.0(1.4) |
| 13 | 55.7(1.8) | 82.1(1.6) |
| 15 | 58.1(1.3) | 82.2 (0.8) |
| 17 | 59.6(1.0) | 81.5(1.1) |
| 19 | 61.7(1.5) | 80.6(1.0) |
| 21 | 60.9(2.2) | 79.9(1.2) |
| 23 | 58.9(1.8) | 79.9(1.0) |
| 25 | 57.7(1.4) | 80.6(1.1) |
| 27 | 56.4(1.1) | 80.2(1.4) |
| 29 | 56.9(1.6) | 79.2(1.0) |
| 31 | 55.6(3.0) | 79.8(1.4) |
| 33 | 54.1(1.3) | 80.3(1.3) |
| 35 | 54.7(2.1) | 78.6 (0.8) |

| (b) Number of hidden units.^{b} | | |
|---|---|---|
| Hidden units | Linker [%] | Non-linker [%] |
| 0^{c} | 60.9(0.4) | 82.4(0.5) |
| 2 | 61.7(1.5) | 80.6(1.0) |
| 3 | 61.1(1.7) | 81.6(0.9) |
| 4 | 61.5(1.6) | 80.7(0.7) |
| 5 | 63.6(1.4) | 79.3(1.3) |
| 10 | 63.3(2.1) | 79.4(1.2) |
| 15 | 62.8(0.9) | 79.2(1.1) |
| 20 | 64.1(1.4) | 79.5(0.9) |

| (c) Training data set size.^{d} | | |
|---|---|---|
| Dataset size* | Linker [%] | Non-linker [%] |
| 0.1 | 39.0(1.8) | 75.5(0.6) |
| 0.2 | 50.4(1.9) | 70.8(1.7) |
| 0.3 | 47.5(1.5) | 79.3 (1.3) |
| 0.4 | 52.1(1.9) | 75.7(1.0) |
| 0.5 | 53.2(2.0) | 79.0(1.1) |
| 0.6 | 52.4(1.7) | 80.8(1.0) |
| 0.7 | 56.2(1.8) | 79.8(1.5) |
| 0.8 | 57.9(0.8) | 81.3(1.0) |
| 0.9 | 60.3(2.1) | 80.0(0.9) |
| 1.0 | 61.7(1.5) | 80.6(1.0) |

The following conditions: window size (a), the number of hidden units (b) and the size of training data set (c) were changed and learning was executed using the three-layer neural network. By calculating the correct answer rates of the linker sequence and the non-linker sequence using a single test method (See Materials and methods), the learning efficiency was evaluated. The sequence segment with the output value of neural network larger than 0.5 was predicted as a linker sequence. The others were predicted as a non-linker sequence. Learning was started with at-random initial parameters and executed 10 times independently. The correct answer rates of the linker and the non-linker sequences were averaged among 10 times of independent learning and indicated in Table. The standard deviation is shown in the parentheses.

The number of ^{a}hidden units was set to 2. The ^{b}window size was 19 residues. ^{c}0 indicates that there is no hidden layer. The ^{d}window size and the number of hidden units were 19 and 2, respectively. The proportion of etraining data set to the initial size.

**Table 3:**

| Influence of threshold value and cut-off value on prediction efficiency | | | | | | | | | |
|---|---|---|---|---|---|---|---|---|---|
| (a) Specificity. | | | | | | | | | |
| Threshold | Cut-off | | | | | | | | |
| | 0.9 | 0.8 | 0.7 | 0.6 | 0.5 | 0.4 | 0.3 | 0.2 | 0.1 |
| 0.9 | 63.6 | ----- | ----- | ----- | ----- | ----- | ----- | ----- | ----- |
| 0.8 | 72.7 | 52.6 | ----- | ----- | ----- | ----- | ----- | ----- | ----- |
| 0.7 | 72.7 | 57.9 | 50.0 | ----- | ----- | ----- | ----- | ---- | ----- |
| 0.6 | 81.8 | 63.2 | 62.5 | 56.5 | ----- | ----- | ----- | ----- | ----- |
| 0.5 | 81.8 | 63.2 | 65.6 | 58.7 | 58.8 | ----- | ----- | ----- | ----- |
| 0.4 | 81.8 | 63.2 | 65.6 | 60.9 | 60.8 | 55.2 | ----- | ----- | ----- |
| 0.3 | 81.8 | 63.2 | 65.6 | 60.9 | 60.8 | 55.2 | 51.6 | ----- | ----- |
| 0.2 | 81.8 | 63.2 | 65.6 | 60.9 | 60.8 | 58.6 | 54.7 | 54.6 | ----- |
| 0.1 | 81.8 | 63.2 | 65.6 | 60.9 | 62.8 | 60.3 | 56.3 | 56.1 | 56.1 |

| (b) Sensitivity. | | | | | | | | | |
|---|---|---|---|---|---|---|---|---|---|
| Threshold | Cut-off | | | | | | | | |
| | 0.9 | 0.8 | 0.7 | 0.6 | 0.5 | 0.4 | 0.3 | 0.2 | 0.1 |
| 0.9 | 8.1 | ----- | ----- | ----- | ----- | ----- | ----- | ----- | ----- |
| 0.8 | 9.2 | 11.5 | ----- | ----- | ----- | ----- | ----- | ----- | ----- |
| 0.7 | 9.2 | 12,6 | 18.4 | ----- | ----- | ----- | ----- | ----- | ----- |
| 0.6 | 10.3 | 13.8 | 23.0 | 29.9 | ----- | ----- | ----- | ----- | ----- |
| 0.5 | 10.3 | 13.8 | 25.3 | 32.2 | 35.6 | ----- | ----- | ----- | ----- |
| 0.4 | 10.3 | 13.8 | 25.3 | 33.3 | 36.8 | 37.9 | ----- | ----- | ----- |
| 0.3 | 10.3 | 13.8 | 25.3 | 33.3 | 36.8 | 37.9 | 39.1 | ----- | ----- |
| 0.2 | 10.3 | 13.8 | 25.3 | 33.3 | 36.8 | 40.2 | 41.4 | 42.5 | ----- |
| 0.1 | 10.3 | 13.8 | 25.3 | 33.3 | 37.9 | 41.4 | 42.5 | 43.7 | 43.7 |

Using the smoothing window of 19 residues, the domain linker in a protein sequence was predicted, and the prediction efficiency in the first rank prediction region was evaluated by the 10-fold jackknife test. The two values used for evaluation (specificity (a) and sensitivity (b)) were the same as those in Figs. 3a and 3b.

**Table A**

| Group 1 | | |
|---|---|---|
| w(i,j) | | |
| | 1 | 2 |
| 0 | 0.203088 | 0.540009 |
| 1 | 0.073914 | -0.34164 |
| 2 | 0.668079 | 0.503217 |
| 3 | 0.045715 | -0.61632 |
| 4 | 0.111587 | -0.17979 |
| 5 | 0.182084 | -0.0401 |
| 6 | -0.3307 | 0.707415 |
| 7 | 0.219901 | 0.514386 |
| 8 | -0.09145 | -0.14363 |
| 9 | -0.80034 | 0.021658 |
| 10 | -0.05301 | 0.191661 |
| 11 | 0.708844 | 0.486389 |
| 12 | 0.010888 | -0.26662 |
| 13 | -0.41839 | -0.50119 |
| 14 | -0.46904 | 0.190709 |
| 15 | 0.326836 | -0.12006 |
| 16 | -0.08283 | -0.35478 |
| 17 | -0.00795 | -0.22021 |
| 18 | 0.119587 | 0.215764 |
| 19 | 0.031814 | 0.236334 |
| 20 | 0.101783 | 0.26889 |
| 21 | 0.241188 | -0.28814 |
| 22 | -0.41516 | -0.15032 |
| 23 | 0.656729 | 0.145216 |
| 24 | -0.16417 | -0.26117 |
| 25 | -0 24376 | 0.412418 |
| 26 | 0.227849 | -0.42203 |
| 27 | -0.09348 | 0.408046 |
| 28 | 0.153017 | 0.374756 |
| 29 | 0.209754 | -0.22188 |
| 30 | -0.20783 | -0.30559 |
| 31 | 0.206758 | -0.00058 |
| 32 | 0.409745 | 0.683895 |
| 33 | -0.13617 | -0.1969 |
| 34 | -0.66977 | -0.25687 |
| 35 | -0.17179 | -0.03489 |
| 36 | -0.02782 | 0.299192 |
| 37 | 0.050957 | -0.59742 |
| 38 | -0.17204 | -0.31799 |
| 39 | 0.078222 | 0.21067 |
| 40 | 0.179898 | -0.12665 |
| 41 | 0.08324 | 0.370715 |
| 42 | 0.211288 | -0.01238 |
| 43 | 0.169011 | 0.01512 |
| 44 | 0.384231 | 0.359081 |
| 45 | -0.86572 | 0.271657 |
| 46 | 0.157363 | -0.05606 |
| 47 | -0.42993 | 0.088111 |
| 48 | 0.125666 | 0.315909 |
| 49 | 0.08278 | 0.772704 |
| 50 | 0.347408 | -0.03607 |
| 51 | 0.00797 | -0.47078 |
| 52 | -0.03288 | 0.238103 |
| 53 | 0.540945 | 0.694973 |
| 54 | -0.22537 | -0.25544 |
| 55 | -0.37341 | -0.41868 |
| 56 | -0.20714 | -0.05525 |
| 57 | -0.06712 | 0.261499 |
| 58 | 0.198648 | -0.38155 |
| 59 | -0.14564 | -0.2884 |
| 60 | 0.386566 | 0.29794 |
| 61 | -0.21057 | 0.088406 |
| 62 | -0.108 | 0.621091 |
| 63 | 0.189822 | -0.04068 |
| 64 | 0.375172 | -0.24881 |
| 65 | 0.280784 | 0.350218 |
| 66 | -0.32876 | -0.03357 |
| 67 | -0.07806 | 0.01148 |
| 68 | -0.26105 | -0.01629 |
| 69 | 0.387278 | 0.437011 |
| 70 | 0.386287 | 0.923562 |
| 71 | 0.185638 | 0.239484 |
| 72 | 0.199535 | -0.69202 |
| 73 | -0.28438 | 0.395351 |
| 74 | 0.756292 | 0.665594 |
| 75 | -0.12696 | -0.15193 |
| 76 | -0.23617 | -0.7661 |
| 77 | -0.09949 | -0.05336 |
| 78 | 0.04634 | 0.137315 |
| 79 | -0.23178 | 0.00718 |
| 80 | -0.03971 | -0.50462 |
| 81 | -0.31114 | 0.530159 |
| 82 | -0.23345 | -0.0257 |
| 83 | -0.02918 | 0.592355 |
| 84 | -0.23439 | 0.085195 |
| 85 | 0.13202 | -0.17814 |
| 86 | 0.261043 | 0.189141 |
| 87 | -0.04655 | -0.13789 |
| 88 | -0.12989 | -0.06276 |
| 89 | -0.51844 | 0.145467 |
| 90 | 0.295651 | 0.301802 |
| 91 | 0.290119 | 0.991052 |
| 92 | 0.04461 | 0.390948 |
| 93 | -0.01422 | -0.78845 |
| 94 | 0.134781 | -0.19037 |
| 95 | 0.474398 | 0.989826 |
| 96 | 0.091282 | -0.37682 |
| 97 | -0.869 | -0.45437 |
| 98 | -0.23552 | -0.13247 |
| 99 | 0.191084 | 0.418961 |
| 100 | -0.6409 | 0.101467 |
| 101 | 0.421567 | -0.65302 |
| 102 | 0.284741 | 0.052028 |
| 103 | -0.11986 | 0.01357 |
| 104 | 0.285669 | 0.029401 |
| 105 | -0.25297 | -0.03396 |
| 106 | 0.014272 | -0.00808 |
| 107 | 0.231999 | 0.211252 |
| 108 | -0.18804 | -0.12474 |
| 109 | 0.087 | -0.12682 |
| 110 | -0.22814 | -0.02755 |
| 111 | 0.244127 | 0367347 |
| 112 | 0.784543 | 0.520689 |
| 113 | 0.149655 | 0.784079 |
| 114 | -0.23133 | -0.41153 |
| 115 | 0.004895 | -0.04649 |
| 116 | 0.384475 | 0.859132 |
| 117 | -0.04573 | -0.03756 |
| 118 | -0.62681 | -0.74889 |
| 119 | 0.197454 | -0.3442 |
| 120 | 0.291285 | 0.407792 |
| 121 | -0.58478 | 0.206976 |
| 122 | 0.238565 | -0.33292 |
| 123 | 0.097992 | 0.357675 |
| 124 | 0.092729 | 0.226479 |
| 125 | 0.550985 | -0.09568 |
| 126 | -0.06271 | -0.18487 |
| 127 | -0.10729 | 0.01074 |
| 128 | 0.210412 | 0.347196 |
| 129 | -0.62222 | -0.26147 |
| 130 | -0.25796 | -0.27077 |
| 131 | -0.12156 | 0.071659 |
| 132 | -0.01946 | 0.129441 |
| 133 | 0.891879 | 0.355866 |
| 134 | 0.564503 | 0.630488 |
| 135 | -0.23093 | -0.34267 |
| 136 | 0.023624 | -0.03566 |
| 137 | 0.565664 | 0.561007 |
| 138 | 0.084232 | -0.48613 |
| 139 | -0.9251 | -0.81282 |
| 140 | -0.16212 | -0.41277 |
| 141 | 0.231087 | 0.098628 |
| 142 | -0.38896 | -0.16256 |
| 143 | -0.32491 | -0.2981 |
| 144 | 0.182849 | 0.078623 |
| 145 | -0.05575 | 0.314276 |
| 146 | 0.185952 | 0.307593 |
| 147 | -0.09747 | -0.26393 |
| 148 | 0.17624 | -0.35769 |
| 149 | 0.23492 | 0.080185 |
| 150 | -0.31363 | -0.38283 |
| 151 | 0.058098 | -0.10503 |
| 152 | -0.16272 | 0.214434 |
| 153 | -0.05524 | -0.03954 |
| 154 | 0.622912 | 0.623841 |
| 155 | 0.645335 | 0.620295 |
| 156 | 0.040316 | -0.1983 |
| 157 | -0.20348 | 0.433101 |
| 158 | 0.372777 | 0.352405 |
| 159 | -0.14011 | -0.51238 |
| 160 | -0.92278 | -0.79862 |
| 161 | -0.54901 | 0.149817 |
| 162 | -0.01294 | 0.571202 |
| 163 | 0.021641 | -0.62211 |
| 164 | -0.69912 | 0.157707 |
| 165 | 0.574073 | 0.142712 |
| 166 | 0.322987 | 0.005772 |
| 167 | 0.618337 | 0.269614 |
| 168 | 0.265902 | -0.15868 |
| 169 | 0.157827 | -0.20402 |
| 170 | 0.028886 | 0.051689 |
| 171 | -0.13465 | -0.55666 |
| 172 | 0.258128 | -0.57963 |
| 173 | 0.213903 | 0.300525 |
| 174 | 0.006395 | -0.05051 |
| 175 | 0.527014 | 0.397299 |
| 176 | -0.08341 | 0.818489 |
| 177 | 0.096983 | -0.249 |
| 178 | 0.206032 | 0.230246 |
| 179 | 0.477328 | 0.691801 |
| 180 | -0.41699 | -0.3035 |
| 181 | -0.57723 | -0.9143 |
| 182 | -0.45925 | -0.01211 |
| 183 | -0.17188 | 0.349711 |
| 184 | -0.22653 | -0.24533 |
| 185 | -0.78692 | 0.092476 |
| 186 | 0.334388 | 0.844846 |
| 187 | 0.655526 | -0.18564 |
| 188 | 0.368002 | 0.885076 |
| 189 | 0.195082 | -0.13708 |
| 190 | 0.059913 | 0.063141 |
| 191 | 0.096481 | 0.305493 |
| 192 | 0.192202 | -0.73329 |
| 193 | -0.13854 | -0.19136 |
| 194 | -0.31815 | 0.416714 |
| 195 | 0.367023 | -0.38544 |
| 196 | 0.286686 | 0.570619 |
| 197 | 0.3929 | 0.595546 |
| 198 | -0.22844 | 0.259292 |
| 199 | 0.25547 | 0.457686 |
| 200 | 0.234665 | 0.970347 |
| 201 | -0.62163 | -0.47735 |
| 202 | -0.67553 | -0.99274 |
| 203 | 0.107656 | -0.25714 |
| 204 | 0.205029 | 0.16812 |
| 205 | 0.097486 | -0.3854 |
| 206 | -0.53177 | -0.08877 |
| 207 | 0.380016 | 0.534568 |
| 208 | 0.45693 | 0.153908 |
| 209 | 0.32634 | 0.806303 |
| 210 | -0.17631 | -0.14437 |
| 211 | -0.0411 | -0.06376 |
| 212 | 0.23951 | 0.045609 |
| 213 | -0.20442 | -0.74475 |
| 214 | 0.073167 | -0.24842 |
| 215 | 0.189712 | -0.08041 |
| 216 | 0.005198 | 0.025968 |
| 217 | 0.101933 | 0.568057 |
| 218 | 0.399463 | 0.662669 |
| 219 | -0.40578 | 0.0777 |
| 220 | 0.125337 | 0.431644 |
| 221 | 0.411373 | 0.486051 |
| 222 | -0.78261 | -0.31995 |
| 223 | -1.22404 | -0.95589 |
| 224 | 0.08699 | -0.27955 |
| 225 | -0.09821 | 0.621336 |
| 226 | 0.042753 | -0.45847 |
| 227 | -0.11693 | -0.36604 |
| 228 | 0.113745 | 0.476587 |
| 229 | 0.173725 | 0.270702 |
| 230 | 0.56185 | 0.323922 |
| 231 | 0.06301 | 0.001923 |
| 232 | -0.31059 | -0.20397 |
| 233 | 0.324997 | 0.018771 |
| 234 | -0.09743 | -0.68422 |
| 235 | -0.01322 | 0.030533 |
| 236 | -0.08388 | -0.1557 |
| 237 | 0.189697 | 0.088263 |
| 238 | 0.16064 | 0.551251 |
| 239 | -0.01986 | 0.568367 |
| 240 | -0.39143 | 0.136758 |
| 241 | 0.440537 | 0.034732 |
| 242 | 0.392792 | 0.330706 |
| 243 | -0.39351 | -0.05948 |
| 244 | -1.17077 | -0.88137 |
| 245 | -0.38548 | 0.012554 |
| 246 | 0.345199 | 0.274505 |
| 247 | -0.6181 | -0.20843 |
| 248 | -0.13399 | -0.33174 |
| 249 | 0.104228 | 0.356645 |
| 250 | 0.301217 | 0.126347 |
| 251 | 0.448494 | 0.163406 |
| 252 | -0.15862 | -0.1854 |
| 253 | -0.21469 | -0.11044 |
| 254 | 0.197129 | 0.263244 |
| 255 | -0.06038 | -0.33234 |
| 256 | 0.098681 | 0.009518 |
| 257 | -0.0969 | -0.03526 |
| 258 | 0.281643 | 0.483559 |
| 259 | 0.010048 | 0.919913 |
| 260 | 0.435673 | -0.0995 |
| 261 | -0.31441 | 0.097275 |
| 262 | -0.02226 | 0.388633 |
| 263 | 0.33509 | 0.696228 |
| 264 | -0.25108 | -0.34716 |
| 265 | -0.90538 | -1.08562 |
| 266 | 0.141516 | -0.00531 |
| 267 | 0.487108 | 0.025541 |
| 268 | -0.02694 | -0.26978 |
| 269 | -0.20007 | -0.10958 |
| 270 | 0.222975 | 0.143381 |
| 271 | 0.102519 | 0.318553 |
| 272 | 0.189818 | 0.425075 |
| 273 | 0.066414 | 0.278496 |
| 274 | -0.13978 | -0.1304 |
| 275 | 0.609217 | 0.031532 |
| 276 | -0.50278 | -0.19433 |
| 277 | 0.411463 | -0.42302 |
| 278 | -0.27966 | 0.028935 |
| 279 | 0.694426 | 0.149943 |
| 280 | 0.627737 | 0.671108 |
| 281 | 0.038077 | 0.042256 |
| 282 | -0.2655 | 0.03135 |
| 283 | 0.102474 | 0.110377 |
| 284 | -0.09849 | 0.322938 |
| 285 | -0.27829 | 0.017574 |
| 286 | -1.02283 | -0.92786 |
| 287 | -0.01837 | 0.121062 |
| 288 | 0.237061 | 0.034332 |
| 289 | -0.48873 | 0.299139 |
| 290 | -0.27517 | -0.27876 |
| 291 | -0.14755 | 0.175789 |
| 292 | 0.345262 | 0.030499 |
| 293 | 0.014736 | 0.527607 |
| 29,4 | -0.16378 | 0.161211 |
| 295 | -0.33541 | 0.062575 |
| 296 | -0.00391 | 0.403422 |
| 297 | -0.3426 | -0.27167 |
| 298 | 0.18699 | -0.24662 |
| 299 | 0.108613 | -0.18845 |
| 300 | 0.508756 | 0.380611 |
| 301 | 0.731858 | 1.000181 |
| 302 | 0.114055 | -0.36009 |
| 303 | 0.082556 | 0.026083 |
| 304 | -0.06738 | 0.119676 |
| 305 | 0.039332 | -0.04198 |
| 306 | -0.11006 | -0.15986 |
| 307 | -0.88112 | -0.63456 |
| 308 | 0.155289 | -0.01426 |
| 309 | 0.109575 | 0.469614 |
| 310 | -0.20505 | 0.036813 |
| 311 | -0.18698 | -0.49412 |
| 312 | -0.04873 | 0.168336 |
| 313 | 0.025702 | 0.05031 |
| 314 | -0.11124 | 0.407873 |
| 315 | 0.047223 | -0.23643 |
| 316 | 0.102958 | -0.12006 |
| 317 | 0.674179 | 0.260172 |
| 318 | -0.41698 | 0.249571 |
| 319 | -0.30771 | 0.010681 |
| 320 | 0.1453 | -0.55156 |
| 321 | 0.163701 | 0.425897 |
| 322 | 0.530241 | 0.817036 |
| 323 | -0.03604 | -0.03902 |
| 324 | 0.106241 | 0.052858 |
| 325 | -0.20991 | 0.031123 |
| 326 | 0.196667 | 0.281562 |
| 327 | -0.06811 | -0.28679 |
| 328 | -0.56776 | -0.75427 |
| 329 | 0.299402 | -0.33616 |
| 330 | 0.168059 | 0.031208 |
| 331 | 0.352322 | -0.30052 |
| 332 | -0.17216 | -0.38732 |
| 333 | -0.27658 | -0.0851 |
| 334 | -0.3196 | -0.10739 |
| 335 | 0.195742 | 0.206005 |
| 336 | 0.010308 | -0.20822 |
| 337 | -0.07463 | -0.09805 |
| 338 | 0.039709 | 0.252356 |
| 339 | -0.22698 | 0.105322 |
| 340 | -0.28974 | -0.08327 |
| 341 | -0.01719 | -0.19148 |
| 342 | 0.340217 | 0.47778 |
| 343 | 0.855064 | 1.043365 |
| 344 | 0.002245 | -0.05562 |
| 345 | 0.048565 | -0.15503 |
| 346 | -0.1008 | -0.0194 |
| 347 | 0.161311 | 0.317004 |
| 348 | 0.006362 | -0.20268 |
| 349 | -0.74142 | -0.45124 |
| 350 | -0.03248 | -0.04255 |
| 351 | 0.031161 | 0.041716 |
| 352 | 0.277543 | -0.07988 |
| 353 | 0.176521 | -0.59229 |
| 354 | -0.23469 | -0.0568 |
| 355 | -0.03005 | 0.274288 |
| 356 | 0.100855 | 0.513823 |
| 357 | 0.168584 | -0.16726 |
| 358 | 0.076166 | 0.125704 |
| 359 | 0.42765 | 0.140564 |
| 360 | -0.42414 | 0.382035 |
| 361 | -0.22894 | -0.0216 |
| 362 | -0.34243 | -0.0781 |
| 363 | 0.216098 | -0.07901 |
| 364 | 0.551773 | 1.2368 |
| 365 | -0.09594 | -0.11456 |
| 366 | -0.0232 | -0.20889 |
| 367 | -0.26975 | 0.117923 |
| 368 | 0.608954 | -0.04884 |
| 369 | -0.27152 | -0.11366 |
| 370 | -0.69291 | -0.63739 |
| 371 | -0.16959 | -0.00889 |
| 372 | -0.05624 | 0.24408 |
| 373 | 0.406214 | -0.35149 |
| 374 | -0.02814 | -0.31822 |
| 375 | -0.11775 | -0.26461 |
| 376 | 0.172854 | 0.105598 |
| 377 | 0.349553 | -0.02751 |
| 378 | 0.131891 | 0.065268 |
| 379 | 0.120444 | 0.100008 |
| 380 | 0.458291 | 0.502448 |
| 381 | 0.443249 | -0.41384 |
| 382 | -0.0834 | -0.48195 |
| 383 | 0.064858 | 0.058266 |
| 384 | 0.168691 | -0.13751 |
| 385 | 0.756834 | 0.961917 |
| 386 | -0.1738 | -0.20047 |
| 387 | -0.13101 | -0.18184 |
| 388 | -0.11993 | -0.00069 |
| 389 | 0.290256 | 0.081142 |
| 390 | -0.35059 | 0.049965 |
| 391 | -0.16127 | -0.74512 |
| 392 | -0.1623 | 0.031976 |
| 393 | 0.211564 | 0.25765 |
| 394 | 0.24337 | -0.09502 |
| 395 | -0.1533 | -0.31831 |
| 396 | 0.174432 | -0.15268 |
| 397 | 0.076752 | 0.13494 |
| 398 | 0.057971 | 0.313684 |
| 399 | 0.187533 | 0.027739 |

| Group 1 | | |
|---|---|---|
| v(j) | | |
| 0 | 3.2501 | |
| 1 | -5.21239 | |
| 2 | -6.36906 | |

| Group 2 | | |
|---|---|---|
| w(ij) | | |
| | 1 | 2 |
| 0 | 0.372319 | 1.012758 |
| 1 | -1.341 | 0.650946 |
| 2 | 0.158913 | 0.96759 |
| 3 | -1.00242 | 0.502232 |
| 4 | -0.16249 | 0.109527 |
| 5 | -0.04493 | -0.0061 |
| 6 | 0.147951 | 0.828177 |
| 7 | 0.257626 | 1.502491 |
| 8 | -0.42083 | -0.05306 |
| 9 | 0.04632 | -0.55298 |
| 10 | 0.5877 | -0.12828 |
| 11 | -0.07568 | 1.047878 |
| 12 | -0.66223 | 0.201755 |
| 13 | 0.518818 | -2.15565 |
| 14 | -0.04026 | -0.27853 |
| 15 | -0.0951 | -0.62544 |
| 16 | -0.30661 | -1.02384 |
| 17 | -0.83816 | 0.543225 |
| 18 | 0.837488 | -0.21466 |
| 19 | 1.31166 | 0.003249 |
| 20 | -0.09556 | 0.160277 |
| 21 | -0.22429 | 0.005239 |
| 22 | -1.08283 | 0.177379 |
| 23 | 1.85618 | 0.677984 |
| 24 | 0.550711 | -0.92495 |
| 25 | 0.61898 | -0.53054 |
| 26 | -1.25602 | 0.431499 |
| 27 | 0.836531 | 0.709338 |
| 28 | 0.172603 | 1.268029 |
| 29 | 0.544312 | -0.54946 |
| 30 | 0.439839 | -1.27576 |
| 31 | -0.9683 | 1.0389 |
| 32 | -0.26756 | 0.404665 |
| 33 | 0.186216 | -0.57616 |
| 34 | -0.59601 | -0.53179 |
| 35 | -1.17389 | 0.801059 |
| 36 | -0.36422 | -0.0952 |
| 37 | 0.006947 | -0.96672 |
| 38 | -0.36351 | -0.47753 |
| 39 | 0.545638 | 0.025779 |
| 40 | -0.36275 | 0.127718 |
| 41 | 0.124485 | 0.920747 |
| 42 | -0.03199 | -0.13435 |
| 43 | -0.09835 | -0.15629 |
| 44 | 1.171092 | 1.222355 |
| 45 | 0.643286 | -1.22703 |
| 46 | -0.46178 | 0.200579 |
| 47 | -0.65874 | 0.238926 |
| 48 | 1.396822 | -0.07879 |
| 49 | 0.926215 | -0.10695 |
| 50 | -0.78907 | 0.7949 |
| 51 | -0.41946 | -0.18274 |
| 52 | 0.804891 | -0.43246 |
| 53 | 0.006097 | 0.887291 |
| 54 | -0.44191 | 0.150472 |
| 55 | -0.7983 | -0.32103 |
| 56 | -0.56179 | -0.41367 |
| 57 | -0.31169 | 0.380215 |
| 58 | -0.33279 | 0.190591 |
| 59 | -0.72536 | -0.47715 |
| 60 | 0.585753 | 0.099597 |
| 61 | -0.80454 | 0.564453 |
| 62 | 0.453927 | 0.248351 |
| 63 | -0.08668 | -0.04731 |
| 64 | 0.318061 | -0.84727 |
| 65 | 0.374398 | 0.757071 |
| 66 | -2.0298 | 1.146123 |
| 67 | 0.394106 | -0.39591 |
| 68 | 0.07358 | -0.70301 |
| 69 | -0.68274 | 1.441549 |
| 70 | -0.46442 | 1.017186 |
| 71 | -0.71161 | 1.377589 |
| 72 | -0.11208 | -1.47182 |
| 73 | 0.767579 | 0.188171 |
| 74 | 0.272972 | 0.790575 |
| 75 | 0.029222 | -0.75555 |
| 76 | -0.9388 | -0.33266 |
| 77 | 0.563326 | -0.28903 |
| 78 | 0.953385 | -0.61675 |
| 79 | -0.45069 | -0.52235 |
| 80 | -0.371 | -0.16591 |
| 81 | 0.170516 | 0.027167 |
| 82 | 0.329378 | 0.473275 |
| 83 | 1.230148 | 0.066737 |
| 84 | 0.107705 | -0.01789 |
| 85 | -0.11121 | -0.46777 |
| 86 | 0.611088 | 0.969042 |
| 87 | -0.75603 | 0.690166 |
| 88 | 0.546101 | -0.57099 |
| 89 | -0.03037 | -0.54039 |
| 90 | 1.474246 | 0.332466 |
| 91 | 0.204416 | 1.429161 |
| 92 | -0.14068 | 0.514587 |
| 93 | -1.41905 | 0.199062 |
| 94 | 0.216501 | -0.44243 |
| 95 | 0.03831 | 0.868207 |
| 96 | 0.296135 | -0.56985 |
| 97 | -1.38752 | -0.76682 |
| 98 | 0.206328 | -0.63806 |
| 99 | 1.174771 | 0.124625 |
| 100 | -0.41639 | -0.10495 |
| 101 | -0.27166 | -0.54396 |
| 102 | -0.16883 | -0.72151 |
| 103 | 0.407663 | 0.218976 |
| 104 | -0.55194 | 0.169801 |
| 105 | -0.23534 | 0.006364 |
| 106 | 0.226047 | -0.80968 |
| 107 | 0.516791 | 1.117572 |
| 108 | -0.974 | 0.409229 |
| 109 | -0.48793 | 0.055412 |
| 110 | -0.85389 | 0.437169 |
| 111 | 0.949932 | -0.6671 |
| 112 | 0.5633 | 1.540877 |
| 113 | 0.526601 | 0.635268 |
| 114 | -1.12373 | -0.47794 |
| 115 | -0.2104 | 0.019839 |
| 116 | 0.747487 | 0.255723 |
| 117 | -0.11946 | -0.26685 |
| 118 | -1.35075 | -0.86309 |
| 119 | 0.053518 | -0.768 |
| 120 | -0.17937 | 0.765414 |
| 121 | -0.15649 | -0.48113 |
| 122 | -0.96195 | 0.414535 |
| 123 | 0.683285 | -0.98484 |
| 124 | 0.640423 | 0.074378 |
| 125 | 0.848435 | -0.88792 |
| 126 | 0.005374 | 0.052965 |
| 127 | 0.490916 | -0.9179 |
| 128 | 0.325312 | 1.215089 |
| 129 | -0.10178 | -0.26361 |
| 130 | -0.71463 | 0.56387 |
| 131 | 0.197467 | -0.27329 |
| 132 | -0.9659 | 0.649583 |
| 133 | 1.535152 | 0.41254 |
| 134 | 1.051094 | -0.00066 |
| 135 | -0.24396 | -0.58386 |
| 136 | 0.003446 | -0.25114 |
| 137 | 0.558898 | 0.715059 |
| 138 | 0.3027 | -0.71344 |
| 139 | -0.84002 | -2.00214 |
| 140 | 0.121945 | -0.44956 |
| 141 | -0.39661 | 0.56633 |
| 142 | -0.91024 | 0.092194 |
| 143 | -0.20685 | -0.3164 |
| 144 | -0.42944 | 0.76597 |
| 145 | 0.601729 | 1.575967 |
| 146 | 0.37399 | -0.24323 |
| 147 | -0.1151 | 0.022806 |
| 148 | 0.099057 | -0.49125 |
| 149 | 0.563675 | 0.427817 |
| 150 | 1.040476 | -2.26792 |
| 151 | -0.88453 | 0.579925 |
| 152 | 0.461455 | 0.21274 |
| 153 | 0.320121 | 0.002335 |
| 154 | -0.03817 | 1.98842 |
| 155 | 0.889309 | 0.400192 |
| 156 | -1.20325 | 0.185965 |
| 157 | -0.16815 | 0.58407 |
| 158 | -0.02384 | 0.760548 |
| 159 | -0.4854 | 0.116441 |
| 160 | -0.76274 | -1.17413 |
| 161 | -0.42853 | 0.136514 |
| 162 | -0.25117 | 0.788685 |
| 163 | -0.81991 | -0.60464 |
| 164 | 1.093789 | -1.29857 |
| 165 | 0.593176 | -0.62777 |
| 166 | 0.042685 | 1.250965 |
| 167 | 0.289241 | 0.201878 |
| 168 | -0.10597 | 0.136875 |
| 169 | -0.13298 | -0.12669 |
| 170 | -0.25962 | 0.58148 |
| 171 | -0.22509 | -0.9229 |
| 172 | 0.092411 | -0.32242 |
| 173 | 0.049033 | 0.970155 |
| 174 | -0.12387 | -0.12311 |
| 175 | 1.123553 | 1.601295 |
| 176 | 1.605461 | 0.525174 |
| 177 | -0.33026 | -0.47233 |
| 178 | 1.329003 | -0.77797 |
| 179 | 0.797318 | 1.285923 |
| 180 | -0.82889 | -0.61139 |
| 181 | -1.17017 | -1.09782 |
| 182 | -0.06474 | -0.59703 |
| 183 | 0.020001 | -0.69653 |
| 184 | -0.44051 | -0.5325 |
| 185 | -0.91604 | 0.388778 |
| 186 | 0.313204 | 0.834129 |
| 187 | 0.446538 | 0.391983 |
| 188 | -0.1375 | 1.045966 |
| 189 | -0.27902 | 0.168854 |
| 190 | 0.213499 | -0.5981 |
| 191 | 0.524226 | 0.29399 |
| 192 | -1.876 | 0.114566 |
| 193 | 0.331433 | -1.34881 |
| 194 | 0.330727 | 0.165592 |
| 195 | 0.638544 | -0.81778 |
| 196 | 0.393752 | 1.091602 |
| 197 | 1.259493 | -0.05325 |
| 198 | -0.22225 | -0.32938 |
| 199 | 0.31073 | 0.566817 |
| 200 | 0.601091 | 1.423425 |
| 201 | -0.42536 | -0.39793 |
| 202 | -0.82215 | -1.75331 |
| 203 | -0.48023 | 0.198024 |
| 204 | -0.63781 | 0.1369 |
| 205 | 0.191438 | -0.6548 |
| 206 | -0.98536 | 0.31134 |
| 207 | 0.138424 | 0.77689 |
| 208 | -0.37989 | 1.705708 |
| 209 | 0.497788 | 0.001009 |
| 210 | -0.14845 | -0.1907 |
| 211 | -0.46655 | -0.15832 |
| 212 | 0.609589 | 0.646876 |
| 213 | -0.80251 | -0.72485 |
| 214 | -1.53593 | 0.878273 |
| 215 | 0.021097 | -0.08568 |
| 216 | -0.29809 | 0.00275 |
| 217 | 1.435665 | 0.654431 |
| 218 | 0.905449 | 0.519054 |
| 219 | -0.84481 | 0.443573 |
| 220 | 0.818234 | 0.359483 |
| 221 | 1.039553 | 0.620431 |
| 222 | -0.71191 | 0.12189 |
| 223 | -1.55452 | -2.1478 |
| 224 | -0.20686 | -0.87571 |
| 225 | -1.0579 | 0.255759 |
| 226 | -0.19342 | -0.27488 |
| 227 | 1.367741 | -1.18942 |
| 228 | 1.015088 | 0.373095 |
| 229 | 1.039317 | 0.363051 |
| 230 | 0.741473 | 0.944602 |
| 231 | -0.02939 | 0.050053 |
| 232 | 0.460047 | -0.65877 |
| 233 | 0.498954 | 0.414528 |
| 234 | 0.007725 | -2.18768 |
| 235 | 0.268561 | 0.838417 |
| 236 | -0.20237 | 0.169613 |
| 237 | -0.07271 | 0.875462 |
| 238 | -0.03225 | 1.018183 |
| 239 | -0.35942 | 1.141722 |
| 240 | -0.20693 | -0.23387 |
| 241 | -0.59737 | 1.700581 |
| 242 | 0.020339 | 1.171419 |
| 243 | 0.089375 | -1.81856 |
| 244 | -1.79811 | -1.14135 |
| 245 | 0.549497 | -0.52375 |
| 246 | 0.111344 | 0.262793 |
| 247 | -1.18526 | 0.798752 |
| 248 | -0.63376 | -0.30982 |
| 249 | 1.30076 | -0.29873 |
| 250 | 0.888363 | 0.25456 |
| 251 | 1.300921 | 0.228738 |
| 252 | 0.012754 | -0.24326 |
| 253 | -0.33606 | -0.24743 |
| 254 | 0.977908 | -0.18158 |
| 255 | -0.04509 | -0.71121 |
| 256 | -0.23876 | -0.06482 |
| 257 | -0.02321 | -0.73439 |
| 258 | 0.099253 | 1.016878 |
| 259 | -0.0417 | 1.372833 |
| 260 | -0.06396 | -0.07946 |
| 261 | 0.383551 | -0.26515 |
| 262 | 1.326307 | -0.06171 |
| 263 | -0.28182 | 1.62259 |
| 264 | 0.502595 | -1.252 |
| 265 | -1.13057 | -2.3503 |
| 266 | -0.09228 | -0.30353 |
| 267 | -0.59805 | 0.410668 |
| 268 | -0.47716 | -0.29089 |
| 269 | -0.58518 | 0.211163 |
| 270 | -0.55333 | 1.1767 |
| 271 | 0.094785 | 0.800725 |
| 272 | 1.324693 | -0.31817 |
| 273 | -0.06387 | 0.00125 |
| 274 | -1.50464 | 1.020169 |
| 275 | 1.245549 | -0.24367 |
| 276 | -0.67602 | -0.3428 |
| 277 | 0.528288 | -0.59713 |
| 278 | 0.024628 | 0.118675 |
| 279 | 1.055138 | 0.026115 |
| 280 | 0.859912 | 1.269743 |
| 281 | 1.258145 | -0.71006 |
| 282 | -0.50994 | 0.291778 |
| 283 | 0.958029 | 0.299932 |
| 284 | 0.689574 | 0.024824 |
| 285 | -1.07561 | 0.471378 |
| 286 | -1.91763 | -0.62226 |
| 287 | -1.25017 | 0.766226 |
| 288 | -0.16323 | -0.10854 |
| 289 | 0.638055 | -0.82443 |
| 290 | -0.53975 | -0.33419 |
| 291 | 0.758639 | -0.15319 |
| 292 | 0.594179 | 0.570446 |
| 293 | -0.92564 | 0.960015 |
| 294 | -0.13725 | 0.237896 |
| 295 | 0.289032 | -0.08296 |
| 296 | -0.30306 | 0.836385 |
| 297 | -0.33999 | -1.03909 |
| 298 | -1.37385 | 0.605332 |
| 299 | 0.31271 | -0.55184 |
| 300 | 0.665469 | 0.580574 |
| 301 | 1.942278 | 0.893087 |
| 302 | -0.6842 | 0.414846 |
| 303 | -0.05879 | 0.018329 |
| 304 | 0.803861 | -0.19056 |
| 305 | -0.61378 | 0.550721 |
| 306 | 0.892449 | -1.32746 |
| 307 | -1.32872 | -0.86773 |
| 308 | -0.38608 | 0.126183 |
| 309 | -0.70359 | 1.03929 |
| 310 | 0.415473 | 0.029884 |
| 311 | -0.26547 | -0.04058 |
| 312 | 0.819376 | -0.25439 |
| 313 | -0.30077 | 0.664709 |
| 314 | 0.612671 | -0.62634 |
| 315 | 0.170665 | -0.03717 |
| 316 | 0.249139 | 0.094595 |
| 317 | 0.584117 | 0.50475 |
| 318 | -0.16904 | -1.10622 |
| 319 | -1.16225 | 0.454448 |
| 320 | -1.04308 | 0.580959 |
| 321 | 0.947568 | -0.24702 |
| 322 | 0.46843 | 1.812657 |
| 323 | -1.00285 | 0.836803 |
| 324 | 0.153991 | 0.082174 |
| 325 | 0.749477 | 0.101108 |
| 326 | 0.127364 | 0.671505 |
| 327 | -0.28706 | -0.61516 |
| 328 | 0.318896 | -1.41377 |
| 329 | 0.677223 | -0.06426 |
| 330 | -0.22088 | -0.69879 |
| 331 | 0.596426 | -1.05072 |
| 332 | 0.291061 | -0.35945 |
| 333 | -0.73066 | 1.099099 |
| 334 | -0.88041 | 0.896239 |
| 335 | 0.808179 | -0.88718 |
| 336 | 0.188898 | -0.23301 |
| 337 | -0.21541 | 0.373246 |
| 338 | -0.08762 | 0.914606 |
| 339 | 0.118484 | -0.20604 |
| 340 | -0.24408 | 0.251664 |
| 341 | -0.37165 | 0.461679 |
| 342 | 0.089567 | 0.603273 |
| 343 | 1.496688 | 1.466543 |
| 344 | -0.05072 | -0.25358 |
| 345 | 0.313925 | -0.41294 |
| 346 | 0.053316 | 0.749362 |
| 347 | -0.74389 | 0.411311 |
| 348 | -0.49302 | -0.25245 |
| 349 | -0.94967 | -0.96243 |
| 350 | 0.851304 | -0.41661 |
| 351 | 0.345168 | -0.70767 |
| 352 | -1.01369 | 0.879443 |
| 353 | 0.01378 | -0.3087 |
| 354 | 0.701879 | -0.79491 |
| 355 | 0.572887 | -0.42668 |
| 356 | -0.08216 | -0.10615 |
| 357 | -0.02387 | 0.181898 |
| 358 | 0.877753 | -0.2666 |
| 359 | 0.324874 | 1.059339 |
| 360 | -0.8376 | 0.46615 |
| 361 | -0.44131 | 0.541288 |
| 362 | -0.08335 | 0.157274 |
| 363 | 0.066947 | -0.27572 |
| 364 | 1.137957 | 2.041129 |
| 365 | 0.300565 | -0.50854 |
| 366 | 0.238039 | -0.37083 |
| 367 | 0.020584 | -0.02529 |
| 368 | 1.333457 | -0.61684 |
| 369 | 0.182297 | -0.42132 |
| 370 | -2.02979 | -0.38779 |
| 371 | 0.556706 | 0.002565 |
| 372 | 0.639737 | -0.94327 |
| 373 | 1.380703 | -1.56491 |
| 374 | -0.56515 | 0.013118 |
| 375 | -1.1856 | 0.670355 |
| 376 | -0.72614 | 0.44601 |
| 377 | -0.5484 | -0.1112 |
| 378 | 0.003803 | -0.1694 |
| 379 | 0.393805 | -0.70671 |
| 380 | 1.49297 | 1.159131 |
| 381 | -0.70885 | 0.204981 |
| 382 | -0.64565 | 0.045964 |
| 383 | 0.469698 | 0.142748 |
| 384 | -1.23385 | 1.509698 |
| 385 | 1.029039 | 2.167971 |
| 386 | -1.13576 | -0.61285 |
| 387 | -0.02462 | -0.83687 |
| 388 | -0.00175 | -0.07921 |
| 389 | 0.756253 | -0.37463 |
| 390 | 0.543368 | -1.08814 |
| 391 | -0.35125 | -0.78552 |
| 392 | -0.86242 | -0.03181 |
| 393 | -0.29751 | 0.254151 |
| 394 | 0.818977 | -0.73301 |
| 395 | -0.45858 | 0.213372 |
| 396 | 0.597384 | -0.43315 |
| 397 | -0.80248 | 1.288501 |
| 398 | -0.19609 | -0.08565 |
| 399 | -0.1102 | -0.11805 |

| Group 2 | | |
|---|---|---|
| w(ij) | | |
| | 1 | 2 |
| 0 | 0.372319 | 1.012758 |
| 1 | -1.341 | 0.650946 |
| 2 | 0.158913 | 0.96759 |
| 3 | -1.00242 | 0.502232 |
| 4 | -0.16249 | 0.109527 |

**Table C**

| Group 3 | | |
|---|---|---|
| w(i,j) | | |
| | 1 | 2 |
| 0 | 1.004024 | -0.11681 |
| 1 | -0.46811 | 0.090162 |
| 2 | 1.279157 | -0.19382 |
| 3 | -0.30628 | -0.37219 |
| 4 | -0.14028 | -0.15035 |
| 5 | -0.2048 | 0.133447 |
| 6 | 0.512491 | -0.01194 |
| 7 | 0.63078 | -0.28511 |
| 8 | -1.02646 | 0.842553 |
| 9 | -0.62444 | -0.12475 |
| 10 | 0.472281 | -0.81161 |
| 11 | 0.306864 | 0.63061 |
| 12 | -0.16558 | -0.18881 |
| 13 | -1.06502 | 0.597906 |
| 14 | 0.272965 | 0.034676 |
| 15 | -0.57892 | 0.63626 |
| 16 | -0.37242 | -0.97125 |
| 17 | -0.38615 | 0.08074 |
| 18 | 0.07122 | 0.149479 |
| 19 | 0.755653 | 0.223882 |
| 20 | 0.268192 | -0.15909 |
| 21 | -0.2046 | -0.13816 |
| 22 | -0.0853 | 0.070648 |
| 23 | 0.892944 | 0.704875 |
| 24 | 0.146346 | -0.791 |
| 25 | 0.170655 | 0.145587 |
| 26 | -0.83426 | 0.209631 |
| 27 | 0.698428 | 0.389035 |
| 28 | 0.785289 | -0.54712 |
| 29 | -0.64214 | 1.009625 |
| 30 | -1.29797 | 0.402818 |
| 31 | 0.039817 | 0.07894 |
| 32 | 0.61725 | 0.618425 |
| 33 | -0.40266 | 0.478541 |
| 34 | -0.26985 | -1.16237 |
| 35 | 0.080986 | -0.04654 |
| 36 | -0.3608 | 0.160113 |
| 37 | -0.55668 | -0.37711 |
| 38 | -0.18491 | -0.69771 |
| 39 | 0.479744 | -0.2725 |
| 40 | 0.062613 | 0.333443 |
| 41 | 0.672461 | -0.19654 |
| 42 | 0.209104 | 0.186025 |
| 43 | 0.614902 | -1.10572 |
| 44 | 1.134287 | -0.16237 |
| 45 | 0.234847 | -0.71651 |
| 46 | 0.686253 | -0.37688 |
| 47 | -0.79735 | 0.253434 |
| 48 | 1.015096 | -0.3108 |
| 49 | 0.75879 | 0.263073 |
| 50 | -0.0865 | 0.683639 |
| 51 | -1.03435 | 0.206723 |
| 52 | 0.438253 | -0.18217 |
| 53 | 0.236015 | 0.894676 |
| 54 | -0.3544 | -0.4623 |
| 55 | -0.45392 | -0.58569 |
| 56 | -0.79325 | 0.684121 |
| 57 | -0.2426 | 0.542804 |
| 58 | -0.27223 | -0.73384 |
| 59 | -0.58165 | -0.34843 |
| 60 | 0.115739 | 0.34983 |
| 61 | 0.260375 | 0.091938 |
| 62 | 0.398343 | 0.233472 |
| 63 | 0.152738 | -0.15343 |
| 64 | 0.106383 | -0.18249 |
| 65 | 0.728098 | 0.290297 |
| 66 | -0.336 | -0.28259 |
| 67 | 0.389201 | -0.54929 |
| 68 | -0.90409 | 0.453672 |
| 69 | 0.426757 | 0.538328 |
| 70 | 0.859309 | 0.930478 |
| 71 | 0.493995 | 0.151622 |
| 72 | -1.0182 | 0.026609 |
| 73 | 0.651485 | -0.20388 |
| 74 | 0.299455 | 0.396555 |
| 75 | -0.29099 | -0.22434 |
| 76 | -0.94351 | -0.11843 |
| 77 | 0.086563 | -0.31442 |
| 78 | -0.58351 | 0.355236 |
| 79 | -0.53903 | -0.57365 |
| 80 | -0.16276 | -0.71377 |
| 81 | -0.11496 | 0.259748 |
| 82 | 0.12623 | -0.41488 |
| 83 | 0.654674 | 0.100566 |
| 84 | 0.202198 | 0.211111 |
| 85 | 0.396006 | -0.44005 |
| 86 | 0.663665 | -0.0656 |
| 87 | 0.31313 | -0.71306 |
| 88 | 0.514124 | -0.77319 |
| 89 | -0.22935 | -0.27617 |
| 90 | 0.372575 | 0.740254 |
| 91 | 0.264275 | 1.078466 |
| 92 | 0.734117 | 0.652704 |
| 93 | -0.68451 | -0.22033 |
| 94 | 0.646702 | -1.08029 |
| 95 | 0.990196 | -0.11291 |
| 96 | -0.32513 | 0.084341 |
| 97 | -0.98137 | -0.37282 |
| 98 | -0.06306 | 0.428022 |
| 99 | -0.13921 | 0.666978 |
| 100 | -0.33762 | -0.2141 |
| 101 | -0.75245 | 0.753085 |
| 102 | 0.240273 | -0.50352 |
| 103 | -0.46653 | 0.39949 |
| 104 | 0.288331 | 0.417016 |
| 105 | 0.157725 | 0.135273 |
| 106 | 0.041753 | 0.092251 |
| 107 | 0.147789 | 0.186064 |
| 108 | -0.9583 | 0.389773 |
| 109 | 0.373819 | -0.49631 |
| 110 | -0.42647 | -0.19777 |
| 111 | 0.074202 | 0.616781 |
| 112 | 0.85043 | 0.857786 |
| 113 | 0.801465 | -0.1226 |
| 114 | 0.030552 | -0.5568 |
| 115 | -0.29244 | 0.129129 |
| 116 | 0.584148 | 0.274931 |
| 117 | -0.67056 | 0.165075 |
| 118 | -0.87811 | -0.9584 |
| 119 | -0.50145 | 0.3473 |
| 120 | 0.799634 | -0.10651 |
| 121 | -0.03293 | -0.39887 |
| 122 | -0.04378 | -0.67914 |
| 123 | 0.512023 | -0.21647 |
| 124 | 0.78011 | -0.10479 |
| 125 | -0.00434 | 0.080991 |
| 126 | 0.188919 | 0.126331 |
| 127 | 0.197557 | 0.291773 |
| 128 | 0.42123 | 0.474027 |
| 129 | -0.20866 | -1.27725 |
| 130 | -0.01356 | -0.33619 |
| 131 | -0.69968 | 0.582187 |
| 132 | 0.746966 | 0.125134 |
| 133 | 1.226108 | 0.133789 |
| 134 | 0.97259 | -0.38866 |
| 135 | -0.34146 | -0.10497 |
| 136 | -0.1678 | -0.08602 |
| 137 | 0.39727 | 0.354463 |
| 138 | -0.28935 | 0.310911 |
| 139 | -1.31728 | -0.72753 |
| 140 | -0.215 | -0.49316 |
| 141 | 0.432077 | 0.240804 |
| 142 | -0.44211 | -0.04486 |
| 143 | -0.24664 | -0.21749 |
| 144 | -0.384 | 0.746762 |
| 145 | 0.686701 | -0.12241 |
| 146 | 0.604833 | 0.519606 |
| 147 | 0.028166 | 0.287481 |
| 148 | 0.230852 | -0.74712 |
| 149 | 0.368127 | 0.111856 |
| 150 | -0.78333 | -0.24773 |
| 151 | 0.062378 | -0.1906 |
| 152 | -0.14611 | 0.093142 |
| 153 | 0.210439 | 0.507843 |
| 154 | 0.321131 | 0.956007 |
| 155 | 0.110984 | 1.129606 |
| 156 | 0.107698 | -1.24675 |
| 157 | 0.122315 | 0.099841 |
| 158 | 0.455235 | 0.512434 |
| 159 | -0.20897 | -0.25961 |
| 160 | -1.28075 | -0.83038 |
| 161 | -0.70688 | -0.01295 |
| 162 | 0.689556 | -0.28957 |
| 163 | -1.0605 | -0.08662 |
| 164 | -0.05183 | -0.32778 |
| 165 | 0.138294 | 0.317154 |
| 166 | 0.690033 | -0.20754 |
| 167 | 0.510691 | 0.722132 |
| 168 | 0.289157 | -0.22229 |
| 169 | 0.491521 | -0.69939 |
| 170 | 0.06764 | 0.069653 |
| 171 | -0.22002 | -1.14676 |
| 172 | -0.19473 | -0.37497 |
| 173 | -0.06457 | 0.140806 |
| 174 | 0.199647 | 0.144141 |
| 175 | 0.611402 | 0.010185 |
| 176 | 0.714286 | 0.638965 |
| 177 | -0.77794 | 0.223457 |
| 178 | 0.139636 | 0.68296 |
| 179 | 1.172761 | 0.140248 |
| 180 | -0.0795 | -0.37251 |
| 181 | -1.96427 | -0.07096 |
| 182 | -0.29195 | -0.4436 |
| 183 | 0.028678 | 0.002673 |
| 184 | -0.85479 | 0.000457 |
| 185 | 0.588077 | -1.12861 |
| 186 | -0.15922 | 1.248564 |
| 187 | 0.469895 | 0.412343 |
| 188 | 0.631877 | 0.818812 |
| 189 | -0.1148 | -0.13338 |
| 190 | 0.200086 | 0.294969 |
| 191 | -0.33438 | 0.279061 |
| 192 | -1.39349 | 0.160891 |
| 193 | -0.05931 | -0.05823 |
| 194 | -0.66762 | 0.309202 |
| 195 | 0.104639 | -0.35225 |
| 196 | 0.383507 | 0.803746 |
| 197 | 0.785425 | 0.906542 |
| 198 | -0.07847 | -0.12003 |
| 199 | 0.797546 | -0.26118 |
| 200 | 0.682677 | 0.157548 |
| 201 | -0.26744 | -1.14416 |
| 202 | -1.89516 | -0.70392 |
| 203 | -0.24401 | -0.72596 |
| 204 | -0.09464 | 0.206922 |
| 205 | -0.40848 | -0.78097 |
| 206 | -0.12837 | -0.3297 |
| 207 | 1.248755 | -0.49065 |
| 208 | 1.0963 | 0.327233 |
| 209 | 0.547934 | 0.515923 |
| 210 | -0.00832 | 0.035282 |
| 211 | 0.264242 | -0.05309 |
| 212 | -0.45123 | -0.14118 |
| 213 | -1.06745 | -0.23329 |
| 214 | 0.867713 | -1.50369 |
| 215 | 0.055919 | -0.08365 |
| 216 | 0.359941 | -0.40581 |
| 217 | 0.843012 | -0.03312 |
| 218 | 0.871078 | -0.05446 |
| 219 | 0.231425 | -0.65604 |
| 220 | -0.60082 | 1.656698 |
| 221 | 0.741195 | -0.484 |
| 222 | -1.12097 | 0.070659 |
| 223 | -1.57549 | -0.739 |
| 224 | 0.125157 | -0.63895 |
| 225 | -0.26437 | 1.142433 |
| 226 | -0.68609 | 0406983 |
| 227 | -0.3541 | 0.422875 |
| 228 | 0.368056 | 0.733312 |
| 229 | 0.772901 | 0.400143 |
| 230 | 1.266734 | 0.492368 |
| 231 | -0.08848 | -0.17902 |
| 232 | -0.35565 | 0.361561 |
| 233 | 0.412036 | 0.36919 |
| 234 | -1.38829 | -0.05899 |
| 235 | 0.199105 | 0.341281 |
| 236 | -0.14544 | 0.177778 |
| 237 | 0.230189 | 0.031033 |
| 238 | 1.093614 | 0.193318 |
| 239 | 0.089004 | 0.2415 |
| 240 | -0.67759 | 0.609855 |
| 241 | 0.693831 | 0.288255 |
| 242 | 1.478346 | -0.42766 |
| 243 | -0.56983 | -0.03365 |
| 244 | -0.75739 | -2.06033 |
| 245 | -0.54685 | 0.325194 |
| 246 | -0.15521 | 0.448378 |
| 247 | -0.77507 | 0.039176 |
| 248 | 0.295671 | -0.53819 |
| 249 | 0.137191 | 0.69708 |
| 250 | 1.265553 | -0.03233 |
| 251 | 0.996088 | 0.047599 |
| 252 | 0.296115 | 0.124905 |
| 253 | 0.656914 | -0.88604 |
| 254 | 0.673108 | -0.07355 |
| 255 | -0.22631 | -0.66768 |
| 256 | -0.26885 | 0.831377 |
| 257 | -0.28345 | -0.05506 |
| 258 | 0.412438 | -0.03448 |
| 259 | 0.492824 | 0.651686 |
| 260 | 0.06211 | -0.33171 |
| 261 | -1.15656 | 0.539162 |
| 262 | 0.203141 | 0.665158 |
| 263 | 1.14548 | 0.098247 |
| 264 | -0.20716 | -0.83843 |
| 265 | -1.47386 | -0.84748 |
| 266 | 0.336032 | -0.8546 |
| 267 | 0.046214 | 0.289208 |
| 268 | -0.62178 | 0.272184 |
| 269 | -1.0668 | 0.692154 |
| 270 | 0.585225 | -0.35786 |
| 271 | 1.103219 | 0.381376 |
| 272 | 0.788853 | -0.31099 |
| 273 | -0.17332 | 0.11223 |
| 274 | -0.36651 | 0.130302 |
| 275 | -0.01107 | 0.850712 |
| 276 | -0.78903 | -0.11641 |
| 277 | 0.252346 | -0.10787 |
| 278 | 0.051208 | -1.04722 |
| 279 | 0.012939 | 0.44276 |
| 280 | 0.799078 | 0.990284 |
| 281 | -0.12157 | -0.25303 |
| 282 | -0.6013 | 0.245574 |
| 283 | 0.801383 | -0.41376 |
| 284 | 0.820691 | 0.280123 |
| 285 | 0.220597 | -0.36296 |
| 286 | -1.20743 | -1.21132 |
| 287 | 0.209962 | -0.41378 |
| 288 | -0.13633 | -0.08769 |
| 289 | 0.031633 | -0.19123 |
| 290 | -0.85594 | 0.307278 |
| 291 | 0.144258 | 0.536252 |
| 292 | 0.881918 | 0.140548 |
| 293 | 0.645941 | -0.5031 |
| 294 | 0.262111 | -0.25639 |
| 295 | 0.232752 | -0.13855 |
| 296 | 0.821786 | -0.02311 |
| 297 | -0.35687 | -0.52199 |
| 298 | -0.57111 | 0.773281 |
| 299 | -0.41137 | 0.000981 |
| 300 | 0.502704 | 0.000514 |
| 301 | 1.692603 | 0.859202 |
| 302 | 0.132702 | -0.4733 |
| 303 | 0.133975 | -0.47971 |
| 304 | 0.272025 | 0.216747 |
| 305 | -0.69142 | 0.335123 |
| 306 | 0.036624 | 0.239196 |
| 307 | -1.68968 | -0.00324 |
| 308 | -0.66983 | 0.502012 |
| 309 | 0.26929 | -0.19238 |
| 310 | -0.34765 | 0.144632 |
| 311 | -0.1718 | -0.41873 |
| 312 | -0.08424 | 0.276866 |
| 313 | -0.06493 | 0.006073 |
| 314 | 0.296196 | 0.081631 |
| 315 | 0.213089 | 0.010418 |
| 316 | 0.277913 | -0.18024 |
| 317 | 0.766437 | -0.06923 |
| 318 | -0.20061 | -0.18397 |
| 319 | -0.35767 | 0.668918 |
| 320 | -0.10929 | -0.19674 |
| 321 | -0.49762 | 1.314274 |
| 322 | 1.382855 | 0.509434 |
| 323 | -0.12215 | -0.29356 |
| 324 | -0.68324 | 0.233548 |
| 325 | 0.282519 | -0.26659 |
| 326 | 0.333216 | -0.14135 |
| 327 | 0.211095 | -0.82173 |
| 328 | -1.42946 | 0.264724 |
| 329 | -0.20359 | -0.33235 |
| 330 | 0.228757 | -0.18728 |
| 331 | 0.03754 | 0.205635 |
| 332 | 0.533825 | -0.64817 |
| 333 | -0.15608 | 0.136506 |
| 334 | 0.28726 | -0.2505 |
| 335 | 0.078657 | 0.074542 |
| 336 | -0.26028 | 0.280049 |
| 337 | 0.378086 | -0.23957 |
| 338 | 0.693161 | 0.428142 |
| 339 | 0.703408 | -1.45698 |
| 340 | 0.055301 | 0.280806 |
| 341 | 0.261535 | -0.41249 |
| 342 | 0.794976 | -0 38405 |
| 343 | 1.476265 | 1.181076 |
| 344 | -0.83566 | 1.164971 |
| 345 | -0.11267 | -0.64174 |
| 346 | 0.161657 | -0.56449 |
| 347 | -0.68506 | 0.955127 |
| 348 | 0.220672 | 0.021767 |
| 349 | -0.80982 | -0.51308 |
| 350 | -0.43622 | 0.048359 |
| 351 | 0.177509 | -0.72598 |
| 352 | -0.06145 | 0.651952 |
| 353 | 0.104504 | -0.30518 |
| 354 | -0.4938 | 0.706649 |
| 355 | 1.244981 | -0.59617 |
| 356 | 0.145796 | 0.655866 |
| 357 | -0.09185 | 0.226241 |
| 358 | -0.08146 | 0.41829 |
| 359 | 0.776445 | 0.553408 |
| 360 | 0.167289 | -0.01266 |
| 361 | 0.178662 | -0.33074 |
| 362 | 0.576612 | -0.55005 |
| 363 | 0.68667 | -0.57215 |
| 364 | 2.122255 | 1.240154 |
| 365 | 0.003564 | -0.58875 |
| 366 | -0.71716 | 0.522011 |
| 367 | -0.39368 | -0.07848 |
| 368 | -0.47967 | -0.42041 |
| 369 | -0.82776 | 0.481101 |
| 370 | -1.37468 | 0.029261 |
| 371 | -0.44288 | -0.13636 |
| 372 | 0.074483 | -0.29835 |
| 373 | 0.270493 | 0.184273 |
| 374 | -0.3248 | -0.04902 |
| 375 | -0.22869 | -0.31825 |
| 376 | 0.53391 | -0.31017 |
| 377 | 0.159034 | -0.05819 |
| 378 | -0.07994 | -0.24517 |
| 379 | 0.441122 | -0.71809 |
| 380 | 0.330793 | 0.425578 |
| 381 | -0.25331 | -0.59126 |
| 382 | -0.42893 | 0.273508 |
| 383 | 0.128794 | 0.38432 |
| 384 | 0.387389 | -0.2666 |
| 385 | 1.895239 | 0.821941 |
| 386 | -0.04176 | -0.0793 |
| 387 | -0.45132 | 0.055102 |
| 388 | 0.245882 | -0.99002 |
| 389 | 0.377565 | 0.3972 |
| 390 | -0.25513 | -0.56847 |
| 391 | -0.70826 | -0.57396 |
| 392 | -0.59585 | 0.137021 |
| 393 | 0.259558 | -0.09784 |
| 394 | 0.359762 | -0.29718 |
| 395 | -0.65384 | 0.626671 |
| 396 | -0.12596 | -0.14852 |
| 397 | -0.29259 | 1.007973 |
| 398 | 0.159272 | -0.22977 |
| 399 | -0.01964 | -0.00385 |

| Group 3 | | |
|---|---|---|
| v(j) | | |
| 0 | 4.927978 | |
| 1 | -10.0383 | |
| 2 | -8.69324 | |

**Table D**

| Group 4 | | |
|---|---|---|
| w(i,j) | | |
| | 1 | 2 |
| 0 | 0.226206 | 0.260618 |
| 1 | -0.03189 | -0.21085 |
| 2 | 0.52392 | 0.253769 |
| 3 | -0.58775 | 0.144325 |
| 4 | -0.16012 | -0.10151 |
| 5 | -0.5876 | 0.160045 |
| 6 | 0.279785 | 0.170879 |
| 7 | 0.614079 | 0.133685 |
| 8 | 0.26442 | -0.16267 |
| 9 | -0.21516 | -0.3054 |
| 10 | -0.00563 | 0.265494 |
| 11 | 0.647089 | 0.220283 |
| 12 | 0.305374 | -0.00304 |
| 13 | -0.36445 | -0.49975 |
| 14 | -0.11731 | -0.23575 |
| 15 | 0.105189 | -0.10202 |
| 16 | -0.00651 | -0.25626 |
| 17 | -0.42596 | 0.331674 |
| 18 | 0.404073 | -0.16025 |
| 19 | -0.08717 | 0.179923 |
| 20 | 0.708343 | -0.22046 |
| 21 | -0.07864 | -0.12575 |
| 22 | -0.34943 | 0.195537 |
| 23 | 0.034287 | 0.655379 |
| 24 | -0.42965 | -0.00546 |
| 25 | 0.107411 | -0.16686 |
| 26 | -0.05767 | -0.56613 |
| 27 | 0.388889 | -0.03338 |
| 28 | 0.189386 | 0.487292 |
| 29 | -0.43662 | 0.505805 |
| 30 | -0.66538 | -0.07828 |
| 31 | -0.10182 | 0.381624 |
| 32 | 0.477485 | 0.469298 |
| 33 | -0.1221 | -0.05484 |
| 34 | -0.59457 | -0.26283 |
| 35 | -0.0667 | -0.28251 |
| 36 | 0.304533 | -0.51715 |
| 37 | -0.18205 | -0.38069 |
| 38 | -0.07302 | -0.41194 |
| 39 | 0.084175 | -0.1292 |
| 40 | 0.057485 | -0.1273 |
| 41 | 0.574239 | -0.19857 |
| 42 | 0.224194 | -0.28833 |
| 43 | -0.10035 | 0.242529 |
| 44 | 0.067762 | 0.738802 |
| 45 | -0.07279 | -0.24517 |
| 46 | -0.05828 | -0.17968 |
| 47 | -0.40972 | -0.20438 |
| 48 | 0.426567 | 0.245457 |
| 49 | 0.246013 | 0.442851 |
| 50 | 0.002712 | 0.534569 |
| 51 | -0.52675 | -0.15654 |
| 52 | 0.336688 | 0.24233 |
| 53 | 0.660565 | 0.714213 |
| 54 | -0.10583 | -0.16144 |
| 55 | -0.64909 | -0.16975 |
| 56 | -0.35712 | 0.021783 |
| 57 | -0.06857 | 0.210661 |
| 58 | -0.03571 | -0.06023 |
| 59 | -0.34567 | -0.08102 |
| 60 | 0.437818 | -0.21721 |
| 61 | -0.1234 | -0.21718 |
| 62 | 0.371482 | 0.200683 |
| 63 | -0.185 | 0.045429 |
| 64 | 0.372766 | -0.33343 |
| 65 | 0.443291 | 0.38682 |
| 66 | -0.15587 | -0.14673 |
| 67 | -0.39113 | 0.217053 |
| 68 | -0.5104 | 0.073388 |
| 69 | 0.368508 | 0.303623 |
| 70 | 0.401565 | 0.443822 |
| 71 | 0.094551 | 0.425654 |
| 72 | -0.30696 | -0.50007 |
| 73 | 0.212491 | 0.250549 |
| 74 | 0.647447 | 0.59292 |
| 75 | -0.06403 | -0.10011 |
| 76 | -0.60491 | -0.36691 |
| 77 | -0.00165 | -0.37519 |
| 78 | -0.11133 | 0.174124 |
| 79 | -0.15852 | -0.29007 |
| 80 | -0.29174 | -0.16216 |
| 81 | 0.35238 | -0.08113 |
| 82 | -0.07812 | -0.20428 |
| 83 | 0.478907 | 0.301337 |
| 84 | 0.118891 | 0.042763 |
| 85 | 0.311708 | -0.42851 |
| 86 | 0.344308 | -0.04858 |
| 87 | -0.33733 | 0.14195 |
| 88 | -0.3803 | 0.071193 |
| 89 | -0.11079 | -0.18699 |
| 90 | 0.512906 | 0.045017 |
| 91 | 0.112473 | 0.546731 |
| 92 | 0.692633 | -0.03599 |
| 93 | -0.52251 | -0.48746 |
| 94 | 0.155087 | 0.112051 |
| 95 | 0.283569 | 0.861488 |
| 96 | -0.17636 | 0.113391 |
| 97 | -0.92332 | -0.30994 |
| 98 | -0.40473 | 0.100675 |
| 99 | 0.179164 | -0.0087 |
| 100 | -0.42849 | 0.116815 |
| 101 | -0.09302 | -0.02803 |
| 102 | 0.258587 | -0.40879 |
| 103 | -0.01173 | 0.190435 |
| 104 | 0.269888 | 0.199216 |
| 105 | -0.13057 | -0.00024 |
| 106 | 0.13323 | -0.18031 |
| 107 | 0.40161 | 0.217409 |
| 108 | -0.37429 | -0.02991 |
| 109 | -0.12809 | -0.08833 |
| 110 | -0.10525 | 0.139387 |
| 111 | 0.153842 | 0.389767 |
| 112 | 0.471743 | 0.065518 |
| 113 | 0.479758 | 0.398661 |
| 114 | -0.47459 | -0.52318 |
| 115 | 0.068511 | -0.00164 |
| 116 | 0.466496 | 0.656382 |
| 117 | -0.3289 | 0.278205 |
| 118 | -1.27668 | -0.26538 |
| 119 | -0.3896 | -0.11537 |
| 120 | 0.42313 | -0.28983 |
| 121 | 0.051053 | -0.27401 |
| 122 | 0.046605 | -0.31091 |
| 123 | -0.08976 | 0.108483 |
| 124 | 0.504903 | -0.23784 |
| 125 | 0.056955 | 0.246386 |
| 126 | 0.252427 | 0.052024 |
| 127 | 0.085108 | -0.15773 |
| 128 | 0.180587 | 0.545152 |
| 129 | -0.16724 | -0.31275 |
| 130 | -0.18565 | -0.30719 |
| 131 | 0.128329 | 0.069173 |
| 132 | 0.139314 | 0.17111 |
| 133 | 0.593687 | 0.370089 |
| 134 | 0.669274 | 0.457737 |
| 135 | -1.0218 | -0.02481 |
| 136 | 0.020255 | -0.06774 |
| 137 | 0.730902 | 0.172791 |
| 138 | 0.028517 | -0.13515 |
| 139 | -1.17361 | -0.5307 |
| 140 | -0.28338 | -0.10519 |
| 141 | 0.480372 | -0.33086 |
| 142 | -0.26465 | -0.18666 |
| 143 | -0.24505 | -0.06034 |
| 144 | -0.21471 | 0.478091 |
| 145 | 0.062021 | 0.245054 |
| 146 | 0.128703 | 0.251266 |
| 147 | -0.08979 | 0.120986 |
| 148 | -0.01686 | -0.11908 |
| 149 | 0.093827 | 0.553642 |
| 150 | -0.03957 | -0.55645 |
| 151 | -0.29266 | -0.16066 |
| 152 | 0.390273 | 0.293393 |
| 153 | -0.2161 | 0.300892 |
| 154 | 0.700162 | -0.04379 |
| 155 | 0.657845 | 0.460867 |
| 156 | -0.24593 | -0.42937 |
| 157 | -0.00383 | 0.355383 |
| 158 | 0.440665 | 0.768201 |
| 159 | -0.15086 | -0.08878 |
| 160 | -0.70712 | -0.87748 |
| 161 | -0.42352 | -0.08051 |
| 162 | 0.513725 | -0.08209 |
| 163 | -0.48877 | -0.18008 |
| 164 | -022873 | 0.040272 |
| 165 | -0.00113 | 0.29397 |
| 166 | 0.106515 | 0.119573 |
| 167 | 0.141129 | 0.310612 |
| 168 | 0.029283 | -0.07189 |
| 169 | 0.254885 | -0.36133 |
| 170 | 0.146097 | 0.155699 |
| 171 | -0.31281 | -0.53023 |
| 172 | -0.25084 | -0.14917 |
| 173 | 0.141674 | 0.332842 |
| 174 | 0.037511 | -0.14144 |
| 175 | 0.306236 | 0.235262 |
| 176 | 0.227363 | 0.672372 |
| 177 | -0.02763 | -0.74887 |
| 178 | 0.324277 | 0.347386 |
| 179 | 0.571938 | 0.283112 |
| 180 | -0.33717 | 0.146416 |
| 181 | -0.91176 | -0.73728 |
| 182 | -0.03258 | -0.57903 |
| 183 | -0.00981 | 0.144192 |
| 184 | -0.32812 | -0.17407 |
| 185 | 0.154753 | -0.50136 |
| 186 | 0.563866 | 0.308207 |
| 187 | 0.382776 | 0.019374 |
| 188 | 0.439278 | 0.664556 |
| 189 | 0.219328 | -0.22488 |
| 190 | -0.38653 | 0.326004 |
| 191 | 0.314489 | 0.012771 |
| 192 | -0.12701 | -0.81362 |
| 193 | -0.2957 | -0.43017 |
| 194 | 0.041101 | 0.311955 |
| 195 | 0.145308 | -0.28147 |
| 196 | 0.561174 | 0.110213 |
| 197 | 0.392436 | 0.634688 |
| 198 | -0.18019 | -0.25681 |
| 199 | -0.00207 | 0.641755 |
| 200 | 0.628524 | -0.05038 |
| 201 | -0.35407 | -0.50832 |
| 202 | -1.1832 | -0.64462 |
| 203 | -0 50521 | -0.06 |
| 204 | -0.05322 | 0.282016 |
| 205 | -0.05472 | -0.36064 |
| 206 | -0.34314 | -0.13726 |
| 207 | 0.422846 | 0.552068 |
| 208 | 0.245241 | 0.234947 |
| 209 | 0.422916 | 0.323113 |
| 210 | 0.295644 | 0.170715 |
| 211 | 0.252945 | -0.1877 |
| 212 | 0.171743 | -0.07606 |
| 213 | -0.39141 | -0.75132 |
| 214 | 0.102703 | -0.58376 |
| 215 | 0.30197 | -0.05727 |
| 216 | 0.219068 | -0.12696 |
| 217 | 0.16692 | 0.60087 |
| 218 | 0.518199 | 0.743352 |
| 219 | 0.151034 | -0.6938 |
| 220 | -0.05764 | 0.754374 |
| 221 | 0.735271 | 0.374059 |
| 222 | -0.36743 | -0.2232 |
| 223 | -0.95533 | -1.10203 |
| 224 | -0.32752 | -0.22155 |
| 225 | 0.353274 | 0.033745 |
| 226 | -0.4163 | 0.078438 |
| 227 | -0.12173 | -0.25926 |
| 228 | 0.268961 | 0.499232 |
| 229 | 0.102849 | 0.422606 |
| 230 | 0.177013 | 0.707539 |
| 231 | 0.184536 | -0.18362 |
| 232 | -0.29692 | 0.191906 |
| 233 | 0.422856 | 0.403739 |
| 234 | -0.56147 | -0.3524 |
| 235 | 0.331275 | -0.53025 |
| 236 | 0.208699 | 0.121352 |
| 237 | 0.321185 | -0.17841 |
| 238 | 0.63918 | 0.152929 |
| 239 | 0.016557 | 0.582623 |
| 240 | -0.00078 | -0.32827 |
| 241 | 0.602267 | 0.241723 |
| 242 | 0.580199 | 0.182785 |
| 243 | 0.072041 | -0.29027 |
| 244 | -0.92459 | -0.89049 |
| 245 | 0.025638 | -0.35368 |
| 246 | -0.01213 | 0.098191 |
| 247 | -0.35373 | -0.06859 |
| 248 | -0.02719 | -0.30683 |
| 249 | 0.530257 | 0.486047 |
| 250 | 0.334835 | 0.084108 |
| 251 | 0.445446 | 0.580003 |
| 252 | 0.178144 | -0.13768 |
| 253 | 0.446267 | -0.61053 |
| 254 | 0.22687 | 0.2438 |
| 255 | -0.8244 | 0.007268 |
| 256 | 0.036487 | -0.21761 |
| 257 | 0.210414 | -0.13334 |
| 258 | 0.198165 | 0.180186 |
| 259 | 0.385193 | 0.707844 |
| 260 | 0.252956 | 0.076905 |
| 261 | -0.30304 | -0.19392 |
| 262 | 0.267532 | 0.49041 |
| 263 | 0.568239 | 0.146866 |
| 264 | 0.019128 | -0.45084 |
| 265 | -0.96245 | -0.79859 |
| 266 | -0.14419 | -0.27452 |
| 267 | 0.319705 | 0.282828 |
| 268 | -0.06563 | -0.05245 |
| 269 | 0.0002 | -0.32114 |
| 270 | 0.228603 | 0.338158 |
| 271 | 0.398017 | 0.471874 |
| 272 | 0.675209 | 0.24046 |
| 273 | -0.17874 | 0.000091 |
| 274 | 0.08205 | -0.33205 |
| 275 | 0.528481 | 0.345893 |
| 276 | -0.36679 | -0.61998 |
| 277 | -0.03875 | 0.045072 |
| 278 | 0.26725 | -0.40661 |
| 279 | 0.684031 | -0.00746 |
| 280 | 0.444083 | 0.565414 |
| 281 | 0.168172 | -0.02131 |
| 282 | -0.46121 | -0.06202 |
| 283 | -0.16477 | 0.680022 |
| 284 | 0.217985 | 0.367969 |
| 285 | 0.215731 | -0.35663 |
| 286 | -1.16002 | -0.49627 |
| 287 | -0.20349 | -0.15535 |
| 288 | -0.04902 | 0.141569 |
| 289 | -0.12404 | 0.212393 |
| 290 | -0.275 | -0.25014 |
| 291 | 0.152998 | 0.248768 |
| 292 | 0.240205 | 0.226874 |
| 293 | 0.411988 | 0.297382 |
| 294 | -0.22425 | -0.1374 |
| 295 | -0.31402 | 0.152802 |
| 296 | 0.288638 | 0.443179 |
| 297 | -0.32416 | -0.91627 |
| 298 | 0.08197 | -0.24439 |
| 299 | -0.17465 | -0.43857 |
| 300 | 0.718813 | 0.073667 |
| 301 | 0.549763 | 0.835362 |
| 302 | 0.038374 | -0.08445 |
| 303 | -0.04175 | -0.35171 |
| 304 | 0.405471 | -0.08403 |
| 305 | -0.31725 | 0.123633 |
| 306 | -0.12411 | 0.073884 |
| 307 | -0.87963 | -0.58426 |
| 308 | -0.50685 | 0.138949 |
| 309 | 0.408485 | -0.27883 |
| 310 | -0.16015 | 0.019151 |
| 311 | -0.62211 | 0.12792 |
| 312 | 0.20478 | -0.09979 |
| 313 | 0.304819 | 0.075326 |
| 314 | 0.284068 | 0.028721 |
| 315 | -0.08562 | 0.2851 |
| 316 | 0.116882 | -0.04446 |
| 317 | 0.670848 | 0.138119 |
| 318 | -0.35138 | -0.47389 |
| 319 | -0.04829 | -0.17167 |
| 320 | -0.62068 | -0.0673 |
| 321 | 0.164085 | 0.400686 |
| 322 | 0.679365 | 0.631526 |
| 323 | -0.20465 | 0.222757 |
| 324 | -0.05834 | -0.14604 |
| 325 | 0.259994 | -0.11419 |
| 326 | 0.140722 | 0.405258 |
| 327 | -0.09553 | 0.087806 |
| 328 | -0.89708 | -0.41049 |
| 329 | -0.05374 | -0.17161 |
| 330 | -0.23111 | 0.410405 |
| 331 | 0.052623 | -0.05698 |
| 332 | -0.43436 | 0.116803 |
| 333 | 0.176257 | -0.12436 |
| 334 | 0.255225 | -0.10801 |
| 335 | 0.209227 | 0.160554 |
| 336 | 0.152583 | 0.140399 |
| 337 | 0.108238 | -0.20629 |
| 338 | 0.489354 | 0.080487 |
| 339 | -0.38701 | -0.2711 |
| 340 | -0.57375 | 0.14515 |
| 341 | -0.35949 | -0.24821 |
| 342 | 0.404413 | 0.042078 |
| 343 | 0.83004 | 0.973249 |
| 344 | -0.22586 | -0.18182 |
| 345 | -0.10795 | -0.18211 |
| 346 | 0.326448 | -0.21616 |
| 347 | 0.037056 | 0.188999 |
| 348 | 0.207069 | -0.43474 |
| 349 | -0.79309 | -0.41817 |
| 350 | -0.10995 | -0.13448 |
| 351 | -0.13583 | 0.196779 |
| 352 | -0.09454 | 0.249088 |
| 353 | 0.114098 | -0.51201 |
| 354 | -0.06277 | -0.0066 |
| 355 | 0.030739 | 0.104943 |
| 356 | 0.089245 | 0.506509 |
| 357 | 0.13851 | -0.16745 |
| 358 | 0.346465 | -0.05318 |
| 359 | 0.305717 | 0.390758 |
| 360 | -0.57124 | -0.07996 |
| 361 | -0.14735 | -0.08012 |
| 362 | 0.316356 | -0.70561 |
| 363 | 0.234631 | -0.02486 |
| 364 | 0.808535 | 1.168878 |
| 365 | -0.00351 | -0.31577 |
| 366 | 0.088283 | -0.05286 |
| 367 | 0.040512 | 0.063009 |
| 368 | -0.30793 | 0.464784 |
| 369 | -0.1417 | 0.25236 |
| 370 | -0.78908 | -0.10603 |
| 371 | -0.09926 | -0.15619 |
| 372 | -0.11163 | 0.245076 |
| 373 | -0.17555 | 0.33526 |
| 374 | 0.194532 | -0.35185 |
| 375 | 0.072285 | -0.21255 |
| 376 | 0.1249 | -0.04503 |
| 377 | 0.073888 | 0.058349 |
| 378 | -0.01345 | 0.065294 |
| 379 | 0.170292 | -0.18619 |
| 380 | 0.166905 | 0.421758 |
| 381 | -0.0171 | -0.58313 |
| 382 | -0.33802 | -0.02872 |
| 383 | -0.26185 | 0.126446 |
| 384 | -0.1691 | 0.345999 |
| 385 | 1.230522 | 0.848091 |
| 386 | -0.49941 | 0.114222 |
| 387 | -0.26152 | -0.08266 |
| 388 | 0.475755 | -0.56818 |
| 389 | 0.501029 | 0.063689 |
| 390 | 0.017664 | -0.08095 |
| 391 | -0.56184 | -0.16015 |
| 392 | -0.44203 | -0.23736 |
| 393 | 0.081059 | 0.277815 |
| 394 | -0.02677 | 0.32758 |
| 395 | 0.18334 | -0.15914 |
| 396 | 0.197635 | -0.09194 |
| 397 | 0.253548 | -0.09238 |
| 398 | 0.228668 | 0.041099 |
| 399 | -0.23404 | -0.28024 |

| Group 4 | | |
|---|---|---|
| v(j) | | |
| 0 | 2.880628 | |
| 1 | -5.78703 | |
| 2 | -5.36282 | |

**Table E**

| Group 5 | | |
|---|---|---|
| w(i,j) | | |
| | 1 | 2 |
| 0 | 1.633116 | -0.01787 |
| 1 | -0.62108 | -0.20829 |
| 2 | 1.913093 | -0.01412 |
| 3 | -1.96856 | 0.80515 |
| 4 | 0.133583 | 0.027592 |
| 5 | 0.469761 | 0.156819 |
| 6 | 0.71116 | 0.743258 |
| 7 | 0.812836 | 0.046079 |
| 8 | -0.88466 | 0.708408 |
| 9 | -1.90587 | 0.02119 |
| 10 | 1.066909 | -0.36633 |
| 11 | 0.576728 | 0.349386 |
| 12 | 0.576573 | -0.62547 |
| 13 | -2.29197 | 0.687983 |
| 14 | 0.238057 | -1.24159 |
| 15 | 0.457516 | 0.286093 |
| 16 | -0.26544 | -1.71114 |
| 17 | 0.296042 | -0.70806 |
| 18 | -0.18413 | 0.80496 |
| 19 | 0.952597 | -0.72077 |
| 20 | -0.22207 | 1.208819 |
| 21 | -0.2052 | -0.13841 |
| 22 | -0.07908 | -0.49014 |
| 23 | 1.947971 | 0.716275 |
| 24 | 0.446668 | -1.57593 |
| 25 | -0.15773 | 0.020541 |
| 26 | -0.68954 | 0.802026 |
| 27 | 1.51186 | -0.62119 |
| 28 | 1.090407 | 0.719696 |
| 29 | -1.20834 | 1.642169 |
| 30 | -2.14508 | 0.957761 |
| 31 | 0.396216 | -0.04474 |
| 32 | 0.551327 | 1.113978 |
| 33 | 0.31785 | -1.1189 |
| 34 | -0.7388 | -1.05682 |
| 35 | -0.82589 | 0.104796 |
| 36 | -0.01086 | 0.449585 |
| 37 | -1.00865 | -1.37757 |
| 38 | -0.05227 | 0.105677 |
| 39 | 0.132099 | 0.263383 |
| 40 | 0.402687 | -0.75319 |
| 41 | 0.760481 | 0.752159 |
| 42 | 0.208942 | 0.186062 |
| 43 | 0.875639 | -1.09463 |
| 44 | 1.836774 | -1.92769 |
| 45 | -0.66355 | 0.157748 |
| 46 | 0.569171 | 0.187531 |
| 47 | -0.97359 | 0.217252 |
| 48 | 1.298208 | 0.193359 |
| 49 | 1.833575 | -0.09301 |
| 50 | -0.04765 | 0.930874 |
| 51 | -1.29108 | -0.28887 |
| 52 | 0.741605 | 0.83145 |
| 53 | 1.617258 | 0.665168 |
| 54 | 0.509606 | -0.34202 |
| 55 | -0.52289 | -1.16473 |
| 56 | -1.65447 | 0.702827 |
| 57 | -0.52738 | 1.006644 |
| 58 | -0.47908 | -1.474 |
| 59 | -1.24247 | 0.674448 |
| 60 | 0.212803 | 0.261198 |
| 61 | 0.23612 | -0.85479 |
| 62 | -0.4217 | 0.729907 |
| 63 | 0.151497 | -0.15399 |
| 64 | -0.2407 | 0.802181 |
| 65 | -0.05103 | 0.173889 |
| 66 | -0.02474 | -1.09451 |
| 67 | 0.74556 | -0.99378 |
| 68 | -1.02571 | 0.591872 |
| 69 | 0.150945 | 0.832713 |
| 70 | 0.745685 | 0.907195 |
| 71 | 1.841285 | -0.10294 |
| 72 | -1.4037 | -0.17811 |
| 73 | 1.247343 | -0.68575 |
| 74 | 0.363718 | 2.242145 |
| 75 | -0.3419 | 0.164293 |
| 76 | -1.98196 | -0.13119 |
| 77 | -0.30677 | -0.47691 |
| 78 | -0.50804 | 1.467378 |
| 79 | -0.16275 | -2.2218 |
| 80 | -0.84948 | -0.70157 |
| 81 | -0.661 | 1.245141 |
| 82 | 1.271082 | -1.24958 |
| 83 | 1.750265 | -0.56693 |
| 84 | 0.202354 | 0.211588 |
| 85 | 0.647557 | -0.95091 |
| 86 | 1.874839 | -0.47279 |
| 87 | 0.526894 | -1.59479 |
| 88 | -0.3158 | 0.545521 |
| 89 | -0.6846 | -0.36199 |
| 90 | 1.142325 | 0.379102 |
| 91 | -0.02355 | 2.276324 |
| 92 | 0.66365 | 0.797738 |
| 93 | -2.67646 | 0.567422 |
| 94 | 0.400623 | -0.49895 |
| 95 | 2.054157 | -0.77646 |
| 96 | -0.13674 | 0.080102 |
| 97 | -1.36038 | -1.48188 |
| 98 | 0.122525 | -0.16783 |
| 99 | -0.0449 | 1.166012 |
| 100 | -0.28944 | -0.63102 |
| 101 | -1.24365 | 1.511372 |
| 102 | 0.537764 | -0.80321 |
| 103 | -0.04347 | 1.270253 |
| 104 | 0.922993 | -0.30641 |
| 105 | 0.156597 | 0.134695 |
| 106 | -0.15585 | 0.407672 |
| 107 | 0.998183 | 0.457523 |
| 108 | -1.51947 | 0.685985 |
| 109 | 0.742291 | -1.48412 |
| 110 | -1.08993 | -0.70698 |
| 111 | -0.81266 | 2.116249 |
| 112 | 0.90585 | 0.080458 |
| 113 | 1.54171 | 0.931925 |
| 114 | -0.2484 | -2.08013 |
| 115 | -0.25322 | 0.127254 |
| 116 | 1.237261 | 0.442228 |
| 117 | 0.030239 | -0.78845 |
| 118 | -2.21477 | -0.41647 |
| 119 | -1.41758 | 1.112989 |
| 120 | 2.362344 | -1.32122 |
| 121 | -0.05788 | -0.25831 |
| 122 | -0.11173 | -0.19132 |
| 123 | -0.00859 | 0.318572 |
| 124 | 1.48446 | -0.01536 |
| 125 | 0.404989 | -0.01714 |
| 126 | 0.188271 | 0.126396 |
| 127 | 0.459736 | -0.18947 |
| 128 | 0.854089 | 0.193115 |
| 129 | -0.45512 | -0.22194 |
| 130 | 1.081616 | -1.41959 |
| 131 | -0.65735 | -0.02727 |
| 132 | 1.540419 | -0.16958 |
| 133 | 0.859011 | 1.064669 |
| 134 | 0.499077 | 0.496344 |
| 135 | -0.70174 | 0.232365 |
| 136 | -0.04475 | 0.124903 |
| 137 | 0.677028 | 1.069718 |
| 138 | -0.49249 | 0.137786 |
| 139 | -3.05996 | -0.45445 |
| 140 | -0.49001 | -0.84128 |
| 141 | 0.187077 | 1.204593 |
| 142 | 0.648683 | -0.62179 |
| 143 | -0.4145 | 0.175266 |
| 144 | 0.839112 | -0.09491 |
| 145 | 0.892383 | -1.48356 |
| 146 | -0.23322 | 0.904961 |
| 147 | 0.027881 | 0.287417 |
| 148 | 0.342177 | -1.37657 |
| 149 | 0.226559 | 0.137022 |
| 150 | -1.93716 | 0.36371 |
| 151 | -0.48932 | -0.31886 |
| 152 | 0.498358 | -0.67656 |
| 153 | 0.738419 | 0.864068 |
| 154 | 1.248411 | 1.185542 |
| 155 | 0.716607 | 0.811932 |
| 156 | 0.093749 | -2.65489 |
| 157 | 0.024369 | 1.119003 |
| 158 | 2.087017 | 0.536435 |
| 159 | 0.445107 | -0.7034 |
| 160 | -2.35185 | -1.04278 |
| 161 | -1.02991 | -0.06381 |
| 162 | 1.155033 | -0.79636 |
| 163 | -1.37801 | 0.649245 |
| 164 | -0.15361 | -0.25945 |
| 165 | -0.24387 | 0.184499 |
| 166 | 0.476368 | 0.72066 |
| 167 | -0.06049 | 1.422042 |
| 168 | 0.289742 | -0.22153 |
| 169 | 1.011297 | -1.65898 |
| 170 | 0.007675 | 0.054371 |
| 171 | -0.11519 | -2.14812 |
| 172 | 0.667691 | -0.68922 |
| 173 | 0.90545 | -0.10237 |
| 174 | 0.048318 | -0.1431 |
| 175 | 0.763572 | -1.00072 |
| 176 | 1.972264 | 1.59214 |
| 177 | -1.79713 | 0.918227 |
| 178 | -0.09704 | 1.490765 |
| 179 | 0.848521 | 1.400365 |
| 180 | -0.95535 | 0.91044 |
| 181 | -3.94267 | 0.300783 |
| 182 | 0.333388 | -1.05365 |
| 183 | 0.106396 | -0.10122 |
| 184 | -0.72442 | -0.11626 |
| 185 | 1.139524 | -2.60956 |
| 186 | 0.182929 | 2.023504 |
| 187 | -0.00534 | -0.43591 |
| 188 | 0.788548 | 1.763997 |
| 189 | -0.11575 | -0.13344 |
| 190 | -0.70834 | 0.929717 |
| 191 | 0.696337 | 0.015223 |
| 192 | -2.07644 | 0.245698 |
| 193 | -0.83276 | -0.01836 |
| 194 | 0.776188 | 0.464094 |
| 195 | -0.09738 | 0.169003 |
| 196 | 0.891282 | 0.466628 |
| 197 | 1.50897 | -0.24904 |
| 198 | 0.03632 | -0.08794 |
| 199 | 1.513318 | -0.92179 |
| 200 | 1.131784 | 0.743998 |
| 201 | -0.54813 | -0.69265 |
| 202 | -320059 | -1.15822 |
| 203 | -0.42477 | -0.15737 |
| 204 | 0.367595 | 0.252744 |
| 205 | -1.17282 | -0.95093 |
| 206 | -1.30467 | -0.59389 |
| 207 | 1.671015 | -0.41244 |
| 208 | 1.813753 | 0.846436 |
| 209 | 0.863894 | 0.20288 |
| 210 | -0.0082 | 0.035545 |
| 211 | 0.306919 | -0.13532 |
| 212 | 0.118673 | 0.748655 |
| 213 | -2.11774 | 0.737975 |
| 214 | 0.775423 | -1.47389 |
| 215 | 0.709271 | -0.49501 |
| 216 | 0.146263 | -0.16975 |
| 217 | 1.567843 | 0.006499 |
| 218 | 2.11808 | -0.5554 |
| 219 | -0.06215 | -0.86154 |
| 220 | -0.32676 | 1.85614 |
| 221 | 1.058951 | -0.12573 |
| 222 | -0.98641 | -0.94748 |
| 223 | -2.55545 | -2.30878 |
| 224 | -0.04588 | -1.36072 |
| 225 | -0.39746 | 1.483424 |
| 226 | -1.19669 | 0.43933 |
| 227 | -1.37894 | 0.597146 |
| 228 | 0.929822 | 1.003409 |
| 229 | 0.330506 | 0.293568 |
| 230 | 2.281329 | 1.664459 |
| 231 | -0.08808 | -0.17784 |
| 232 | 0.747359 | -0.25745 |
| 233 | 0.319927 | 1.155909 |
| 234 | -1.18401 | -0.98042 |
| 235 | 0.333317 | 0.343803 |
| 236 | 0.21802 | -0.92137 |
| 237 | 1.066362 | -0.69973 |
| 238 | 2.387336 | -0.16661 |
| 239 | 0.975425 | 0.050598 |
| 240 | -1.11853 | 1.241668 |
| 241 | 0.475428 | 0.624026 |
| 242 | 0.38673 | -0.26307 |
| 243 | -2.05334 | 0.526326 |
| 244 | -1.5451 | -3.22796 |
| 245 | -1.22025 | 0.680508 |
| 246 | 0.512797 | 0.376656 |
| 247 | -0.56901 | -0.64517 |
| 248 | -0.25595 | -0.45231 |
| 249 | 0.066816 | 1.410666 |
| 250 | 1.020443 | 0.903051 |
| 251 | 1.584772 | 1.419337 |
| 252 | 0.295863 | 0.125109 |
| 253 | 0.294461 | -0.50519 |
| 254 | 1.750022 | 0.092489 |
| 255 | -0.871 | -0.47788 |
| 256 | 0.007856 | 0.668028 |
| 257 | -0.81328 | -0.31354 |
| 258 | 0.747123 | -0.32865 |
| 259 | -0.31632 | 1.739569 |
| 260 | 0.825831 | -0.36991 |
| 261 | -0.50468 | -0.02081 |
| 262 | 0.387979 | 0.584358 |
| 263 | 2.049293 | 0.13737 |
| 264 | -0.482 | -0.91783 |
| 265 | -2.81911 | -1.86775 |
| 266 | -0.19503 | -0.47003 |
| 267 | -0.02671 | 1.415572 |
| 268 | -0.38073 | -0.70344 |
| 269 | -1.4107 | 0.214772 |
| 270 | 1.253706 | -1.22195 |
| 271 | 1.879221 | 0.772873 |
| 272 | 0.455635 | 0.833817 |
| 273 | -0.17495 | 0.112013 |
| 274 | -0.7729 | 0.539989 |
| 275 | 0.590563 | 1.456178 |
| 276 | -1.18563 | -0.69358 |
| 277 | 0.380529 | 0.139288 |
| 278 | 0.463008 | -2.35149 |
| 279 | 0.047245 | 1.532602 |
| 280 | 2.095466 | 1.328176 |
| 281 | -0.74064 | 0.522017 |
| 282 | -1.07015 | 0.326975 |
| 283 | 1.914589 | -1.44314 |
| 284 | 0.133123 | 1.229839 |
| 285 | -0.70828 | -0.26286 |
| 286 | -1.60192 | -1.50848 |
| 287 | 0.131394 | -1.63553 |
| 288 | 0.448256 | 0.917572 |
| 289 | 0.291321 | -0.70406 |
| 290 | -1.77845 | 0.461537 |
| 291 | 0.801541 | -0.34689 |
| 292 | 0.655769 | 0.720574 |
| 293 | 1.317247 | -0.91426 |
| 294 | 0.261885 | -0.25623 |
| 295 | 0.263911 | -1.02605 |
| 296 | 0.551645 | -0.11998 |
| 297 | -0.94442 | -1.69194 |
| 298 | -1.12475 | 1.437829 |
| 299 | -0.43916 | -0.96252 |
| 300 | 1.16488 | 0.471043 |
| 301 | 2.832753 | 1.553714 |
| 302 | 0.736882 | -1.83349 |
| 303 | 0.612951 | -1.67105 |
| 304 | 0.454548 | -0.40769 |
| 305 | 0.284457 | 0.576541 |
| 306 | -0.86674 | 1.215636 |
| 307 | -1.85671 | -1.11827 |
| 308 | -1.42227 | 1.3999 |
| 309 | 0.145514 | 1.420671 |
| 310 | -0.09195 | -0.2457 |
| 311 | 0.113107 | -0.59437 |
| 312 | 0.644385 | 0.318136 |
| 313 | -1.42941 | 1.89067 |
| 314 | 0.332982 | 0.671281 |
| 315 | 0.211443 | 0.0099 |
| 316 | 0.1645 | -0.63417 |
| 317 | 2.226396 | 0.745519 |
| 318 | -1.3663 | 0.554042 |
| 319 | -0.62514 | 0.776205 |
| 320 | -0.42821 | -0.10153 |
| 321 | -0.96343 | 2.018122 |
| 322 | 2.592806 | -0.40131 |
| 323 | -0.51963 | -0.89171 |
| 324 | 0.080479 | 0.257162 |
| 325 | 0.125237 | -0.36136 |
| 326 | -0.09777 | 0.463747 |
| 327 | 1.339665 | -1.16691 |
| 328 | -2.19904 | -0.1217 |
| 329 | 0.116225 | -0.5574 |
| 330 | 0.370282 | -0.55109 |
| 331 | -0.56585 | 0.575709 |
| 332 | 0.078517 | -1.25867 |
| 333 | -0.63998 | 0.139579 |
| 334 | 0.878082 | -0.32057 |
| 335 | 0.723166 | 0.771149 |
| 336 | -0.26075 | 0.280087 |
| 337 | 0.847563 | -0.94227 |
| 338 | 1.261161 | 0.568843 |
| 339 | 0.617729 | -1.67872 |
| 340 | -0.02624 | -0.32565 |
| 341 | -0.12063 | -0.5062 |
| 342 | 1.413222 | 1.316965 |
| 343 | 1.767599 | 1.794284 |
| 344 | -2.13529 | 1.665581 |
| 345 | 0.53001 | -0.56849 |
| 346 | 0.650829 | -0.85844 |
| 347 | -1.99032 | 1.966636 |
| 348 | 0.619084 | -1.25124 |
| 349 | -1.44217 | -1.70657 |
| 350 | -0.31124 | 0.920554 |
| 351 | 0.764848 | -0.49393 |
| 352 | 0.044589 | 0.703631 |
| 353 | 0.211831 | -1.07207 |
| 354 | -1.00136 | 1.054915 |
| 355 | 1.173388 | -0.26242 |
| 356 | 0.741422 | 0.03033 |
| 357 | -0.09607 | 0.22436 |
| 358 | -0.74147 | 1.634693 |
| 359 | -0.11593 | 2.330206 |
| 360 | -0.17286 | 0.041886 |
| 361 | 0.00867 | -0.38863 |
| 362 | 0.088977 | -0.68523 |
| 363 | 0.998564 | -0.79101 |
| 364 | 3.295628 | 2.146997 |
| 365 | -0.75167 | -0.21617 |
| 366 | -1.60686 | 0.913739 |
| 367 | -0.66005 | 0.546999 |
| 368 | -0.56738 | -0.43853 |
| 369 | 0.114157 | -1.19931 |
| 370 | -2.02121 | -0.52243 |
| 371 | -0.04816 | -0.56476 |
| 372 | 0.051841 | 0.325243 |
| 373 | -0.08187 | 0.030018 |
| 374 | -0.35163 | -0.90398 |
| 375 | 1.225754 | -2.09676 |
| 376 | 1.128187 | -0.05179 |
| 377 | 1.518524 | -0.30576 |
| 378 | -0.08343 | -0.24672 |
| 379 | 0.804333 | -1.01293 |
| 380 | 1.121503 | 0.944903 |
| 381 | -1.25018 | -0.9489 |
| 382 | -1.05705 | 0.429744 |
| 383 | 0.24272 | 0.560046 |
| 384 | 0.477673 | -0.07328 |
| 385 | 2.923389 | 1.499489 |
| 386 | -1.47505 | 0.75497 |
| 387 | -0.9743 | 0.229118 |
| 388 | 0.087532 | -0.46502 |
| 389 | 1.594751 | -0.82819 |
| 390 | -0.91633 | 0.077167 |
| 391 | -1.4445 | -0.53334 |
| 392 | 0.025976 | -0.66656 |
| 393 | 1.32135 | -0.40929 |
| 394 | 0.78529 | -0.20118 |
| 395 | -0.25673 | 0.420163 |
| 396 | -0.56328 | 0.202355 |
| 397 | -0.67384 | 0.439696 |
| 398 | 0.664373 | -0.7367 |
| 399 | -0.04978 | -0.01886 |

| Group 5 | | |
|---|---|---|
| v(j) | | |
| 0 | 9.196142 | |
| 1 | -18.677 | |
| 2 | -17.1693 | |

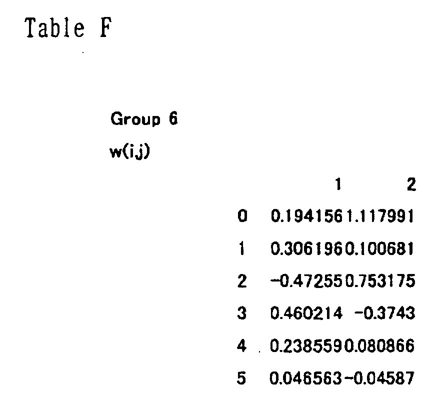

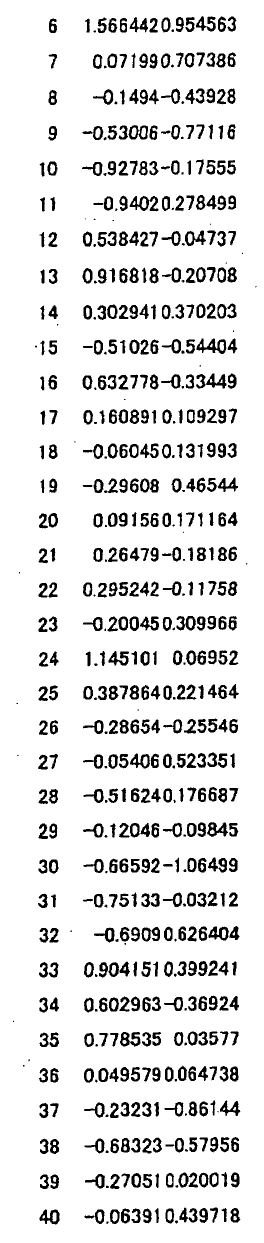

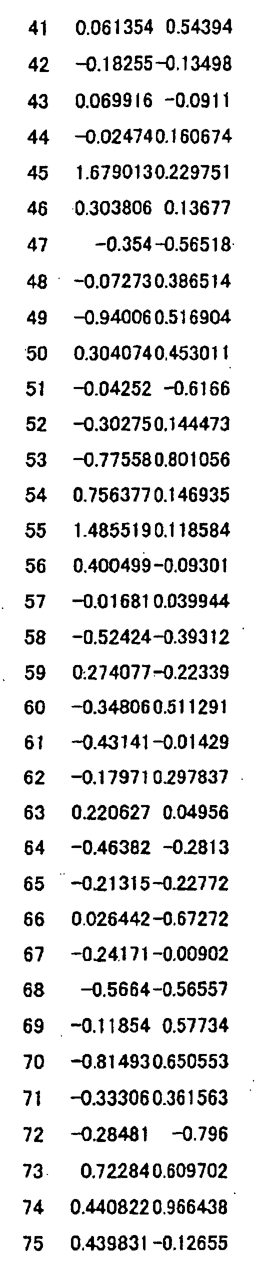

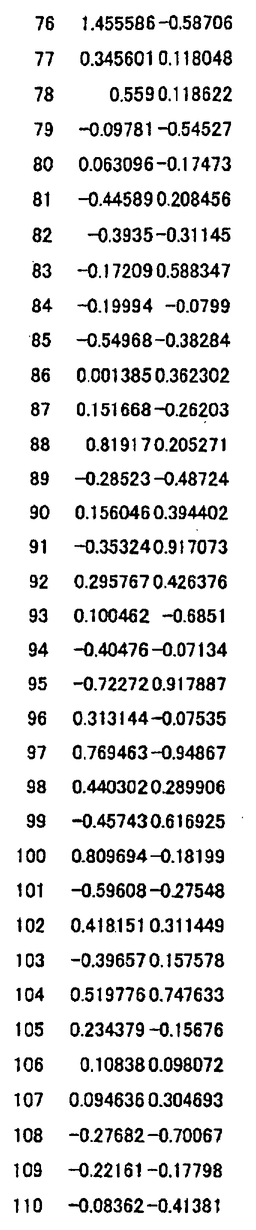

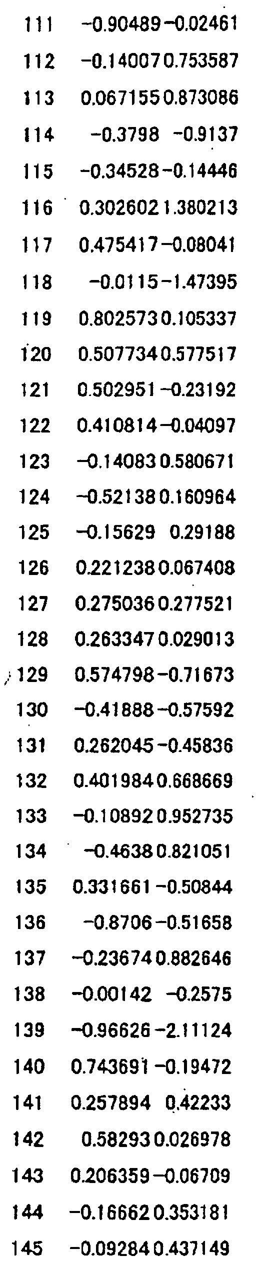

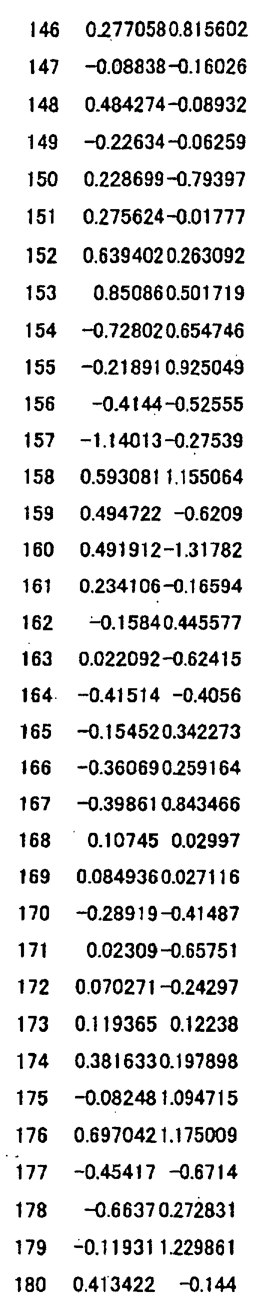

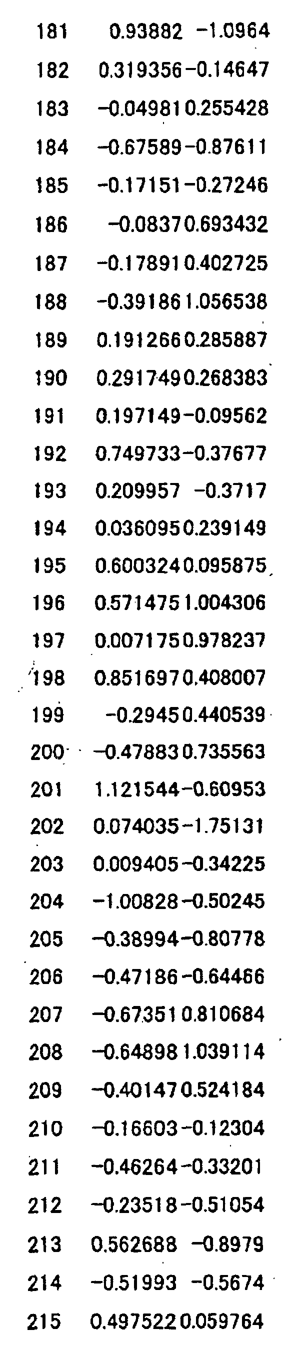

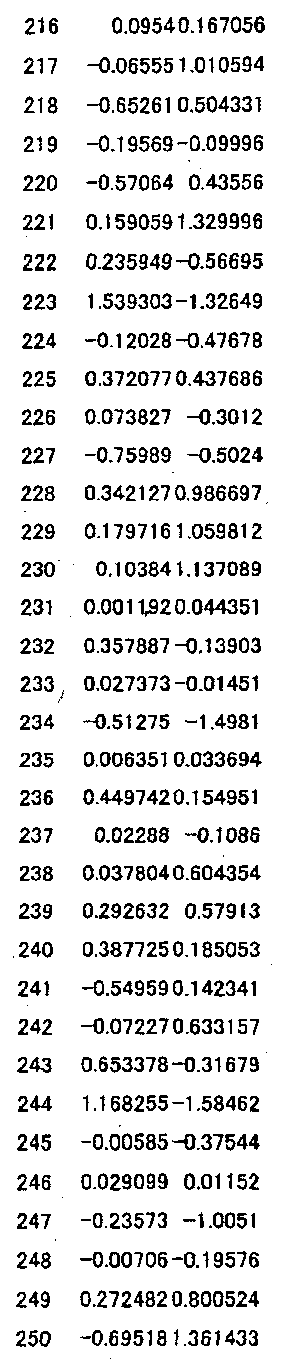

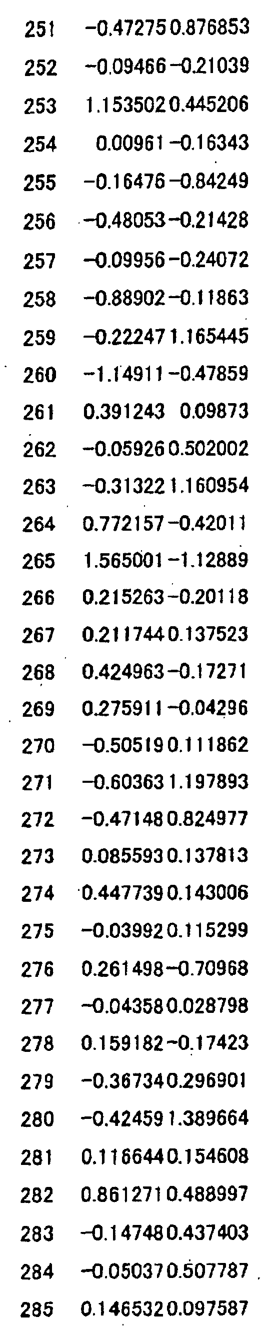

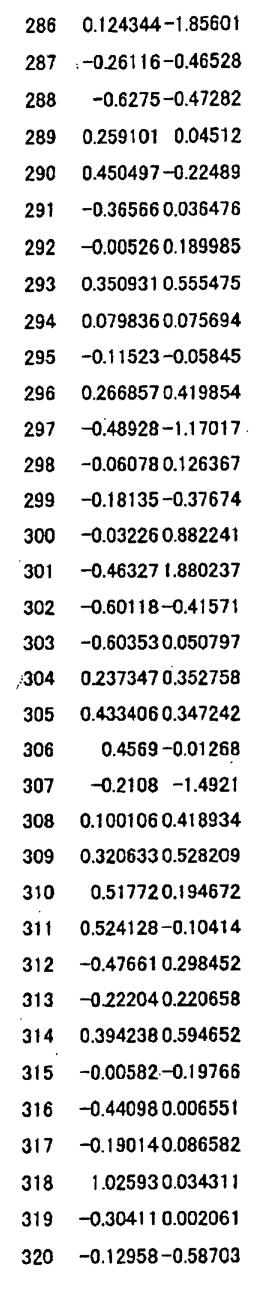

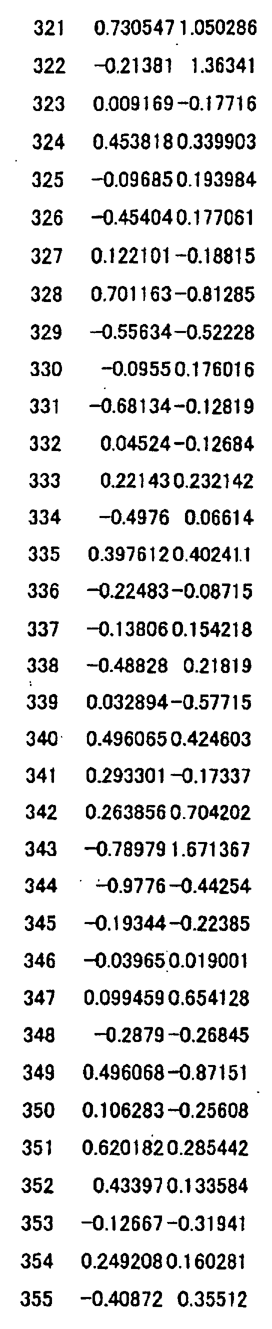

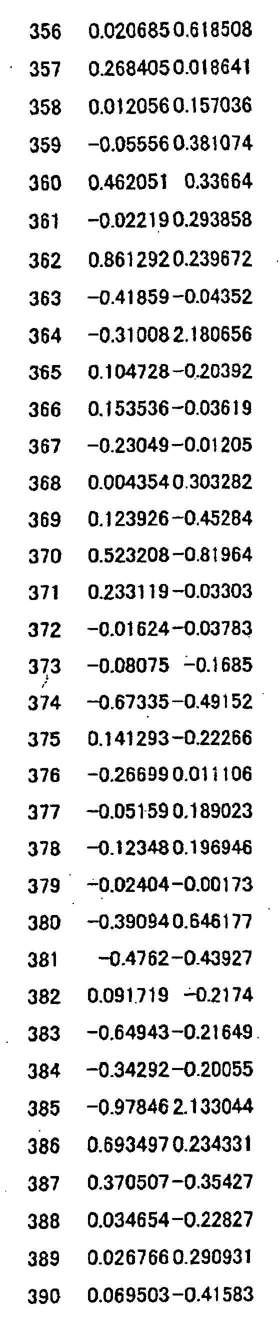

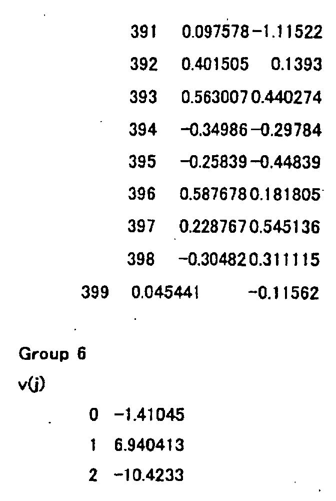

**Table G**

| Group 7 | | |
|---|---|---|
| w(ij) | | |
| | 1 | 2 |
| 0 | -0.16116 | 0.952026 |
| 1 | -0.32202 | 0.025618 |
| 2 | 0.577338 | 2.091769 |
| 3 | 0.547314 | -0.82647 |
| 4 | -0.21961 | -0.11748 |
| 5 | 0.259493 | 0.01009 |
| 6 | 0.037505 | 0.557986 |
| 7 | 0.984537 | 0.451684 |
| 8 | 1.179184 | -1.06802 |
| 9 | 0.134425 | -0.68651 |
| 10 | -0.47375 | 0.523121 |
| 11 | 0.297758 | 0.151143 |
| 12 | -0.6666 | 0.452625 |
| 13 | -0.9244 | -0.14154 |
| 14 | -0.76154 | 0.447989 |
| 15 | 0.19593 | -0.54365 |
| 16 | -1.20564 | -0.08764 |
| 17 | 0.559777 | -0.5726 |
| 18 | -0.28401 | 0.204275 |
| 19 | 0.282494 | 0.592133 |
| 20 | 0.223333 | 0.136176 |
| 21 | 0.172376 | 0.045282 |
| 22 | -1.67958 | 0.501968 |
| 23 | 1.004569 | 0.640574 |
| 24 | -1.74296 | 0.69751 |
| 25 | -0.27663 | 0.290629 |
| 26 | -0.01177 | -0.3867 |
| 27 | 0.047008 | 0.595556 |
| 28 | 0.173677 | 0.759059 |
| 29 | 0.369319 | -0.39987 |
| 30 | 0.523654 | -0.7363 |
| 31 | 0.737732 | -0.03419 |
| 32 | 0.207484 | 0.360778 |
| 33 | -0.11674 | 0.099155 |
| 34 | -0.9762 | -0.34034 |
| 35 | -0.33133 | -0.12398 |
| 36 | 0.962934 | -0.52256 |
| 37 | -0.20453 | -0.66836 |
| 38 | -0.66838 | 0.169429 |
| 39 | 1.027828 | -0.65309 |
| 40 | 0.31039 | 0.058132 |
| 41 | 0.316571 | 0.206955 |
| 42 | 0.016275 | -0.21301 |
| 43 | -0.33655 | 0.362337 |
| 44 | -0.31345 | 0.798333 |
| 45 | -0.90054 | -0.08791 |
| 46 | 0.288776 | 0.20399 |
| 47 | -0.09455 | -0.13539 |
| 48 | 0.51467 | 0.210544 |
| 49 | 0.230771 | 0.86127 |
| 50 | -0.6495 | 0.225224 |
| 51 | 0.158882 | -0.66128 |
| 52 | -0.25408 | 0.905009 |
| 53 | 1.208907 | 0.639759 |
| 54 | 0.218249 | -0.26201 |
| 55 | -0.68484 | -0.19948 |
| 56 | 0.225061 | -0.63116 |
| 57 | 0.491608 | -0.46188 |
| 58 | 0.014772 | -0.26975 |
| 59 | -0.11951 | -0.33449 |
| 60 | 0.077088 | 0.733437 |
| 61 | 0.641571 | -0.05755 |
| 62 | 0.351657 | 0.337222 |
| 63 | 0.008663 | 0.234405 |
| 64 | -0.28145 | 0.194142 |
| 65 | -0.2022 | 1.109594 |
| 66 | -0.21643 | -0.25816 |
| 67 | -0.0611 | 0.252675 |
| 68 | -0.336 | -0.30978 |
| 69 | 0.891509 | 0.367366 |
| 70 | 0.480627 | 1.348569 |
| 71 | -0.72904 | 0.687807 |
| 72 | -0.07202 | -1.21576 |
| 73 | -1.38784 | 0.794157 |
| 74 | 0.030905 | 0.545277 |
| 75 | -0.36854 | -0.08744 |
| 76 | -0.43898 | -1.07104 |
| 77 | 0.072127 | -0.29637 |
| 78 | 0.038991 | -0.3736 |
| 79 | 0.066868 | -0.87201 |
| 80 | -0.6034 | 0.262139 |
| 81 | 1.143917 | -0.91287 |
| 82 | 0.318877 | -0.11209 |
| 83 | 0.290785 | 0.334883 |
| 84 | -0.24084 | -0.16428 |
| 85 | 0.793954 | -0.72614 |
| 86 | 0.0681 | 0.532904 |
| 87 | 0.055778 | -0.47404 |
| 88 | -0.65457 | 0.714498 |
| 89 | -0.12146 | 0.218392 |
| 90 | 0.880572 | 0.050742 |
| 91 | 1.178395 | 0.301675 |
| 92 | -0.01813 | 0.75307 |
| 93 | 0.219745 | -0.9708 |
| 94 | -1.00945 | 0.671983 |
| 95 | 0.576366 | 0.399846 |
| 96 | 0.381798 | -0.23557 |
| 97 | -0.62553 | -0.87244 |
| 98 | 0.568739 | -0.93272 |
| 99 | 0.287672 | 0.645434 |
| 100 | -0.84269 | -0.00767 |
| 101 | 0.548424 | -0.31304 |
| 102 | -0.61381 | 0.363308 |
| 103 | -0.24645 | 0.30292 |
| 104 | -0.41172 | 0.513523 |
| 105 | -0.27476 | -0.19388 |
| 106 | 0.209509 | 0.048639 |
| 107 | 0.28158 | 0.136281 |
| 108 | 0.068161 | -0.97228 |
| 109 | 0.155841 | 0.0172 |
| 110 | 0.00633 | -0.40658 |
| 111 | 0.907108 | -0.17721 |
| 112 | -0.11386 | 0.775573 |
| 113 | 0.444104 | 0.605973 |
| 114 | -0.00253 | -1.08272 |
| 115 | -0.47473 | -0.07385 |
| 116 | 0.492717 | 0.878332 |
| 117 | -0.30503 | -0.07812 |
| 118 | -1.08111 | -0.96659 |
| 119 | 0.185648 | -0.13622 |
| 120 | -0.37399 | 0.99358 |
| 121 | -0.0055 | -0.79363 |
| 122 | -0.96044 | 0.333197 |
| 123 | -0.03455 | -0.0014 |
| 124 | -0.12856 | 0.451339 |
| 125 | 0.247729 | 0.081733 |
| 126 | 0.263341 | 0.271675 |
| 127 | 0.246978 | -0.21531 |
| 128 | 0.005498 | 0.117313 |
| 129 | -0.41252 | -0.49146 |
| 130 | 0.226321 | -0.54646 |
| 131 | -0.46116 | 0.097586 |
| 132 | 0.92645 | -0.14832 |
| 133 | 0.723156 | 1.507419 |
| 134 | 0.697545 | 0.33707 |
| 135 | -0.53302 | -0.44478 |
| 136 | -0.14883 | 0.013437 |
| 137 | 0.710592 | 0.679529 |
| 138 | 0.233794 | -0.78629 |
| 139 | -1.41571 | -1.15975 |
| 140 | -0.61608 | -0.31949 |
| 141 | 0.34281 | 0.363431 |
| 142 | -0.30402 | -0.41221 |
| 143 | -0.21014 | -0.08596 |
| 144 | 0.37367 | -0.26087 |
| 145 | -0.16392 | 0.854498 |
| 146 | -0.28934 | 0.656717 |
| 147 | -0.22147 | -0.09179 |
| 148 | 0.050573 | -0.35 |
| 149 | -0.12584 | 0.408706 |
| 150 | -0.34467 | -0.61728 |
| 151 | 0.500646 | -0.47403 |
| 152 | -0.25914 | -0.27107 |
| 153 | 0.746127 | -0.33074 |
| 154 | 1.44325 | 0.908748 |
| 155 | -0.29912 | 1.122012 |
| 156 | -0.37679 | -0.534 |
| 157 | 0.320957 | -0.27257 |
| 158 | -0.2564 | 0.639578 |
| 159 | 0.627944 | -1.11724 |
| 160 | -1.68237 | -0.9094 |
| 161 | -0.66335 | 0.123786 |
| 162 | 0.556378 | 0.256135 |
| 163 | -0.27528 | -0.79806 |
| 164 | -1.11223 | 0.831075 |
| 165 | -0.42788 | 0.391855 |
| 166 | 0.779897 | -0.11279 |
| 167 | 0.683911 | 0.799801 |
| 168 | -0.02281 | -0.20089 |
| 169 | -0.43741 | 0.182329 |
| 170 | 0.118584 | 0.104221 |
| 171 | -0.45789 | -0.36884 |
| 172 | -0.25323 | 0.284032 |
| 173 | 0.480395 | 0.030552 |
| 174 | 0.571073 | -0.40809 |
| 175 | 0.5511 | 0.627068 |
| 176 | 0.494763 | 0.466723 |
| 177 | -0.14581 | -0.16282 |
| 178 | 0.119332 | 0.33166 |
| 179 | 0.263196 | 0.827155 |
| 180 | -0.72626 | -0.18538 |
| 181 | -0.16067 | -1.81726 |
| 182 | -0.47213 | -0.31826 |
| 183 | 0.173686 | -0.25636 |
| 184 | -0.1471 | -0.73623 |
| 185 | -0.83421 | 0.269216 |
| 186 | -0.08911 | 0.699163 |
| 187 | 0.729552 | -0.36486 |
| 188 | 0.511894 | 0.938879 |
| 189 | 0.024353 | 0.098312 |
| 190 | 0.09891 | -0.18622 |
| 191 | 0.028666 | 0.360353 |
| 192 | 0.150558 | -0.99021 |
| 193 | -0.01256 | -0.18229 |
| 194 | 0.206479 | 0.011154 |
| 195 | 0.347881 | -0.03464 |
| 196 | 0.46512 | 0.608844 |
| 197 | -0.63944 | 1.131016 |
| 198 | -0.5466 | 0.471751 |
| 199 | -0.50893 | 0.775994 |
| 200 | 0.410304 | 0.794308 |
| 201 | -0.30276 | -0.9032 |
| 202 | -0.70618 | -1.92498 |
| 203 | 0.263135 | -0.48577 |
| 204 | 0.259449 | -0.31257 |
| 205 | 0.041894 | -0.71755 |
| 206 | -0.67119 | -0.00392 |
| 207 | 0.71847 | 0.273196 |
| 208 | 1.152892 | 0.29791 |
| 209 | -0.35021 | 1.304214 |
| 210 | -0.28575 | -0.03429 |
| 211 | 0.006433 | -0.32892 |
| 212 | 0.392356 | -0.51691 |
| 213 | 0.836076 | -2.1572 |
| 214 | -0.26051 | 0.351812 |
| 215 | 0.458575 | -0.2674 |
| 216 | 0.004712 | 0.241106 |
| 217 | -0.67989 | 1.429458 |
| 218 | 0.696202 | 0.531781 |
| 219 | -0.49787 | 0.254954 |
| 220 | 0.921626 | 0.209449 |
| 221 | -0.15413 | 0.723596 |
| 222 | -0.28136 | -0.66827 |
| 223 | -1.37797 | -1.65337 |
| 224 | -0.2317 | -0.47489 |
| 225 | -0.05378 | 0.00638 |
| 226 | -0.20323 | -0.20444 |
| 227 | -0.7349 | 0.215366 |
| 228 | 0.222201 | 0.719393 |
| 229 | -0.11264 | 1.197522 |
| 230 | 0.073209 | 1.618749 |
| 231 | -0.08599 | -0.00337 |
| 232 | -0.42299 | 0.211071 |
| 233 | 0.473687 | -0.36608 |
| 234 | -0.40803 | -0.80058 |
| 235 | 0.447448 | 0.107415 |
| 236 | -0.21912 | -0.26223 |
| 237 | 0.145861 | 0.584819 |
| 238 | 0.052241 | 0.841711 |
| 239 | -0.21356 | 0.615208 |
| 240 | 0.184003 | -0.35891 |
| 241 | 1.012649 | -0.0815 |
| 242 | -0.1204 | 0.719037 |
| 243 | -0.31649 | -0.65588 |
| 244 | -1.75328 | -0.93674 |
| 245 | -0.12473 | -0.44169 |
| 246 | 0.001966 | 0.086703 |
| 247 | 0.202073 | -0.85561 |
| 248 | 0.066234 | -0.28649 |
| 249 | 0.110118 | 0.129543 |
| 250 | 0.130556 | 1.557635 |
| 251 | 0.039979 | 0.869844 |
| 252 | 0.226513 | -0.16183 |
| 253 | -0.23691 | -0.01831 |
| 254 | -0.11286 | 0.295167 |
| 255 | -0.70859 | -0.21826 |
| 256 | 0.337634 | 0.272778 |
| 257 | 0.328478 | -0.94128 |
| 258 | 1.119938 | -0.22344 |
| 259 | 0.73273 | 1.161907 |
| 260 | -0.17326 | 0.683648 |
| 261 | 0.473915 | -0.3837 |
| 262 | 0.870057 | 0.079204 |
| 263 | 0.19465 | 0.798324 |
| 264 | -0.9505 | 0.136983 |
| 265 | -1.87403 | -0.88188 |
| 266 | -0.33104 | -0.40796 |
| 267 | 0.492426 | -0.35027 |
| 268 | -0.26282 | -0.23165 |
| 269 | 0.276232 | -0.39329 |
| 270 | 0.918487 | -0.232 |
| 271 | 0.628287 | 1.132801 |
| 272 | -0.71454 | 1.243445 |
| 273 | -0.18391 | -0.09509 |
| 274 | 0.154282 | -0.29892 |
| 275 | -0.5197 | 0.593967 |
| 276 | -0.5339 | -0.00346 |
| 277 | -0.04428 | 0.304153 |
| 278 | -0.05151 | -0.14267 |
| 279 | 0.569344 | 0.822627 |
| 280 | 1.277337 | 0.533094 |
| 281 | 0.037228 | 0.429038 |
| 282 | -0.04906 | -0.12204 |
| 283 | -0.36835 | 0.865235 |
| 284 | -0.19801 | 0.235206 |
| 285 | -0.03786 | 0.174635 |
| 286 | -0.62758 | -1.85625 |
| 287 | 0.064 | -0.2429 |
| 288 | 0.492912 | -0.28661 |
| 289 | 0.543405 | -0.38539 |
| 290 | -0.39653 | -0.42192 |
| 291 | -0.31924 | 0.399616 |
| 292 | 0.477591 | 0.434302 |
| 293 | -0.95127 | 0.85898 |
| 294 | 0.120295 | -0.01517 |
| 295 | -0.09382 | 0.103287 |
| 296 | -0.28005 | 0.863913 |
| 297 | -0.44324 | -0.62813 |
| 298 | -0.084 | 0.454225 |
| 299 | -0.07921 | -0.14424 |
| 300 | 0.131555 | 0.814075 |
| 301 | -0.21798 | 1.835027 |
| 302 | 0.533602 | -0.73434 |
| 303 | 0.40985 | -0.45349 |
| 304 | 0.106818 | 0.176583 |
| 305 | 0.084243 | -0.94748 |
| 306 | -0.1197 | -0.23802 |
| 307 | -0.77802 | -1.32853 |
| 308 | 0.723337 | -0.87406 |
| 309 | 0.156401 | 0.212868 |
| 310 | -0.82775 | 0.731181 |
| 311 | -0.09839 | -0.35822 |
| 312 | 0.377462 | -0.06259 |
| 313 | -0.29508 | 0.686754 |
| 314 | 0.258617 | 0.059888 |
| 315 | -0.27161 | -0.18004 |
| 316 | -0.06366 | 0.536997 |
| 317 | 0.494787 | 0.263148 |
| 318 | -0.229 | -0.28755 |
| 319 | 0.035704 | 0.294238 |
| 320 | -0.00665 | -0.44558 |
| 321 | 0.60288 | 0.517194 |
| 322 | 0.322324 | 1.062177 |
| 323 | 0.305631 | -0.62619 |
| 324 | -0.7877 | 0.712856 |
| 325 | -0.48418 | 0.552661 |
| 326 | 0.51245 | -0.93216 |
| 327 | -0.42594 | -0.0971 |
| 328 | -0.99706 | -0.6507 |
| 329 | 0.090135 | 0.083225 |
| 330 | -0.58068 | 0.070852 |
| 331 | 0.364399 | -0.17893 |
| 332 | -0.02607 | -0.14066 |
| 333 | 0.509021 | -0.76237 |
| 334 | -0.50758 | 1.123283 |
| 335 | 0.273302 | 0.230054 |
| 336 | 0.199687 | 0.276129 |
| 337 | 0.398315 | -0.07461 |
| 338 | -0.04843 | 0.285003 |
| 339 | -0.8104 | 0.361751 |
| 340 | -0.66519 | 0.609338 |
| 341 | -1.8071 | 0.657019 |
| 342 | 1.072492 | 0.49836 |
| 343 | 0.945935 | 1.252245 |
| 344 | 0.463992 | -0.35137 |
| 345 | 0.544405 | -0.52372 |
| 346 | 0.208211 | 0.102906 |
| 347 | 0.071478 | -0.56062 |
| 348 | 0.477881 | -0.74869 |
| 349 | -0.54452 | -1.11115 |
| 350 | -0.13797 | -0.23512 |
| 351 | -0.2446 | -0.16621 |
| 352 | -0.46765 | 0.371339 |
| 353 | -0.29119 | 0.109423 |
| 354 | 0.937551 | -1.11605 |
| 355 | 0.116678 | 0.900321 |
| 356 | -0.06633 | 0.93897 |
| 357 | 0.006084 | -0.04327 |
| 358 | -0.14393 | 0.314732 |
| 359 | -0.29552 | 0.34999 |
| 360 | 0.101242 | -0.30007 |
| 361 | -0.48111 | 0.627135 |
| 362 | -0.53688 | 0.448549 |
| 363 | 0.513632 | 0.105445 |
| 364 | 1.068519 | 1.835874 |
| 365 | 0.524791 | -0.60243 |
| 366 | 0.165395 | -0.32997 |
| 367 | -0.39774 | -0.07011 |
| 368 | -0.14967 | -0.26553 |
| 369 | -0.45352 | -0.20844 |
| 370 | -0.44374 | -1.15758 |
| 371 | 0.193073 | -0.03592 |
| 372 | 0.090713 | -0.24465 |
| 373 | 0.103573 | 0.154867 |
| 374 | -0.02979 | 0.115943 |
| 375 | 0.224572 | -0.48044 |
| 376 | -0.0975 | 0.889975 |
| 377 | 0.293523 | 0.357257 |
| 378 | -0.0797 | 0.152286 |
| 379 | -0.13368 | 0.136809 |
| 380 | 0.040422 | 0.564384 |
| 381 | -0.61705 | 0.321536 |
| 382 | 0.634972 | -0.71585 |
| 383 | 0.101148 | 0.111547 |
| 384 | -0.02348 | 0.397552 |
| 385 | 0.91179 | 1.208421 |
| 386 | -0.15862 | -0.10794 |
| 387 | -0.10705 | -0.45336 |
| 388 | 0.047635 | -0.48201 |
| 389 | -0.35233 | 0.268381 |
| 390 | -0.60686 | 0.001003 |
| 391 | -0.03156 | -1.36357 |
| 392 | 0.165383 | -0.48752 |
| 393 | -0.49348 | 0.412971 |
| 394 | 0.284205 | -0.19159 |
| 395 | -0.34574 | 0.03731 |
| 396 | -0.11658 | -0.15478 |
| 397 | 0.54125 | 0.570973 |
| 398 | 0.110871 | 0.145109 |
| 399 | 0.34038 | 0.103448 |

| Group 7 | | |
|---|---|---|
| v(j) | | |
| 0 | 5.144898 | |
| 1 | -9.0301 | |
| 2 | -10.2899 | |

**Table H**

| Group 8 | | |
|---|---|---|
| w(ij) | | |
| | 1 | 2 |
| 0 | 0.280176 | 0.322336 |
| 1 | 0.089863 | -0.16466 |
| 2 | 0.258712 | 0.13301 |
| 3 | -0.35689 | -0.10317 |
| 4 | 0.072041 | -0.08645 |
| 5 | -0.23186 | 0.270893 |
| 6 | 0.31259 | 0.16543 |
| 7 | 0.559172 | -0.00685 |
| 8 | 0.06183 | -0.15552 |
| 9 | -0.15374 | -0.21874 |
| 10 | 0.101713 | 0.085875 |
| 11 | 0.558737 | 0.326798 |
| 12 | 0.046735 | -0.00874 |
| 13 | -0.24817 | -0.4984 |
| 14 | -0.05777 | -0.02885 |
| 15 | 0.206622 | 0.054918 |
| 16 | -0.20067 | -0.2843 |
| 17 | -0.15782 | 0.154129 |
| 18 | 0.261983 | -0.03436 |
| 19 | -0.19116 | 0.10826 |
| 20 | 0.384408 | -0.0457 |
| 21 | -0.07824 | -0.12549 |
| 22 | -0.2621 | 0.151674 |
| 23 | 0.05061 | 0.419858 |
| 24 | -0.40798 | 0.043756 |
| 25 | 0.03181 | 0.065562 |
| 26 | -0.0728 | -0.35157 |
| 27 | 0.18568 | 0.048925 |
| 28 | 0.258083 | 0.374686 |
| 29 | -0.20178 | 0.150815 |
| 30 | -0.37952 | -0.13445 |
| 31 | -0.07022 | 0.128067 |
| 32 | 0.487422 | 0.357583 |
| 33 | -0.13862 | -0.076 |
| 34 | -0.50341 | -0.2973 |
| 35 | -0.16533 | -0.12502 |
| 36 | 0.326894 | -0.25499 |
| 37 | -0.24026 | -0.42517 |
| 38 | -0.21263 | -0.38549 |
| 39 | 0.063399 | -0.03075 |
| 40 | 0.121922 | -0.03443 |
| 41 | 0.321519 | -0.0844 |
| 42 | 0.224381 | -0.28818 |
| 43 | 0.027942 | 0.194588 |
| 44 | 0.125309 | 0481723 |
| 45 | -0.14902 | -0.07481 |
| 46 | -0.07075 | 0.080686 |
| 47 | -0.23067 | 0.02413 |
| 48 | 0.262883 | 0.383931 |
| 49 | 0.170966 | 0.311139 |
| 50 | -0.04542 | 0.210747 |
| 51 | -0.26566 | -0.11295 |
| 52 | 0.204875 | 0.106507 |
| 53 | 0.411018 | 0.59082 |
| 54 | -0.0726 | -0.09807 |
| 55 | -0.44838 | -0.25068 |
| 56 | -0.26283 | 0.077592 |
| 57 | 0.053487 | 0.200935 |
| 58 | -0.08799 | -0.06156 |
| 59 | -0.19695 | -0.31923 |
| 60 | 0.199526 | 0.080912 |
| 61 | -0.07185 | -0.19526 |
| 62 | 0.196895 | 0.332062 |
| 63 | -0.18528 | 0.045242 |
| 64 | 0.307743 | -0.15154 |
| 65 | 0.273907 | 0.263797 |
| 66 | -0.176 | -0.15571 |
| 67 | 0.044056 | -0.05496 |
| 68 | -0.30912 | -0.04222 |
| 69 | 0.449623 | 0.328544 |
| 70 | 0.408023 | 0.352031 |
| 71 | 0.047199 | 0.197917 |
| 72 | -0.14097 | -0.20277 |
| 73 | 0.121769 | 0.144908 |
| 74 | 0.457205 | 0.433156 |
| 75 | -0.16274 | -0.09385 |
| 76 | -0.60701 | -0.25247 |
| 77 | -0.07979 | -0.11969 |
| 78 | -0.00318 | 0.257171 |
| 79 | -0.1114 | -0.10213 |
| 80 | -0.19517 | -0.20672 |
| 81 | 0.176544 | 0.049347 |
| 82 | -0.10583 | -0.14123 |
| 83 | 0.356354 | 0.167297 |
| 84 | 0.11891 | 0.042833 |
| 85 | 0.180655 | -0.20791 |
| 86 | 0.280233 | 0.099587 |
| 87 | -0.19843 | 0.12152 |
| 88 | -0.13518 | 0.130912 |
| 89 | -0.04634 | -0.11816 |
| 90 | 0.324811 | 0.214844 |
| 91 | 0.167347 | 0.391105 |
| 92 | 0.520048 | -0.06311 |
| 93 | -0.3756 | -0.26741 |
| 94 | -0.00007 | 0.143016 |
| 95 | 0.257771 | 0.641781 |
| 96 | -0.15785 | -0.11424 |
| 97 | -0.62828 | -0.51594 |
| 98 | -0.35792 | 0.070469 |
| 99 | 0.285154 | 0.138717 |
| 100 | -0.24297 | -0.05282 |
| 101 | -0.2569 | -0.09424 |
| 102 | 0.149283 | -0.15182 |
| 103 | -0.2092 | 0.192871 |
| 104 | 0.230196 | 0.059552 |
| 105 | -0.13162 | -0.00127 |
| 106 | 0.044484 | 0.028085 |
| 107 | 0.192866 | 0.09894 |
| 108 | -0.233 | -0.09201 |
| 109 | -0.13998 | 0.01842 |
| 110 | -0.15383 | 0.110923 |
| 111 | 0.173836 | 0.274321 |
| 112 | 0.51414 | 0.133339 |
| 113 | 0.182077 | 0.371687 |
| 114 | -0.29869 | -0.42132 |
| 115 | 0.053145 | 0.03305 |
| 116 | 0.352281 | 0.588561 |
| 117 | -0.18262 | -0.06152 |
| 118 | -0.79579 | -0.57692 |
| 119 | -0.12687 | -0.1593 |
| 120 | 0.316487 | 0.038593 |
| 121 | 0.017199 | -0.17629 |
| 122 | -0.09134 | -0.22363 |
| 123 | -0.04756 | 0.228905 |
| 124 | 0.252189 | -0.09371 |
| 125 | 0.116935 | 0.12619 |
| 126 | 0.251119 | 0.050925 |
| 127 | 0.127259 | -0.0269 |
| 128 | 0.05564 | 0.288694 |
| 129 | -0.25431 | -0.24257 |
| 130 | -0.03116 | -0.12309 |
| 131 | -0.05097 | 0.022442 |
| 132 | 0.04139 | 0.249297 |
| 133 | 0.529803 | 0.28221 |
| 134 | 0.361491 | 0.42698 |
| 135 | -0.51547 | -0.00114 |
| 136 | 0.053323 | 0.010736 |
| 137 | 0.696979 | 0.243455 |
| 138 | -0.04103 | -0.13276 |
| 139 | -0.87638 | -0.56972 |
| 140 | -0.29005 | 0.0786 |
| 141 | 0.394238 | -0.04498 |
| 142 | -0.11369 | -0.22259 |
| 143 | -0.32284 | 0.008446 |
| 144 | -0.08911 | 0.2045 |
| 145 | -0.10728 | 0.052944 |
| 146 | 0.177407 | 0.098888 |
| 147 | -0.09009 | 0.120616 |
| 148 | -0.07467 | 0.01718 |
| 149 | -0.00037 | 0.392235 |
| 150 | -0.22564 | -0.21368 |
| 151 | -0.05539 | 0.048398 |
| 152 | 0.221042 | 0.003341 |
| 153 | -0.21499 | 0.160504 |
| 154 | 0.641006 | 0.106168 |
| 155 | 0.363684 | 0.414426 |
| 156 | -0.1965 | -0.29292 |
| 157 | 0.185528 | 0.232695 |
| 158 | 0.269108 | 0.635684 |
| 159 | -0.17473 | -0.232 |
| 160 | -0.76539 | -0.79342 |
| 161 | -0.16027 | 0.078819 |
| 162 | 0.308538 | 0.108572 |
| 163 | -0.29246 | -0.25191 |
| 164 | -0.15875 | -0.10026 |
| 165 | 0.145114 | 0.27188 |
| 166 | 0.172042 | 0.061138 |
| 167 | 0.295894 | 0.293787 |
| 168 | 0.028913 | -0.07227 |
| 169 | 0.026949 | -0.12841 |
| 170 | 0.087546 | 0.153603 |
| 171 | -0.18152 | -0.34885 |
| 172 | -0.0285 | -0.1266 |
| 173 | 0.018039 | 0.117074 |
| 174 | 0.013201 | -0.17427 |
| 175 | 0.328484 | 0.283735 |
| 176 | 0.263523 | 0.462784 |
| 177 | -0.04506 | -0.39274 |
| 178 | 0.21313 | 0.100068 |
| 179 | 0.639239 | 0.458409 |
| 180 | -0.29708 | -0.15314 |
| 181 | -0.75986 | -0.8363 |
| 182 | 0.140368 | -0.35048 |
| 183 | -0.02458 | 0.328242 |
| 184 | -0.17431 | -0.43726 |
| 185 | 0.000826 | -0.37192 |
| 186 | 0.376571 | 0.251457 |
| 187 | 0.334845 | -0.00291 |
| 188 | 0.462598 | 0.616313 |
| 189 | 0.218829 | -0.22532 |
| 190 | -0.12587 | 0.119885 |
| 191 | 0.276037 | 0.059948 |
| 192- | -0.15675 | -0.40897 |
| 193 | -0.25608 | -0.043 |
| 194 | -0.01207 | 0.085644 |
| 195 | 0.003494 | -0.09893 |
| 196 | 0.571325 | 0.162064 |
| 197 | 0.398344 | 0.495579 |
| 198 | -0.08543 | -0.22323 |
| 199 | 0.008196 | 0.408179 |
| 200 | 0.591552 | 0.060628 |
| 201 | -0.49251 | -0.30129 |
| 202 | -1.07518 | -0.71723 |
| 203 | -0.29767 | -0.10512 |
| 204 | 0.099298 | 0.197993 |
| 205 | -0.19574 | -0.24457 |
| 206 | -0.37491 | -0.12382 |
| 207 | 0.329921 | 0.421738 |
| 208 | 0.105327 | -0.01787 |
| 209 | 0.432718 | 0.221158 |
| 210 | 0.294576 | 0.169892 |
| 211 | 0.200918 | -0.17751 |
| 212 | 0.155954 | -0.10067 |
| 213 | -0.32383 | -0.38157 |
| 214 | 0.182018 | -0.27661 |
| 215 | 0.032786 | -0.17018 |
| 216 | 0.222737 | -0.08613 |
| 217 | 0.07883 | 0.595989 |
| 218 | 0.516062 | 0.610738 |
| 219 | 0.148437 | -0.38454 |
| 220 | 0.09305 | 0.514056 |
| 221 | 0.619208 | 0.253326 |
| 222 | -0.50617 | -0.26182 |
| 223 | -1.03036 | -0.98533 |
| 224 | -0.09114 | -0.09227 |
| 225 | 0.430771 | 0.115833 |
| 226 | -0.24198 | -0.08795 |
| 227 | -0.1943 | -0.24671 |
| 228 | 0.256378 | 0.37642 |
| 229 | 0.097133 | 0.178745 |
| 230 | 0.291176 | 0.598428 |
| 231 | 0.185446 | -0.18283 |
| 232 | -0.1262 | 0.081021 |
| 233 | 0.364879 | 0.20601 |
| 234 | -0.16759 | -0.33473 |
| 235 | 0.354533 | -0.13748 |
| 236 | 0.088811 | 0.048252 |
| 237 | 0.275667 | 0.066499 |
| 238 | 0.553402 | 0.198148 |
| 239 | 0.192956 | 0.252252 |
| 240 | 0.046442 | -0.11814 |
| 241 | 0.549325 | 0.021857 |
| 242 | 0.534248 | 0.197887 |
| 243 | -0.18942 | -0.26986 |
| 244 | -1.0251 | -0.7881 |
| 245 | 0.085048 | -0.29609 |
| 246 | 0.286335 | 0.242831 |
| 247 | -0.35344 | -0.03213 |
| 248 | -0.05745 | -0.3493 |
| 249 | 0.261177 | 0.485355 |
| 250 | 0.429397 | 0.036518 |
| 251 | 0.304101 | 0.37675 |
| 252 | 0.178639 | -0.13729 |
| 253 | 0.146889 | -0.20496 |
| 254 | 0.311676 | 0.069606 |
| 255 | -0.2809 | 0.066729 |
| 256 | 0.173884 | -0.00731 |
| 257 | 0.082149 | -0.12322 |
| 258 | 0.131881 | 0.256422 |
| 259 | 0.436154 | 0.519177 |
| 260 | 0.19433 | 0.131613 |
| 261 | -0.05006 | -0.10751 |
| 262 | 0.356847 | 0.239002 |
| 263 | 0.557269 | 0.137655 |
| 264 | -0.20516 | -0.27195 |
| 265 | -0.89702 | -0.78432 |
| 266 | -0.18417 | -0.20021 |
| 267 | 0.330243 | 0.174138 |
| 268 | 0.065072 | -0.16737 |
| 269 | -0.05387 | -0.20715 |
| 270 | 0.223589 | 0.123392 |
| 271 | 0.157142 | 0.260878 |
| 272 | 0.489482 | 0.289157 |
| 273 | -0.17691 | 0.001684 |
| 274 | 0.079506 | -0.13101 |
| 275 | 0.284311 | 0.107616 |
| 276 | -0.14871 | -0.3219 |
| 277 | 0.075672 | 0.086463 |
| 278 | 0.03304 | -0.24115 |
| 279 | 0.451536 | 0.09847 |
| 280 | 0.393575 | 0.47111 |
| 281 | 0.215062 | -0.08996 |
| 282 | -0.1232 | 0.106244 |
| 283 | 0.052652 | 0.321821 |
| 284 | 0.146523 | 0.361367 |
| 285 | -0.08415 | -0.18466 |
| 286 | -0.98776 | -0.64694 |
| 287 | -0.14207 | -0.01228 |
| 288 | 0.22634 | 0.09001 |
| 289 | -0.06194 | 0.277908 |
| 290 | -0.20718 | -0.25136 |
| 291 | 0.019461 | 0.093787 |
| 292 | 0.023885 | 0.067402 |
| 293 | 0.378495 | 0.283371 |
| 294 | -0.22544 | -0.13846 |
| 295 | -0.10132 | 0.020483 |
| 296 | 0.2432 | 0.240385 |
| 297 | -0.19961 | -0.41433 |
| 298 | 0.104077 | -0.14921 |
| 299 | 0.053988 | -0.32661 |
| 300 | 0.38022 | 0.138622 |
| 301 | 0.562018 | 0.715657 |
| 302 | -0.13685 | -0.14249 |
| 303 | -0.03016 | -0.22117 |
| 304 | 0.14678 | -0.04298 |
| 305 | -0.10325 | -0.00728 |
| 306 | -0.1241 | -0.11444 |
| 307 | -0.76743 | -0.67222 |
| 308 | -0.27217 | 0.198293 |
| 309 | 0.218046 | -0.1459 |
| 310 | -0.04301 | 0.159191 |
| 311 | -0.58886 | -0.0227 |
| 312 | 0.092836 | -0.1229 |
| 313 | 0.194934 | -0.01003 |
| 314 | 0.244997 | -0.00846 |
| 315 | -0.08548 | 0.285201 |
| 316 | -0.03473 | 0.141617 |
| 317 | 0.438175 | 0.051332 |
| 318 | -0.16444 | -0.26022 |
| 319 | -0.07391 | 0.202322 |
| 320 | -0.28044 | -0.0554 |
| 321 | 0.114254 | 0.401794 |
| 322 | 0.492382 | 0.57594 |
| 323 | -0.0815 | -0.15213 |
| 324 | -0.03754 | -0.04391 |
| 325 | 0.157412 | 0.035032 |
| 326 | -0.02602 | 0.392123 |
| 327 | -0.17738 | -0.14248 |
| 328 | -0.59422 | -0.45361 |
| 329 | 0.009462 | -0.02529 |
| 330 | -0.16892 | 0.339293 |
| , 331 | 0.209446 | 0.089063 |
| 332 | -0.24768 | -0.05874 |
| 333 | 0.128149 | -0.20183 |
| 334 | 0.045111 | -0.16833 |
| 335 | 0.076539 | 0.080288 |
| 336 | 0.152465 | 0.140161 |
| 337 | 0.002925 | -0.04547 |
| 338 | 0.344921 | 0.020747 |
| 339 | -0.16712 | -0.1798 |
| 340 | -0.28057 | 0.172974 |
| 341 | -0.28399 | -0.09391 |
| 342 | 0.242239 | 0.080815 |
| 343 | 0.629515 | 0.717999 |
| 344 | -0.36706 | -0.14904 |
| 345 | -0.18594 | -0.05377 |
| 346 | 0.122529 | -0.03742 |
| 347 | 0.209078 | 0.088422 |
| 348 | 0.142492 | -0.3696 |
| 349 | -0.49413 | -0.46858 |
| 350 | 0.017413 | -0.03532 |
| 351 | 0.022092 | 0.02744 |
| 352 | 0.021223 | 0.167044 |
| 353 | -0.08818 | -0.33604 |
| 354 | -0.10013 | -0.07328 |
| 355 | -0.10447 | 0.035356 |
| 356 | 0.158499 | 0.269667 |
| 357 | 0.137598 | -0.16839 |
| 358 | 0.053401 | 0.09205 |
| 359 | 0.295167 | 0.254434 |
| 360 | -0.22037 | 0.040353 |
| 361 | 0.1523 | 0.007335 |
| 362 | 0.160472 | -0.38438 |
| 363 | 0.079779 | 0.095844 |
| 364 | 0.593924 | 0.902876 |
| 365 | -0.07806 | -0.24758 |
| 366 | -0.03983 | -0.17643 |
| 367 | -0.02031 | 0.142277 |
| 368 | -0.06825 | 0.348749 |
| 369 | -0.2862 | 0.084045 |
| 370 | -0.68083 | -0.2086 |
| 371 | -0.05227 | -0.0774 |
| 372 | 0.043616 | 0.013121 |
| 373 | 0.193444 | 0.212376 |
| 374 | 0.038471 | -0.24379 |
| 375 | 0.016123 | -0.24717 |
| 376 | -0.04567 | 0.058567 |
| 377 | 0.179515 | 0.190871 |
| 378 | -0.01504 | 0.063935 |
| 379 | 0.035176 | 0.008966 |
| 380 | 0.195784 | 0.384433 |
| 381 | 0.055274 | -0.34632 |
| 382 | -0.25716 | 0.151064 |
| 383 | -0.09593 | 0.058775 |
| 384 | 0.019821 | 0.176833 |
| 385 | 0.901357 | 0.659182 |
| 386 | -0.38373 | -0.08401 |
| 387 | -0.33863 | -0.0348 |
| 388 | 0.247882 | -0.15263 |
| 389 | 0.382067 | -0.01182 |
| 390 | 0.023522 | -0.09082 |
| 391 | -0.45018 | -0.25501 |
| 392 | -0.0551 | -0.19082 |
| 393 | 0.288189 | 0.113233 |
| 394 | 0.081899 | 0.318285 |
| 395 | -0.04854 | -0.16885 |
| 396 | 0.052214 | -0.11094 |
| 397 | 0.137644 | -0.2618 |
| 398 | 0.194715 | 0.197988 |
| 399 | -0.23755 | -0.28356 |

| Group 8 | | |
|---|---|---|
| v(j) | | |
| 0 | 2.513683 | |
| 1 | -4.36612 | |
| 2 | -3.85445 | |

**Table I**

| Group 9 | | |
|---|---|---|
| w(ij) | | |
| | 1 | 2 |
| 0 | 0.64194 | 0.270091 |
| 1 | 0.195859 | -0.6188 |
| 2 | 0.534558 | 0.496142 |
| 3 | -0.87565 | 0.061475 |
| 4 | -0.06192 | 0.345997 |
| 5 | -1.18645 | 0.933661 |
| 6 | 0.467275 | 0.126926 |
| 7 | 0.984155 | -0.39572 |
| 8 | 0.151643 | -0.12714 |
| 9 | 0.320599 | -0.7048 |
| 10 | 0.52666 | -0.06082 |
| 11 | 1.089887 | 0.035408 |
| 12 | -0.0923 | 0.225278 |
| 13 | -0.72464 | -0.25572 |
| 14 | -0.86248 | 0.050058 |
| 15 | 0.147439 | 0.053642 |
| 16 | -0.29571 | -0.51335 |
| 17 | -0.15325 | 0.069256 |
| 18 | 0.953717 | -0.46928 |
| 19 | -0.24658 | 0.612544 |
| 20 | 0.803891 | -0.25394 |
| 21 | -0.0769 | -0.12496 |
| 22 | -0.14417 | -0.63508 |
| 23 | 0.576635 | 0.957538 |
| 24 | -0.74671 | -0.28942 |
| 25 | 0.429834 | -0.13955 |
| 26 | -0.24778 | -0.4306 |
| 27 | 0.581436 | -0.0015 |
| 28 | 0.721117 | 0.116565 |
| 29 | -0.66842 | 0.77073 |
| 30 | -0.85377 | 0.434075 |
| 31 | 0.054877 | 0.509492 |
| 32 | 0.441406 | 0.826331 |
| 33 | 0.112802 | -0.07728 |
| 34 | -1.08547 | -0.48129 |
| 35 | -0.26093 | -0.46607 |
| 36 | -0.04708 | -0.29622 |
| 37 | -1.11634 | 0.063518 |
| 38 | -0.03217 | -0.69024 |
| 39 | 0.560496 | -0.14397 |
| 40 | 0.103567 | 0.052875 |
| 41 | 1.17473 | -0.59104 |
| 42 | 0.224769 | -0.28789 |
| 43 | 0.014872 | -0.11585 |
| 44 | 0.35228 | 1.081893 |
| 45 | -0.31757 | -0.11967 |
| 46 | 0.121239 | -0.07055 |
| 47 | -0.8264 | -0.08918 |
| 48 | 0.097376 | 0.713038 |
| 49 | 0.623651 | 1.05684 |
| 50 | -0.34583 | 0.247849 |
| 51 | -0.91097 | 0.395358 |
| 52 | 0.63771 | -0.10862 |
| 53 | 0.657779 | 1.129134 |
| 54 | -0.0481 | -0.50822 |
| 55 | -0.81004 | -0.25981 |
| 56 | -0.58872 | 0.189189 |
| 57 | -0.20744 | -0.27762 |
| 58 | -0.19968 | -0.09627 |
| 59 | 0.426767 | -0.89817 |
| 60 | 0.653613 | -0.39879 |
| 61 | 0.028338 | -0.21747 |
| 62 | 0.752471 | 0.257402 |
| 63 | -0.1843 | 0.04568 |
| 64 | 0.277822 | -0.74439 |
| 65 | 0.692065 | 0.601465 |
| 66 | -0.15557 | -0.34936 |
| 67 | -0.12144 | 0.157933 |
| 68 | -0.6335 | 0.165339 |
| 69 | 0.858233 | 0.331915 |
| 70 | 0.226071 | 0.656136 |
| 71 | 0.199787 | -0.34098 |
| 72 | -0.83458 | -0.5201 |
| 73 | -0.36468 | 0.620908 |
| 74 | 0.259614 | 1.166547 |
| 75 | 0.117573 | -0.25142 |
| 76 | -1.15267 | -0.0683 |
| 77 | 0.26628 | -0.79707 |
| 78 | 0.382105 | -0.55314 |
| 79 | -0.38318 | -0.21845 |
| 80 | 0.234626 | -0.32187 |
| 81 | -0.12815 | 0.031953 |
| 82 | 0.259737 | 0.082435 |
| 83 | 0.910008 | 0.966931 |
| 84 | 0.121696 | 0.044944 |
| 85 | 0.275588 | -0.74298 |
| 86 | 0.458071 | -0.35126 |
| 87 | -0.49655 | 0.00218 |
| 88 | -0.11105 | -0.06496 |
| 89 | -0.2502 | 0.079157 |
| 90 | 0.523925 | -0.23767 |
| 91 | 0.229805 | 1.023343 |
| 92 | 1.035111 | -0.08909 |
| 93 | -0.68921 | -0.11272 |
| 94 | -0.17698 | 0.102316 |
| 95 | 0.33117 | 1.367461 |
| 96 | 0.140862 | 0.098976 |
| 97 | -1.15189 | -0.96311 |
| 98 | -0.77562 | 0.398092 |
| 99 | 0.847465 | -0.07587 |
| 100 | -0.61258 | -0.04538 |
| 101 | 0.074122 | -0.18041 |
| 102 | -0.2131 | 0.164791 |
| 103 | 0.286545 | 0.424462 |
| 104 | 0.42088 | -0.23242 |
| 105 | -0.12945 | 0.000264 |
| 106 | -0.11031 | -0.1573 |
| 107 | 0.382466 | -0.39352 |
| 108 | -0.51306 | -0.06702 |
| 109 | -0.02756 | -0.09547 |
| 110 | -0.50884 | 0.212841 |
| 111 | -0.07683 | 0.819798 |
| 112 | 0.354268 | 0.353191 |
| 113 | 0.27911 | -0.14657 |
| 114 | -0.6447 | -0.28158 |
| 115 | 0.306664 | -0.28371 |
| 116 | 1.089572 | 0.766528 |
| 117 | -0.08145 | 0.182469 |
| 118 | -1.2225 | -0.68656 |
| 119 | -0.1273 | -0.18348 |
| 120 | 0.969078 | -0.4354 |
| 121 | -0.05033 | -0.37569 |
| 122 | 0.216231 | -0.61049 |
| 123 | -0.70487 | 0.544751 |
| 124 | 0.770164 | 0.46746 |
| 125 | 0.207107 | 0.563171 |
| 126 | 0.251933 | 0.051569 |
| 127 | -0.14306 | 0.095492 |
| 128 | 0.53051 | 0.370838 |
| 129 | -0.8089 | 0.238253 |
| 130 | 0.199101 | -0.44365 |
| 131 | -0.85422 | 0.441722 |
| 132 | 0.462494 | 0.007296 |
| 133 | 0.599888 | 0.389471 |
| 134 | 0.526516 | 0.564856 |
| 135 | -1.37953 | 0.51871 |
| 136 | 0.298681 | -0.4019 |
| 137 | 1.694115 | 0.157266 |
| 138 | 0.206048 | -0.47354 |
| 139 | -1.39902 | -0.87779 |
| 140 | -0.69132 | 0.08969 |
| 141 | 0.857214 | -0.62908 |
| 142 | -0.15679 | -0.69497 |
| 143 | -0.07203 | -0.27461 |
| 144 | -0.91135 | 1.145603 |
| 145 | 0.727183 | -0.01054 |
| 146 | 0.418721 | 0.336123 |
| 147 | -0.08732 | 0.122543 |
| 148 | -0.898 | 0.331623 |
| 149 | 0.053974 | 0.560811 |
| 150 | 0.050149 | -0.25342 |
| 151 | 0.458407 | -0.4585 |
| 152 | -0.14336 | 0.15496 |
| 153 | -0.11636 | 0.192769 |
| 154 | 0.555904 | 0.376013 |
| 155 | 0.182186 | 0.965759 |
| 156 | -0.09942 | -0.38636 |
| 157 | 0.028325 | 0.143316 |
| 158 | 0.871104 | 1.423339 |
| 159 | -0.06471 | -0.34219 |
| 160 | -1.03126 | -1.19913 |
| 161 | -0.16424 | -0.12197 |
| 162 | 0.746217 | -0.0971 |
| 163 | -0.66526 | -0.32622 |
| 164 | -0.22201 | -0.30147 |
| 165 | 0.563351 | 0.033633 |
| 166 | -0.06711 | 0.145258 |
| 167 | 0.605177 | 0.045812 |
| 168 | 0.030987 | -0.07084 |
| 169 | -0.12778 | 0.075078 |
| 170 | -0.15755 | -0.20566 |
| 171 | 0.342908 | -0.42727 |
| 172 | 0.200543 | -0.64354 |
| 173 | -0.43139 | 0.161183 |
| 174 | -0.13837 | -0.11641 |
| 175 | 0.057848 | -0.08861 |
| 176 | -0.02743 | 0.755987 |
| 177 | 0.315783 | -0.46494 |
| 178 | 0.056731 | 0.794653 |
| 179 | 1.011103 | 0.159911 |
| 180 | -0.26479 | 0.312825 |
| 181 | -1.75305 | -0.00027 |
| 182 | 0.241128 | -1.00732 |
| 183 | 0.227055 | 0.460513 |
| 184 | -0.06852 | -0.91193 |
| 185 | -0.07212 | -0.84389 |
| 186 | 0.571736 | 0.309804 |
| 187 | 0.537941 | 0.265783 |
| 188 | 1.233532 | 0.810271 |
| 189 | 0.219658 | -0.22491 |
| 190 | -0.36104 | 0.332115 |
| 191 | -0.18942 | -0.1638 |
| 192 | -0.59689 | -0.13726 |
| 193 | -0.33822 | -0.20329 |
| 194 | -0.2269 | -0.07741 |
| 195 | 0.308725 | -0.47266 |
| .196 | 0.763413 | -0.09072 |
| 197 | 0.822728 | 0.866146 |
| 198 | 0.21017 | -0.77585 |
| 199 | -0.02319 | 0.512316 |
| 200 | 0.903219 | -0.38413 |
| 201 | -0.69516 | -0.36682 |
| 202 | -1.77259 | -0.48219 |
| 203 | -0.12357 | -0.49763 |
| 204 | 0.268101 | 0.335958 |
| 205 | -0.47952 | -0.00869 |
| 206 | -0.64648 | -0.43127 |
| 207 | -0.20566 | 0.83273 |
| 208 | 0.891432 | 0.582017 |
| 209 | 1.595405 | 0.526094 |
| 210 | 0.295802 | 0.170744 |
| 211 | -0.06414 | -0.06092 |
| 212 | 0.181292 | -0.18714 |
| 213 | -0.84168 | -0.43137 |
| 214 | 0.795056 | -0.82129 |
| 215 | -0.17349 | -0.06386 |
| 216 | 0.524684 | -0.6431 |
| 217 | 0.01955 | 0.584466 |
| 218 | 0.334903 | 0.589175 |
| 219 | 0.484624 | -1.27308 |
| 220 | -0.2432 | 1.417159 |
| 221 | 1.328315 | 0.388687 |
| 222 | -0.30025 | -0.7474 |
| 223 | -1.88373 | -0.8222 |
| 224 | -0.12477 | -0.68928 |
| 225 | 0.459323 | -0.1235 |
| 226 | -0.02454 | 0.040108 |
| 227 | 0.094795 | -0.91173 |
| 228 | 0.245644 | 1.019559 |
| 229 | 0.203867 | 0.750493 |
| 230 | 0.232651 | 1.883079 |
| 231 | 0.235931 | -0.1407 |
| 232 | 0.002532 | -0.31216 |
| 233 | 0.793494 | 0.803846 |
| 234 | -0.85145 | -0.56047 |
| 235 | 0.979351 | -0.42222 |
| 236 | -0.06487 | -0.31285 |
| 237 | 1.144863 | -1.12495 |
| 238 | -0.15415 | 0.123716 |
| 239 | -0.26907 | 0.60704 |
| 240 | 0.417199 | -0.66759 |
| 241 | 0.54443 | 0.19883 |
| 242 | 0.512661 | 0.526665 |
| 243 | -0.19581 | -0.69199 |
| 244 | -1.83611 | -0.56734 |
| 245 | 0.248137 | -0.6183 |
| 246 | 0.719724 | 0.362173 |
| 247 | -1.45562 | 0.896144 |
| 248 | 0.036063 | -1.10999 |
| 249 | 0.276427 | 0.731965 |
| 250 | 1.563736 | 0.884842 |
| 251 | 0.355988 | 1.087338 |
| 252 | 0.17992 | -0.13668 |
| 253 | 0.371447 | -0.84377 |
| 254 | 0.494653 | 0.44325 |
| 255 | -0.49123 | 0.280616 |
| 256 | 0.836888 | -0.6744 |
| 257 | 0.306617 | -0.94787 |
| 258 | 0.853347 | -0.42568 |
| 259 | 0.313188 | 0.99838 |
| 260 | -0.09518 | 0.606475 |
| 261 | -0.24398 | -0.58032 |
| 262 | -0.01009 | 1.018463 |
| 263 | 0.712298 | 0.2833 |
| 264 | 0.09528 | -0.97263 |
| 265 | -1.83872 | -0.47492 |
| 266 | -0.46719 | -0.38963 |
| 267 | 0.253523 | 0.75298 |
| 268 | -0.99408 | 0.54079 |
| 269 | -0.46788 | -0.42138 |
| 270 | 0.10059 | 0.465181 |
| 271 | 1.628881 | 0.571075 |
| 272 | 0.985786 | -0.09001 |
| 273 | -0.17393 | 0.003292 |
| 274 | -0.09132 | -0.08775 |
| 275 | 0.601023 | 0.176045 |
| 276 | -0.60317 | -0.3847 |
| 277 | 0.59004 | -0.6693 |
| 278 | 0.356249 | -1.22756 |
| 279 | 1.16766 | -0.20239 |
| 280 | 0.553931 | 1.286648 |
| 281 | 0.035759 | 0.291821 |
| 282 | -0.43328 | -0.04207 |
| 283 | -0.1205 | 0.660251 |
| 284 | 0.804468 | 0.247399 |
| 285 | 0.002066 | -0.56318 |
| 286 | -1.33456 | -0.59814 |
| 287 | -0.39328 | 0.112915 |
| 288 | -0.16621 | 0.528415 |
| 289 | -0.78284 | 0.734089 |
| 290 | -0.36778 | -0.32289 |
| 291 | -0.24177 | 0.30388 |
| 292 | 0.883634 | -0.02213 |
| 293 | 0.526107 | 0.212735 |
| 294 | -0.22489 | -0.1385 |
| 295 | 0.415039 | -0.90147 |
| 296 | 0.153491 | 0.352736 |
| 297 | -0.11253 | -0.96807 |
| 298 | 0.467165 | -0.54412 |
| 299 | -0.17126 | -0.52193 |
| 300 | 0.788337 | -0.03039 |
| 301 | 0.911138 | 1.3102 |
| 302 | 0.093481 | -0.34812 |
| 303 | -0.55441 | 0.029816 |
| 304 | 0.489211 | -0.18274 |
| 305 | -0.39533 | 0.276446 |
| 306 | -0.59687 | -02716 |
| 307 | -0.99818 | -0.6321 |
| 308 | -0.54105 | 0.468147 |
| 309 | 0.363936 | 0.326605 |
| 310 | -0.51659 | 0.208887 |
| 311 | -0.94323 | 0.14807 |
| 312 | 0.044745 | 0.167918 |
| 313 | 0.677847 | 0.158515 |
| 314 | 0.659608 | -0.02807 |
| 315 | -0.08393 | 0.286155 |
| 316 | 0.162055 | -0.23945 |
| 317 | 1.259513 | 0.195843 |
| 318 | -0.05268 | 0.022288 |
| 319 | 0.545569 | -0.35745 |
| 320 | -0.52506 | -0.16193 |
| 321 | -0.35724 | 1.065798 |
| 322 | 0.149871 | 0.989745 |
| 323 | 0.155403 | 0.023116 |
| 324 | 0.180607 | -0.29687 |
| 325 | 0.254375 | -0.55448 |
| 326 | -0.10757 | 0.616046 |
| 327 | -0.21484 | -0.19326 |
| 328 | -1.32521 | -0.04685 |
| 329 | -0.52195 | 0.338382 |
| 330 | -0.52643 | 1.246406 |
| 331 | 0.350233 | -0.58487 |
| 332 | -0.45791 | -0.1388 |
| 333 | 0.242386 | -0.48422 |
| 334 | 0.489285 | -0.79099 |
| 335 | 0.445327 | 0.045959 |
| 336 | 0.152633 | 0.140344 |
| 337 | -0.03022 | -0.30771 |
| 338 | 0.632293 | -0.02374 |
| 339 | -0.08615 | -0.03418 |
| 340 | -0.44081 | 0.866435 |
| 341 | -0.50159 | -0.51748 |
| 342 | 0.81282 | -0.01571 |
| 343 | 0.067787 | 1.553558 |
| 344 | -0.47632 | 0.196224 |
| 345 | 0.387457 | -0.65912 |
| 346 | 0.52038 | -0.61349 |
| 347 | -0.11048 | -0.17568 |
| 348 | -0.32033 | -0.31679 |
| 349 | -0.8788 | -0.29749 |
| 350 | -0.45012 | 0.616855 |
| 351 | 0.189491 | -0.21047 |
| 352 | -0.0252 | -0.31013 |
| 353 | 0.09285 | -0.59475 |
| 354 | -0.06975 | -0.37393 |
| 355 | 0.453887 | 0.055217 |
| 356 | 0.434582 | 0.847264 |
| 357 | 0.138895 | -0.16725 |
| 358 | 0.460854 | -0.73421 |
| 359 | -0.08043 | 0.8153 |
| 360 | -0.22652 | 0.363883 |
| 361 | 0.276865 | 0.1623 |
| 362 | 0.657328 | -1.52977 |
| 363 | 0.277363 | 0.223051 |
| 364 | 1.130451 | 1.645333 |
| 365 | -0.48611 | 0.011101 |
| 366 | -0.09968 | -0.25949 |
| 367 | 0.364756 | -0.4449 |
| 368 | -0.39767 | 0.877105 |
| 369 | -0.79214 | 0.425876 |
| 370 | -1.20164 | -0.11292 |
| 371 | 0.281444 | -0.20106 |
| 372 | 0.303414 | 0.427254 |
| 373 | -0.92178 | 0.557361 |
| 374 | 0.533701 | -1.18621 |
| 375 | -0.15805 | -0.73345 |
| 376 | 0.456479 | 0.000646 |
| 377 | 0.295776 | 0.496952 |
| 378 | -0.01329 | 0.065039 |
| 379 | -0.09384 | -0.20761 |
| 380 | 0.274427 | 0.269854 |
| 381 | 0.188822 | -0.66758 |
| 382 | 0.047133 | -0.22507 |
| 383 | -0.23114 | -0.49506 |
| 384 | -0.1771 | 0.367024 |
| 385 | 1.483081 | 1.216784 |
| 386 | -0.73488 | 0.075664 |
| 387 | -0.68143 | 0.103813 |
| 388 | 0.28584 | -0.85768 |
| 389 | 0.930243 | -0.29447 |
| 390 | -0.60416 | 0.289829 |
| 391 | -0.88622 | -0.58707 |
| 392 | -0.48878 | 0.360653 |
| 393 | -0.008 | 0.765181 |
| 394 | -0.34795 | 0.509356 |
| 395 | 0.283503 | -0.64571 |
| 396 | 0.229828 | -0.32588 |
| 397 | 0.897132 | 0.403366 |
| 398 | 0.805111 | 0.137891 |
| 399 | -0.06116 | -0.16817 |

| Group 9 | | |
|---|---|---|
| v(j) | | |
| 0 | 4.981966 | |
| 1 | -9.82405 | |
| 2 | -9.23957 | |

**Table J**

| Group 10 | | |
|---|---|---|
| w(ij) | | |
| | 1 | 2 |
| 0 | -0.21773 | 0.95167 |
| 1 | -0.08082 | 0.0675 |
| 2 | 0.133668 | 1.193804 |
| 3 | 0.544682 | -1.04487 |
| 4 | 0.121715 | -0.07394 |
| 5 | 0.326843 | -0.42653 |
| 6 | -0.67617 | 1.009579 |
| 7 | 0.382046 | 0.386103 |
| 8 | 1.511935 | -1.72435 |
| 9 | 0.608665 | -1.12193 |
| 10 | -0.06424 | 0.815661 |
| 11 | 0.752652 | -0.13895 |
| 12 | -0.43834 | 0.581571 |
| 13 | -0.86096 | -0.01378 |
| 14 | -0.5169 | 0.538929 |
| 15 | -0.06988 | -0.18176 |
| 16 | -1.88976 | 0.394621 |
| 17 | 0.154164 | 0.029392 |
| 18 | -0.18418 | 0.371262 |
| 19 | 0.369377 | 0.498222 |
| 20 | 0.239975 | 0.43862 |
| 21 | 0.172868 | 0.046495 |
| 22 | -1.1767 | 0.339212 |
| 23 | 0.038298 | 0.411596 |
| 24 | -2.11033 | 0.659546 |
| 25 | 0.298284 | -0.01726 |
| 26 | 0.118495 | -0.50437 |
| 27 | 0.325695 | 0.418034 |
| 28 | 0.8627 | 0.856154 |
| 29 | 0.784064 | -1.05789 |
| 30 | 0.730496 | -1.5156 |
| 31 | 0.343097 | 0.34106 |
| 32 | 0.395478 | 1.366663 |
| 33 | 0.199538 | -0.09937 |
| 34 | -1.52002 | 0.147602 |
| 35 | -0.05017 | -0.20997 |
| 36 | -0.52503 | 0.316586 |
| 37 | -0.20434 | -0.97047 |
| 38 | -0.11874 | -0.17555 |
| 39 | 0.893663 | -0.39776 |
| 40 | -0.09813 | 0.417118 |
| 41 | 0.430623 | 0.189829 |
| 42 | 0.015809 | -0.21414 |
| 43 | -0.14313 | 0.117916 |
| 44 | -0.29495 | 0.944132 |
| 45 | -1.162 | 0.174987 |
| 46 | 0.099015 | 0.337924 |
| 47 | -0.34787 | -0.2085 |
| 48 | 0.515055 | 0.596587 |
| 49 | 0.340882 | 0.967424 |
| 50 | -0.08268 | 0.306925 |
| 51 | 0.277343 | -0.46136 |
| 52 | -0.43352 | 0.970509 |
| 53 | 1.124498 | 1.18225 |
| 54 | -0.14135 | -0.0326 |
| 55 | -1.23821 | 0.004672 |
| 56 | 0.263903 | -0.90628 |
| 57 | 0.82547 | -0.41619 |
| 58 | 0.02184 | -0.87756 |
| 59 | 0.025358 | -1.06669 |
| 60 | 0.003618 | 0.78061 |
| 61 | 0.609521 | -0.26741 |
| 62 | 0.74983 | 0.028416 |
| 63 | 0.008452 | 0.234547 |
| 64 | 0.365827 | -0.06829 |
| 65 | -0.49862 | 0.912657 |
| 66 | 0.185391 | -0.80148 |
| 67 | 0.381624 | -0.30506 |
| 68 | -0.7952 | 0.071444 |
| 69 | 0.924077 | 0.092822 |
| 70 | 0.367975 | 0.785521 |
| 71 | -0.38631 | 0.497334 |
| 72 | -0.01782 | -0.77508 |
| 73 | -1.16561 | 0.818246 |
| 74 | -0.48721 | 1.496814 |
| 75 | -0.39069 | -0.2942 |
| 76 | -0.60262 | -1.15507 |
| 77 | 0.407577 | 0.183629 |
| 78 | 0.437982 | -0.13769 |
| 79 | -0.64203 | -0.2094 |
| 80 | -0.04004 | -0.7484 |
| 81 | 0.444955 | -0.02859 |
| 82 | 0.155416 | -0.30453 |
| 83 | -0.06453 | 0.354482 |
| 84 | -0.23992 | -0.1623 |
| 85 | 1.000023 | -0.91666 |
| 86 | 0.323839 | 0.607188 |
| 87 | 0.398023 | -0.17633 |
| 88 | -0.7008 | 0.428521 |
| 89 | -0.35049 | 0.471606 |
| 90 | 0.807497 | 0.234673 |
| 91 | 1.032899 | -0.44715 |
| 92 | -0.58409 | 0.79558 |
| 93 | -0.41526 | -0.77405 |
| 94 | 0.056802 | 0.402432 |
| 95 | 0.699936 | 0.246205 |
| 96 | 0.259323 | -0.31317 |
| 97 | -0.66119 | -1.32467 |
| 98 | 0.263412 | -0.57359 |
| 99 | 0.264639 | 0.284937 |
| 100 | -0.50496 | -0.12251 |
| 101 | 0.16423 | -0.00587 |
| 102 | -0.74334 | 0.738508 |
| 103 | 0.082901 | 0.745391 |
| 104 | -0.4341 | 0.63456 |
| 105 | -0.27487 | -0.19392 |
| 106 | -0.20281 | -0.2119 |
| 107 | 0.401397 | 0.326238 |
| 108 | 0.238096 | -0.99372 |
| 109 | 0.461596 | -0.71014 |
| 110 | -0.17323 | -0.01865 |
| 111 | 1.113207 | -0.48887 |
| 112 | -0.21206 | 1.151766 |
| 113 | -0.30801 | 1.118044 |
| 114 | -1.27072 | 0.032129 |
| 115 | -0.95366 | 0.138042 |
| 116 | 0.459428 | 0.874064 |
| 117 | -0.13406 | 0.059186 |
| 118 | -0.71757 | -1.42382 |
| 119 | 0.05319 | -0.30797 |
| 120 | -0.32224 | 0.743598 |
| 121 | -0.30567 | -0.23633 |
| 122 | -0.48825 | -0.16081 |
| 123 | 0.836827 | -0.52256 |
| 124 | 0.395397 | 0.466756 |
| 125 | 0.591145 | -0.20207 |
| 126 | 0.263215 | 0.271603 |
| 127 | -0.74747 | 0.48582 |
| 128 | -0.38176 | 0.239157 |
| 129 | 0.098815 | -0.6565 |
| 130 | 0.279631 | -0.19188 |
| 131 | -0.505 | -0.24193 |
| 132 | 0.078652 | 0.27227 |
| 133 | 0.545278 | 1.099666 |
| 134 | 0.623631 | 0.610526 |
| 135 | -0.47941 | -0.73372 |
| 136 | 0.1908 | -0.212 |
| 137 | 0.58491 | 0.802174 |
| 138 | 0.215447 | -0.79666 |
| 139 | -0.5143 | -1.85654 |
| 140 | 0.470532 | -0.92079 |
| 141 | -0.12043 | 0.137829 |
| 142 | -0.17338 | -0.22141 |
| 143 | -1.06062 | -0.03656 |
| 144 | 0.671523 | -0.16176 |
| 145 | -0.11988 | 0.97522 |
| 146 | 0.219223 | 1.163602 |
| 147 | -0.22213 | -0.09253 |
| 148 | -0.65502 | 0.275044 |
| 149 | -0.11003 | 0.213207 |
| 150 | -0.28553 | -0.41543 |
| 151 | 0.019128 | -0.09822 |
| 152 | -0.11046 | -0.0706 |
| 153 | 0.137203 | -0.37862 |
| 154 | 1.491766 | 0.797081 |
| 155 | -0.01711 | 1.110665 |
| 156 | -0.42072 | -0.45854 |
| 157 | 0.334536 | 0.052784 |
| 158 | 0.140428 | 1.078279 |
| 159 | 0.16883 | -0.83192 |
| 160 | -0.95643 | -1.52285 |
| 161 | -0.68153 | -0.08786 |
| 162 | 0.624607 | -0.00196 |
| 163 | -0.15398 | -0.59526 |
| 164 | -1.39393 | 0.482341 |
| 165 | 0.473873 | -0.21725 |
| 166 | 0.490911 | -0.05932 |
| 167 | 0.733999 | 0.422976 |
| 168 | -0.02329 | -0.20126 |
| 169 | -0.10513 | -0.12856 |
| 170 | 0.375006 | -0.36407 |
| 171 | -0.33327 | -0.17987 |
| 172 | -0.37175 | 0.494296 |
| 173 | 0.702191 | -0.7595 |
| 174 | -0.16296 | -0.20259 |
| 175 | 0.152321 | 0.460986 |
| 176 | 0.697848 | 0.3066 |
| 177 | -0.14361 | -0.67665 |
| 178 | 0.81453 | -0.05581 |
| 179 | 0.687745 | 0.68682 |
| 180 | -1.25889 | 0.645092 |
| 181 | -0.02834 | -1.93654 |
| 182 | 0.050488 | -0.26644 |
| 183 | -0.48807 | 0.197827 |
| 184 | -0.46939 | -0.29067 |
| 185 | -0.33725 | 0.067898 |
| 186 | -0.04078 | 0.960604 |
| 187 | 0.634126 | -0.49156 |
| 188 | -0.14168 | 1.674543 |
| 189 | 0.02444 | 0.098474 |
| 190 | -0.14505 | 0.205176 |
| 191 | -0.16419 | 0.442674 |
| 192 | 0.044461 | -1.07263 |
| 193 | -1.36962 | 0.37259 |
| 194 | 0.283042 | 0.017251 |
| 195 | 1.092625 | -1.1232 |
| 196 | 0.247437 | 0.548705 |
| 197 | -0.39745 | 1.194135 |
| 198 | -0.44046 | 0.627115 |
| 199 | 0.069683 | 0.592096 |
| 200 | 0.225729 | 1.428233 |
| 201 | -1.25359 | -0.42427 |
| 202 | -1.17756 | -1.58033 |
| 203 | 0.503496 | -0.76863 |
| 204 | 1.031094 | -0.74216 |
| 205 | -0.12463 | -0.30107 |
| 206 | 0.096206 | -1.13019 |
| 207 | 1.021226 | 0.31877 |
| 208 | 1.269505 | 0.67148 |
| 209 | -0.46299 | 1.010138 |
| 210 | -0.28538 | -0.03308 |
| 211 | -0.06883 | 0.204001 |
| 212 | -0.01832 | 0.269239 |
| 213 | 0.297059 | -1.84782 |
| 214 | -0.07365 | -0.21822 |
| 215 | 0.486585 | 0.0699 |
| 216 | -0.83033 | 0.736992 |
| 217 | -0.12023 | 1.029522 |
| 218 | 1.02821 | 0.25679 |
| 219 | -0.22914 | 0.055263 |
| 220 | 1.012032 | 0.268538 |
| 221 | -0.2231 | 0.942085 |
| 222 | 0.272282 | -1.05414 |
| 223 | -2.01859 | -1.0958 |
| 224 | -0.08049 | -0.64881 |
| 225 | -0.29718 | 0.184306 |
| 226 | -0.55353 | 0.089595 |
| 227 | -0.05476 | -0.60637 |
| 228 | -0.05174 | 1.126084 |
| 229 | -0.16872 | 0.432311 |
| 230 | 0.65904 | 1.033112 |
| 231 | -0.08572 | -0.00212 |
| 232 | -0.29466 | -0.06132 |
| 233 | 0.578632 | 0.215785 |
| 234 | -0.96778 | -0.43407 |
| 235 | 0.002677 | 0.094515 |
| 236 | 0.193565 | -0.54194 |
| 237 | -0.46957 | 0.377909 |
| 238 | 1.197912 | 0.404643 |
| 239 | -0.78557 | 1.067509 |
| 240 | 0.009357 | -0.09093 |
| 241 | 0.488222 | 0.474727 |
| 242 | 0.52839 | 0.732205 |
| 243 | -0.87273 | -0.85902 |
| 244 | -1.38837 | -1.05511 |
| 245 | -0.89398 | 0.066645 |
| 246 | 0.855543 | -0.80416 |
| 247 | 0.506373 | -1.26234 |
| 248 | 0.335109 | -0.8094 |
| 249 | -0.06034 | 0.774042 |
| 250 | -0.12714 | 1.181986 |
| 251 | 0.395557 | 1.203972 |
| 252 | 0.226989 | -0.16054 |
| 253 | -0.58576 | 0.31283 |
| 254 | 0.182666 | 0.053203 |
| 255 | -0.63325 | -0.17037 |
| 256 | 0.400883 | 0.028283 |
| 257 | 0.112598 | -0.59169 |
| 258 | 0.600046 | -0.2114 |
| 259 | 0.50731 | 0.637549 |
| 260 | -0.11214 | 0.468035 |
| 261 | -0.03818 | 0.333437 |
| 262 | 0.890646 | 0.21398 |
| 263 | 1.325245 | 0.366913 |
| 264 | -0.11456 | -0.64253 |
| 265 | -1.90257 | -1.00335 |
| 266 | -0.68849 | -0.06369 |
| 267 | -0.0315 | 0.449778 |
| 268 | 0.239412 | -0.88748 |
| 269 | 0.212653 | -0.81674 |
| 270 | 0.344784 | 0.58365 |
| 271 | 0.953292 | 1.068973 |
| 272 | -0.71044 | 1.393535 |
| 273 | -0.18484 | -0.09612 |
| 274 | 0.376895 | -0.20359 |
| 275 | -0.64558 | 1.150552 |
| 276 | -0.91855 | -0.51135 |
| 277 | 1.010612 | -0.55235 |
| 278 | -0.52861 | -0.02539 |
| 279 | -0.03456 | 0.647398 |
| 280 | 0.962356 | 1.377247 |
| 281 | -0.90009 | 1.121584 |
| 282 | 0.310246 | -0.18279 |
| 283 | 0.03641 | 0.195487 |
| 284 | 0.119913 | 0.214807 |
| 285 | -0.0332 | -0.15499 |
| 286 | -0.31766 | -2.14717 |
| 287 | -0.10553 | -0.30634 |
| 288 | 0.096125 | -0.07956 |
| 289 | 0.705596 | -0.71083 |
| 290 | -0.74559 | -0.48972 |
| 291 | -0.10237 | 0.925293 |
| 292 | 0.649804 | 0.455141 |
| 293 | -0.47315 | 1.168144 |
| 294 | 0.119503 | -0.01637 |
| 295 | -0.433 | 0.20022 |
| 296 | 0.058918 | 0.780589 |
| 297 | -0.28718 | -1.12224 |
| 298 | 0.144755 | 0.160832 |
| 299 | -0.40157 | -0.87417 |
| 300 | 0.662664 | 0.204028 |
| 301 | -0.00299 | 2.032077 |
| 302 | 0.418736 | -0.58364 |
| 303 | 0.412196 | -0.12008 |
| 304 | 0.169921 | 0.314581 |
| 305 | -0.01066 | -0.4368 |
| 306 | -0.1617 | -0.02143 |
| 307 | -0.21742 | -1.50086 |
| 308 | -0.19351 | 0.038954 |
| 309 | -0.34873 | 0.58912 |
| 310 | -0.67432 | 0.244386 |
| 311 | -0.44883 | -0.0935 |
| 312 | 0.782133 | -0.16698 |
| 313 | -0.38938 | 0.479967 |
| 314 | 0.328822 | 0.044201 |
| 315 | -0.27238 | -0.18091 |
| 316 | -0.06375 | -0.36066 |
| 317 | 1.015702 | 1.01279 |
| 318 | -1.25294 | 0.096562 |
| 319 | -0.24241 | 0.341134 |
| 320 | -0.20625 | -0.71412 |
| 321 | 0.936838 | -0.1063 |
| 322 | 0.178143 | 1.737094 |
| 323 | -0.06339 | -0.24368 |
| 324 | -0.71818 | 0.701858 |
| 325 | -0.04926 | 0.555514 |
| 326 | 0.413655 | -0.31184 |
| 327 | -0.07405 | -0.26802 |
| 328 | -1.05429 | -0.66335 |
| 329 | -0.67127 | 0.739118 |
| 330 | -0.18322 | -0.04423 |
| 331 | 0.70219 | -0.67887 |
| 332 | -0.21005 | -0.06677 |
| 333 | 0.696555 | -0.8612 |
| 334 | -0.17799 | 0.40026 |
| 335 | 0.369617 | 0.059646 |
| 336 | 0.199605 | 0.276308 |
| 337 | 0.688028 | -0.36144 |
| 338 | 0.366221 | 0.669716 |
| 339 | -0.82291 | -0.06005 |
| 340 | -0.23427 | 0.030383 |
| 341 | -1.87436 | 0.983992 |
| 342 | 0.037124 | 0.483859 |
| 343 | 0.931052 | 1.781862 |
| 344 | 0.55304 | -0.45553 |
| 345 | 0.600632 | -0.27261 |
| 346 | 0.404765 | 0.116244 |
| 347 | -0.07397 | -0.25744 |
| 348 | 0.647364 | -1.00598 |
| 349 | -0.48945 | -0.85349 |
| 350 | -0.08483 | -0.1437 |
| 351 | -0.99018 | 0.26505 |
| 352 | -0.39191 | 0.282081 |
| 353 | -0.26311 | -0.36914 |
| 354 | 0.960139 | -0.83258 |
| 355 | -0.53822 | 0.811772 |
| 356 | 0.671682 | 0.385085 |
| 357 | 0.006271 | -0.04253 |
| 358 | -0.21623 | 0.290006 |
| 359 | 0.402823 | 0.711615 |
| 360 | -0.54409 | -0.14294 |
| 361 | 0.193133 | 0.309053 |
| 362 | -0.97294 | 0.238346 |
| 363 | 0.399154 | -008528 |
| 364 | 1.132518 | 2.528306 |
| 365 | 0.811664 | -0.75203 |
| 366 | 0.25782 | -0.12548 |
| 367 | -0.10651 | 0.237355 |
| 368 | -0.50943 | 0.20882 |
| 369 | -0.1833 | -0.8121 |
| 370 | -0.59408 | -1.18243 |
| 371 | -0.66789 | 0.517471 |
| 372 | 0.989984 | -0.7456 |
| 373 | -0.12962 | -0.03808 |
| 374 | 0.161323 | -0.45044 |
| 375 | -0.07859 | -0.00279 |
| 376 | -0.31234 | 0.317121 |
| 377 | 0.160675 | 0.48443 |
| 378 | -0.08068 | 0.151237 |
| 379 | 0.654037 | -0.45605 |
| 380 | 0.522271 | 0.924588 |
| 381 | -0.68717 | 0.180251 |
| 382 | 0.783095 | -0.91222 |
| 383 | -0.14511 | -0.14484 |
| 384 | 0.387458 | -0.06218 |
| 385 | 1.21814 | 1.69288 |
| 386 | -0.80553 | 0.24397 |
| 387 | -0.11478 | -0.50692 |
| 388 | 0.223987 | -0.47254 |
| 389 | -0.57047 | 0.515589 |
| 390 | -0.78901 | 0.427246 |
| 391 | -0.53284 | -0.89689 |
| 392 | 0.139725 | -0.43812 |
| 393 | 0.037648 | 0.294196 |
| 394 | -0.16659 | 0.162129 |
| 395 | -0.68619 | -0.263 |
| 396 | 0.272476 | -0.25536 |
| 397 | 0.015712 | -0.22681 |
| 398 | 0.201703 | 0.609574 |
| 399 | 0.176496 | -0.09298 |

| Group 10 | | |
|---|---|---|
| v(j) | | |
| 0 | 5.761896 | |
| 1 | -10.274 | |
| 2 | -11.409 | |

### Abbreviation

BLAST: Basic Local Alignment Search Tool
DSC: Determination of Secondary structure Class
DSSP: Dictionary of Secondary Structures of Proteins
PDB: Protein Data Bank
PHD: Profile network from HeiDelberg
SCOP: Structural Classification of Proteins

### [Embodiment 4]

A non-redundant protein sequence data set whose structure is known and which has been disclosed on the Internet, nr-PDB, was prepared as a basic data set. Among data in this data set, only data including two or more domains defined in SCOP, a structural classification database, in 1 sequence was collected. The structure of the sequences were further examined, regions with a loop structure of 4 residues or more were selected, and those existing on the boundary between adjoining two domains were defined as domain linkers, while the others and not existing either of the N/C terminals were defined as non-domain linker loops, and the respective data sets were prepared.

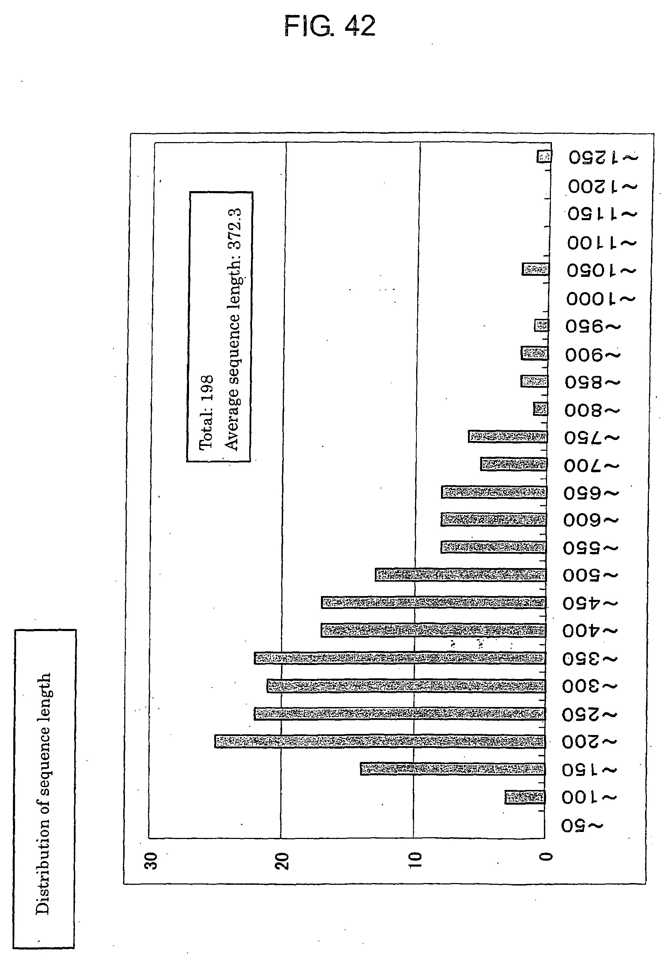

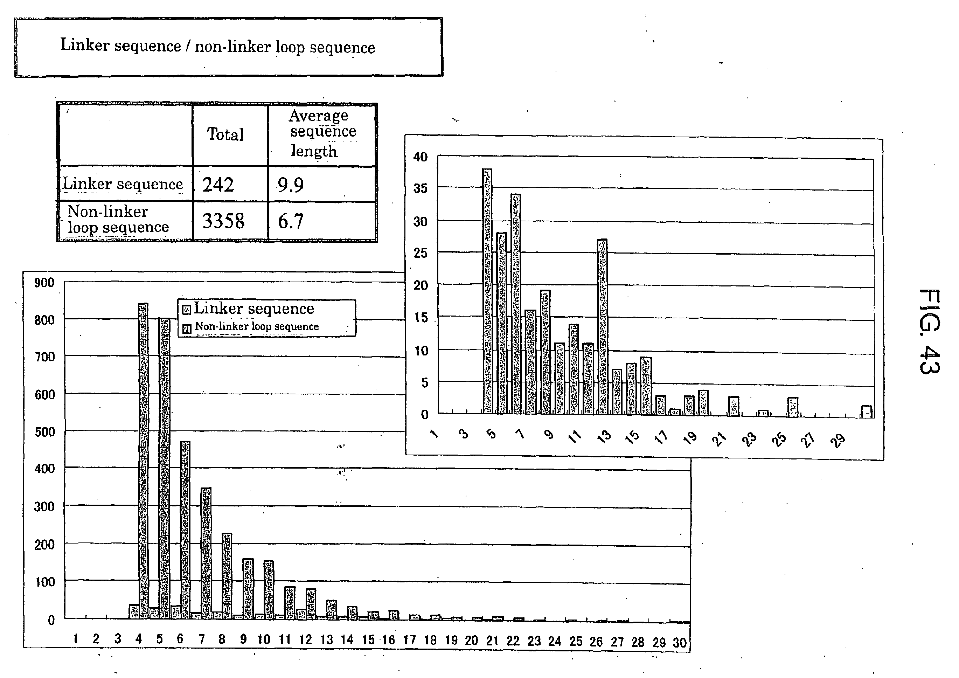

Distribution of sequence length in the multi-domain protein data set including one or more above defined domain linkers is shown in Fig. 42. Also, the summary of the linker sequence and the non-linker loop sequence existing in the sequence data set is shown in Fig. 43.

### [Embodiment 5]

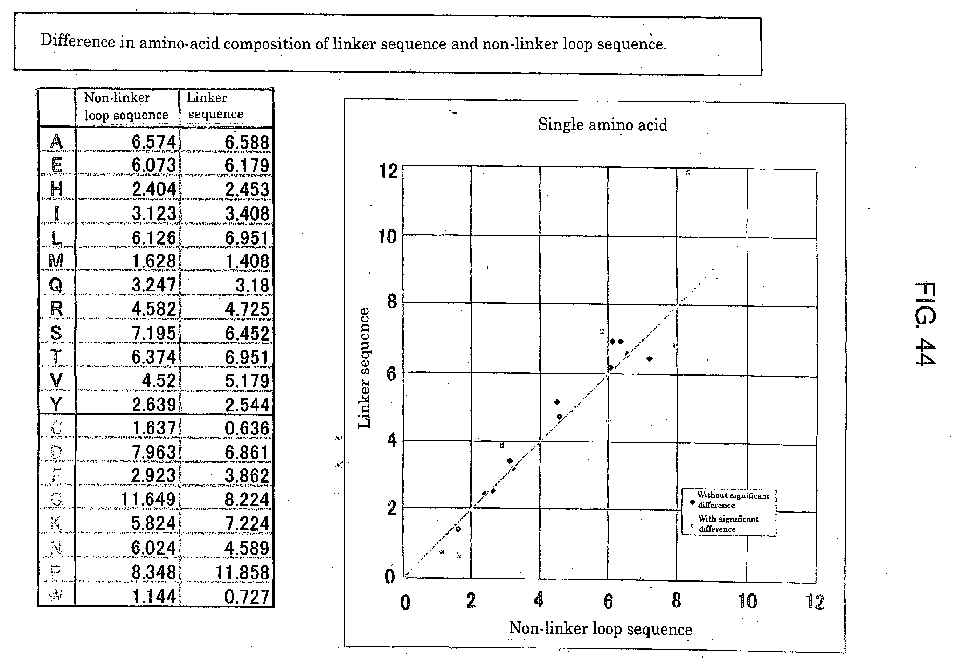

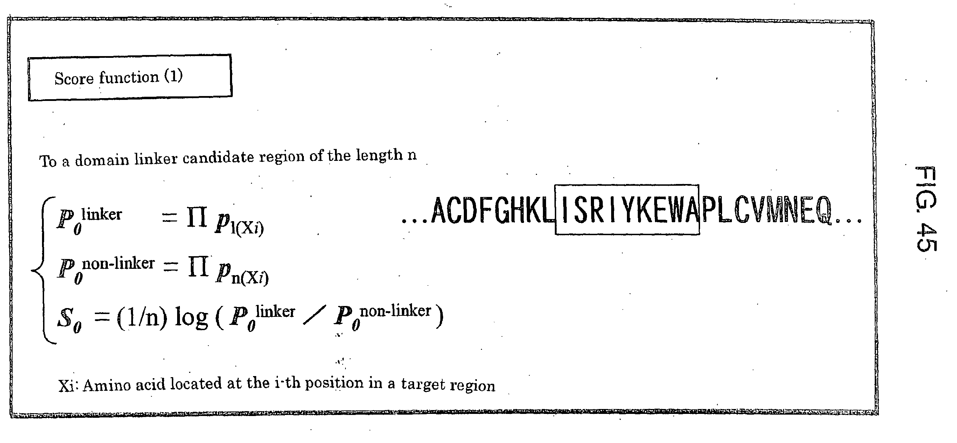

The occurrence frequencies P_{Xaa}^{L} and P_{Xaa}^{N} of the amino acid Xₐₐ in each data set of domain linker and non-domain linker loop are shown in Fig. 44. Using these numeral values, a probability that a linker candidate sequence can exist as a domain linker or a non-domain linker loop is calculated, respectively, and which is how much larger is indicated as a score S₀ in the equation in Fig. 45.

### [Embodiment 6]

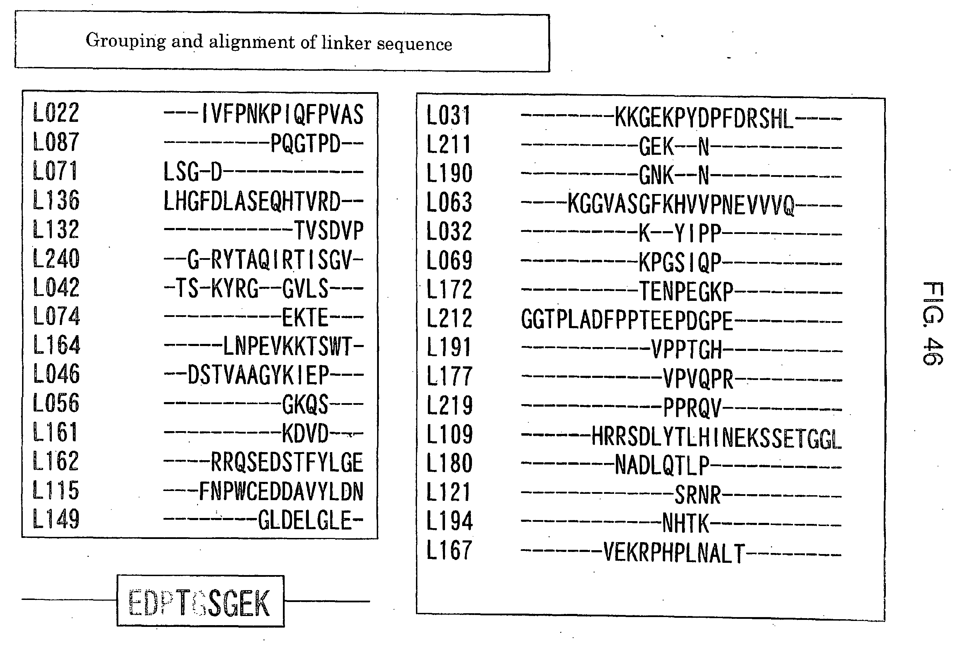

As shown in Fig. 46, a pattern consisting of some types of 2 residues exists in a linker sequence. Similarly to the case for an arbitrary amino acid, this is analyzed based on the difference in occurrence frequency between the domain linker and the non-domain linker loop.

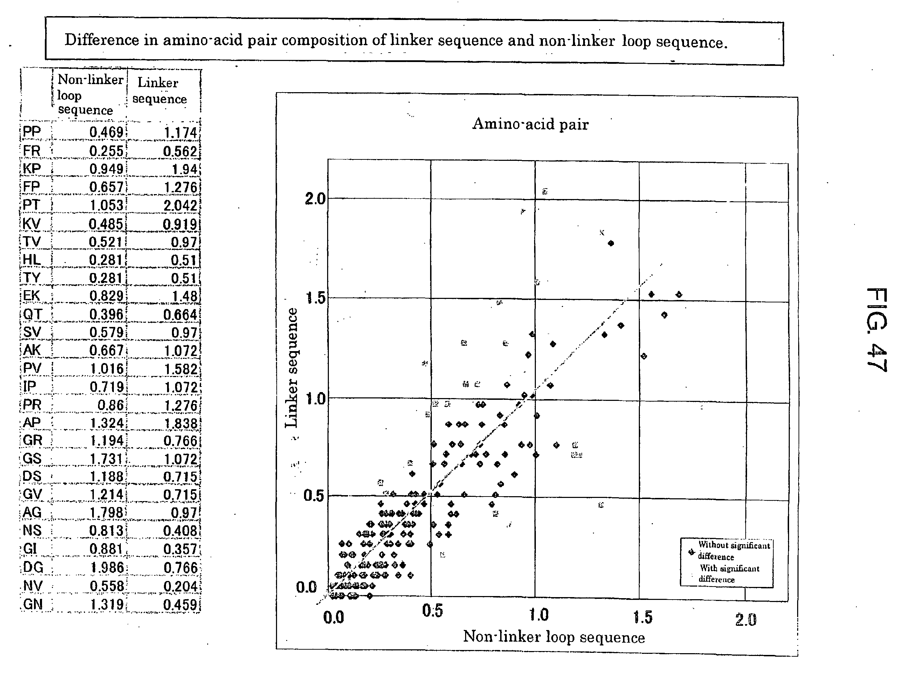

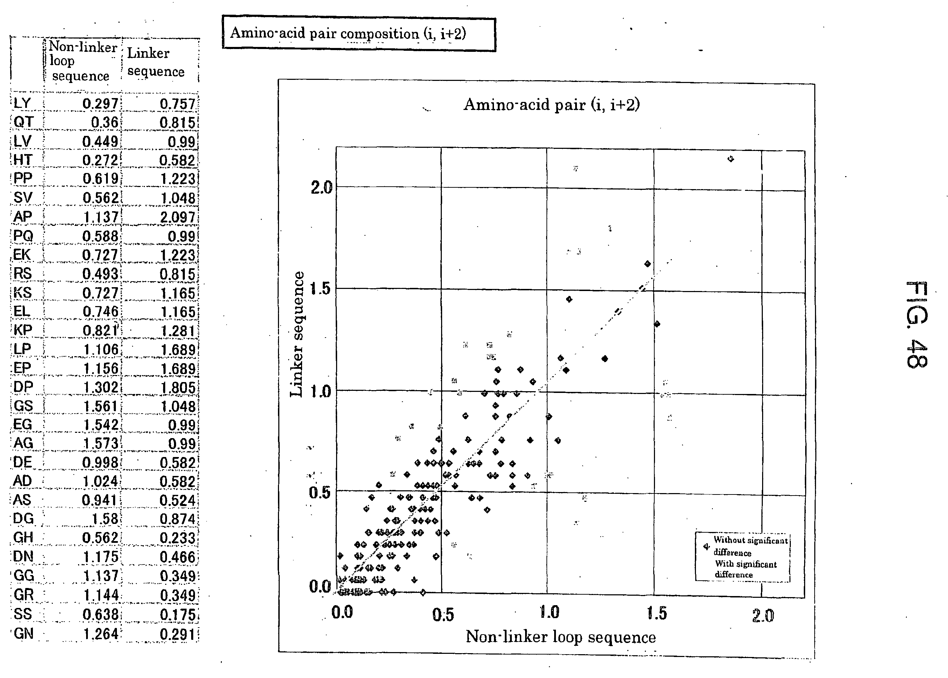

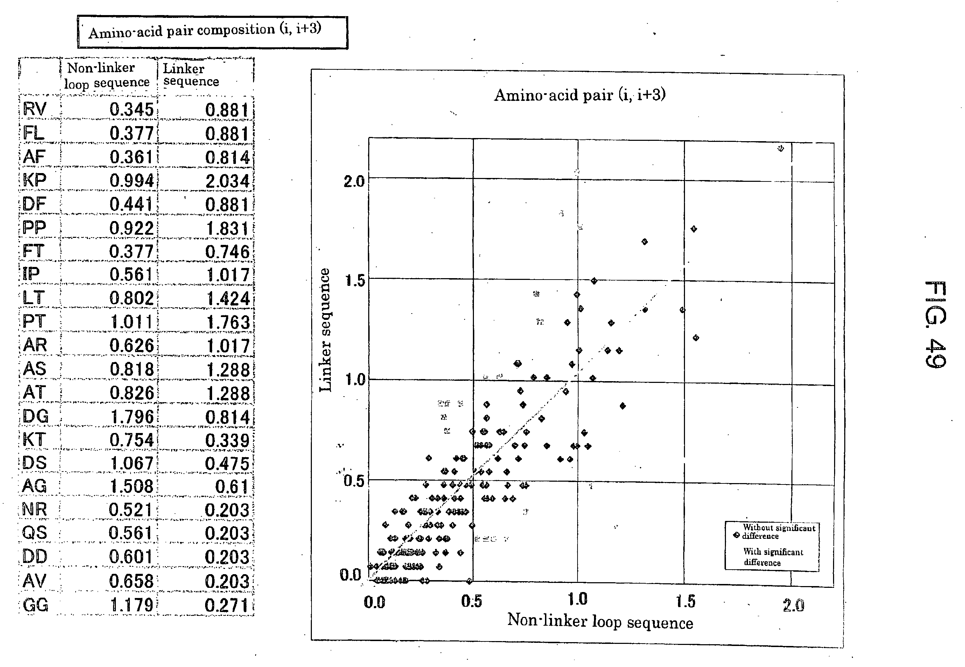

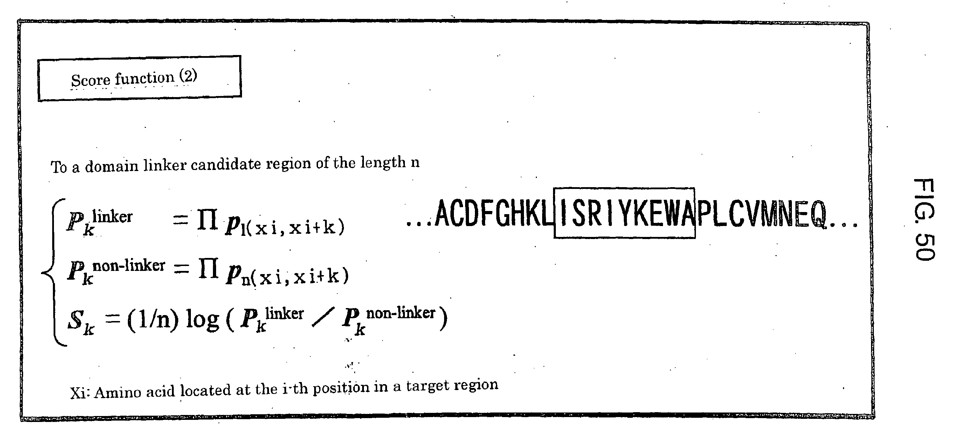

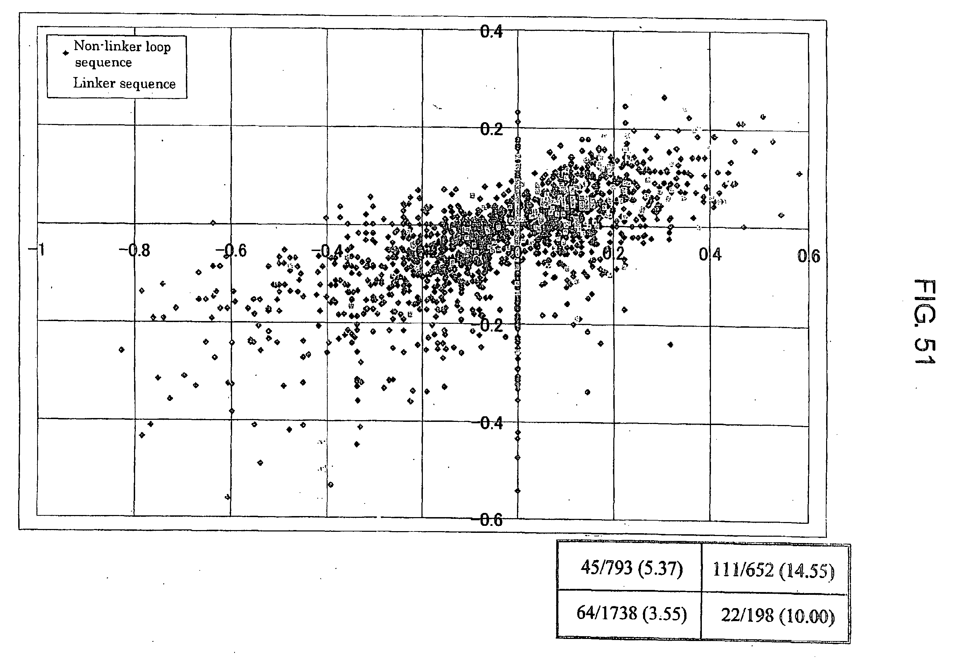

In each of the data sets for the domain linker and the non-domain linker loop prepared in Embodiment 4, occurrence probabilities P_{XaaYaa(m)}^{L} and P_{XaaYaa(m)}^{N} of the amino-acid residue pair Xₐₐ and Yₐₐ (the order of Xₐₐ and Yₐₐ does not matter) with m pieces (m is an integer, m = 0, 1, 2) of arbitrary amino-acid residues between them are shown in Figs. 47 through 49. Using these numeral values, a probability that a linker candidate sequence can exist as a domain linker or a non-domain linker loop is calculated, respectively, and which is how much larger is indicated as a score Sₖ (k = 1 through 3) in the equation in Fig. 50. The calculation of the linker degree discrimination score according to a preferred embodiment of the present application was carried out for the prepared 242 pieces of linker sequences and 3381 pieces of non-linker sequences, and the distribution of each sequence is shown in Fig. 51 with F₁s on the horizontal axis and F₁p on the vertical axis.

### [Embodiment 7]

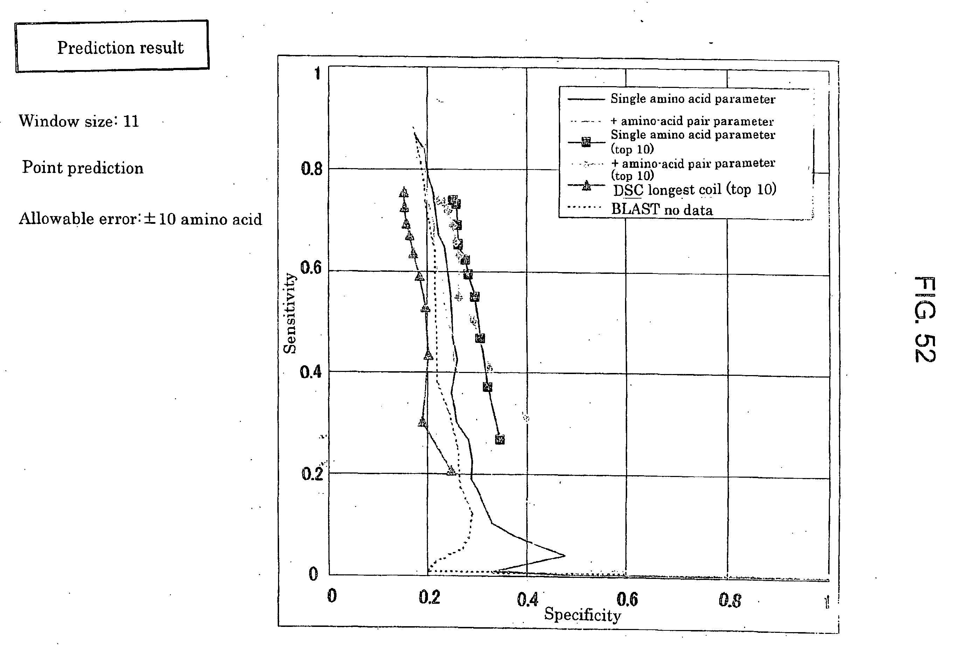

The results of domain linker prediction executed for the multi-domain protein data sets defined in Embodiment 4 in 6 different methods are shown in Fig. 52. The results with the best prediction efficiency were obtained when scores explained in Embodiments 5 and 6 were used in combination. The legend in the graph of Fig. 52 shows, in the order from above, the case where the threshold value is changed using the score F₁₂s, the case where the threshold value is changed using the score F₁₂ (=F₁₂s+αF₁₂p), the case where the top 1 through 10 were taken using the score F₁₂, the case where the top 1 through 10 were taken using the score F₁₂ (=F₁₂s+αF₁₂p), the case where the loop predicted by the secondary structure prediction tool DSC was predicted as a linker in the order of length, and the case where the threshold value was changed using the score F₁₁ (=F₁₁s+αF₁₁p). In the graph of Fig. 52, the horizontal axis: specificity = number of linker prediction successes / prediction presented number, the vertical axis: sensitivity = number of linker prediction successes / number of existing linkers.

### [Embodiment 8]

The Jackknife test of this predicting method was executed for the multi-domain protein data set defined in Embodiment 4. That is, the data set was divided into 5 partial sets, parameters were set using the sequence groups included in 4 of them, and domain linker prediction was made for the remaining 1 sequence group. This was repeated for the 5 partial sets. The average of correct answer rate (specificity) by this method was 35.6%.

### References

Altschul, S. F., Gish, W., Miller, W. Myers, E. W. & Lipman, D. J. (1990) Basic loacl alignment search tool. *J. Mol. Biol.* **215**, 403-410.
Altschul, S. F., Madden, T. L., Schaffer, A. A., Zhang, J., Zhang, Z., Miller, W. & Lipman, D. J. (1997) Gapped BLAST and PSI-BLAST: a new generation of protein database search programs. *Nucleic Acids Res*. **25,** 3389-3402.
Argos, P. (1990) An investigation of oligopeptides linking domains in protein tertiary structures and possible candidates for general gene fusion. *J. Mol. Biol*. **211,** 943-958.
Busetta, B. & Barrans, Y. (1984) The prediction of protein domains. *Biochim. Biophys. Acta* **790,** 117-124.
Campbell, I. D. & Downing, A. K. (1994) Building protein structure and function from modular units. *Trends Biotechnology* **12,** 168-72.
Chandonia, J. M. & Karplus, M. (1995) Neural networks for secondary structure and structural class predictions. *Protein Sci*. **4,** 275-285.
Chou, P. Y. & Fasman, G. D. (1974) Prediction of protein conformation. *Biochemistry* **13,** 222-245.
Chou, K. C., Liu, W. M., Maggiora, G. M. & Zhang, C. T. (1998) Prediction and classification of domain structural classes. *Proteins* **31,** 97-103.
Cohen, F. E., Abarbanel, R. M., Kuntz, I. D. & Fletterick, R. J. (1983) Secondary structure assignment for α/β proteins by a combinatorial approach. *Biochemistry* **22,** 4894-4904.
Corpet, F., Gouzy, J. & Kahn, D. (1998) The ProDom database of protein domain families. *Nucleic Acids Res.* **26,** 323-326.
Demeler, B. & Zhou, G. (1991) Neural network optimization for *E. coli* promoter prediction. *Nucleic Acids Res.* **19,** 1593-1599.
Dosztányi, Z., Fiser, A. & Simon, I. (1997) Stabilization centers in proteins: identification, characterization and predictions. *J. Mol. Biol*. **272,** 597-612.
Garnier, J., Osguthorpe, D. J. & Robson, B. (1978) Analysis of the accuracy and implications of simple methods for predicting the secondary structure of globular proteins. *J. Mol. Biol*. **120,** 97-120.
Gerstein, M., Lesk, A. M. & Chothia, C. (1994) Structural mechanisms for domain movements in proteins. *Biochemistry* **33,** 6739-6749.
Henikoff, S., Greene, E. A., Pietrokovski, S., Bork, P., Attwood, T. K. & Hood, L. (1997) Gene families: the taxonomy of protein paralogs and chimeras. *Science* **278,** 609-614.
Hirst, J. D. & Sternberg, M. J. E. (1992) Prediction of structural and functional features of protein and nucleic acid sequences by artificial neural networks. *Biochemistry* **31,** 7211-7128.
Holbrook, S. R., Muskal, S. M. & Kim, S. H. (1990). Predicting surface exposure of amino acids from protein sequences. *Protein Eng*. **3,** 659-665.
Horton, P. B. & Kanehisa, M. (1992) An assessment of neural network and statistical approaches for prediction of E.coli promoter sites. *Nucleic Acids Res*. **20,** 4331-4338.
Kabsh, W. & Sander, C. (1983) Dictionary of protein secondary structure: pattern recognition of hydrogen-bonded and geometrical features. *Biopolymers* **22,** 2577-2637.
Kikuchi, T., Nemethy, G. & Scheraga, H. A. (1988) Prediction of the location of structural domains in globular proteins. *J. Protein Chem*. **7,** 427-471.
King, R. D. & Sternberg, M. J. E. (1990) Machine learning approach for the prediction of protein secondary structure. *J. Mol*. *Biol*. **216,** 441-457.
King, R. D. & Sternberg, M. J. E. (1996) Identification and application of the concepts important for accurate and reliable protein secondary structure prediction. *Protein Sci*. **5,** 2298-2310.
Kranlis, P. J. (1991) MOLSCRIPT: a program to produce both detailed and schematic plots of protein structures. *J. Appl*. *Crystallogr*. **24,** 946-950.
Kuroda, Y., Tani, K., Matsuo, Y. & Yokoyama, S. (2000) Automated search of natively folded protein fragments for high-throughput structure determination in structural genomics. *Protein Sci*. **9,** 2313-21.
Lim, V. I. (1974) Structural principles of the globular organization of protein chains. A stereochemical theory of globular protein secondary structure. *J. Mol*. *Biol*. **88,** 857-872.
Merrit, E. A. & Murphy, M. E. P. (1994) Raster3D version 2.0. A program for photorealistic molecular graphics. *Acta Crystallogr*. **D50,** 869-863.
Murzin, A. G., Brenner, S. E., Hubbard, T. & Chothia, C. (1995) SCOP: a structural classification of proteins database for the investigation of sequences and structures. *J. Mol*. *Biol*. **247,** 536-540.
Ptitsyn, O. B. & Finkelsteia, A. V. (1983) Theory of protein secondary structure and algorithm of its prediction. *Biopolymers* **22,**15-25.
Qian, N. & Sejnowski, J. (1988) Predicting the secondary structure of globular proteins using neural network models. *J. Mol*. *Biol*. **202,** 865-884.
Radhakrishnan, I., Pérez-Alvarado, G. C., Parker, D., Dyson, H. J., Montininy, M. R. & Wright, P. E. (1999) Structural analyses of CREB-CBP transcriptional activator-coactivator complexes by NMR spectroscopy: implications for mapping the boundaries of structural domains. *J. Mol*. *Biol*. **287,**859-865.
Richardson, J. S. (1981) The anatomy and taxonomy of protein structure. *Adv. Protein Chem.* **34,** 246-253.
Romero, P., Obradovic, Z., Li, X., Garner, E. C., Brown, C. J. & Dunker, A. K. (2001) Sequence complexity of disordered protein. *Proteins* **42,** 38-48.
Post, B. & Sander, C. (1993) Prediction of protein secondary structure at better than 70% accuracy. *J*. *Mol*. *Biol*. **232,** 584-599.
Rumelhart, D. E., Hinton, G. E. & Williams, R. J. (1986) Learning representations by back-propagating errors. *Nature* **323,** 533-536.
Shepherd, A. J., Gorse, D. & Thornton, J. M. (1999) Prediction of the location and type of β-turns in proteins using neural networks. *Protein Sci*. **8,** 1045-1055.
Sonnhammer, E. L. L.& Kahn, D. (1994) Modular arrangement of proteins as inferred from analysis of homology. *Protein Sci*. **3,** 482-492.
Sternberg, M. J. E., Bates, P. A., Kelley, L. A. & MacCallum, R. M. (1999) Progress in protein structure prediction: assessment of CASP3. *Curr*. *Opin. Struct*. *Biol*. **9,** 368-373.
Uberbacher, E. C. & Mural, R. J. (1991) Locating protein-coding regions in human DNA sequences by a multiple sensor - neural network approach. *Proc. Natl*. *Acad*. *Sci*., *USA* **88,** 11261-11265.
Vonderviszt, F. & Simon, I. (1996) A possible way for prediction of domain boundaries in globular proteins from amino acid sequence. *Biochem*. *Biophys*. *Res. Commun*. **139,** 11-17.
Wheelan, S. J., Marchler-Bauer, A. & Bryant, S. H. (2000) Domain size distributions can predict domain boundaries. *Bioinformatics* **16,** 613-618.
Wider, G. & Wüthrich, K. (1999) NMR spectroscopy of large molecules and multimolecular assemblies in solution. *Curr*. *Opin. Struct*. *Biol*. **9,** 594-601.
Wilmot, C. M. & Thornton, J. M. (1988) Analysis and prediction of the different types of β-turn in proteins. *J. Mol*. *Biol*. **203,** 221-232.
Zvelebil, M. J., Barton, G. J., Taylor, W. R. & Sternberg, M. J. E. (1987) Prediction of protein secondary structure and active sites using the alignment of homologous sequences. *J*. *Mol. Biol*. **195,** 957-961.

Alroy, I. & Yarden, Y., *FEBS Letters,* **410.** 83-86, (1997)
Altschul, S. F. *et al.., Nuc. Acids Res*., **25,** 3389-3402, (1997)
Arjunan, P. *et al*., *J. Mol. Biol*., **256,** 590-600, (1996)
Beerli, R. R. and Hynes, N. E., *J. Biol. Chem*., **271,** 6071-6076, (1996)
Brown, P. O. & Botstein, D., *Nature Genet.,* **21,** 33-37, (1999)
Busetta, B. & Barrans, Y., *Biochem. Biophys. Acta*., **790,** 117-124, (1984)
Carraway, K. L. *et al., J. Biol. Chem,* **269,** 14303-14306, (1994a)
Carraway, K. L. & Cantley, L. C., *Cell,* **78,** 5-8, (1994b)
Chandonia, J. & Karplus, M., *Protein Sci*., **4,** 275-285, (1995)
Chou, K. C., Liu, W. M., Maggiora, G. M. and Zhang, C. T., *Proteins,* 31, 97-103, (1998)
Chou, M. M. & Blenis, J., *Cell,* **85,** 573-583, (1996)
Corpet, F., Gouzy, J. and Kahn, D., *Nuc. Acids Res*., **26,** 323-326, (1998)
Dosztányi, Z., Fiser, A. and Simon, I., *J*. *Mol*. *Biol*., **272,** 597-612, (1997)
Elenius, K. Paul, S., Allison, G., Sun, J. and Klagsbrun, M., *EMBO J.,* **16,** 1268-1278, (1997)
Funahashi, K., *Neural Networks*, **2,** 183-192, (1989)
Gaskell, A., Crennell, S. and Taylor, G., *Structure*, **3,** 1197-1205, (1995)
Graus-Porta, D., Beerli, R. and Hynes, N. E., *Mol. Cell*. *Biol*., **15,** 1182-1191, (1995)
Guy, P. M., Platko, J. V., Cantley, L. C., Carione, R. A. and Carraway, K. L., *Proc*. *Natl*. *Acad. Sci*. *USA*, **91,** 8132-8136, (1994)
Higashiyama, S., Abraham, J. A., Miller, J., Fiddes, J. C. and Klagsbrun, M., *Science,* **251,** 936-939, (1991)
Hirst, A. D. & Sternberg, M. J. E., *Biochemistry*, **31,** 7211-7218, (1992)
Holley, L. H. & Karplus, M., *Proc. Natl*. *Acad. Sci*. *USA*, **86,** 152-156, (1989)
Hubbard, S. J., *Biochem*. *Biophys. Acta*., **1382,** 191-206, (1998)
Hynes, N. E. & Stem, D. F., *Biochim*. *Biophys*. *Acta*., **1198,** 165-184, (1994)
Kabsh, W. & Sander, C., *Biopolymers,* **22,** 2577-2637, (1983)
Karunagaran, D. *et al*., *EMBO J*., **15,** 254-264, (1996)
King, R. D. & Sternberg, M. J., *Protein Sci*., **5,** 2298-2310, (1996)
Kneller, D. G., Cohen, F. E. and Langridge, R., *J. Mol*. *Biol*., **214,** 171-182, (1990)
Kosa, P. F., Ghosh, G., DeDecker, B. S. and Sigler, P. B., *Proc*. *Natl*. *Acad*. *Sci*. *USA*, **94,** 6042-6047, (1997)
Kraus, M. H., Issing, W., Miki, T. Popescu, N. C. and Aronson, S. A., *Proc*. *Natl*. *Acad. Sci. USA*, **86,** 9193-9197, (1989)
Marquardt, H., Hunkapiller, M. W., Hood, L. E. and Todaro, G., J., *Science*, **223,** 1079-1082, (1984)
Muchmore, C. R., Krahn, J. M., Kim., J. H., Zalkin, H. and Smith, J. L., *Protein Sci*., **7,** 39-51, (1998)
Murzin, A. G., Brenner, S. E., Hubbard, T. and Chothia, C.. *J. Mol*. *Biol*., **247,** 536-540, (1995)
Plowman, G. D. *et al*., *Proc. Natl*. *Acad. Sci*. *USA,* **90,** 1746-1750, (1993a)
Plowman, G. D. *et al*., *Nature*, **366,** 473-475, (1993b)
Qian, N. & Sejnowski, T. J., *J. Mol*. *Biol*., **202,** 865-884, (1988)
Riese, D. J., Bermingham, Y. and van Raaij, *Oncogene*, **12,** 345-353, (1996)
Post, B. & Sander, C., *J. Mol. Biol*., **232,** 584-599, (1993)
Rumelhart, D. E., Hinton, G. E. and Williams, R. J., *Nature,* **323,** 533-536, (1986)
Savage, C. R., Jr., Inagami, T. and Cohen, S., *J*. *Biol*. *Chem*., **241,** 7612-7621, (1972)
Shing, Y. *et al*., *Science*, **259,** 1604-1607, (1993)
Shoyab, M., Plowman, G. D., McDonald, V. L., Bradley, J. G. and Todaro, G. J., *Science,* **243,** 1074-1076, (1989)
Tzahar, E. *et al*. *EMBO J*., **16,** 4938-4950, (1998)
Uberbacher, E. C. & Mural, R. J., *Proc. Natl*. *Acad*. *Sci. USA*, **88,** 11261-11265, (1991)
Ullrich, A. *et al*., *Nature,* 309, 418-425, (1984)
Vondervisat, F. & Simon, I., *Biochem*. *Biophys. Res. Commun*., **139,** 11-17, (1986)
Wen, D. *et al*., *Cell,* **69,** 559-572, (1992)
Yamamoto, T. *et al*., *Nature,* **319,** 230-234, (1986)

All the publications, patents and patent applications quoted in this specification are incorporated as they are in this specification as reference.

### Industrial applicability

By this invention, a linker sequence of a protein can be predicted.

Also, by this invention, characteristics of a sequence of a domain linker were identified. Using these characteristics, a linker sequence can be detected in an amino-acid sequence of a protein, and as a result, a structural domain region of a protein can be predicted.

When the linker sequence can be predicted, a protein can be divided into structural domains. It is difficult to analyze the structure, of a protein with large molecular weight, but if a protein can be divided into structural domains with small molecular weights, structural analysis and functional analysis per structural domain would be enabled, and functional analysis of a protein would progress at a significant speed.

## Claims

1. A method of training a neural network to identify a linker sequence of a protein consisting of 2 or more structural domains comprising:
a dividing step for dividing an amino-acid sequence of a protein consisting of 2 or more structural domains of a data set into a linker sequence and a non-linker sequence;
a window setting step for taking a window of a range of 5 to 35 residues within the amino-acid sequence of the protein consisting of two or more structural domains of the data set;
a sequence classifying step in which, if an amino-acid residue located at the center of the window constitutes a part of the linker sequence, a numeral value is granted to classify the amino-acid sequence in the winder as a positive sequence and if the amino-acid residue located at the center of the window constitutes a part of the non-linker sequence, a numeral value is granted to classify the amino-acid sequence in the window as a negative sequence; and
a learning step for repeatedly learning to optimize a weight parameter of a hierarchical neural network by a back-propagation method,
in which a value representing an amino-acid sequence in the window in numerals is input to the hierarchical neural network to acquire an output value, the error between the output value and the numeral value which classifies the amino-acid sequence in the window either as a positive sequence or as a negative sequence is calculated, and the weight parameter of the hierarchical neural network is so detemined that the error becomes minimal.

2. A method of predicting a linker sequence of a protein whose structure is unknown comprising:
a window setting step for taking a window of a range of 5 to 35 residues within an amino-acid sequence of a protein whose structure is unknown;
an input / output step for obtaining an output value by inputting a value of the amino-acid sequence in the window represented in numerals into a hierarchical neutral network having trained by the method of claim 1;
a predicted value granting step for granting the output value to an amino-acid residue located at the center of the window as a predicted value;
a step of repeating the input / output step and the predicted value granting step, with the position of the window being moved within a desired range of the amino-acid sequence of the protein whose structure is unknown; and
a linker sequence predicting step for predicting as a linker sequence a region cosisting of amino-acid residues with the predicted values larger than a preset threshold value.

3. A method as set forth in claim 2 comprising, following the step of repeating the input / output step and the predicted value granting step:
an average value calculating step for obtaining an average value by taking a new window of a range more than the predetermined number of residues within the amino-acid sequence of the protein whose structure is unknown and smoothing the predicted values over the amino-acid residues within this window; and
a step for repeating the average value calculating step, with the position of the new window being moved within a desired range of the amino-acid sequence of the protein whose structure is unknown, and in the linker sequence predicting step, a linker sequence is predicted by the threshold with respect to the average value of the predicted values.

4. A method as set forth in claim 3, wherein in the linker sequence predicting step, if the largest of the predicted values for the amino-acid residues in a region consisting of amino-acid residues whose average value of the predicted values, is larger than a preset threshold value is larger than a preset cut-off value, that region is predicted as a linker sequence.

5. A system for predicting a linker sequence of a protein whose structure is unknown comprising an amino-acid sequence input means for inputting numerals that represent the amino-acid sequence of the protein whose structure is unknown, a window setting means for taking a window in the amino-acid sequence of the protein whose structure is unknown, an in-window amino-acid sequence input means by which numerals that represent the amino-acid sequence in the window are input into a hierarchical neural network trained to identify the linker sequence of a protein consisting of 2 or more structural domains, an output value calculating means for having the hierarchical neural network calculate an output value, a predicted value granting means for granting the output value to the amino-acid residue located at the center of the window as a predicted value, a window-position moving means for moving the position of the window within a desired range of the amino-acid sequence of the protein whose structure is unknown, a smoothing window setting means for taking a new window of a range more than the predetermined number of residues in the amino-acid sequence of the protein whose structure is unknown, an average value calculating means for obtaining an average value by smoothing predicted values over the amino-acid residues in the new window, a smoothing window moving means for moving the position of the new window within a desired range of the amino-acid sequence of the protein whose structure is unknown, and a linker sequence predicting means for predicting as a linker sequence a region consisting of the amino-acid residues whose average value of the predicted values is larger than a preset threshold value.

6. A program for having a computer function as a system for predicting a linker sequence of a protein whose structure is unknown **characterized in that** the system comprises an amino-acid sequence input means for inputting numerals that represent the amino-acid sequence of the protein whose structure is unknown, a window setting means for taking a window in the amino-acid sequence of the protein whose structure is unknown, an in-window amino-acid sequence input means by which numerals that represent the amino-acid sequence in the window are input into a hierarchical neural network trained to identify the linker sequence of a protein consisting of 2 or more structural domains, an output value calculating means for having the hierarchical neural network calculate an output value, a predicted value granting means for granting the output value to the amino-acid residue located at the center of the window as a predicted value, a window-position moving means for moving the position of the window within a desired range of the amino-acid sequence of the protein whose structure is unknown, a smoothing window setting means for taking a new window of a range more than the predetermined number of residues in the amino-acid sequence of the protein whose structure is unknown, an average value calculating means for obtaining an average value by smoothing predicted values over the amino-acid residues in the new window, a smoothing window moving means for moving the position of the new window within a desired range of the amino-acid sequence of the protein whose structure is unknown, and a linker sequence predicting means for predicting as a linker sequence a region consisting of the amino-acid residues whose average value of the predicted values is larger than a preset threshold value.

7. A computer readable recording medium having recorded thereon a program for having a computer function as a system for predicting a linker sequence of a protein whose structure is unknown **characterized in that** the system comprises an amino-acid sequence input means for inputting numerals that represent the amino-acid sequence of the protein whose structure is unknown, a window setting means for taking a window in the amino-acid sequence of the protein whose structure is unknown, an in-window amino-acid sequence input means by which numerals that represent the amino-acid sequence in the window are input into a hierarchical neural network trained to identify the linker sequence of a protein consisting of 2 or more structural domains, an output value calculating means for having the hierarchical neural network calculate an output value, a predicted value granting means for granting the output value to the amino-acid residue located at the center of the window as a predicted value, a window-position moving means for moving the position of the window within a desired range of the amino-acid sequence of the protein whose structure is unknown, a smoothing window setting means for taking a new window of a range more than the predetermined number of residues in the amino-acid sequence of the protein whose structure is unknown, an average value calculating means for obtaining an average value by smoothing predicted values over the amino-acid residues in the new window, a smoothing window moving means for moving the position of the new window within a desired range of the amino-acid sequence of the protein whose structure is unknown, and a linker sequence predicting means for predicting as a linker sequence a region consisting of the amino-acid residues whose average value of the predicted values is larger than a preset threshold value.

8. A method of producing a protein fragment corresponding to one or more structural domains located closer to the N-terminal side than a predicted linker sequence comprising a step for producing at least one of the protein fragments obtained by cutting off a protein at any of the following portions (i), (ii) or (iii):
(i) an arbitrary portion of at least one linker sequence predicted by the method as set forth in any of claims 2 through 4;
(ii) any of portions located between the C-terminal of at least one linker sequence predicted by the method as set forth in any of claims 2 through 4 and the 50^{th} amino-acid residue as counted therefrom to the C-terminal side of the protein; or
(iii) any of portions located between the N-terminal of at least one linker sequence predicted by the method as set forth in any of claims 2 through 4 and the 15^{th} amino-acid residue as counted therefrom to the N-terminal side of the protein.

9. A method of producing a protein fragment corresponding to one or more structural domains located closer to the C-terminal side than a predicted linker sequence comprising a step for producing at least one of the protein fragments obtained by cutting off a protein at any of the following portions (i), (iv) or (v):
(i) an arbitrary portion of at least one linker sequence predicted by the method as set forth in any of claims 2 through 4;
(iv) any of portions located between the N-terminal of at least one linker sequence predicted by the method as set forth in any of claims 2 through 4 and the 50^{th} amino-acid residue as counted therefrom to the N-terminal side of the protein; or
(v) any of portions located between the C-terminal of at least one linker sequence predicted by the method as set forth in any of claims 2 through 4 and the 15^{th} amino-acid residue as counted therefrom to the C-terminal side of the protein.

10. A method of analyzing a protein fragment corresponding to one or more structural domains located closer to the N-terminal side than a predicted linker sequence comprising a step for analyzing at least one of the protein fragments obtained by cutting off a protein at any of the following portions (i), (ii) or (iii):
(i) an arbitrary portion of at least one linker sequence predicted by the method as set forth in any of claims 2 through 4;
(ii) any of portions located between the C-terminal of at least one linker sequence predicted by the method as set forth in any of claims 2 through 4 and the 50^{th} amino-acid residue as counted therefrom to the C-terminal side of the protein; or
(iii) any of portions located between the N-terminal of at least one linker sequence predicted by the method as set forth in any of claims 2 through 4 and the 15^{th} amino-acid residue as counted therefrom to the N-terminal side of the protein.

11. A method of analyzing a protein fragment corresponding to one or more structural domains located closer to the C-terminal side than a predicted linker sequence comprising a step for analyzing at least one of the protein fragments obtained by cutting off a protein at any of the following portions (i), (iv) or (v):
(i) an arbitrary portion of at least one linker sequence predicted by the method as set forth in any of claims 2 through 4;
(iv) any of portions located between the N-terminal of at least one linker sequence predicted by the method as set forth in any of claims 2 through 4 and the 50^{th} amino-acid residue counted therefrom to the N-terminal side of the protein; or
(v) any of portions located between the C-terminal of at least one linker sequence predicted by the method as set forth in any of claims 2 through 4 and the 15^{th} amino-acid residue as counted therefrom to the C-terminal side of the protein.

12. A method of constructing a linker sequence database comprising a step for recording in a recording medium the amino-acid sequence data for the linker sequence predicted by the method as set forth in any of claims 2 through 4.

13. A method of constructing a structural domain database comprising a step for recording in a recording medium the amino-acid sequence data for the structural domain obtained by cutting off a protein at an arbitrary portion of at least one linker sequence predicted by the method as set forth in any of the claims 2 through 4.

14. A peptide which has a sequence pattern satisfying the conditions of (i) and (ii) below and can function as a domain linker of a multi-domain protein:
(i) when a sequence fragment consisting of 19 residues in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈ {0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
the value of the following g(x) should be in a range of 0.5 to 1.0:$\text{g} {\text{(x) = τ(ν}}_{\text{0}} {\text{+ ν}}_{\text{1}} {\text{f}}_{\text{1}} {\text{(x) + ν}}_{\text{2}} {\text{f}}_{\text{2}} \text{(x))}$ 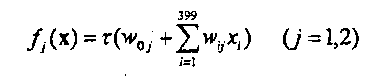 $\text{τ(} \text{u} \text{) = 1/(1+} {\text{e}}^{\text{-}} {\text{}}^{\text{u}} \text{)}$ (where a combination of wᵢⱼ(i=0, ·····, 399; j=1,2) and vⱼ(j=0, 1, 2) is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
(ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.

15. A method of predicting a region having a sequence pattern satisfying the conditions of (i) and (ii) below as a linker sequence of protein:
(i) when a sequence fragment consisting of 19 residues in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈ {0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
the value of the following g(x) should be in a range of 0.5 to 1.0:$\text{g} {\text{(x) = τ(ν}}_{\text{0}} {\text{+ ν}}_{\text{1}} {\text{f}}_{\text{1}} {\text{(x) + ν}}_{\text{2}} {\text{f}}_{\text{2}} \text{(x))}$ 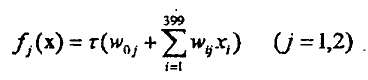 $\text{τ(} \text{u} \text{) = 1/(1+} {\text{e}}^{\text{-}} {\text{}}^{\text{u}} \text{)}$ (where a combination of wᵢⱼ(i=0, ·····, 399; j=1,2) and vⱼ(j=0, 1, 2) is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
(ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.

16. A method of dividing a protein into structural domains **characterized in that** the protein is cut off at an arbitrary portion of a region having a sequence pattern satisfying the conditions of (i) and (ii) below:
(i) when a sequence fragment consisting of 19 residues in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈ {0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" **in that** order and for the 21-bit binary sequence, only those matching the amino acid types of the represented residues are 1, while the others are 0).
the value of the following g(x) sould be in a range of 0.5 to 1.0:$\text{g} {\text{(x) = τ(ν}}_{\text{0}} {\text{+ ν}}_{\text{1}} {\text{f}}_{\text{1}} {\text{(x) + ν}}_{\text{2}} {\text{f}}_{\text{2}} \text{(x))}$ 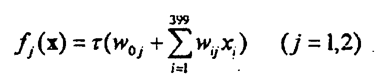 $\text{τ} \text{(} \text{u} \text{) = 1/(1 +} {\text{e}}^{\text{-}} {\text{}}^{\text{u}} \text{)}$ (where a combination of wᵢⱼ(i=0,·····, 399; j=1,2) and vⱼ(j=0, 1, 2)is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
(ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.

17. A method of producing a protein fragment comprising a step for producing at least one of the protein fragments obtained by cutting off a protein at an arbitrary portion of a region having a sequence pattern satisfying the conditions of (i) and (ii) below:
(i) when a sequence fragment consisting of 19 residues in succession is represented numerically by an equation x:$\text{x = (} {\text{x}}_{\text{1}} \text{,} {\text{x}}_{\text{2}} \text{,···,} {\text{x}}_{\text{399}} \text{)(} {\text{x}}_{\text{i}} \text{∈ {0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
the value of the following g(x) should be in a range of 0.5 to 1.0:$\text{g} {\text{(x) = τ(ν}}_{\text{0}} {\text{+ ν}}_{\text{1}} {\text{f}}_{\text{1}} {\text{(x) + ν}}_{\text{2}} {\text{f}}_{\text{2}} \text{(x))}$ 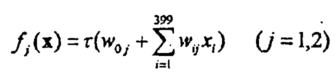 $\text{τ} \text{(} \text{u} \text{) = 1/(1 +} {\text{e}}^{\text{-u}} \text{)}$ (where a combination of wᵢⱼ(i=0,·····, 399; j=1,2) and vⱼ(j=0, 1, 2)is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
(ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.

18. A method of analyzing a protein fragment comprising a step for analyzing at least one of the protein fragments obtained by cutting off protein at an arbitrary portion of a region having a sequence pattern satisfying the conditions of (i) and (ii) below:
(i) when a sequence fragment consisting of 19 residues in succession is represented numerically by an equation x:$\text{x = (} {\text{x}}_{\text{1}} \text{,} {\text{x}}_{\text{2}} \text{,···} {\text{x}}_{\text{399}} \text{)(} {\text{x}}_{\text{i}} \text{∈{0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
the value of the following g(x) should be in a range of 0.5 to 1.0:$\text{g} {\text{(x) = τ(ν}}_{\text{0}} {\text{+ ν}}_{\text{1}} {\text{f}}_{\text{1}} {\text{(x) + ν}}_{\text{2}} {\text{f}}_{\text{2}} \text{(x))}$ 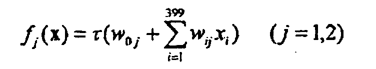 $\text{τ} \text{(} \text{u} \text{) = 1/(1 +} {\text{e}}^{\text{-}} {\text{}}^{\text{u}} \text{)}$ (where a combination of wᵢⱼ(i=0,·····, 399; j=1,2) and vⱼ(j=0, 1, 2)is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
(ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.

19. A method of producing a new multi-domain protein by designing a new linker sequence with a peptide having a sequence pattern satisfying the conditions of (i) and (ii) below and by connecting at least two protein fragments:
(i) when a sequence fragment consisting of 19 in succession is represented numerically by an equation x:$\text{x=(} {\text{x}}_{\text{1}} \text{,} {\text{x}}_{\text{2}} \text{,···,} {\text{x}}_{\text{399}} \text{)(} {\text{x}}_{\text{i}} \text{∈ {0,1} (} \text{i} \text{= 1,···,399))}$ (where, x = (x₁, x₂,·····, x₃₉₉) is a 399-bit (= 19 x 21) binary sequence obtained as a result of arrangement in series of 21-bit binary sequences associated with amino acid types according to the sequence of the 19 residues of the sequence fragment, and the bit sequence corresponds to "alanine (A), cysteine (C), aspartic acid (D), glutamic acid (E), phenylalanine (F), glycine(G), histidine (H), isoleucine (I), lysine (K), leucine (L), methionine (M), asparagines (N), proline (P), glutamine (Q), arginine (R), serine (S), threonine (T), valine (V), tryptophan (W), tyrosine (Y), others (X)" in that order and for the 21-bit binary sequence, only those matching the amino acid types of the represented residues are 1, while the others are 0),
the value of the following g(x) shoud be in a range of 0.5 to 1.0:$\text{g} {\text{(x) = τ(ν}}_{\text{0}} {\text{+ ν}}_{\text{1}} {\text{f}}_{\text{1}} {\text{(x) + ν}}_{\text{2}} {\text{f}}_{\text{2}} \text{(x))}$ 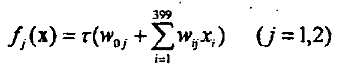 $\text{τ(} \text{u} {\text{) = 1/(1 + e}}^{\text{-u}} \text{)}$ (where a combination of wᵢⱼ(i=0,·····, 399; j=1,2) and vⱼ(j=0, 1, 2)is selected from the group consisting of the combinations of Group 1 in Table A, the combinations of Group 2 in Table B, the combinations of Group 3 in Table C, the combinations of Group 4 in Table D, the combinations of Group 5 in Table E, the combinations of Group 6 in Table F, the combinations of Group 7 in Table G, the combinations of Group 8 in Table H, the combinations of group 9 in Table I, and the combinations of Group 10 in Table J);
(ii) a central residue of the sequence fragment x=(x₁, x₂,·····, x₃₉₉) with the value of g(x) in the range of 0.5 to 1.0 should be included, with an amino acid within 9 residues before and after the central residue being optionally further included.

20. A method comprising:
i) a step for extracting a linker sequence and a non-linker loop sequence from a database of multi-domain proteins of known structures; and
ii) a step for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino-acid residue Xₐₐ occurring in a linker sequence and a non-linker loop sequence, respectively) and the probabilities P_{XaaYaa(m)}^{L} and P_{XaaYaa(m)}^{N} of occurrence of the amino-acid residues Xₐₐ and Yₐₐ as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring in the linker sequence and the non-linker loop sequence, respectively, as interrupted by m amino acid residues (the order of Xₐₐ and Yₐₐ does not matter)), said method predicting and/or detecting a linker sequence in a multi-domain protein of unknown structure from the characteristics in terms of the amino-acid sequence of the linker sequence extracted in step i).

21. A system comprising:
i) a means for extracting a linker sequence and a non-linker loop sequence from a database of multi-domain proteins of known structures i; and
ii) a means for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino-acid residue Xₐₐ occurring in a linker sequence and a non-linker loop sequence, respectively) and the probabilities P_{XaaYaa(m)}^{L} and P_{XaaYaa(m)}^{N} of occurrence of the amino-acid residues Xₐₐ and Yₐₐ as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring in the linker sequence and then-linker loop sequence, respectively, as interrupted by m amino acid residues (the order of Xₐₐ and Yₐₐ does not matter)), said system predicting and/or detecting a linker sequence in a multi-domain protein of unknown structure from the characteristics in terms of the amino-acid sequence of the linker sequence extracted by the means of i).

22. A program for having a computer function as a system for predicting and / or detecting a linker sequence in a multi-domain protein of unknown structure from the characateristics in terms of its amino acid sequence, the system comprising
i) a means for extracting a linker sequence and a non-linker loop sequence from a database of multi-domain proteins of known structures; and
ii) a means for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino-acid residue Xₐₐ occurring in a linker sequence and a non-linker loop sequence, respectively) and the probabilities P_{XaaYaa(m)}^{L} and P_{XaaYaa(m)}^{N} of occurrence of the amino-acid residues Xₐₐ and Yₐₐ as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring in the linker sequence and the non-linker loop sequence, respectively, as interrupted by m amino acid residues (the order of Xₐₐ and Yₐₐ does not matter)).

23. A structural domain predicting method comprising a step in which a protein fragment generated by cutting off a multi-domain protein of unknown structure at any of the portions of a linker sequence in the multi-domain protein after it was predicted by the method as set forth in claim 20 is predicted as a structural domain.

24. A protein producing method comprising a step for producing a protein having the same amino-acid sequence as the structural domain predicted by the method as set forth in claim 23.

25. A protein analyzinig method comprising a step for analyzing a protein having the same amino-acid sequence as the structural domain predicted by the method as set forth in claim 23.

26. A system for calculating a parameter of an occurrence trend of an amino-acid residue comprising:
i) a means for extracting a linker sequence and a non-linker loop sequence from a database of multi-domain proteins of known structures;
ii) a means for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino acid residue Xₐₐ occurring in a linker sequence and a non-linker loop sequence, respectively)
iii) a means for obtaining an occurrence trend parameter S_{Xaa} of the amino-acid residue Xₐₐ by the following equation :${\text{S}}_{\text{Xaa}} {\text{=log (P}}_{\text{Xaa}} {\text{}}^{\text{L}} {\text{/P}}_{\text{Xaa}} {\text{}}^{\text{N}} \text{)}$ (where S_{Xaa}=0 if there is no statistically significant difference between P_{Xaa}^{L} and P_{Xaa}^{N}).

27. A program for having a computer function as a system for calculating a parameter representing an occurrence trend of an arbitrary amino-acid residue, the system comprising:
i) a means for extracting a linker sequence and a non-linker loop sequence from a database of multi-domain proteins of known structures;
ii) a means for obtaining, based on statistical processing of amino-acid sequence of each domain, the probabilities P_{Xaa}^{L} and P_{Xaa}^{N} of occurrence of an amino-acid residue Xₐₐ (where P_{Xaa}^{L} and P_{Xaa}^{N} are the probabilities of the amino acid residue Xₐₐ occurring in a linker sequence and a non-linker loop sequence, respectively); and
iii) a means for obtaining an occurrence trend parameter S_{Xaa} of the amino.acid residue Xₐₐ by the following equation:${\text{S}}_{\text{Xaa}} {\text{=log (P}}_{\text{Xaa}} {\text{}}^{\text{L}} {\text{/P}}_{\text{Xaa}} {\text{}}^{\text{N}} \text{)}$ (where S_{Xaa}=0 if there is no statistically significant difference between P_{Xaa}^{L} and P_{Xaa}^{N}).

28. A system for calculating a parameter of an appearance trend of an amino-acid residue pair comprising:
i) a means for extracting a linker sequence and a non-linker loop sequence from a database of multi-domain proteins of known structures;
ii) a means for obtaining, based on statistical processing of amino acid sequence of each domain, the probabilities P_{XaaYaa(m)}^{L} and P_{XaaYaa}(m)^{N} of occurrence of amino-acid residues Xₐₐ and Yₐₐ (the order of Xₐₐ and Yₐₐ does not matter) as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring (the order of Xₐₐ and Yₐₐ does not matter) in a linker sequence and a non-linker loop sequence, respectively, as interrupted by m amino-acid residues (m is an integer, m = 0, 1, 2)) for the cases where m is 0, 1 and 2, respectively; and
iii) a means for obtaining an occurrence trend parameter S_{XaaYaa(m)} of the pair of amino acid residues Xₐₐ and Yₐₐ by the following equation:${\text{S}}_{\text{XaaYaa(m)}} {\text{=log (P}}_{\text{XaaYaa(m)}} {\text{}}^{\text{L}} {\text{/P}}_{\text{XaaYaa(m)}} {\text{}}^{\text{N}} \text{)}$ (where S_{Xaa}=0 if there is no statistically significant difference between P_{XaaYaa(m)}^{L} and P_{XaaYaa(m)}^{N}).

29. A program for having a computer function as a system for calculating a parameter representing an occurrence trend of an arbitrary amino-acid residue pair, the system comprising:
i) a means for extracting a linker sequence and a non-linker loop sequence from a database of multi-domain proteins of known structures;
ii) a means for obtaining, based on statistical processing of amino acid sequence of each domain, the probabilities P_{XaaYaa(m)}^{L} and P_{XaaYaa(m)}^{N} of occurrence of amino-acid residues Xₐₐ and Yₐₐ (the order of Xₐₐ and Yₐₐ does not matter) as interrupted by m (m is an integer, m = 0, 1, 2) arbitrary amino-acid residues (where P_{XaaYaa(m)}^{L} and P_{XaaYaa(m)}^{N} are the probabilities of the amino-acid residues Xₐₐ and Yₐₐ occurring (the order of Xₐₐ and Yₐₐ does not matter) in a linker sequence and a non-linker loop sequence, respectively, as interrupted by m amino-acid residues (m is an integer, m = 0, 1, 2)) for the cases where m is 0, 1 and 2, respectively; and
iii) a means for obtaining an occurrence trend parameter S_{XaaYaa(m)} of the pair of amino-acid residues Xₐₐ and Yₐₐ by the following equation:${\text{S}}_{\text{XaaYaa(m)}} {\text{=log (P}}_{\text{XaaYaa(m)}} {\text{}}^{\text{L}} {\text{/P}}_{\text{XaaYaa(m)}} {\text{}}^{\text{N}} \text{)}$ (where S_{Xaa}=0 if there is no statistically significant difference between P_{XaaYaa(m)}^{L} and P_{XaaYaa(m)}^{N}).

30. A system for obtaining a linker degree determination score F₁ for an amino-acid sequence with L₁ amino-acid residues (L₁ is an integer of 1 or more but not more than 21), the system comprising:
i) a means for obtaining a linker trend score F₁s of an amino-acid residue Aₖ by the following equation: 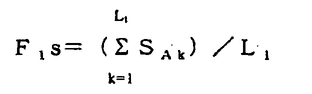 (where S_{Ak}=log(P_{Ak}^{L}/P_{Ak}^{N})
where S_{Ak}=0 if there is no statistically significant difference between P_{Ak}^{L} and P_{Ak}^{N};
P_{Ak}^{L} and P_{Ak}^{N} are the probabilities of the amino-acid residue Aₖ occurring in a linker sequence and a non-linker loop sequence, respectively);
ii) a means for obtaining a linker trend score F₁p of the pair of amino-acid residues Aₖ and Aₖ₊₍ₘ₊₁₎, as interrupted by m arbitrary amino-acid residues (m is an integer, m = 0, 1, 2), by the following equation: 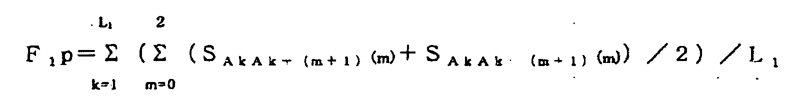 (where S_{AkAk+(m+1)(m)}=log(P_{AkAk+(m+1)(m)}^{L}/P_{AkAk+(m+1)(m)}^{N}) and S_{AkAk-(m+1)(m)}=log(P_{AkAk-(m+1)(m)}^{L}/P_{AkAk-(m+1)(m)}^{N})
where S_{AkAk+(m+1)(m)}=0 or S_{AkAk-(m+1)(m)}=0 if there is no statistically significant difference between P_{AkAk+(m+1)(m)}^{L} and P_{AkAk+(m+1)(m)}^{N} or between P_{AkAk-(m+1)(m)}^{L} and P_{AkAk-(m+1)(m)}^{N};
P_{AkAk+(m+1)(m)}^{L} and P_{AkAk+(m+1)(m)}^{N} are the probabilities of the arbitrary amino-acid residues Aₖ and Aₖ₊₍ₘ₊₁₎ occurring in a linker sequence and a non-linker loop sequence, respectively (the order of Aₖ and Aₖ₊₍ₘ₊₁₎ does not matter), and P_{AkAk-(m+1)(m)}^{L} and P_{AkAk-(m+1)(m)}^{N} are the probabilities of the arbitrary amino-acid residues Aₖ and Aₖ₋₍ₘ₊₁₎ occurring in the linker sequence and the non-linker loop sequence, respectively (the order of Aₖ and Aₖ₋₍ₘ₊₁₎ occurring does not matter)); and
iii) a means for obtaining a linker degree determination score F₁ by the following equation below:${\text{F}}_{\text{1}} {\text{=F}}_{\text{1}} {\text{s+ α}}_{\text{1}} {\text{F}}_{\text{1}} \text{p}$ (where 0≦ α₁ ≦ 1).

31. A program for having a computer function as a system for obtaining a linker degree determination score F₁ for an amino-acid sequence with L₁ amino-acid residues (L₁ is an integer of 1 or more but not more than 21), the system comprising:
i) a means for obtaining a linker trend score F₁s of an amino-acid residue Aₖ by the following equation: 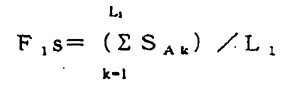 (where S_{Ak}=log(P_{Ak}^{L}/P_{Ak}^{N})
where S_{Ak}=0 if there is no statistically significant difference between P_{AK}^{L} and P_{AK}^{N};
P_{Ak}^{L} and P_{AK}^{N} are the probabilities of the amino-acid residue Aₖ occurring in a linker sequence and a non-linker loop sequence, respetively);
ii) a means for obtaining a linker trend score F₁p of the pair of amino-acid residues Aₖ and Aₖ₊₍ₘ₊₁₎, as interrupted by m arbitrary amino-acid residues (m is an integer, m = 0, 1, 2), by the following equation: 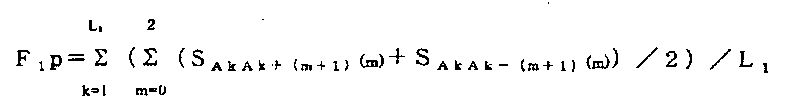 (where S_{AkAk+(m+1)(m)}=log(P_{AkAk+(m+1)(m)}^{L}/P_{AkAk+(m+1)(m)}^{N}) and${\text{S}}_{\text{AkAk-(m+1)(m)}} {\text{=log(P}}_{\text{AkAk-(m+1)(m)}} {\text{}}^{\text{L}} {\text{/P}}_{\text{AkAk-(m+1)(m)}} {\text{}}^{\text{N}} \text{)}$ where S_{AkAk+(m+1)(m)}=0 or S_{AkAk-(m+1)(m)}=0 if there is no statistically significant difference between P_{AkAk+(m+1)(m)}^{L} and P_{AkAk+(m+1)(m)}^{N} or between P_{AkAk-(m+1)(m)}^{L} and P_{AkAk-(m+1)(m)}^{N};
P_{AkAk+(m+1)(m)}^{L} and P_{AkAk+(m+1)(m)}^{N} are the probabilities of the arbitrary amino-acid residues Aₖ and Aₖ₊₍ₘ₊₁₎ occurring in a linker sequence and a non-linker loop sequence, respectively (the order of Aₖ and Aₖ₊₍ₘ₊₁₎ does not matter), and P_{AkAk-(m+1)(m)}^{L} and P_{AkAk-(m+1)(m)}^{N} are the probabilities of the arbitrary amino-acid residues Aₖ and Aₖ₋₍ₘ₊₁₎ occurring in the linker sequence and the non-linker loop sequence, respectively (the order of Ak and Aₖ₋₍ₘ₊₁₎ does not matter)); and
iii) a means for obtaining a linker degree determination score F₁ by the following equation:${\text{F}}_{\text{1}} {\text{=F}}_{\text{1}} {\text{s+ α}}_{\text{1}} {\text{F}}_{\text{1}} \text{p}$ (where 0≦ α₁≦ 1).

32. A method of obtaining a linker degree determination score F₁₁(i) for an amino-acid residue Ai at a position i in an amino-acid sequence with L₂ amino-acid residues (L₂ is an integer of 22 or more) by taking a window of w amino-acid residues before and after the amino-acid residue at the position i (i is an integer of 1 or more but not more than L₂) comprising:
i) a step for obtaining a linker trend determination score F₁₁s(i) of an amino-acid residue Aₖ by the folloing equation: 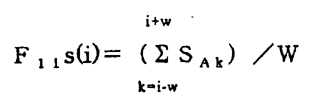 (where W is the window width, and W=2w+1,${\text{S}}_{\text{Ak}} {\text{=log(P}}_{\text{Ak}} {\text{}}^{\text{L}} {\text{/P}}_{\text{Ak}} {\text{}}^{\text{N}} \text{)}$ where S_{Ak}=0 if there is no statistically significant difference between P_{Ak}^{L} and P_{Ak}^{N};
P_{Ak}^{L} and P_{Ak}^{N} are the probabilities of the amino-acid residue Aₖ occurring in a linker sequence and a non-linker loop sequence, respectively);
ii) a step for obtaining the linker trend score F₁₁p(i) of the pair of amino-acid residues Aᵢ and Aᵢ₊₍ₘ₊₁₎, as interrupted by m arbitrary amino-acid residues (m is an integer, m = 0, 1, 2), by the following equation: 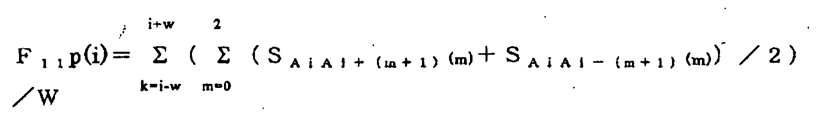 (where S_{AiAi+(m+1)(m)}=log(P_{AiAi+(m+1)(m)}^{L}/P_{AiAi+(m+1)(m)}^{N}) and${\text{S}}_{\text{AiAi-(m+1)(m)}} {\text{=log(P}}_{\text{AiAi-(m+1)(m)}} {\text{}}^{\text{L}} {\text{/P}}_{\text{AiAi-(m+1)(m)}} {\text{}}^{\text{N}} \text{)}$ where S_{AiAi+(m+1)(m)}=0 or S_{AiAi-(m+1)(m)}=0 if there is no statistically significant diffrerence between P_{AiAi+(m+1)(m)}^{L} and P_{AiAi+(m+1)(m)}^{N} or between P_{AiAi-(m+1)(m)}^{L} and P_{AiAi-(m+1)(m)}^{N};
P_{AiAi+(m+1)(m)}^{L} and P_{AiAi+(m+1)(m)}^{N} are the probabilities of the pair of the arbitrary amino-acid residues Aᵢ and Aᵢ₊₍ₘ₊₁₎ occurring in a linker sequence and a non-linker loop sequence, respectively (the order of Aᵢ and Aᵢ₊₍ₘ₊₁₎ does not matter), and P_{AiAi-(m+1)(m)}^{L} and P_{AiAi-(m+1)(m)}^{N} are the probabilities of the pair of the arbitrary amino-acid residues Aᵢ and Aᵢ₋₍ₘ₊₁₎ occurring in the linker sequence and the non-linker loop sequence, respectively (the order of Aᵢ and Aᵢ₋₍ₘ₊₁₎ does not matter)); and
iii) a step for obtaining the linker degree determination score F₁₁(i) of the amino-acid residue Ai at the position i by the following equation:${\text{F}}_{\text{11}} {\text{(i)=F}}_{\text{11}} {\text{s(i)+α}}_{\text{11}} {\text{F}}_{\text{11}} \text{p(i)}$ (where 0≦ α₁₁≦1).

33. A system for obtaining a linker degree determination score F₁₁(i) for an amino-acid residue Ai at a position i in an amino-acid sequence with L₂ amino-acid residues (L₂ is an integer of 22 or more) by taking a window of w amino-acid residues before and after the amino-acid residue at the position i (i is an integer of 1 or more but not more than L₂) comprising:
i) a step for obtaining a linker trend determination score F₁₁s(i) of an amino-acid residue Aₖ by following equation: 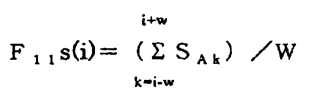 (where W is the window width, and W=2w+1.${\text{S}}_{\text{Ak}} {\text{=log(P}}_{\text{Ak}} {\text{}}^{\text{L}} {\text{/P}}_{\text{Ak}} {\text{}}^{\text{N}} \text{)}$ where S_{Ak}=0 if there is no statistically significant difference between P_{Ak}^{L} and P_{Ak}^{N};
P_{Ak}^{L} and P_{Ak}^{N} are the probabilities of the amino-acid residue Aₖ occurring in a linker sequence and a non-linker loop sequence, respectively);
ii) a step for obtaining the linker trend score F₁₁p(i) of the pair of amino-acid residues A, and Aᵢ₊₍ₘ₊₁₎, as interrupted by m arbitrary amino-acid residues (m is an integer, m = 0, 1, 2), by the following equation: 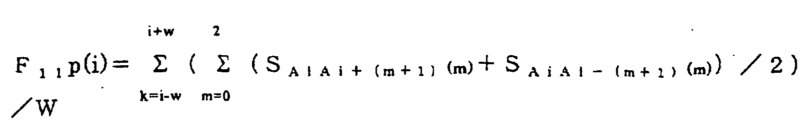 (where S_{AiAi+(m+1)(m)}=log(P_{AiAi+(m+1)(m)}^{L}/P_{AiAi+(m+1)(m)}^{N}) and${\text{S}}_{\text{AiAi-(m+1)(m)}} {\text{=log(P}}_{\text{AiAi-(m+1)(m)}} {\text{L/P}}_{\text{AiAi-(m+1)(m)}} {\text{}}^{\text{N}} \text{)}$ where S_{AiAi+(m+1)(m)}=0 or S_{AiAi-(m+1)(m)}=0 if there is no statistically significant diffrerence between P_{AiAi+(m+1)(m)}^{L} and P_{AiAi+(m+1)(m)}^{N} or between P_{AiAi-(m+1)(m)}^{L} and P_{AiAi-(m+1)(m)}^{N};
P_{AiAi+(m+1)(m)}^{L} and P_{AiAi+(m+1)(m)}^{N} are the probabilities of the pair of the arbitrary amino-acid residues Aᵢ and Aᵢ₊₍ₘ₊₁₎ occurring in a linker sequence and a non-linker loop sequence, respectively (the order of A, and Aᵢ₊₍ₘ₊₁₎ does not matter), and P_{AiAi-(m+1)(m)}^{L} and P_{AiAi-(m+1)(m)}^{N} are the probabilities of the pair of the arbitrary amino-acid residues Aᵢ and Aᵢ₋₍ₘ₊₁₎ occurring in the linker sequence and the non-linker loop sequence, respectively (the order of Aᵢ and Aᵢ₋₍ₘ₊₁₎ does not matter)); and
iii) a step for obtaining the linker degree determination score F₁₁(i) of the amino-acid residue Ai at the position i by the following equation:${\text{F}}_{\text{11}} {\text{(i)=F}}_{\text{11}} {\text{s(i)+ α}}_{\text{11}} {\text{F}}_{\text{11}} \text{p(i)}$ (where 0 ≦ α₁₁≦1).

34. A program for having a computer function as a system for obtaining a linker degree determination score F₁₁(i) for an amino-acid residue Ai at a position i in an amino-acid sequence with L₂ amino-acid residues (L₂ is an integer of 22 or more) by taking a window of w amino-acid residues before and after the amino-acid residue at the position i (i is an integer of 1 or more but not more than L₂), the system comprising:
i) a step for obtaining a linker trend score F₁₁s(i) of an amino-acid residue Aₖ by the following equation: 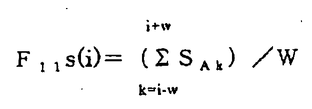 (where W is the window width, and W=2w+1,${\text{S}}_{\text{Ak}} {\text{=log(P}}_{\text{Ak}} {\text{}}^{\text{L}} {\text{/P}}_{\text{Ak}} {\text{}}^{\text{N}} \text{)}$ where S_{Ak}=0 if there is no statistically significant diffrerence between P_{Ak}^{L} and P_{Ak}^{N};
P_{Ak}^{L} and P_{Ak}^{N} are the probabilities of the amino-acid residue Aₖ occurring in a linker sequence and a non-linker loop sequence, respectively);
ii) a step for obtaining the linker trend score F₁₁p(i) of the pair of amino-acid residues Aᵢ and Aᵢ₊₍ₘ₊₁₎, as interrupted by m arbitrary amino-acid residues (m is an integer, m = 0, 1, 2), by the following equation: 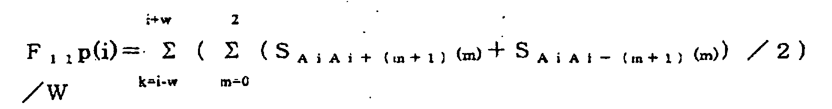 (where S_{AiAi+(m+1)(m)}=log(P_{AiAi+(m+1)(m)}^{L}/P_{AiAi+(m+1)(m)}^{N}) and${\text{S}}_{\text{AiAi-(m+1)(m)}} {\text{=log(P}}_{\text{AiAi-(m+1)(m)}} {\text{}}^{\text{L}} {\text{/P}}_{\text{AiAi-(m+1)(m)}} {\text{}}^{\text{N}} \text{)}$ where S_{AiAi+(m+1)(m)}=0 or S_{AiAi-(m+1)(m)}=0 if there is no statistically significant diffrerence between P_{AiAi+(m+1)(m)}^{L} and P_{AiAi+(m+1)(m)}^{N} or between P_{AiAi-(m+1)(m)}^{L} and P_{AiAi-(m+1)(m)}^{N};
P_{AiAi+(m+1)(m)}^{L} and P_{AiAi+(m+1)(m)}^{N} are the probabilities of the pair of the arbitrary amino-acid residues Aᵢ and Aᵢ₊₍ₘ₊₁₎ occurring in a linker sequence and a non-linker loop sequence, respectively (the order of Aᵢ and Aᵢ₊₍ₘ₊₁₎ does not matter), and P_{AiAi-(m+1)(m)}^{L} and P_{AiAi-(m+1)(m)}^{N} are the probabilities of the pair of the arbitrary amino-acid residues Aᵢ and Aᵢ₋₍ₘ₊₁₎ occurring in the linker sequence and the non-linker loop sequence, respectively (the order of A, and Aᵢ₋₍ₘ₊₁₎ does not matter)); and
iii) a step for obtaining the linker degree determination score F₁₁(i) of the amino acid residue Ai at the position i by the following equation:${\text{F}}_{\text{11}} {\text{(i)=F}}_{\text{11}} {\text{s(i)+ α}}_{\text{11}} {\text{F}}_{\text{11}} \text{p(i)}$ (where 0≦ α₁₁≦1).

35. A method by which a linker degree determination score F₁₂(i) of an amino-acid residue Ai at a position i in an amino-acid sequence seq.0 with L₂ amino-acid residues (L₂ is an interger of 22 or more) for which the existence of n homologous sequences seq.1∼seq.n (n is an integer of 1 or more) is known is obtained by taking a window with w amino-acid residues before and after the amino-acid residue at the position i (i is an integer of 1 or more but not more than 22), the method comprising:
i) a step for identifying an amino-acid residue Aᵢ^{k} in a seq.k (k is an integer of 1 or more but not more than n) corresponding to an amino-acid residue Ai⁰ at a position i in the seq.0 by aligning seq.0 and seq.1∼seq.n;
ii) a step for obtaining parameters S'_{Ai}, S'_{AiAi+(m+1)}(m) and S'_{AiAi-(m+1)}(m) for the amino-acid residue Ai at the position i by the following equation: 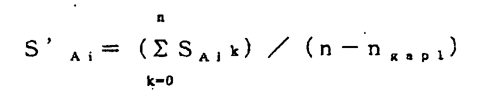 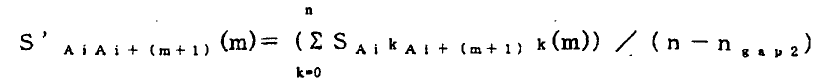 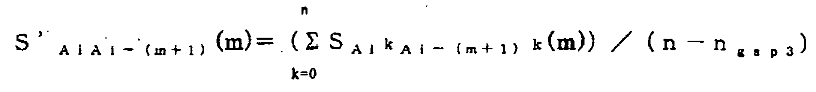 (where n_{gap1} is the number of gaps occurring in Aᵢ^{k},${\text{S}}_{\text{Ai}} {\text{k=log(P}}_{\text{Ai}} {\text{k}}^{\text{L}} {\text{/P}}_{\text{Ai}} {\text{k}}^{\text{N}} \text{)}$ where S_{Ai}k=0 if there is no statistically significant difference between P_{Ai}k^{L} and P_{Ai}k^{N};
P_{Ai}k^{L} and P_{Ai}k^{N} are the probabilities of the amino-acid residue Aᵢ^{k} occurring in a linker sequence and a non-linker loop sequence, respectively;
wherein n_{gap2} is the number of gaps occurring in Aᵢ^{k} or Aᵢ₊₍ₘ₊₁₎ ^{k},${\text{S}}_{\text{Ai}} {\text{k}}_{\text{Ai+(m+1)}} {\text{k(m)= log(P}}_{\text{Ai}} {\text{k}}_{\text{Ai+(m+1)}} {\text{k}}_{\text{(m)}} {\text{}}^{\text{L}} {\text{/P}}_{\text{Ai}} {\text{k}}_{\text{Ai+(m+1)}} {\text{k}}_{\text{(m)}} {\text{}}^{\text{N}} \text{)}$ where S_{Ai}k_{Ai+(m+1)}k₍ₘ₎=0 if there is no statistically significant difference between P_{Ai}k_{Ai+(m+1)}k₍ₘ₎^{L} and P_{Ai}k_{Ai+(m+1)}k ₍ₘ₎^{N};
P_{Ai}k_{Ai+(m+1)}k₍ₘ₎^{L} and P_{Ai}k_{Ai+(m+1)}k₍ₘ₎^{N} are the probabilities of the amino-acid residues Aᵢ^{k} and Aᵢ₊₍ₘ₊₁₎^{k} occurring in a linker sequence and a non-linker loop sequence, respectively (the order of Aᵢ^{k} and Aᵢ₊₍ₘ₊₁₎^{k} does not matter) as interrupted by m arbitrary amino-acid residues (m is an integer, m = 0, 1, 2);
and wherein n_{gap3} is the number of gaps occurring in Aᵢ^{k} or Aᵢ₋₍ₘ₊₁₎ ^{k},${\text{S}}_{\text{Ai}} {\text{k}}_{\text{Ai-(m+1)}} {\text{k(m)= log(P}}_{\text{Ai}} {\text{k}}_{\text{Ai-(m+1)}} {\text{k}}_{\text{(m)}} {\text{}}^{\text{L}} {\text{/P}}_{\text{Ai}} {\text{k}}_{\text{Ai-(m+1)}} {\text{k}}_{\text{(m)}} {\text{}}^{\text{N}} \text{)}$ where S_{Ai}k_{Ai-(m+1)}k₍ₘ₎=0 if there is no statistically significant difference between P_{Ai}k_{Ai-(m+1)}k₍ₘ₎^{L} and P_{Ai}k_{Ai-(m+1)}k₍ₘ₎^{N};
P_{Ai}k_{Ai-(m+1)}k₍ₘ₎^{L} and P_{Ai}k_{Ai-(m+1)}k₍ₘ₎^{N} are the probabilities of the amino-acid residues Aᵢ^{k} and Aᵢ₋₍ₘ₊₁₎^{k} occurring in a linker sequence and a non-linker loop sequence, respectively (the order of Aᵢ^{k} and Aᵢ₋₍ₘ₊₁₎^{k} does not matter) as interrupted by m arbitrary amino-acid residues (m is an integer, m = 0, 1, 2));
iii) a step for obtaining a linker trend score F₁₂s(i) of an amino-acid residue by the following equation: 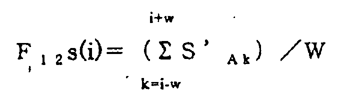
iv) a step for obtaining a linker trend score F₁₂p(i) of an arbitrary amino-acid residue pair by the following equation: 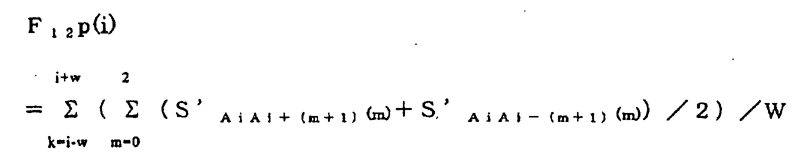 and
v) a step for obtaining the linker degree determination score F₁₂(i) for the amino-acid residue Ai at the position i by the following equation:${\text{F}}_{\text{12}} {\text{(i)=F}}_{\text{12}} {\text{s(i)+ α}}_{\text{12}} {\text{F}}_{\text{12}} \text{p(i)}$ (where 0≦ α₁₂≦ 1).

36. A system by which a linker degree determination score F₁₂(i) of an amino-acid residue Ai at a position i in an amino-acid sequence seq.0 with L₂ amino-acid residues (L₂ is an interger of 22 or more) for which the existence of n homologous sequences seq.1∼seq.n (n is an integer of 1 or more) is known is obtained by taking a window with w amino-acid residues before and after the amino-acid residue at the position i (i is an integer of 1 or more but not more than 22), the system comprising:
i) a means for identifying an amino-acid residue Aᵢ^{k} in a seq.k (k is an integer of 1 or more but not more than n) corresponding to an amino-acid residue Ai⁰ at the position i in the seq.0 by aligning seq.0 and seq.1∼seq.n;
ii) a means for obtaining parameters for the amino-acid residue Ai at the position i, S'_{Ai}, S'_{AiAi+(m+1)}(m) and S'_{AiAi-(m+1)}(m), by the following equation: 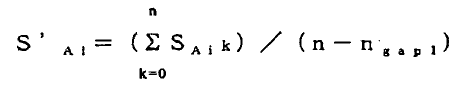 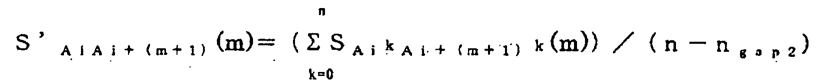 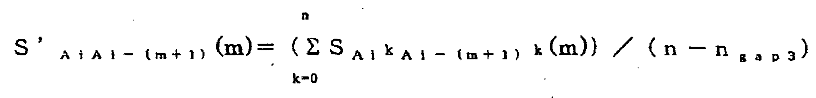 (where n_{gap1} is the number of gaps occurring in Aᵢ^{k},${\text{S}}_{\text{Ai}} {\text{k=log(P}}_{\text{Ai}} {\text{k}}^{\text{L}} {\text{/P}}_{\text{Ai}} {\text{k}}^{\text{N}} \text{)}$ where S_{Ai}^{k}=0 if there is no statistically significant difference between P_{Ai}k^{L} and P_{Ai}k^{N};
P_{Ai}k^{L} and P_{Ai}k^{N} are the probabilities of the amino-acid residue Aᵢ^{k} occurring in a linker sequence and a non-linker loop sequence, respectively;
wherein n_{gap2} is the number of gaps occurring in Aᵢ^{k} or Aᵢ₊₍ₘ₊₁₎^{k},${\text{S}}_{\text{Ai}} {\text{k}}_{\text{Ai+(m+1)}} {\text{k(m)= log(P}}_{\text{Ai}} {\text{k}}_{\text{Ai+(m+1)}} {\text{k}}_{\text{(m)}} {\text{}}^{\text{L}} {\text{/P}}_{\text{Ai}} {\text{k}}_{\text{Ai+(m+1)}} {\text{k}}_{\text{(m)}} {\text{}}^{\text{N}} \text{)}$ where S_{Ai}k_{Ai+(m+1)}k₍ₘ₎=0 if there is no statistically significant difference between P_{Ai}k_{Ai+(m+1)}k₍ₘ₎^{L} and P_{Ai}k_{Ai+(m+1)}k ₍ₘ₎^{N};
P_{Ai}k_{Ai+(m+1)}k₍ₘ₎^{L} and P_{Ai}k_{Ai+(m+1)}k₍ₘ₎^{N} are the probabilities of the amino-acid residues Aᵢ^{k} and Aᵢ₊₍ₘ₊₁₎^{k} occurring in the linker sequence and the non-linker loop sequence, respectively (the order of Aᵢ^{k} and Aᵢ₊₍ₘ₊₁₎^{k} does not matter) as interrupted by m arbitrary amino-acid residues (m is an integer, m = 0, 1, 2);
and wherein n_{gap3} is the number of gaps occurring in Aᵢ^{k} or Aᵢ₋₍ₘ₊₁₎ ^{k},${\text{S}}_{\text{Ai}} {\text{k}}_{\text{Ai-(m+1)}} {\text{k(m)= log(P}}_{\text{Ai}} {\text{k}}_{\text{Ai-(m+1)}} {\text{k}}_{\text{(m)}} {\text{}}^{\text{L}} {\text{/P}}_{\text{Ai}} {\text{k}}_{\text{Ai-(m+1)}} {\text{k}}_{\text{(m)}} {\text{}}^{\text{N}} \text{)}$ where S_{Ai}k_{Ai-(m+1)}k₍ₘ₎=0 if there is no statistically significant difference between P_{Ai}k_{Ai-(m+1)}k₍ₘ₎^{L} and P_{Ai}k_{Ai-(m+1)}k₍ₘ₎^{N};
P_{Ai}k_{Ai-(m+1)}k₍ₘ₎^{L} and P_{Ai}k_{Ai-(m+1)}k₍ₘ₎^{N} are the probabilities of the amino-acid residues Aᵢ^{k} and Aᵢ₋₍ₘ₊₁₎^{k} occurring in the linker sequence and the non-linker loop sequence, respectively (the order of Aᵢ^{k} and Aᵢ₋₍ₘ₊₁₎^{k} does not matter) as interrupted by m arbitrary amino acid residues (m is an integer, m = 0, 1, 2));
iii) a means for obtaining a linker trend score F₁₂s(i) of an amino-acid residue by the following equation; 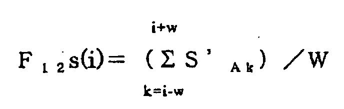
iv) a means for obtaining a linker trend score F₁₂p(i) of an arbitrary amino-acid residue pair by the following equation; 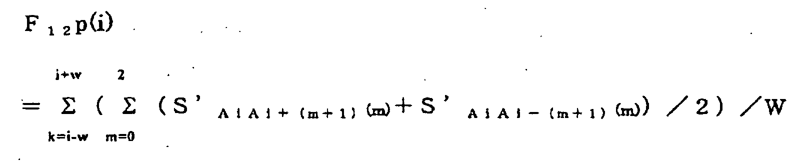 and
v) a means for obtaining the linker degree determination score F₁₂(i) for the amino-acid residue Ai at the position i by the following equation:${\text{F}}_{\text{12}} {\text{(i)=F}}_{\text{12}} {\text{s(i)+ α}}_{\text{12}} {\text{F}}_{\text{12}} \text{p(i)}$ (where 0≦ α₁₂≦1).

37. A program for having a computer function as a system by which a linker degree determination score F₁₂(i) of an amino-acid residue Ai at a position i in an amino-acid sequence seq.0 with L₂ amino-acid residues (L₂ is an interger of 22 or more) for which the existence of n homologous sequences seq.1∼seq.n (n is an integer of 1 or more) is known is obtained by taking a window with w amino-acid residues before and after the amino-acid residue at the position i (i is an integer of 1 or more but not more than 22), the system comprising:
i) a means for identifying an amino acid residue Aᵢ^{k} in a seq.k (k is an integer of 1 or more but not more than n) corresponding to an amino-acid residue Ai⁰ at the position i in the seq.0 by aligning seq.0 and seq.1∼seq.n;
ii) a means for obtaining parameters for the amino-acid residue Ai at the position i, S'_{Ai}, S'_{AiAi+(m+1)}(m) and S'_{AiAi-(m+1)}(m), by the following equation: 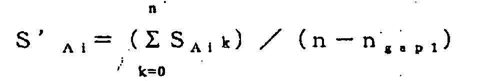 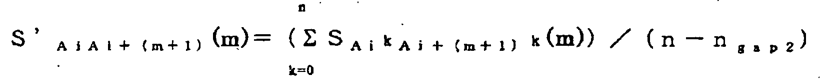 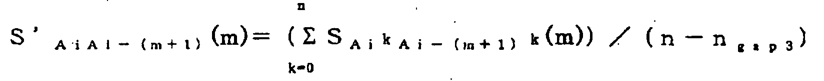 (where n_{gap1} is the number of gaps occurring in Aᵢ^{k},${\text{S}}_{\text{Ai}} {\text{k=log(P}}_{\text{Ai}} {\text{k}}^{\text{L}} {\text{/P}}_{\text{Ai}} {\text{k}}^{\text{N}} \text{)}$ where S_{Ai}k=0 if there is no statistically significant difference between P_{Ai}k^{L} and P_{Ai}k^{N};
P_{Ai}k^{L} and P_{Ai}k^{N} are the probabilities of the amino-acid residue Aᵢ^{k} occurring in a linker sequence and a non-linker loop sequence, respectively;
wherein n_{gap2} is the number of gaps occurring in Aᵢ^{k} or Aᵢ₊₍ₘ₊₁₎^{k},${\text{S}}_{\text{Ai}} {\text{k}}_{\text{Ai+(m+1)}} {\text{k(m)= log(P}}_{\text{Ai}} {\text{k}}_{\text{Ai+(m+1)}} {\text{k}}_{\text{(m)}} {\text{}}^{\text{L}} {\text{/P}}_{\text{Ai}} {\text{k}}_{\text{Ai+(m+1)}} {\text{k}}_{\text{(m)}} {\text{}}^{\text{N}} \text{)}$ where S_{Ai}k_{Ai+(m+1)}k₍ₘ₎=0 if there is no statistically significant difference between P_{Ai}k_{Ai+(m+1)}k₍ₘ₎^{L} and P_{Ai}k_{Ai+(m+1)}k₍ₘ₎^{N};
P_{Ai}k_{Ai+(m+1)}k₍ₘ₎^{L} and P_{Ai}k_{Ai+(m+1)}k₍ₘ₎^{N} are the probabilities of the amino-acid residues Aᵢ^{k} and Aᵢ₊₍ₘ₊₁₎^{k} occurring in the linker sequence and the non-linker loop sequence, respectively (the order of Aᵢ^{k} and Aᵢ₊₍ₘ₊₁₎^{k} does not matter) as interrupted by m arbitrary amino-acid residues (m is an integer, m = 0, 1, 2);
and wherein n_{gap3} is the number of gaps occurring in Aᵢ^{k} or Aᵢ₋₍ₘ₊₁₎^{k},${\text{S}}_{\text{Ai}} {\text{k}}_{\text{Ai-(m+1)}} {\text{k}}_{\text{(m)}} {\text{= log(P}}_{\text{Ai}} {\text{k}}_{\text{Ai-(m+1)}} {\text{k}}_{\text{(m)}} {\text{}}^{\text{L}} {\text{/P}}_{\text{Ai}} {\text{k}}_{\text{Ai-(m+1)}} {\text{k}}_{\text{(m)}} {\text{}}^{\text{N}} \text{)}$ where S_{Ai}k_{Ai-(m+1)}k₍ₘ₎=0 if there is no statistically significant difference between P_{Ai}k_{Ai-(m+1)}k₍ₘ₎^{L} and P_{Ai}k_{Ai-(m+1)}k₍ₘ₎^{N};
P_{Ai}k_{Ai-(m+1)}k₍ₘ₎^{L} and P_{Ai}k_{Ai-(m+1)}k₍ₘ₎^{N} are the probabilities of the amino-acid residues Aᵢ^{k} and Aᵢ₋₍ₘ₊₁₎^{k} occurring in the linker sequence and the non-linker loop sequence, respectively (the order of Aᵢ^{k} and Aᵢ₋₍ₘ₊₁₎^{k} does not matter) as interrupted by m arbitrary amino-acid residues (m is an integer, m = 0, 1, 2);
iii) a means for obtaining a linker trend score F₁₂s(i) of an amino-acid residue by the following equation; 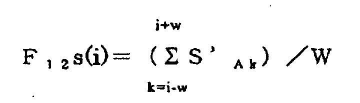
iv) a means for obtaining a linker trend score F₁₂ₚ(i) of an arbitrary amino-acid residue pair by the following equation; 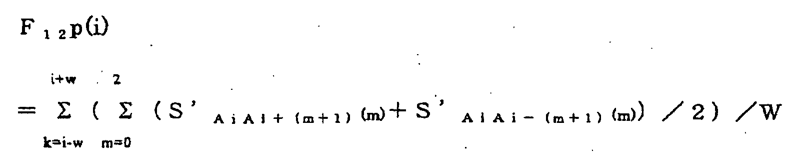 and
v) a means for obtaining the linker degree determination score F₁₂(i) for the amino-acid residue Ai at the position i by the following equation:${\text{F}}_{\text{12}} {\text{(i)=F}}_{\text{12}} {\text{s(i)+ α}}_{\text{12}} {\text{F}}_{\text{12}} \text{p(i)}$ (where 0≦ α₁₂≦ 1).

38. A method of predicting a domain linker portion comprising:
i) a step for obtaining a linker degree determination score of an amino-acid residue Ai at a position i in an amino-acid sequence with L₂ amino-acid residues (L₂ is an interger of 22 or more) according to the method as set forth in claim 32 or 35 (however, a linker degree determination score need not be obtained for 0 to 50 residues at the N and C terminals of the amino-acid sequence);
ii) a step for executing secondary-structure prediction on the amino acid sequence and predicting which regions will take a loop structure;
iii) a step for obtaining regions which are found likely to take a loop structre in the secondary-structure prediction and whose linker degree determination score is greater than 0; and
iv) a step for predicting for each of the regions obtained in iii) that the position at which the linker degree determination score takes a maximum value is the position at which the domain linker exists.

39. A system for predicting a domain linker portion comprising:
i) a means for obtaining a linker degree determination score of an amino acid residue Ai at a position i in an amino-acid sequence with L₂ amino-acid residues (L₂ is an interger of 22 or more) according to the method as set forth in claim 32 or 35 (however, a linker degree determination score need not be obtained for 0 to 50 residues at the N and C terminals of the amino-acid sequence);
ii) a means for executing secondary-structure prediction on the amino-acid sequence and predicting which regions will take a loop structure;
iii) a means for obtaining regions which are found likely to take a loop structure in the secondary-structure prediction and whose linker degree determination score is greater than 0; and
iv) a means for predicting for each of the regions obtained in iii) that the position at which the linker degree determination score takes a maximum value is the position at which the domain linker exists.

40. A program for having a computer function as a system for predicting a domain linker portion, the system comprising:
i) a means for obtaining a linker degree determination score of an amino-acid residue Ai at a position i in an amino-acid sequence with L₂ amino-acid residues (L₂ is an interger of 22 or more) according to the method as set forth in claim 32 or 35 (however, a linker degree determination score need not be obtained for 0 to 50 residues at the N and C terminals of the amino-acid sequence);
ii) a means for executing secondary-structure prediction on the amino-acid sequence and predicting which regions will take a loop structure;
iii) a means for obtaining regions which are found likely to take a loop structure in the secondary-structure prediction and whose linker degree determination score is greater than 0; and
iv) a means for predicting for each of the regions obtained in iii) that the position at which the linker degree determination score takes a maximum value is the position at which the domain linker exists.

41. A method of constructing an amino-acid sequence database comprising:
i) a step for obtaining a linker degree determination score of an amino-acid residue Ai at a position i in an amino-acid sequence with L₂ amino-acid residues (L₂ is an interger of 22 or more) according to the method as set forth in claim 32 or 35 (however, a linker degree determination score need not be obtained for 0 to 50 residues at the N and C terminals of the amino-acid sequence);
ii) a step for executing secondary-structure prediction on the amino-acid sequence and predicting which regions will take a loop structure;
iii) a step for obtaining regions which are found likely to take a loop structure in the secondary-structure prediction and whose linker degree determination score is greater than 0;
iv) a step for selecting from the regions obtained in iii) the one whose maximum value of the linker degree determination score is greater than a lower limit value; and
v) a step for recording in a recording medium the amino-acid sequence of the region selected in iv).

42. A domain linker peptide made of the same amino-acid sequence as the amino-acid sequence of a region whose maximum value of a linker degree determination score is greater than a lower limit value, and which was obtained by a method comprising:
i) a step for obtaining a linker degree determination score of an amino-acid residue Ai at a position i in an amino-acid sequence with L₂ amino acid residues (L₂ is an interger of 22 or more) according to a method as set forth in claim 32 or 35 (however, a linker degree determination score need not be obtained for 0 to 50 residues at the N and C terminals of the amino acid sequence);
ii) a step for executing secondary-structure prediction on the amino-acid sequence and predicting which regions will take a loop structure;
iii) a step for obtaining regions which are found likely to take a loop structure in the secondary-structure prediction and whose linker trend determination score is greater than 0; and
iv) a step for selecting from the regions obtained in iii) the one whose maximum value of the linker degree determination score is greater than the lower limit value.

43. A method of predicting a structural domain comprising a step for predicting about an amino-acid sequence with L₂ amino-acid residues (L₂ is an interger of 22 or more) that a sequence fragment generated by cutting off the amino-acid sequence at any portion of a region including the domain linker portion predicted by the method as set forth in claim 38 or the position at which a domain linker exists is a structural domain.

44. A method as set forth in claim 43, wherein if n domain linker portions are predicted, t of them (t is an integer of 1 or more but not more than n) is selected, all the patterns for cutting an amino acid sequence at that position are considererd, and all the sequence fragments obtained are predicted as structural domains.

45. A system for predicting a structural domain comprising a means for predicting about an amino-acid sequence with L₂ amino-acid residues (L₂ is an interger of 22 or more) that a sequence fragment generated by cutting off the amino-acid sequence at any portion of a region including the domain linker portion predicted by the method as set forth in claim 38 or the position at which a domain linker exists is a structural domain.

46. A program for having a computer function as a system for predicting a structural domain, the system comprising a means for predicting about an amino-acid sequence with L₂ amino-acid residues (L₂ is an interger of 22 or more) that a sequence fragment generated by cutting off the amino-acid sequence at any portion of a region including the domain linker portion predicted by the method as set forth in claim 38 or the position at which a domain linker exists is a structural domain.

47. A method of constructing an amino-acid sequence database comprising a step in which concerning an amino-acid sequence with L₂ amino-acid residues (L₂ is an interger of 22 or more), the amino-acid sequence of a sequence fragment generated by cutting off the first-mentioned amino-acid sequence at any portion of a region including the domain linker portion predicted by the method as set forth in claim 38 or the portion at which a domain linker exists is recorded in a recording medium.

48. A method of producing a protein comprising a step for producing a protein having the same amino-acid sequence as the structural domain predicted by the method as set forth in claim 43.

49. A method of analyzing a protein comprising a step for analyzing a protein having the same amino-acid sequence as the structural domain predicted by the method as set forth in claim 43.

50. A method of producing a protein comprising designing a new multi-domain protein generated by connecting at least 2 protein fragments with a domain linker peptide as set forth in claim 42 and producing this multi-domain protein.
